(19) Europäisches Patentamt
European Patent Office
Office européen des brevets

(11) EP 3 688 173 B1

(12) EUROPEAN PATENT SPECIFICATION

(45) Date of publication and mention of the grant of the patent:
**17.12.2025 Bulletin 2025/51**

(21) Application number: **18792674.6**

(22) Date of filing: **28.09.2018**

(51) International Patent Classification (IPC):
***C12N 15/88*** *(2006.01)* ***A61K 38/46*** *(2006.01)*
***C12N 9/22*** *(2006.01)* ***C12N 15/10*** *(2006.01)*
***C12N 15/113*** *(2010.01)*

(52) Cooperative Patent Classification (CPC):
**C12N 15/88; A61K 38/465; C12N 5/0647; C12N 15/113;** A61K 48/005; C12N 2500/99; C12N 2501/125; C12N 2501/145; C12N 2501/2306; C12N 2501/26; C12N 2501/999; C12N 2510/00

(86) International application number:
**PCT/US2018/053569**

(87) International publication number:
**WO 2019/067999 (04.04.2019 Gazette 2019/14)**

(54) **IN VITRO METHOD OF MRNA DELIVERY USING LIPID NANOPARTICLES**

IN-VITRO-VERFAHREN ZUR MRNA-FREISETZUNG UNTER VERWENDUNG VON LIPIDNANOPARTIKELN

MÉTHODE IN VITRO D'ADMINISTRATION D'ARNM À L'AIDE DE NANOPARTICULES LIPIDIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **29.09.2017 US 201762566232 P**

(43) Date of publication of application:
**05.08.2020 Bulletin 2020/32**

(73) Proprietor: **Intellia Therapeutics, Inc.**
**Cambridge, MA 02139 (US)**

(72) Inventors:
- **STEWART, Morag, Helen**
  **Boston, MA 02114 (US)**
- **VALLASTER, Markus, Parzival**
  **Hopkinton, MA 01748 (US)**
- **MONTI, Anthony**
  **Wrentham, MA 02093 (US)**
- **NARENDRA, Pooja, Kyatsandra**
  **Allston, MA 02134 (US)**
- **HO, Quan**
  **Somerville, MA 02143 (US)**

(74) Representative: **Schlich**
**9 St Catherine's Road**
**Littlehampton**
**West Sussex BN17 5HS (GB)**

(56) References cited:
**WO-A1-2017/083274 WO-A1-2017/134529**
**WO-A2-2010/088537 US-A1- 2014 206 753**

- **HAO YIN ET AL: "Therapeutic genome editing by combined viral and non-viral delivery of CRISPR system components in vivo", NATURE BIOTECHNOLOGY, vol. 34, no. 3, 1 February 2016 (2016-02-01), pages 328 - 333, XP055540393, ISSN: 1087-0156, DOI: 10.1038/nbt.3471**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The introduction of genetic change into stem cells, including hematopoietic stem cells (HSCs), and their progeny is of interest for gene editing and gene therapy methods. Stem cells such as HSCs have proliferative capacities lost in mature cells and committed progenitors making them particularly useful for gene editing technologies. The ability to modify HSCs and stem cells *in vitro* is important, for example, and methods to deliver biological agents to HSCs and other stem cells in culture are needed. There is a particular need for delivery technologies for human HSCs in culture.

HSCs are indispensable for lifelong blood production. HSCs can sustain long-term and functional hematopoiesis due to their ability to both differentiate to produce mature progeny of all myeloid and lymphoid blood lineages or to self-renew to replace the cells that become progressively committed to differentiation. HSCs can be used to restore blood and immune cells in transplant recipients, in immunocompromised patients, or in other patients. Specifically, autologous or allogeneic transplantation of HSCs can be used for the treatment of patients with inherited immunodeficient and autoimmune diseases and diverse hematopoietic disorders to reconstitute the hematopoietic cell lineages and immune system defense.

The present invention provides a method of delivering an mRNA to a stem cell or a stem cell population, the method comprising:

a. preincubating a serum factor, with an LNP composition comprising the mRNA, an amine lipid, a helper lipid, a neutral lipid, and a PEG lipid, wherein the amine lipid is Lipid A

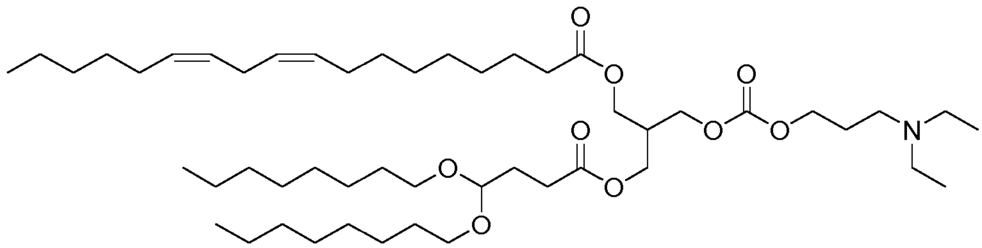

, or a C4-C12 acetal analog of Lipid A;
b. contacting the stem cell or the stem cell population with the preincubated LNP composition *in vitro;* and
c. culturing the stem cell or the stem cell population *in vitro;*

thereby delivering the mRNA to the stem cell or the stem cell population.

In embodiments, methods to deliver components of CRISPR/Cas gene editing systems to HSCs in culture are of particular interest. In embodiments, methods of delivering RNAs, including CRISPR/Cas system components to hematopoietic cell cultures that include HSCs are provided herein. The methods deliver active protein to stem cells, including HSCs, cultured *in vitro* and include contacting the cells with a lipid nanoparticle (LNP) composition that provides an mRNA that encodes the protein. In addition, in embodiments, methods of gene editing in stem cells such as HSCs in vitro, and methods of producing an engineered cell are provided.

In some embodiments, methods of gene editing in HSCs *in vitro,* and methods of producing an engineered HSC cell are provided. In further embodiments, provided herein is a method of delivering an mRNA to a hematopoietic stem and/or progenitor cell (HSPC) or an HSPC population. In some embodiments, the method comprises preincubating a serum factor with an LNP composition comprising the mRNA, an amine lipid, a helper lipid, a neutral lipid, and a PEG lipid. In some embodiments, the method further comprises contacting the HSPC or the HSPC population with the preincubated LNP composition in vitro. In some embodiments, the method further comprises culturing the HSPC or the HSPC population in vitro. In some embodiments, the method results in the delivery of the mRNA to the HSPC or the HSPC population.

In some embodiments, provided herein is a method of introducing a Cas nuclease mRNA and a gRNA to a stem cell, e.g., an HSPC. In some embodiments, the method comprises preincubating a serum factor with an LNP composition comprising the Cas nuclease mRNA, a gRNA, an amine lipid, a helper lipid, a neutral lipid, and a PEG lipid. In some embodiments, the method further comprises contacting the HSPC with the preincubated LNP composition in vitro. In some embodiments, the method further comprises culturing the HSPC. In some embodiments, the method results in the introduction of the Cas nuclease mRNA and gRNA to the HSPC.

In some embodiments, provided herein is a method of producing a genetically engineered stem cell, e.g., HSPC, in vitro. In some embodiments, the method comprises preincubating a serum factor with an LNP composition comprising a Cas nuclease mRNA, a gRNA, an amine lipid, a helper lipid, a neutral lipid, and a PEG lipid. In some embodiments, the method further comprises contacting the HSPC with the preincubated LNP composition *in vitro.* In some embodiments, the method further comprises culturing the HSPC *in vitro.* In some embodiments, the method results in the production of a

genetically engineered HSPC.

In some embodiments, a method of delivering an mRNA to an HSPC or an HSPC population is provided, the method comprising preincubating an LNP composition with a serum factor, contacting the cell or population with the preincubated LNP composition *in vitro;* and culturing the cell or population *in vitro;* thereby delivering the mRNA to the HSPC. In some embodiments, the HSPC is an HSC. In some embodiments, the methods deliver an mRNA, such as a Cas nuclease mRNA, to an HSPC population (e.g., a CD34+ cell population). In certain embodiments, a guide RNA (gRNA), optionally in combination with a Cas nuclease mRNA, is delivered to the cells.

## BRIEF DESCRIPTION OF DRAWINGS

Fig. 1 shows green fluorescent protein (GFP) mRNA delivery in CD34+ bone marrow cells using LNPs.

Fig. 2 shows that mRNA delivery in CD34+ bone marrow cells depends on pre-incubation with serum.

Figs. 3A and 3B show *B2M* editing in CD34+ bone marrow cells with serum pre-incubation, with Fig. 3A depicting the percent of B2M- cells (protein expression knockdown) and Fig. 3B graphing the percent editing achieved in the experiment.

Figs. 4A and 4B show efficient delivery with serum preincubation and ApoE3 preincubation. Fig. 4A depicts the percent of B2M- cells and Fig. 4B provides the percent editing achieved in the experiment.

Fig. 5 shows the effect of LNP pre-incubation with preparations of various serum factors on LNP delivery to CD34+ cells.

Figs. 6A and 6B show viability and editing data for CD34+ cells that were exposed to LNP treatment at varying intervals. Fig. 6A shows viability of CD34+ cells following exposure to LNP at 2, 6, and 24 hours. Fig. 6B provides the percent editing data for the 2, 6, and 24 hour treatment groups.

## DETAILED DESCRIPTION

The present disclosure provides methods of using of LNP compositions of RNAs, including CRISPR/Cas component RNAs (the "cargo"), for *in vitro* delivery to stem cells and stem cell populations (such as CD34+ cells, e.g. HSC-containing cell populations). The methods may exhibit improved properties as compared to prior delivery technologies, for example, the methods provide efficient delivery of the RNAs, while reducing cell death caused by the transfection.

The present disclosure provides a method of delivering an mRNA to a stem cell, e.g., an HSPC or an HSPC population. The method comprises preincubating a serum factor with an LNP composition comprising the mRNA, an amine lipid, a helper lipid, a neutral lipid, and a PEG lipid. The method further comprises contacting the stem cell or the stem cell population (e.g. the HSPC or the HSPC population) with the preincubated LNP composition *in vitro.* The method further comprises culturing the stem cell or the stem cell population (e.g. the HSPC or the HSPC population) *in vitro.* The method results in the delivery of the mRNA to the stem cell or the stem cell population (e.g. the HSPC or the HSPC population). In some embodiments, the mRNA encodes a Cas nuclease.

In some embodiments, provided herein is a method of introducing a Cas nuclease mRNA and a gRNA to a stem cell, e.g., an HSPC or an HSPC population. In some embodiments, the method comprises preincubating a serum factor with an LNP composition comprising the Cas nuclease mRNA, a gRNA, an amine lipid, a helper lipid, a neutral lipid, and a PEG lipid. In some embodiments, the method further comprises contacting the HSPC with the preincubated LNP composition *in vitro.* In some embodiments, the method further comprises culturing the HSPC. In some embodiments, the method results in the introduction of the Cas nuclease mRNA and gRNA to the HSPC.

In some embodiments, provided herein is a method of producing a genetically engineered stem cell, e.g., HSPC, *in vitro.* In some embodiments, the method comprises preincubating a serum factor with an LNP composition comprising a Cas nuclease mRNA, a gRNA, an amine lipid, a helper lipid, a neutral lipid, and a PEG lipid. In some embodiments, the method further comprises contacting the HSPC with the preincubated LNP composition *in vitro.* In some embodiments, the method further comprises culturing the HSPC *in vitro.* In some embodiments, the method results in the production of a genetically engineered HSPC.

In some embodiments, the LNP composition further comprises a gRNA. In some embodiments, the mRNA encodes a Class 2 Cas nuclease. In certain embodiments, the cargo or RNA component includes a Cas nuclease mRNA, such as a Class 2 Cas nuclease mRNA. In certain embodiments, the cargo or RNA component includes a CRISPR/Cas system gRNA or nucleic acids encoding a gRNA. Methods of gene editing and methods of making engineered cells are also provided.

## ***In Vitro* Methods**

The present methods deliver RNAs to stem cells or stem cell populations, such as CD34+ cells *in vitro.* "CD34+ cells" refers to cells that express at their surface CD34 marker. CD34+ cells can be detected and counted using for example flow cytometry and fluorescently labeled anti-human CD34 antibodies.

A method of delivering an mRNA to a stem cell, e.g., an HSPC or an HSPC population, is provided, the method comprising (a) preincubating a serum factor with an LNP composition comprising the mRNA, an amine lipid, a helper lipid, a neutral lipid, and a PEG lipid; (b) contacting the stem cell or the stem cell population (e.g. the HSPC or the HSPC population) with the preincubated LNP composition *in vitro;* and (c) culturing the stem cell or the stem cell population (e.g. the HSPC or the HSPC population) *in vitro;* thereby delivering the mRNA to the stem cell or the stem cell population (e.g. the HSPC or the HSPC population). In some embodiments, the mRNA encodes a Cas nuclease such as a Class 2 Cas nuclease. In some aspects, the Class 2 Cas nuclease mRNA is a Cas9 mRNA or a Cpfl mRNA. In certain embodiments, the Class 2 Cas nuclease is an *S. pyogenes* Cas9. In some embodiments, the LNP composition further comprises a gRNA. In additional embodiments, the methods introduce a Cas nuclease mRNA and a gRNA to an HSPC, the method comprising (a) preincubating a serum factor with an LNP composition comprising the Cas nuclease mRNA, a gRNA, an amine lipid, a helper lipid, a neutral lipid, and a PEG lipid; (b) contacting the HSPC with the preincubated LNP composition *in vitro;* and (c) culturing the HSPC; thereby introducing the Cas nuclease and gRNA to the HSPC.

In various embodiments, the gRNAs of the methods described herein may be a dual-guide RNA (dgRNA) or a single-guide RNA (sgRNA).

In some embodiments of the *in vitro* methods, the LNP transfection may reduce HSPC or CD34+ cell death as compared to known technologies like electroporation. In some embodiments, the LNP transfection may cause less than 5%, less than 10%, less than 20%, less than 30%, or less than 40% cell death. In certain embodiments, post-transfection cell survival is at least 60%, 70%, 80%, 90%, or 95%.

Stem cells are characterized by the ability to self-renew and differentiate into a diverse range of cell types. The two broad types of mammalian stem cells are embryonic stem (ES) cells and adult stem cells. Adult stem cells or progenitor cells may replenish specialized cells. Most adult stem cells are lineage-restricted and may be referred to by their tissue origin. For reference, ES cell lines are derived from the epiblast tissue of the inner cell mass of a blastocyst or early morula stage embryos. ES cells are pluripotent and give rise to derivatives of the three germinal layers, i.e., the ectoderm, endoderm and mesoderm. Induced pluripotent stem cells (iPSCs) are adult cells that have been genetically reprogrammed to an embryonic stem cell-like state by being forced to express genes and factors important for maintaining the defining properties of embryonic stem cells. A "stem cell" may be an ESC, an iPSC, a progenitor cell, or an HSPC, for example.

The terms "hematopoietic stem and/or progenitor cell" and "HSPC" are used interchangeably, and refer to a population of cells comprising both HSCs and hematopoietic progenitor cells ("HPCs"). Such cells are characterized, for example, as CD34+. In exemplary embodiments, HSPCs are isolated from bone marrow. In other exemplary embodiments, HSPCs are isolated from peripheral blood. In other exemplary embodiments, HSPCs are isolated from umbilical cord blood.

HSPCs may be derived from bone marrow, peripheral blood, or umbilical cord blood, and they may be autologous (the patient's own stem cells) or allogeneic (the stem cells come from a donor).

The term "hematopoietic progenitor cells" or "HPCs" as used herein refers to primitive hematopoietic cells that have a limited capacity for self-renewal and the potential for multilineage differentiation (e.g., myeloid, lymphoid), monolineage differentiation (e.g., myeloid or lymphoid) or cell-type restricted differentiation (e.g., erythroid progenitor) depending on placement within the hematopoietic hierarchy (Doulatov et al., Cell Stem Cell 2012).

The term "hematopoietic stem cells" or "HSCs" as used herein also refers to immature blood cells having the capacity to self-renew and to differentiate into more mature blood cells comprising granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), and monocytes (e.g., monocytes, macrophages). It is known in the art that such cells may or may not include CD34+ cells. CD34+ cells are immature cells that express the CD34 cell surface marker. CD34+ cells are believed to include a subpopulation of cells with the stem cell properties defined above. The transplantation of populations of cells, such as HSPCs that contain multipotent HSCs, can be used to treat leukemia, lymphoma, and other other disorders.

HSCs are multipotent cells that can give rise to primitive progenitor cells (e.g., multipotent progenitor cells) and/or progenitor cells committed to specific hematopoietic lineages (e.g., lymphoid progenitor cells). The stem cells committed to specific hematopoietic lineages may be of T cell lineage, B cell lineage, dendritic cell lineage, Langerhans cell lineage and/or lymphoid tissue-specific macrophage cell lineage. In addition, HSCs also refer to long term HSC (LT-HSC) and short term HSC (ST-HSC). ST-HSCs are more active and more proliferative than LT-HSCs. However, LT-HSC have unlimited self renewal (i.e., they survive throughout adulthood), whereas ST-HSC have limited self renewal (i.e., they survive for only a limited period of time). Any of these HSCs can be used in any of the methods described herein. Optionally,

ST-HSCs are useful because they are highly proliferative and thus, quickly increase the number of HSCs and their progeny.

HSCs, HPCs, and HSPC s are optionally obtained from blood products. A blood product includes a product obtained from the body or an organ of the body containing cells of hematopoietic origin. Such sources include bone marrow, umbilical cord, peripheral blood (e.g., mobilized peripheral blood, e.g., moblized with a mobilization agent such as G-CSF or Plerixafor® (AMD3100)), liver, thymus, lymph and spleen. All of the aforementioned blood products (e.g., in crude, un-fractionated, or fractionated forms) can be enriched for cells having HSC characteristics in ways known to those of skill in the art. Similarly, they can be enriched for HPC and/or HSPC population characteristics. In an embodiment, HSCs are characterized as CD34+/CD38-/CD90+/CD45RA-. In embodiments, the HSCs are characterized as CD34+/CD90+/CD49f+ cells. In additional embodiments, the HSCs are characterized as Lineage-CD34+/CD38-/CD90+/CD45RA-. In embodiments, the HSC s are characterized as Lineage-CD34+/CD90+/CD49f+ cells, where "lineage" means omitting markers for terminially differentiated cells e.g., T cells, B cells etc. These can be excluded by staining the cells with antibodies against surface markers expressed by cells that have committed to a hematopoietic lineage. These can include but are not limited to: CD3 (T cell), CD19 (B cell), CD33 (myeloid), CD56 (NK cell), CD235a (Erythroid cells), CD71 (Erythroid cells).

"Enriched" when used in the context of cell population refers to a cell population selected based on the presence of one or more markers, for example, CD34+. A cell population, such as a stem cell population or an HSPC population, refers to eukaryotic mammalian, preferably human, cells isolated from biological sources, for example, blood product or tissues and derived from more than one cell.

During preincubation a serum factor may contact an LNP composition, prior to delivery to the HSPC cell *in vitro.*

Some embodiments of the *in vitro* methods comprise preincubating a serum factor and the LNP composition for about 30 seconds to overnight. In some embodiments, the preincubation step comprises preincubating a serum factor and the LNP composition for about 1 minute to 1 hour. In some embodiments, it comprises preincubating for about 1-30 minutes. In other embodiments, it comprises preincubating for about 1-10 minutes. Still further embodiments comprise preincubating for about 5 minutes. In certain embodiments, the endpoints of the ranges and the values provided above may be ±0.5, 1, 2, 3, or 4 minutes.

In certain embodiments, the preincubating step occurs at about 4°C. In certain embodiments, the preincubating step occurs at about 25°C. In certain embodiments, the preincubating step occurs at about 37°C. The preincubating step may comprise a buffer such as sodium bicarbonate or HEPES. In certain embodiments, the buffer may comprise an HSPC culture medium. In additional embodiments, the buffer may consist of HSPC culture media.

Preincubation of an LNP composition with a serum factor may comprise preincubation with serum, with a serum fraction, or with an isolated serum factor. In some embodiments, the LNP composition is preincubated with serum. The serum may be mammalian, mouse, primate, or human serum. In some embodiments, the LNP composition is pre-incubated with an isolated serum factor. In certain embodiments, the serum factor is an ApoE. In certain embodiments, the serum factor is chosen from ApoE2, ApoE3, and ApoE4. In additional embodiments, the ApoE is a recombinant protein, such as a recombinant human protein. The ApoE may be recombinant human ApoE3. It may be recombinant human ApoE4.

In some embodiments, the methods comprise contacting the stem cell, e.g., HSPC, or stem cell population, e.g., HSPC population, after the preincubation step, e.g. contacting the cells with a preincubated LNP composition. In some embodiments, the methods comprise contacting the stem cell population, such as ES or iPSC population after the preincubation step, e.g. contacting the cells with a preincubated LNP composition. In some embodiments, the methods comprise contacting the cells with a pre-incubated LNP composition for about 1 minute to about 72 hours. In some embodiments, the methods comprise contacting the cells with a pre-incubated LNP composition for about 1 hours to about 24 hours. In some embodiments, the methods comprise contacting the cells with a pre-incubated LNP composition for about 4 hours to about 24 hours. In some embodiments, the methods comprise contacting the cells with a pre-incubated LNP composition for about 4 hours to about 12 hours. In some embodiments, the methods comprise contacting the cells with a pre-incubated LNP composition for about 2 hours to about 12 hours. In some embodiments, the methods comprise contacting the cells with a pre-incubated LNP composition for about 6 hours to about 8 hours. In some embodiments, the methods comprise contacting the cells with a pre-incubated LNP composition for about 6 hours to about 24 hours. In some embodiments, the methods comprise contacting the cells with a pre-incubated LNP composition for about 6 hours to about 24 hours. In some embodiments, the methods comprise contacting the cells with a pre-incubated LNP composition for about 4 hours to about 12 hours. In some embodiments, the methods comprise contacting the cells with a pre-incubated LNP composition for at least about 0.5, 1, 2, 4, 6, 8, 10, or 12 hours. In some embodiments, the methods comprise a washing step after the contacting step. The washing step may comprise media.

In some embodiments, the methods comprise a Cas nuclease mRNA. In some embodiments, the methods comprise a Class 2 Cas nuclease mRNA. In some embodiments, the methods comprise a gRNA nucleic acid, such as a gRNA. In certain embodiments, the methods comprise at least two gRNA nucleic acids. In additional embodiments, the methods comprise 3 or more gRNA nucleic acids. In some embodiments, an mRNA such as a Cas nuclease mRNA and a

gRNA are formulated in a single LNP composition. In some embodiments, the methods comprise an mRNA such as a Cas nuclease mRNA and a gRNA nucleic acid that are co-encapsulated in the LNP composition. In additional embodiments, the methods comprise an mRNA and a gRNA nucleic acid that are separately encapsulated in LNPs. In certain embodiments, an mRNA is formulated in a first LNP composition and a gRNA nucleic acid is formulated in a second LNP composition. In some embodiments, the first and second LNP compositions are administered simultaneously. In other embodiments, the first and second LNP compositions are administered sequentially. In some embodiments of the *in vitro* methods, the first and second LNP compositions are combined prior to the preincubation step. In some embodiments, the first and second LNP compositions are preincubated separately.

In one embodiment, an LNP composition comprising an mRNA encoding a Cas nuclease such as a Class 2 Cas nuclease, may be administered to a cell or cell population, such as, e.g., an HSPC or HSPC population, separately from the administration of a composition comprising a gRNA. In one embodiment, an LNP composition comprising an mRNA encoding a Cas nuclease such as a Class 2 Cas nuclease and a gRNA may be administered, such as to an HSPC or HSPC population, separately from the administration of a template nucleic acid to the cell. In one embodiment, an LNP composition comprising an mRNA encoding a Cas nuclease such as a Class 2 Cas nuclease may be administered, such as to an HSPC or HSPC population, followed by the sequential administration of an LNP composition comprising a gRNA and then a template to the cell or population. In embodiments where an LNP composition comprising an mRNA encoding a Cas nuclease is administered before an LNP composition comprising a gRNA, the administrations may be separated by about 4, 6, 8, 12, 24, 36, 48, or 72 hours; or about 1, 2, or 3 days.

In some embodiments of the *in vitro* methods described herein, the stem cell, HSPC or HSPC population may be cultured *in vitro* after transfection via LNPs.

In some embodiments, the transfected stem cell, HSPC or HSPC population is expanded in a stem cell culture medium, such as an HSPC culture medium. "Expansion" or "expand" in the context of cells refers to an increase in the number of a characteristic cell type, or cell types, from an initial cell population of cells, which may or may not be identical. The initial cells used for expansion may not be the same as the cells generated from expansion. Some embodiments of the *in vitro* methods comprise culturing the HSPC or HSPC population in an HSPC culture medium. Some embodiments further comprise expanding the HSPCs in an HSPC culture medium that comprises a stem cell expander. See, e.g., WO2010/059401 (e.g., compound of Example 1), WO2013/110198, and WO2017115268, which are hereby incorporated by reference regarding suitable compounds for stem cell expansion. "Stem cell expander" refers to a compound which causes cells, e.g., HSPCs, HSCs and/or HPCs to proliferate, e.g., increase in number, at a faster rate relative to the same cell types absent said agent. In one exemplary aspect, the stem cell expander is an inhibitor of the aryl hydrocarbon receptor pathway.

In additional embodiments, the *in vitro* methods further comprise changing the culture media between the contacting and culturing steps. In still further embodiments, the culturing step comprises cell culture medium includes thrombopoietin (Tpo), Flt3 ligand (Flt-3L), and human stem cell factor (SCF). In embodiments, the cell culture medium further includes human interleukin-6 (IL-6). In embodiments, the cell culture medium includes thrombopoietin (Tpo), Flt3 ligand (Flt-3L), and human stem cell factor (SCF).

## CRISPR/Cas Cargo

The CRISPR/Cas cargo delivered via LNP formulation includes an mRNA molecule encoding a protein of interest. For example, an mRNA for expressing a protein such as green fluorescent protein (GFP), and RNA-guided DNA-binding agent, or a Cas nuclease is included. LNP compositions that include a Cas nuclease mRNA, for example a Class 2 Cas nuclease mRNA that allows for expression in a cell of a Cas9 protein are provided. Further, the cargo may contain one or more guide RNAs or nucleic acids encoding guide RNAs. A template nucleic acid, e.g for repair or recombination, may also be included in the composition or a template nucleic acid may be used in the methods described herein.

"mRNA" refers to a polynucleotide that is not DNA and comprises an open reading frame that can be translated into a polypeptide (i.e., can serve as a substrate for translation by a ribosome and amino-acylated tRNAs). mRNA can comprise a phosphate-sugar backbone including ribose residues or analogs thereof, e.g., 2'-methoxy ribose residues. In some embodiments, the sugars of an mRNA phosphate-sugar backbone consist essentially of ribose residues, 2'-methoxy ribose residues, or a combination thereof. In general, mRNAs do not contain a substantial quantity of thymidine residues (e.g., 0 residues or fewer than 30, 20, 10, 5, 4, 3, or 2 thymidine residues; or less than 10%, 9%, 8%, 7%, 6%, 5%, 4%, 4%, 3%, 2%, 1%, 0.5%, 0.2%, or 0.1% thymidine content). An mRNA can contain modified uridines at some or all of its uridine positions.

## CRISPR/Cas Nuclease Systems

One component of the disclosed formulations is an mRNA encoding RNA-guided DNA-binding agent, such as a Cas nuclease.

As used herein, an "RNA-guided DNA binding agent" means a polypeptide or complex of polypeptides having RNA and DNA binding activity, or a DNA-binding subunit of such a complex, wherein the DNA binding activity is sequence-specific and depends on the sequence of the RNA. Exemplary RNA-guided DNA binding agents include Cas cleavases/nickases and inactivated forms thereof ("dCas DNA binding agents"). "Cas nuclease", as used herein, encompasses Cas cleavases, Cas nickases, and dCas DNA binding agents. Cas cleavases/nickases and dCas DNA binding agents include a Csm or Cmr complex of a type III CRISPR system, the Cas10, Csm1, or Cmr2 subunit thereof, a Cascade complex of a type I CRISPR system, the Cas3 subunit thereof, and Class 2 Cas nucleases. As used herein, a "Class 2 Cas nuclease" is a single-chain polypeptide with RNA-guided DNA binding activity. Class 2 Cas nucleases include Class 2 Cas cleavases/nickases (e.g., H840A, D10A, or N863A variants), which further have RNA-guided DNA cleavase or nickase activity, and Class 2 dCas DNA binding agents, in which cleavase/nickase activity is inactivated. Class 2 Cas nucleases include, for example, Cas9, Cpfl, C2c1, C2c2, C2c3, HF Cas9 (e.g., N497A, R661A, Q695A, Q926A variants), HypaCas9 (e.g., N692A, M694A, Q695A, H698A variants), eSPCas9(1.0) (e.g, K810A, K1003A, R1060A variants), and eSPCas9(1.1) (e.g., K848A, K1003A, R1060A variants) proteins and modifications thereof. Cpfl protein, Zetsche et al., Cell, 163: 1-13 (2015), is homologous to Cas9, and contains a RuvC-like nuclease domain. Cpf1 sequences of Zetsche are incorporated by reference in their entirety. *See, e.g.,* Zetsche, Tables S1 and S3. See, *e.g.,* Makarova et al., Nat Rev Microbiol, 13(11): 722-36 (2015); Shmakov et al., Molecular Cell, 60:385-397 (2015).

In some embodiments, the RNA-guided DNA-binding agent is a Class 2 Cas nuclease. In some embodiments, the RNA-guided DNA-binding agent has cleavase activity, which can also be referred to as double-strand endonuclease activity. In some embodiments, the RNA-guided DNA-binding agent comprises a Cas nuclease, such as a Class 2 Cas nuclease (which may be, e.g., a Cas nuclease of Type II, V, or VI). Class 2 Cas nucleases include, for example, Cas9, Cpfl, C2c1, C2c2, and C2c3 proteins and modifications thereof. Examples of Cas9 nucleases include those of the type II CRISPR systems of *S. pyogenes, S. aureus,* and other prokaryotes (see, e.g., the list in the next paragraph), and modified (e.g., engineered or mutant) versions thereof. See, e.g., US2016/0312198 A1; US 2016/0312199 A1. Other examples of Cas nucleases include a Csm or Cmr complex of a type III CRISPR system or the Cas10, Csm1, or Cmr2 subunit thereof; and a Cascade complex of a type I CRISPR system, or the Cas3 subunit thereof. In some embodiments, the Cas nuclease may be from a Type-IIA, Type-IIB, or Type-IIC system. For discussion of various CRISPR systems and Cas nucleases see, e.g., Makarova et al., Nat. Rev. Microbiol. 9:467-477 (2011); Makarova et al., Nat. Rev. Microbiol, 13: 722-36 (2015); Shmakov et al., Molecular Cell, 60:385-397 (2015).

Non-limiting exemplary species that the Cas nuclease can be derived from include *Streptococcus pyogenes, Streptococcus thermophilus, Streptococcus sp., Staphylococcus aureus, Listeria innocua, Lactobacillus gasseri, Francisella novicida, Wolinella succinogenes, Sutterella wadsworthensis, Gammaproteobacterium, Neisseria meningitidis, Campylobacter jejuni, Pasteurella multocida, Fibrobacter succinogene, Rhodospirillum rubrum, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Lactobacillus buchneri, Treponema denticola, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicelulosiruptor becscii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsoni, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospirct sp., Lyngbya sp., Microcoleus chthonoplastes, Oscillatoria sp., Petrotoga mobilis, Thermosipho africanus, Streptococcus pasteurianus, Neisseria cinerea, Campylobacter lari, Parvibaculum lavamentivorans, Corynebacterium diphtheria, Acidaminococcus sp., Lachnospiraceae bacterium ND2006,* and *Acaryochloris marina.*

In some embodiments, the Cas nuclease is the Cas9 nuclease from *Streptococcus pyogenes.* In some embodiments, the Cas nuclease is the Cas9 nuclease from *Streptococcus thermophilus.* In some embodiments, the Cas nuclease is the Cas9 nuclease from *Neisseria meningitidis.* In some embodiments, the Cas nuclease is the Cas9 nuclease is from *Staphylococcus aureus.* In some embodiments, the Cas nuclease is the Cpfl nuclease from *Francisella novicida.* In some embodiments, the Cas nuclease is the Cpfl nuclease from *Acidaminococcus sp.* In some embodiments, the Cas nuclease is the Cpfl nuclease from *Lachnospiraceae* bacterium ND2006. In further embodiments, the Cas nuclease is the Cpf1 nuclease from *Francisella tularensis, Lachnospiraceae bacterium, Butyrivibrio proteoclasticus, Peregrinibacteria bacterium, Parcubacteria bacterium, Smithella, Acidaminococcus, Candidatus Methanoplasma termitum, Eubacterium eligens, Moraxella bovoculi, Leptospira inadai, Porphyromonas crevioricanis, Prevotella disiens,* or *Porphyromonas macacae.* In certain embodiments, the Cas nuclease is a Cpf1 nuclease from an *Acidaminococcus* or *Lachnospiraceae.*

Wild type Cas9 has two nuclease domains: RuvC and HNH. The RuvC domain cleaves the non-target DNA strand, and the HNH domain cleaves the target strand of DNA. In some embodiments, the Cas9 nuclease comprises more

than one RuvC domain and/or more than one HNH domain. In some embodiments, the Cas9 nuclease is a wild type Cas9. In some embodiments, the Cas9 is capable of inducing a double strand break in target DNA. In certain embodiments, the Cas nuclease may cleave dsDNA, it may cleave one strand of dsDNA, or it may not have DNA cleavase or nickase activity. An exemplary Cas9 amino acid sequence is provided as SEQ ID NO: 3. An exemplary Cas9 mRNA ORF sequence, which includes start and stop codons, is provided as SEQ ID NO: 4. An exemplary Cas9 mRNA coding sequence, suitable for inclusion in a fusion protein, is provided as SEQ ID NO: 10.

In some embodiments, chimeric Cas nucleases are used, where one domain or region of the protein is replaced by a portion of a different protein. In some embodiments, a Cas nuclease domain may be replaced with a domain from a different nuclease such as Fok1. In some embodiments, a Cas nuclease may be a modified nuclease.

In other embodiments, the Cas nuclease may be from a Type-I CRISPR/Cas system. In some embodiments, the Cas nuclease may be a component of the Cascade complex of a Type-I CRISPR/Cas system. In some embodiments, the Cas nuclease may be a Cas3 protein. In some embodiments, the Cas nuclease may be from a Type-III CRISPR/Cas system. In some embodiments, the Cas nuclease may have an RNA cleavage activity.

In some embodiments, the RNA-guided DNA-binding agent has single-strand nickase activity, i.e., can cut one DNA strand to produce a single-strand break, also known as a "nick." In some embodiments, the RNA-guided DNA-binding agent comprises a Cas nickase. A nickase is an enzyme that creates a nick in dsDNA, i.e., cuts one strand but not the other of the DNA double helix. In some embodiments, a Cas nickase is a version of a Cas nuclease (e.g., a Cas nuclease discussed above) in which an endonucleolytic active site is inactivated, e.g., by one or more alterations (e.g., point mutations) in a catalytic domain. See, e.g., US Pat. No. 8,889,356 for discussion of Cas nickases and exemplary catalytic domain alterations. In some embodiments, a Cas nickase such as a Cas9 nickase has an inactivated RuvC or HNH domain.

In some embodiments, the RNA-guided DNA-binding agent is modified to contain only one functional nuclease domain. For example, the agent protein may be modified such that one of the nuclease domains is mutated or fully or partially deleted to reduce its nucleic acid cleavage activity. In some embodiments, a nickase is used having a RuvC domain with reduced activity. In some embodiments, a nickase is used having an inactive RuvC domain. In some embodiments, a nickase is used having an HNH domain with reduced activity. In some embodiments, a nickase is used having an inactive HNH domain.

In some embodiments, a conserved amino acid within a Cas protein nuclease domain is substituted to reduce or alter nuclease activity. In some embodiments, a Cas nuclease may comprise an amino acid substitution in the RuvC or RuvC-like nuclease domain. Exemplary amino acid substitutions in the RuvC or RuvC-like nuclease domain include D10A (based on the S. *pyogenes* Cas9 protein). *See, e.g.,* Zetsche et al. (2015) Cell Oct 22:163(3): 759-771. In some embodiments, the Cas nuclease may comprise an amino acid substitution in the HNH or HNH-like nuclease domain. Exemplary amino acid substitutions in the HNH or HNH-like nuclease domain include E762A, H840A, N863A, H983A, and D986A (based on the S. *pyogenes* Cas9 protein). *See, e.g.,* Zetsche et al. (2015). Further exemplary amino acid substitutions include D917A, E1006A, and D1255A (based on the *Francisella novicida* U112 Cpfl (FnCpf1) sequence (UniProtKB - A0Q7Q2 (CPF1_FRATN)).

In some embodiments, an mRNA encoding a nickase is provided in combination with a pair of guide RNAs that are complementary to the sense and antisense strands of the target sequence, respectively. In this embodiment, the guide RNAs direct the nickase to a target sequence and introduce a DSB by generating a nick on opposite strands of the target sequence (i.e., double nicking). In some embodiments, use of double nicking may improve specificity and reduce off-target effects. In some embodiments, a nickase is used together with two separate guide RNAs targeting opposite strands of DNA to produce a double nick in the target DNA. In some embodiments, a nickase is used together with two separate guide RNAs that are selected to be in close proximity to produce a double nick in the target DNA.

In some embodiments, the RNA-guided DNA-binding agent lacks cleavase and nickase activity. In some embodiments, the RNA-guided DNA-binding agent comprises a dCas DNA-binding polypeptide. A dCas polypeptide has DNA-binding activity while essentially lacking catalytic (cleavase/nickase) activity. In some embodiments, the dCas polypeptide is a dCas9 polypeptide. In some embodiments, the RNA-guided DNA-binding agent lacking cleavase and nickase activity or the dCas DNA-binding polypeptide is a version of a Cas nuclease (e.g., a Cas nuclease discussed above) in which its endonucleolytic active sites are inactivated, e.g., by one or more alterations (e.g., point mutations) in its catalytic domains. See, e.g., US 2014/0186958 A1; US 2015/0166980 A1.

In some embodiments, the RNA-guided DNA-binding agent comprises one or more heterologous functional domains (e.g., is or comprises a fusion polypeptide).

In some embodiments, the heterologous functional domain may facilitate transport of the RNA-guided DNA-binding agent into the nucleus of a cell. For example, the heterologous functional domain may be a nuclear localization signal (NLS). In some embodiments, the RNA-guided DNA-binding agent may be fused with 1-10 NLS(s). In some embodiments, the RNA-guided DNA-binding agent may be fused with 1-5 NLS(s). In some embodiments, the RNA-guided DNA-binding agent may be fused with one NLS. Where one NLS is used, the NLS may be linked at the N-terminus or the C-terminus of the RNA-guided DNA-binding agent sequence. It may also be inserted within the RNA-guided DNA binding

agent sequence. In other embodiments, the RNA-guided DNA-binding agent may be fused with more than one NLS. In some embodiments, the RNA-guided DNA-binding agent may be fused with 2, 3, 4, or 5 NLSs. In some embodiments, the RNA-guided DNA-binding agent may be fused with two NLSs. In certain circumstances, the two NLSs may be the same (*e.g.,* two SV40 NLSs) or different. In some embodiments, the RNA-guided DNA-binding agent is fused to two SV40 NLS sequences linked at the carboxy terminus. In some embodiments, the RNA-guided DNA-binding agent may be fused with two NLSs, one linked at the N-terminus and one at the C-terminus. In some embodiments, the RNA-guided DNA-binding agent may be fused with 3 NLSs. In some embodiments, the RNA-guided DNA-binding agent may be fused with no NLS. In some embodiments, the NLS may be a monopartite sequence, such as, *e.g.,* the SV40 NLS, PKKKRKV or PKKKRRV. In some embodiments, the NLS may be a bipartite sequence, such as the NLS of nucleoplasmin, KRPAATKKAGQAKKKK. In a specific embodiment, a single PKKKRKV NLS may be linked at the C-terminus of the RNA-guided DNA-binding agent. One or more linkers are optionally included at the fusion site.

In some embodiments, the heterologous functional domain may be capable of modifying the intracellular half-life of the RNA-guided DNA binding agent. In some embodiments, the half-life of the RNA-guided DNA binding agent may be increased. In some embodiments, the half-life of the RNA-guided DNA-binding agent may be reduced. In some embodiments, the heterologous functional domain may be capable of increasing the stability of the RNA-guided DNA-binding agent. In some embodiments, the heterologous functional domain may be capable of reducing the stability of the RNA-guided DNA-binding agent. In some embodiments, the heterologous functional domain may act as a signal peptide for protein degradation. In some embodiments, the protein degradation may be mediated by proteolytic enzymes, such as, for example, proteasomes, lysosomal proteases, or calpain proteases. In some embodiments, the heterologous functional domain may comprise a PEST sequence. In some embodiments, the RNA-guided DNA-binding agent may be modified by addition of ubiquitin or a polyubiquitin chain. In some embodiments, the ubiquitin may be a ubiquitin-like protein (UBL). Non-limiting examples of ubiquitin-like proteins include small ubiquitin-like modifier (SUMO), ubiquitin cross-reactive protein (UCRP, also known as interferon-stimulated gene-15 (ISG15)), ubiquitin-related modifier-1 (URM1), neuronal-precursor-cell-expressed developmentally downregulated protein-8 (NEDD8, also called Rub1 in *S. cerevisiae*), human leukocyte antigen F-associated (FAT10), autophagy-8 (ATG8) and -12 (ATG12), Fau ubiquitin-like protein (FUB1), membrane-anchored UBL (MUB), ubiquitin fold-modifier-1 (UFM1), and ubiquitin-like protein-5 (UBL5).

In some embodiments, the heterologous functional domain may be a marker domain. Non-limiting examples of marker domains include fluorescent proteins, purification tags, epitope tags, and reporter gene sequences. In some embodiments, the marker domain may be a fluorescent protein. Non-limiting examples of suitable fluorescent proteins include green fluorescent proteins (*e.g.,* GFP, GFP-2, tagGFP, turboGFP, sfGFP, EGFP, Emerald, Azami Green, Monomeric Azami Green, CopGFP, AceGFP, ZsGreen1), yellow fluorescent proteins (*e.g.,* YFP, EYFP, Citrine, Venus, YPet, PhiYFP, ZsYellow1), blue fluorescent proteins (*e.g.,* EBFP, EBFP2, Azurite, mKalamal, GFPuv, Sapphire, T-sapphire,), cyan fluorescent proteins (*e.g.,* ECFP, Cerulean, CyPet, AmCyan1, Midoriishi-Cyan), red fluorescent proteins (*e.g.,* mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (mOrange, mKO, Kusabira-Orange, Monomeric Kusabira-Orange, mTangerine, tdTomato) or any other suitable fluorescent protein. In other embodiments, the marker domain may be a purification tag and/or an epitope tag. Non-limiting exemplary tags include glutathione-S-transferase (GST), chitin binding protein (CBP), maltose binding protein (MBP), thioredoxin (TRX), poly(NANP), tandem affinity purification (TAP) tag, myc, AcV5, AU1, AU5, E, ECS, E2, FLAG, HA, nus, Softag 1, Softag 3, Strep, SBP, Glu-Glu, HSV, KT3, S, S1, T7, V5, VSV-G, 6xHis, 8xHis, biotin carboxyl carrier protein (BCCP), poly-His, and calmodulin. Non-limiting exemplary reporter genes include glutathione-S-transferase (GST), horseradish peroxidase (HRP), chloramphenicol acetyltransferase (CAT), beta-galactosidase, beta-glucuronidase, luciferase, or fluorescent proteins.

In additional embodiments, the heterologous functional domain may target the RNA-guided DNA-binding agent to a specific organelle, cell type, tissue, or organ. In some embodiments, the heterologous functional domain may target the RNA-guided DNA-binding agent to mitochondria.

In further embodiments, the heterologous functional domain may be an effector domain. When the RNA-guided DNA-binding agent is directed to its target sequence, *e.g.,* when a Cas nuclease is directed to a target sequence by a gRNA, the effector domain may modify or affect the target sequence. In some embodiments, the effector domain may be chosen from a nucleic acid binding domain, a nuclease domain (e.g., a non-Cas nuclease domain), an epigenetic modification domain, a transcriptional activation domain, or a transcriptional repressor domain. In some embodiments, the heterologous functional domain is a nuclease, such as a FokI nuclease. See, e.g., US Pat. No. 9,023,649. In some embodiments, the heterologous functional domain is a transcriptional activator or repressor. See, e.g., Qi et al., "Repurposing CRISPR as an RNA-guided platform for sequence-specific control of gene expression," Cell 152:1173-83 (2013); Perez-Pinera et al., "RNA-guided gene activation by CRISPR-Cas9-based transcription factors," Nat. Methods 10:973-6 (2013); Mali et al., "CAS9 transcriptional activators for target specificity screening and paired nickases for cooperative genome engineering," Nat. Biotechnol. 31:833-8 (2013); Gilbert et al., "CRISPR-mediated modular RNA-guided regulation of transcription in eukaryotes," Cell 154:442-51

(2013). As such, the RNA-guided DNA-binding agent essentially becomes a transcription factor that can be directed to bind a desired target sequence using a guide RNA. In certain embodiments, the DNA modification domain is a methylation domain, such as a demethylation or methyltransferase domain. In certain embodiments, the effector domain is a DNA modification domain, such as a base-editing domain. In particular embodiments, the DNA modification domain is a nucleic acid editing domain that introduces a specific modification into the DNA, such as a deaminase domain. *See, e.g.,* WO 2015/089406; US 2016/0304846. The nucleic acid editing domains, deaminase domains, and Cas9 variants described in WO 2015/089406 and U.S. 2016/0304846 are hereby incorporated by reference.

The nuclease may comprise at least one domain that interacts with a guide RNA ("gRNA"). Additionally, the nuclease may be directed to a target sequence by a gRNA. In Class 2 Cas nuclease systems, the gRNA interacts with the nuclease as well as the target sequence, such that it directs binding to the target sequence. In some embodiments, the gRNA provides the specificity for the targeted cleavage, and the nuclease may be universal and paired with different gRNAs to cleave different target sequences. Class 2 Cas nuclease may pair with a gRNA scaffold structure of the types, orthologs, and exemplary species listed above.

**Guide RNA (gRNA)**

In some embodiments of the present disclosure, the cargo for the LNP formulation includes at least one gRNA. The gRNA may guide the Cas nuclease or Class 2 Cas nuclease to a target sequence on a target nucleic acid molecule. In some embodiments, a gRNA binds with and provides specificity of cleavage by a Class 2 Cas nuclease. In some embodiments, the gRNA and the Cas nuclease may form a ribonucleoprotein (RNP), *e.g.,* a CRISPR/Cas complex such as a CRISPR/Cas9 complex. In some embodiments, the CRISPR/Cas complex may be a Type-II CRISPR/Cas9 complex. In some embodiments, the CRISPR/Cas complex may be a Type-V CRISPR/Cas complex, such as a Cpf1/guide RNA complex. Cas nucleases and cognate gRNAs may be paired. The gRNA scaffold structures that pair with each Class 2 Cas nuclease vary with the specific CRISPR/Cas system.

"Guide RNA", "gRNA", and simply "guide" are used herein interchangeably to refer to either a crRNA (also known as CRISPR RNA), or the combination of a crRNA and a trRNA (also known as tracrRNA). The crRNA and trRNA may be associated as a single RNA molecule (single guide RNA, sgRNA) or in two separate RNA molecules (dual guide RNA, dgRNA). "Guide RNA" or "gRNA" refers to each type. The trRNA may be a naturally-occurring sequence, or a trRNA sequence with modifications or variations compared to naturally-occurring sequences.

As used herein, a "guide sequence" refers to a sequence within a guide RNA that is complementary to a target sequence and functions to direct a guide RNA to a target sequence for binding or modification (e.g., cleavage) by an RNA-guided DNA binding agent. A "guide sequence" may also be referred to as a "targeting sequence," or a "spacer sequence." A guide sequence can be 20 base pairs in length, e.g., in the case of *Streptococcus pyogenes* (i.e., Spy Cas9) and related Cas9 homologs/orthologs. Shorter or longer sequences can also be used as guides, e.g., 15-, 16-, 17-, 18-, 19-, 21-, 22-, 23-, 24-, or 25-nucleotides in length. In some embodiments, the target sequence is in a gene or on a chromosome, for example, and is complementary to the guide sequence. In some embodiments, the degree of complementarity or identity between a guide sequence and its corresponding target sequence may be about 75%, 80%, 85%, 90%, 95%, 96%, 97%, 98%, 99%, or 100%. In some embodiments, the guide sequence and the target region may be 100% complementary or identical. In other embodiments, the guide sequence and the target region may contain at least one mismatch. For example, the guide sequence and the target sequence may contain 1, 2, 3, or 4 mismatches, where the total length of the target sequence is at least 17, 18, 19, 20 or more base pairs. In some embodiments, the guide sequence and the target region may contain 1-4 mismatches where the guide sequence comprises at least 17, 18, 19, 20 or more nucleotides. In some embodiments, the guide sequence and the target region may contain 1, 2, 3, or 4 mismatches where the guide sequence comprises 20 nucleotides.

Target sequences for Cas proteins include both the positive and negative strands of genomic DNA (i.e., the sequence given and the sequence's reverse compliment), as a nucleic acid substrate for a Cas protein is a double stranded nucleic acid. Accordingly, where a guide sequence is said to be "complementary to a target sequence", it is to be understood that the guide sequence may direct a guide RNA to bind to the reverse complement of a target sequence. Thus, in some embodiments, where the guide sequence binds the reverse complement of a target sequence, the guide sequence is identical to certain nucleotides of the target sequence (e.g., the target sequence not including the PAM) except for the substitution of U for T in the guide sequence.

The length of the targeting sequence may depend on the CRISPR/Cas system and components used. For example, different Class 2 Cas nucleases from different bacterial species have varying optimal targeting sequence lengths. Accordingly, the targeting sequence may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length. In some embodiments, the targeting sequence length is 0, 1, 2, 3, 4, or 5 nucleotides longer or shorter than the guide sequence of a naturally-occurring CRISPR/Cas system. In certain embodiments, the Cas nuclease and gRNA scaffold will be derived from the same CRISPR/Cas system. In some embodiments, the targeting sequence may comprise or consist of 18-24 nucleotides. In

some embodiments, the targeting sequence may comprise or consist of 19-21 nucleotides. In some embodiments, the targeting sequence may comprise or consist of 20 nucleotides.

In some embodiments, the sgRNA is a "Cas9 sgRNA" capable of mediating RNA-guided DNA cleavage by a Cas9 protein. In some embodiments, the sgRNA is a "Cpfl sgRNA" capable of mediating RNA-guided DNA cleavage by a Cpfl protein. In certain embodiments, the gRNA comprises a crRNA and tracr RNA sufficient for forming an active complex with a Cas9 protein and mediating RNA-guided DNA cleavage. In certain embodiments, the gRNA comprises a crRNA sufficient for forming an active complex with a Cpf1 protein and mediating RNA-guided DNA cleavage. *See* Zetsche 2015.

Certain embodiments of the invention also provide nucleic acids, *e.g.,* expression cassettes, encoding the gRNA described herein. A "guide RNA nucleic acid" is used herein to refer to a guide RNA (*e.g.* an sgRNA or a dgRNA) and a guide RNA expression cassette, which is a nucleic acid that encodes one or more guide RNAs.

In some embodiments, the nucleic acid may be a DNA molecule. In some embodiments, the nucleic acid may comprise a nucleotide sequence encoding a crRNA. In some embodiments, the nucleotide sequence encoding the crRNA comprises a targeting sequence flanked by all or a portion of a repeat sequence from a naturally-occurring CRISPR/Cas system. In some embodiments, the nucleic acid may comprise a nucleotide sequence encoding a tracr RNA. In some embodiments, the crRNA and the tracr RNA may be encoded by two separate nucleic acids. In other embodiments, the crRNA and the tracr RNA may be encoded by a single nucleic acid. In some embodiments, the crRNA and the tracr RNA may be encoded by opposite strands of a single nucleic acid. In other embodiments, the crRNA and the tracr RNA may be encoded by the same strand of a single nucleic acid. In some embodiments, the gRNA nucleic acid encodes an sgRNA. In some embodiments, the gRNA nucleic acid encodes a Cas9 nuclease sgRNA. In come embodiments, the gRNA nucleic acid encodes a Cpfl nuclease sgRNA.

The nucleotide sequence encoding the guide RNA may be operably linked to at least one transcriptional or regulatory control sequence, such as a promoter, a 3' UTR, or a 5' UTR. In one example, the promoter may be a tRNA promoter, *e.g.,* $tRNA^{Lys3}$, or a tRNA chimera. *See* Mefferd et al., RNA. 2015 21:1683-9; Scherer et al., Nucleic Acids Res. 2007 35: 2620-2628. In certain embodiments, the promoter may be recognized by RNA polymerase III (Pol III). Non-limiting examples of Pol III promoters also include U6 and H1 promoters. In some embodiments, the nucleotide sequence encoding the guide RNA may be operably linked to a mouse or human U6 promoter. In some embodiments, the gRNA nucleic acid is a modified nucleic acid. In certain embodiments, the gRNA nucleic acid includes a modified nucleoside or nucleotide. In some embodiments, the gRNA nucleic acid includes a 5' end modification, for example a modified nucleoside or nucleotide to stabilize and prevent integration of the nucleic acid. In some embodiments, the gRNA nucleic acid comprises a double-stranded DNA having a 5' end modification on each strand. In certain embodiments, the gRNA nucleic acid includes an inverted dideoxy-T or an inverted abasic nucleoside or nucleotide as the 5' end modification. In some embodiments, the gRNA nucleic acid includes a label such as biotin, desthiobioten-TEG, digoxigenin, and fluorescent markers, including, for example, FAM, ROX, TAMRA, and AlexaFluor.

In certain embodiments, more than one gRNA nucleic acid, such as a gRNA, can be used with a CRISPR/Cas nuclease system. Each gRNA nucleic acid may contain a different targeting sequence, such that the CRISPR/Cas system cleaves more than one target sequence. In some embodiments, one or more gRNAs may have the same or differing properties such as activity or stability within a CRISPR/Cas complex. Where more than one gRNA is used, each gRNA can be encoded on the same or on different gRNA nucleic acid. The promoters used to drive expression of the more than one gRNA may be the same or different.

**Modified RNAs**

In certain embodiments, the LNP compositions comprise modified RNAs.

Modified nucleosides or nucleotides can be present in an RNA, for example a gRNA or mRNA. A gRNA or mRNA comprising one or more modified nucleosides or nucleotides, for example, is called a "modified" RNA to describe the presence of one or more non-naturally and/or naturally occurring components or configurations that are used instead of or in addition to the canonical A, G, C, and U residues. In some embodiments, a modified RNA is synthesized with a non-canonical nucleoside or nucleotide, here called "modified."

Modified nucleosides and nucleotides can include one or more of: (i) alteration, *e.g.,* replacement, of one or both of the non-linking phosphate oxygens and/or of one or more of the linking phosphate oxygens in the phosphodiester backbone linkage (an exemplary backbone modification); (ii) alteration, *e.g.,* replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.,* removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification). Certain embodiments comprise a 5'

end modification to an mRNA, gRNA, or nucleic acid. Certain embodiments comprise a 3' end modification to an mRNA, gRNA, or nucleic acid. A modified RNA can contain 5' end and 3' end modifications. A modified RNA can contain one or more modified residues at non-terminal locations. In certain embodiments, a gRNA includes at least one modified residue. In certain embodiments, an mRNA includes at least one modified residue.

As used herein, a first sequence is considered to "comprise a sequence with at least X% identity to" a second sequence if an alignment of the first sequence to the second sequence shows that X% or more of the positions of the second sequence in its entirety are matched by the first sequence. For example, the sequence AAGA comprises a sequence with 100% identity to the sequence AAG because an alignment would give 100% identity in that there are matches to all three positions of the second sequence. The differences between RNA and DNA (generally the exchange of uridine for thymidine or vice versa) and the presence of nucleoside analogs such as modified uridines do not contribute to differences in identity or complementarity among polynucleotides as long as the relevant nucleotides (such as thymidine, uridine, or modified uridine) have the same complement (e.g., adenosine for all of thymidine, uridine, or modified uridine; another example is cytosine and 5-methylcytosine, both of which have guanosine or modified guanosine as a complement). Thus, for example, the sequence 5'-AXG where X is any modified uridine, such as pseudouridine, N1-methyl pseudouridine, or 5-methoxyuridine, is considered 100% identical to AUG in that both are perfectly complementary to the same sequence (5'-CAU). Exemplary alignment algorithms are the Smith-Waterman and Needleman-Wunsch algorithms, which are well-known in the art. One skilled in the art will understand what choice of algorithm and parameter settings are appropriate for a given pair of sequences to be aligned; for sequences of generally similar length and expected identity >50% for amino acids or >75% for nucleotides, the Needleman-Wunsch algorithm with default settings of the Needleman-Wunsch algorithm interface provided by the EBI at the www.ebi.ac.uk web server is generally appropriate.

mRNAs

In some embodiments, a composition or formulation disclosed herein comprises an mRNA comprising an open reading frame (ORF), such as, e.g. an ORF encoding an RNA-guided DNA binding agent, such as a Cas nuclease, or Class 2 Cas nuclease as described herein. In some embodiments, an mRNA comprising an ORF encoding an RNA-guided DNA binding agent, such as a Cas nuclease or Class 2 Cas nuclease, is provided, used, or administered. In some embodiments, the ORF encoding an RNA-guided DNA binding agent is a "modified RNA-guided DNA binding agent ORF" or simply a "modified ORF," which is used as shorthand to indicate that the ORF is modified in one or more of the following ways: (1) the modified ORF has a uridine content ranging from its minimum uridine content to 150% of the minimum uridine content; (2) the modified ORF has a uridine dinucleotide content ranging from its minimum uridine dinucleotide content to 150% of the minimum uridine dinucleotide content; (3) the modified ORF has at least 90% identity to any one of SEQ ID NOs: 1, 4, 10, 14, 15, 17, 18, 20, 21, 23, 24, 26, 27, 29, 30, 50, 52, 54, 65, or 66; (4) the modified ORF consists of a set of codons of which at least 75% of the codons are minimal uridine codon(s) for a given amino acid, e.g. the codon(s) with the fewest uridines (usually 0 or 1 except for a codon for phenylalanine, where the minimal uridine codon has 2 uridines); or (5) the modified ORF comprises at least one modified uridine. In some embodiments, the modified ORF is modified in at least two, three, or four of the foregoing ways. In some embodiments, the modified ORF comprises at least one modified uridine and is modified in at least one, two, three, or all of (1)-(4) above.

"Modified uridine" is used herein to refer to a nucleoside other than thymidine with the same hydrogen bond acceptors as uridine and one or more structural differences from uridine. In some embodiments, a modified uridine is a substituted uridine, i.e., a uridine in which one or more non-proton substituents (e.g., alkoxy, such as methoxy) takes the place of a proton. In some embodiments, a modified uridine is pseudouridine. In some embodiments, a modified uridine is a substituted pseudouridine, i.e., a pseudouridine in which one or more non-proton substituents (e.g., alkyl, such as methyl) takes the place of a proton. In some embodiments, a modified uridine is any of a substituted uridine, pseudouridine, or a substituted pseudouridine.

"Uridine position" as used herein refers to a position in a polynucleotide occupied by a uridine or a modified uridine. Thus, for example, a polynucleotide in which "100% of the uridine positions are modified uridines" contains a modified uridine at every position that would be a uridine in a conventional RNA (where all bases are standard A, U, C, or G bases) of the same sequence. Unless otherwise indicated, a U in a polynucleotide sequence of a sequence table or sequence listing in, or accompanying, this disclosure can be a uridine or a modified uridine.

Table 1. Minimal Uridine Codons

| | **Amino Acid** | **Minimal uridine codon** |
|---|---|---|
| A | Alanine | GCA or GCC or GCG |
| G | Glycine | GGA or GGC or GGG |
| V | Valine | GUC or GUA or GUG |

(continued)

| | Amino Acid | Minimal uridine codon |
|---|---|---|
| D | Aspartic acid | GAC |
| E | Glutamic acid | GAA or GAG |
| I | Isoleucine | AUC or AUA or AUG |
| T | Threonine | ACA or ACC or ACG |
| N | Asparagine | AAC |
| K | Lysine | AAG or AAA |
| S | Serine | AGC |
| R | Arginine | AGA or AGG |
| L | Leucine | CUG or CUA or CUC |
| P | Proline | CCG or CCA or CCC |
| H | Histidine | CAC or CAA or CAG |
| Q | Glutamine | CAG or CAA |
| F | Phenylalanine | UUC |
| Y | Tyrosine | UAC |
| C | Cysteine | UGC |
| W | Tryptophan | UGG |
| M | Methionine | AUG |

In any of the foregoing embodiments, the modified ORF may consist of a set of codons of which at least 75%, 80%, 85%, 90%, 95%, 98%, 99%, or 100% of the codons are codons listed in the Table of Minimal Uridine Codons. In any of the foregoing embodiments, the modified ORF may comprise a sequence with at least 90%, 95%, 98%, 99%, or 100% identity to any one of SEQ ID NO: 1, 4, 10, 14, 15, 17, 18, 20, 21, 23, 24, 26, 27, 29, 30, 50, 52, 54, 65, or 66.

In any of the foregoing embodiments, the modified ORF may have a uridine content ranging from its minimum uridine content to 150%, 145%, 140%, 135%, 130%, 125%, 120%, 115%, 110%, 105%, 104%, 103%, 102%, or 101% of the minimum uridine content.

In any of the foregoing embodiments, the modified ORF may have a uridine dinucleotide content ranging from its minimum uridine dinucleotide content to 150%, 145%, 140%, 135%, 130%, 125%, 120%, 115%, 110%, 105%, 104%, 103%, 102%, or 101% of the minimum uridine dinucleotide content.

In any of the foregoing embodiments, the modified ORF may comprise a modified uridine at least at one, a plurality of, or all uridine positions. In some embodiments, the modified uridine is a uridine modified at the 5 position, e.g., with a halogen, methyl, or ethyl. In some embodiments, the modified uridine is a pseudouridine modified at the 1 position, e.g., with a halogen, methyl, or ethyl. The modified uridine can be, for example, pseudouridine, N1-methyl-pseudouridine, 5-methoxyuridine, 5-iodouridine, or a combination thereof. In some embodiments, the modified uridine is 5-methoxyuridine. In some embodiments, the modified uridine is 5-iodouridine. In some embodiments, the modified uridine is pseudouridine. In some embodiments, the modified uridine is N1-methyl-pseudouridine. In some embodiments, the modified uridine is a combination of pseudouridine and N1-methyl-pseudouridine. In some embodiments, the modified uridine is a combination of pseudouridine and 5-methoxyuridine. In some embodiments, the modified uridine is a combination of N1-methyl pseudouridine and 5-methoxyuridine. In some embodiments, the modified uridine is a combination of 5-iodouridine and N1-methyl-pseudouridine. In some embodiments, the modified uridine is a combination of pseudouridine and 5-iodouridine. In some embodiments, the modified uridine is a combination of 5-iodouridine and 5-methoxyuridine.

In some embodiments, at least 10%, 15%, 20%, 25%, 30%, 35%, 40%, 45%, 50%, 55%, 60%, 65%, 70%, 75%, 80%, 85%, 90%, 95%, 98%, 99%, or 100% of the uridine positions in an mRNA according to the disclosure are modified uridines. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are modified uridines, e.g., 5-methoxyuridine, 5-iodouridine, N1-methyl pseudouridine, pseudouridine, or a combination thereof. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-methoxyuridine. In some embodiments, 10%-25%, 15-25%, 25-35%, 35-45%, 45-55%,

55-65%, 65-75%, 75-85%, 85-95%, or 90-100% of the uridine positions in an mRNA according to the disclosure are pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-iodouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-methoxyuridine, and the remainder are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in an mRNA according to the disclosure are 5-iodouridine, and the remainder are N1-methyl pseudouridine.

In any of the foregoing embodiments, the modified ORF may comprise a reduced uridine dinucleotide content, such as the lowest possible uridine dinucleotide (UU) content, e.g. an ORF that (a) uses a minimal uridine codon (as discussed above) at every position and (b) encodes the same amino acid sequence as the given ORF. The uridine dinucleotide (UU) content can be expressed in absolute terms as the enumeration of UU dinucleotides in an ORF or on a rate basis as the percentage of positions occupied by the uridines of uridine dinucleotides (for example, AUUAU would have a uridine dinucleotide content of 40% because 2 of 5 positions are occupied by the uridines of a uridine dinucleotide). Modified uridine residues are considered equivalent to uridines for the purpose of evaluating minimum uridine dinucleotide content.

In some embodiments, the mRNA comprises at least one UTR from an expressed mammalian mRNA, such as a constitutively expressed mRNA. An mRNA is considered constitutively expressed in a mammal if it is continually transcribed in at least one tissue of a healthy adult mammal. In some embodiments, the mRNA comprises a 5' UTR, 3' UTR, or 5' and 3' UTRs from an expressed mammalian RNA, such as a constitutively expressed mammalian mRNA. Actin mRNA is an example of a constitutively expressed mRNA.

In some embodiments, the mRNA comprises at least one UTR from Hydroxysteroid 17-Beta Dehydrogenase 4 (HSD17B4 or HSD), e.g., a 5' UTR from HSD. In some embodiments, the mRNA comprises at least one UTR from a globin mRNA, for example, human alpha globin (HBA) mRNA, human beta globin (HBB) mRNA, or Xenopus laevis beta globin (XBG) mRNA. In some embodiments, the mRNA comprises a 5' UTR, 3' UTR, or 5' and 3' UTRs from a globin mRNA, such as HBA, HBB, or XBG. In some embodiments, the mRNA comprises a 5' UTR from bovine growth hormone, cytomegalovirus (CMV), mouse Hba-a1, HSD, an albumin gene, HBA, HBB, or XBG. In some embodiments, the mRNA comprises a 3' UTR from bovine growth hormone, cytomegalovirus, mouse Hba-a1, HSD, an albumin gene, HBA, HBB, or XBG. In some embodiments, the mRNA comprises 5' and 3' UTRs from bovine growth hormone, cytomegalovirus, mouse Hba-a1, HSD, an albumin gene, HBA, HBB, XBG, heat shock protein 90 (Hsp90), glyceraldehyde 3-phosphate dehydrogenase (GAPDH), beta-actin, alpha-tubulin, tumor protein (p53), or epidermal growth factor receptor (EGFR).

In some embodiments, the mRNA comprises 5' and 3' UTRs that are from the same source, e.g., a constitutively expressed mRNA such as actin, albumin, or a globin such as HBA, HBB, or XBG.

In some embodiments, the mRNA does not comprise a 5' UTR, e.g., there are no additional nucleotides between the 5' cap and the start codon. In some embodiments, the mRNA comprises a Kozak sequence (described below) between the 5' cap and the start codon, but does not have any additional 5' UTR. In some embodiments, the mRNA does not comprise a 3' UTR, e.g., there are no additional nucleotides between the stop codon and the poly-A tail.

In some embodiments, the mRNA comprises a Kozak sequence. The Kozak sequence can affect translation initiation and the overall yield of a polypeptide translated from an mRNA. A Kozak sequence includes a methionine codon that can function as the start codon. A minimal Kozak sequence is NNNRUGN wherein at least one of the following is true: the first N is A or G and the second N is G. In the context of a nucleotide sequence, R means a purine (A or G). In some embodiments, the Kozak sequence is RNNRUGN, NNNRUGG, RNNRUGG, RNNAUGN, NNNAUGG, or RNNAUGG. In some embodiments, the Kozak sequence is rccRUGg with zero mismatches or with up to one or two mismatches to positions in lowercase. In some embodiments, the Kozak sequence is rccAUGg with zero mismatches or with up to one or two mismatches to positions in lowercase. In some embodiments, the Kozak sequence is gccRccAUGG with zero mismatches or with up to one, two, or three mismatches to positions in lowercase. In some embodiments, the Kozak sequence is gccAccAUG with zero mismatches or with up to one, two, three, or four mismatches to positions in lowercase. In some embodiments, the Kozak sequence is GCCACCAUG. In some embodiments, the Kozak sequence is gccgccRccAUGG with zero mismatches or with up to one, two, three, or four mismatches to positions in lowercase.

In some embodiments, the mRNA comprising an ORF encoding an RNA-guided DNA binding agent comprises a sequence having at least 90% identity to SEQ ID NO: 43, optionally wherein the ORF of SEQ ID NO: 43 (i.e., SEQ ID NO: 4) is substituted with an alternative ORF. In some embodiments, the mRNA comprises any of SEQ ID NOs: 10, 14, 15, 17, 18, 20, 21, 23, 24, 26, 27, 29, 30, 50, 52, 54, 65, or 66.

In some embodiments, the degree of identity to the optionally substituted sequence of SEQ ID NO: 43 is 95%. In some embodiments, the degree of identity to the optionally substituted sequence of SEQ ID NO: 4 is 98%. In some embodiments, the degree of identity to the optionally substituted sequence of SEQ ID NO: 43 is 99%. In some embodiments, the degree of identity to the optionally substituted sequence of SEQ ID NO: 43 is 100%.

In some embodiments, an mRNA disclosed herein comprises a 5' cap, such as a Cap0, Cap1, or Cap2. A 5' cap is generally a 7-methylguanine ribonucleotide (which may be further modified, as discussed below e.g. with respect to ARCA) linked through a 5'-triphosphate to the 5' position of the first nucleotide of the 5'-to-3' chain of the mRNA, i.e., the first cap-proximal nucleotide. In Cap0, the riboses of the first and second cap-proximal nucleotides of the mRNA both comprise a 2'-hydroxyl. In Cap1, the riboses of the first and second transcribed nucleotides of the mRNA comprise a 2'-methoxy and a 2'-hydroxyl, respectively. In Cap2, the riboses of the first and second cap-proximal nucleotides of the mRNA both comprise a 2'-methoxy. See, e.g., Katibah et al. (2014) Proc Natl Acad Sci USA 111(33):12025-30; Abbas et al. (2017) Proc Natl Acad Sci USA 114(11):E2106-E2115. Most endogenous higher eukaryotic mRNAs, including mammalian mRNAs such as human mRNAs, comprise Cap1 or Cap2. Cap0 and other cap structures differing from Cap1 and Cap2 may be immunogenic in mammals, such as humans, due to recognition as "non-self" by components of the innate immune system such as IFIT-1 and IFIT-5, which can result in elevated cytokine levels including type I interferon. Components of the innate immune system such as IFIT-1 and IFIT-5 may also compete with eIF4E for binding of an mRNA with a cap other than Cap1 or Cap2, potentially inhibiting translation of the mRNA.

A cap can be included co-transcriptionally. For example, ARCA (anti-reverse cap analog; Thermo Fisher Scientific Cat. No. AM8045) is a cap analog comprising a 7-methylguanine 3'-methoxy-5'-triphosphate linked to the 5' position of a guanine ribonucleotide which can be incorporated *in vitro* into a transcript at initiation. ARCA results in a Cap0 cap in which the 2' position of the first cap-proximal nucleotide is hydroxyl. See, e.g., Stepinski et al., (2001) "Synthesis and properties of mRNAs containing the novel 'anti-reverse' cap analogs 7-methyl(3'-O-methyl)GpppG and 7-methyl(3'deoxy) GpppG," RNA 7: 1486-1495. The ARCA structure is shown below.

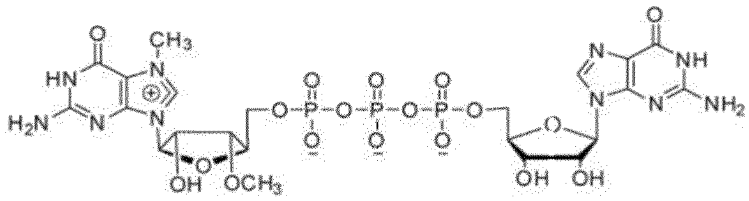

CleanCap™ AG (m7G(5')ppp(5')(2'OMeA)pG; TriLink Biotechnologies Cat. No. N-7113) or CleanCap™ GG (m7G(5')ppp(5')(2'OMeG)pG; TriLink Biotechnologies Cat. No. N-7133) can be used to provide a Cap1 structure co-transcriptionally. 3'-O-methylated versions of CleanCap™ AG and CleanCap™ GG are also available from TriLink Biotechnologies as Cat. Nos. N-7413 and N-7433, respectively. The CleanCap™ AG structure is shown below.

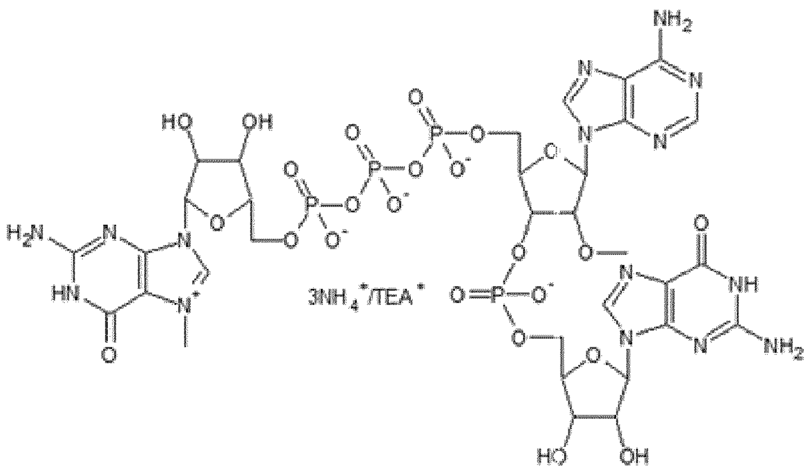

Alternatively, a cap can be added to an RNA post-transcriptionally. For example, Vaccinia capping enzyme is commercially available (New England Biolabs Cat. No. M2080S) and has RNA triphosphatase and guanylyltransferase activities, provided by its D1 subunit, and guanine methyltransferase, provided by its D12 subunit. As such, it can add a 7-methylguanine to an RNA, so as to give Cap0, in the presence of S-adenosyl methionine and GTP. See, e.g., Guo, P. and Moss, B. (1990) Proc. Natl. Acad. Sci. USA 87, 4023-4027; Mao, X. and Shuman, S. (1994) J. Biol. Chem. 269, 24472-24479.

In some embodiments, the mRNA further comprises a poly-adenylated (poly-A) tail. In some embodiments, the poly-A tail comprises at least 20, 30, 40, 50, 60, 70, 80, 90, or 100 adenines, optionally up to 300 adenines. In some embodiments, the poly-A tail comprises 95, 96, 97, 98, 99, or 100 adenine nucleotides. In some instances, the poly-A tail is

"interrupted" with one or more non-adenine nucleotide "anchors" at one or more locations within the poly-A tail. The poly-A tails may comprise at least 8 consecutive adenine nucleotides, but also comprise one or more non-adenine nucleotide. As used herein, "non-adenine nucleotides" refer to any natural or non-natural nucleotides that do not comprise adenine. Guanine, thymine, and cytosine nucleotides are exemplary non-adenine nucleotides. Thus, the poly-A tails on the mRNA described herein may comprise consecutive adenine nucleotides located 3' to nucleotides encoding an RNA-guided DNA-binding agent or a sequence of interest. In some instances, the poly-A tails on mRNA comprise non-consecutive adenine nucleotides located 3' to nucleotides encoding an RNA-guided DNA-binding agent or a sequence of interest, wherein non-adenine nucleotides interrupt the adenine nucleotides at regular or irregularly spaced intervals.

As used herein, "non-adenine nucleotides" refer to any natural or non-natural nucleotides that do not comprise adenine. Guanine, thymine, and cytosine nucleotides are exemplary non-adenine nucleotides. Thus, the poly-A tails on the mRNA described herein may comprise consecutive adenine nucleotides located 3' to nucleotides encoding an RNA-guided DNA-binding agent or a sequence of interest. In some instances, the poly-A tails on mRNA comprise non-consecutive adenine nucleotides located 3' to nucleotides encoding an RNA-guided DNA-binding agent or a sequence of interest, wherein non-adenine nucleotides interrupt the adenine nucleotides at regular or irregularly spaced intervals.

In some embodiments, the mRNA is purified. In some embodiments, the mRNA is purified using a precipation method (*e.g.,* LiCl precipitation, alcohol precipitation, or an equivalent method, *e.g.,* as described herein). In some embodiments, the mRNA is purified using a chromatography-based method, such as an HPLC-based method or an equivalent method (*e.g.,* as described herein). In some embodiments, the mRNA is purified using both a precipitation method (*e.g.,* LiCl precipitation) and an HPLC-based method.

In some embodiments, at least one gRNA is provided in combination with an mRNA disclosed herein. In some embodiments, a gRNA is provided as a separate molecule from the mRNA. In some embodiments, a gRNA is provided as a part, such as a part of a UTR, of an mRNA disclosed herein.

gRNAs

In an aspect, the present disclosure provides for methods of delivering a genome editing system (for example a zinc finger nuclease system, a TALEN system, a meganuclease system or a CRISPR/Cas system) to a cell (or population of cells), for example an HSPC (or population of HSPCs), for example a CD34+ cell (or population of CD34+ cells), wherein the result is a cell (or its progeny) which has increased fetal hemoglobin expression (e.g., when said cell is differentiated into an erythrocyte). Disclosed herein are guide sequences useful in achieving that effect. In embodiments, the genome editing system comprises one or more vectors, e.g., mRNA, encoding the components of the genome editing system. In other embodiments, the genome editing system comprises one or more polypeptides. In a preferred aspect, the methods comprise delivering a CRISPR/Cas system. In embodiments the CRISPR/Cas system comprises a gRNA and a Cas nuclease, for example, complexed in the form of a ribonuclear protein complex (RNP). In other embodiments the CRISPR/Cas system comprises one or more vectors encoding a gRNA and/or a Cas nuclease. In other embodiments the CRISPR/Cas system comprises one or more vectors, e.g., mRNA, encoding a Cas nuclease (e.g., a Class 2 Cas nuclease) and one or more gRNAs. In aspects, the CRISPR/Cas system includes a gRNA described in WO2017/115268, the contents of which are incorporated herein by reference in their entirety. In aspects, the CRISPR/Cas system includes a gRNA comprising a guide sequence complementary to a target sequence within the BCL11a gene or its regulatory elements. In other aspects, the CRISPR/Cas system includes a gRNA comprising a guide sequence complementary to a target sequence within intron 2 of the BCL11a gene (e.g., within a region of intron 2 of the BCL11a gene at or near a GATA1 binding site. In aspects, the CRISPR/Cas system includes a gRNA comprising a guide sequence complementary to a target sequence within the region of intron 2 of the BCL11a gene from ch2:60494000 to ch2:60498000 (according to hg38), for example, within a region of intron 2 of the BCL11a gene from ch2:60494250 to ch2:60496300 (according to hg38). In embodiments, the CRISPR/Cas system includes a gRNA comprising a guide sequence listed in Table 2 of US Provisional Application No. 62/566,232, filed September 29, 2017, which is hereby incorporated by reference.

Exemplary guide sequences of gRNAs which are complementary to target sequences within intron 2 of the BCL11a gene. +58, +62 and +55 refer to the DNAse hypersensitivity sites of the erythroid-specific enhancer region as described in Bauer et al., Science 2013; 342(6155): 253-257.

In other aspects, the CRISPR/Cas system includes a gRNA comprising a guide sequence complementary to a target sequence within the globin locus on chromosome 11. In an aspect, the CRISPR/Cas system includes a gRNA that comprises a guide sequence complementary to a sequence within an HPFH region. As used herein, the term "HPFH region" refers to a genomic site which, when modified (e.g., mutated or deleted), causes increased HbF production in adult red blood cells, and includes HPFH regions identified in the literature (see e.g., the Online Mendelian Inheritance in Man: http://www.omim.org/entry/141749, incorporated herein by reference). In an exemplary embodiment, the HPFH region is a region within or encompassing the beta globin gene cluster on chromosome 11p15. In an exemplary embodiment, the HPFH region is within or encompasses at least part of the delta globin gene and its regulatory elements. In an exemplary embodiment, the HPFH region is a region of the promoter of HBG1. In an exemplary embodiment, the HPFH region is a

region of the promoter of HBG2. In an exemplary embodiment, the HPFH region is a region described in Sankaran VG et al. NEJM (2011) 365:807-814, incorporated herein by reference in its entirety. In an exemplary embodiment, the HPFH region is the French breakpoint deletional HPFH as described in Sankaran VG et al. NEJM (2011) 365:807-814. In an exemplary embodiment, the HPFH region is the Algerian HPFH as described in Sankaran VG et al. NEJM (2011) 365:807-814. In an exemplary embodiment, the HPFH region is the Sri Lankan HPFH as described in Sankaran VG et al. NEJM (2011) 365:807-814. In an exemplary embodiment, the HPFH region is the HPFH-3 as described in Sankaran VG et al. NEJM (2011) 365:807-814. In an exemplary embodiment, the HPFH region is the HPFH-2 as described in Sankaran VG et al. NEJM (2011) 365:807-814. In an embodiment, the HPFH-1 region is the HPFH-3 as described in Sankaran VG et al. NEJM (2011) 365:807-814. In an exemplary embodiment, the HPFH region is the Sri Lankan (δβ)0-thalassemia HPFH as described in Sankaran VG et al. NEJM (2011) 365:807-814. In an exemplary embodiment, the HPFH region is the Sicilian (δβ)0-thalassemia HPFH as described in Sankaran VG et al. NEJM (2011) 365:807-814. In an exemplary embodiment, the HPFH region is the Macedonian (δβ)0-thalassemia HPFH as described in Sankaran VG et al. NEJM (2011) 365:807-814. In an exemplary embodiment, the HPFH region is the Kurdish β0-thalassemia HPFH as described in Sankaran VG et al. NEJM (2011) 365:807-814. In an exemplary embodiment, the HPFH region is the region located at Chr11:5213874-5214400 (hg18). In an exemplary embodiment, the HPFH region is the region located at Chr11:5215943-5215046 (hg18). In an exemplary embodiment, the HPFH region is the region located at Chr11:5234390-5238486 (hg38). In embodiments, the CRISPR/Cas system includes a gRNA comprising a guide sequence comprising a sequence as described in WO2017/077394, the contents of which are incorporated herein by reference in its entirety. In embodiments, the CRISPR/Cas system includes a gRNA comprising a guide sequence comprising a sequence selected from a guide sequence of WO2017/077394. In embodiments, the CRISPR/Cas system includes a gRNA comprising a guide sequence listed in Table 3 of US Provisional Application No. 62/566,232, filed September 29, 2017, which is hereby incorporated by reference.

Exemplary guide sequences directed to the French HPFH (French HPFH; Sankaran VG et al. A functional element necessary for fetal hemoglobin silencing. NEJM (2011) 365:807-814.)

In embodiments, the CRISPR/Cas system includes a gRNA comprising a guide sequence listed in Table 4 of US Provisional Application No. 62/566,232, filed September 29, 2017, which is hereby incorporated by reference.

Exemplary guide sequences may be directed to the HBG1 and/or HBG2 promoter regions.

Chemically Modified gRNA

In some embodiments, the gRNA is chemically modified. A gRNA comprising one or more modified nucleosides or nucleotides is called a "modified" gRNA or "chemically modified" gRNA, to describe the presence of one or more non-naturally and/or naturally occurring components or configurations that are used instead of or in addition to the canonical A, G, C, and U residues. In some embodiments, a modified gRNA is synthesized with a non-canonical nucleoside or nucleotide, is here called "modified." Modified nucleosides and nucleotides can include one or more of: (i) alteration, *e.g.,* replacement, of one or both of the non-linking phosphate oxygens and/or of one or more of the linking phosphate oxygens in the phosphodiester backbone linkage (an exemplary backbone modification); (ii) alteration, *e.g.,* replacement, of a constituent of the ribose sugar, *e.g.,* of the 2' hydroxyl on the ribose sugar (an exemplary sugar modification); (iii) wholesale replacement of the phosphate moiety with "dephospho" linkers (an exemplary backbone modification); (iv) modification or replacement of a naturally occurring nucleobase, including with a non-canonical nucleobase (an exemplary base modification); (v) replacement or modification of the ribose-phosphate backbone (an exemplary backbone modification); (vi) modification of the 3' end or 5' end of the oligonucleotide, *e.g.,* removal, modification or replacement of a terminal phosphate group or conjugation of a moiety, cap or linker (such 3' or 5' cap modifications may comprise a sugar and/or backbone modification); and (vii) modification or replacement of the sugar (an exemplary sugar modification).

In some embodiments, a gRNA comprises a modified uridine at some or all uridine positions. In some embodiments, the modified uridine is a uridine modified at the 5 position, e.g., with a halogen or C1-C6 alkoxy. In some embodiments, the modified uridine is a pseudouridine modified at the 1 position, e.g., with a C1-C6 alkyl. The modified uridine can be, for example, pseudouridine, N1-methyl-pseudouridine, 5-methoxyuridine, 5-iodouridine, or a combination thereof. In some embodiments the modified uridine is 5-methoxyuridine. In some embodiments the modified uridine is 5-iodouridine. In some embodiments the modified uridine is pseudouridine. In some embodiments the modified uridine is N1-methyl-pseudouridine. In some embodiments, the modified uridine is a combination of pseudouridine and N1-methyl-pseudouridine. In some embodiments, the modified uridine is a combination of pseudouridine and 5-methoxyuridine. In some embodiments, the modified uridine is a combination of N1-methyl pseudouridine and 5-methoxyuridine. In some embodiments, the modified uridine is a combination of 5-iodouridine and N1-methyl-pseudouridine. In some embodiments, the modified uridine is a combination of pseudouridine and 5-iodouridine. In some embodiments, the modified uridine is a combination of 5-iodouridine and 5-methoxyuridine.

In some embodiments, at least 10%, 15%, 20%, 25%, 30%, 35%, 40%, 45%, 50%, 55%, 60%, 65%, 70%, 75%, 80%, 85%, 90%, 95%, 98%, 99%, or 100% of the uridine positions in a gRNA according to the disclosure are modified

uridines. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are modified uridines, e.g., 5-methoxyuridine, 5-iodouridine, N1-methyl pseudouridine, pseudouridine, or a combination thereof. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-methoxyuridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-iodouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-methoxyuridine, and the remainder are N1-methyl pseudouridine. In some embodiments, 10%-25%, 15-25%, 25-35%, 35-45%, 45-55%, 55-65%, 65-75%, 75-85%, 85-95%, or 90-100% of the uridine positions in a gRNA according to the disclosure are 5-iodouridine, and the remainder are N1-methyl pseudouridine.

Chemical modifications such as those listed above can be combined to provide modified gRNAs comprising nucleosides and nucleotides (collectively "residues") that can have two, three, four, or more modifications. For example, a modified residue can have a modified sugar and a modified nucleobase. In some embodiments, every base of a gRNA is modified, *e.g.,* all bases have a modified phosphate group, such as a phosphorothioate group. In certain embodiments, all, or substantially all, of the phosphate groups of an gRNA molecule are replaced with phosphorothioate groups. In some embodiments, modified gRNAs comprise at least one modified residue at or near the 5' end of the RNA. In some embodiments, modified gRNAs comprise at least one modified residue at or near the 3' end of the RNA.

In some embodiments, the gRNA comprises one, two, three or more modified residues. In some embodiments, at least 5% (*e.g.,* at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or 100%) of the positions in a modified gRNA are modified nucleosides or nucleotides.

Unmodified nucleic acids can be prone to degradation by, *e.g.,* intracellular nucleases or those found in serum. For example, nucleases can hydrolyze nucleic acid phosphodiester bonds. Accordingly, in one aspect the gRNAs described herein can contain one or more modified nucleosides or nucleotides, *e.g.,* to introduce stability toward intracellular or serum-based nucleases. In some embodiments, the modified gRNA molecules described herein can exhibit a reduced innate immune response when introduced into a population of cells, both *in vivo* and *ex vivo.* The term "innate immune response" includes a cellular response to exogenous nucleic acids, including single stranded nucleic acids, which involves the induction of cytokine expression and release, particularly the interferons, and cell death.

In some embodiments of a backbone modification, the phosphate group of a modified residue can be modified by replacing one or more of the oxygens with a different substituent. Further, the modified residue, *e.g.,* modified residue present in a modified nucleic acid, can include the wholesale replacement of an unmodified phosphate moiety with a modified phosphate group as described herein. In some embodiments, the backbone modification of the phosphate backbone can include alterations that result in either an uncharged linker or a charged linker with unsymmetrical charge distribution.

Examples of modified phosphate groups include, phosphorothioate, phosphoroselenates, borano phosphates, borano phosphate esters, hydrogen phosphonates, phosphoroamidates, alkyl or aryl phosphonates and phosphotriesters. The phosphorous atom in an unmodified phosphate group is achiral. However, replacement of one of the non-bridging oxygens with one of the above atoms or groups of atoms can render the phosphorous atom chiral. The stereogenic phosphorous atom can possess either the "R" configuration (herein Rp) or the "S" configuration (herein Sp). The backbone can also be modified by replacement of a bridging oxygen, (*i.e.,* the oxygen that links the phosphate to the nucleoside), with nitrogen (bridged phosphoroamidates), sulfur (bridged phosphorothioates) and carbon (bridged methylenephosphonates). The replacement can occur at either linking oxygen or at both of the linking oxygens.

The phosphate group can be replaced by non-phosphorus containing connectors in certain backbone modifications. In some embodiments, the charged phosphate group can be replaced by a neutral moiety. Examples of moieties which can replace the phosphate group can include, without limitation, e.g., methyl phosphonate, hydroxylamino, siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo and methyleneoxymethylimino.

In some embodiments, the invention comprises a sgRNA comprising one or more modifications within one or more of the following regions: the nucleotides at the 5' terminus; the lower stem region; the bulge region; the upper stem region; the nexus region; the hairpin 1 region; the hairpin 2 region; and the nucleotides at the 3' terminus. In some embodiments, the modification comprises a 2'-O-methyl (2'-O-Me) modified nucleotide. In some embodiments, the modification comprises a 2'-fluoro (2'-F) modified nucleotide. In some embodiments, the modification comprises a phosphorothioate (PS) bond between nucleotides.

In some embodiments, the first three or four nucleotides at the 5' terminus, and the last three or four nucleotides at the 3' terminus are modified. In some embodiments, the first four nucleotides at the 5' terminus, and the last four nucleotides at the 3' terminus are linked with phosphorothioate (PS) bonds. In some embodiments, the modification comprises 2'-O-Me. In some embodiments, the modification comprises 2'-F.

In some embodiments, the first four nucleotides at the 5' terminus and the last four nucleotides at the 3' terminus are linked with a PS bond, and the first three nucleotides at the 5' terminus and the last three nucleotides at the 3' terminus comprise 2'-O-Me modifications.

In some embodiments, the first four nucleotides at the 5' terminus and the last four nucleotides at the 3' terminus are linked with a PS bond, and the first three nucleotides at the 5' terminus and the last three nucleotides at the 3' terminus comprise 2'-F modifications.

In some embodiments, the sgRNA comprises the modification pattern of SEQ ID NO: 74: (mN*mN*mN*NNNNNNNNNNNNNNNNNGUUUUAGAmMGmCmUmAmGmAmA mAmUmAmGmCAA-GUUAAAAUAAGGCUAGUCCGUUAUCAmAmCmUmUmG mAmAmAmAmAmGmUmGmGmCmAmCmCmGmAmG-mUmCmGmGmUmGmCm U*mU*mU*mU), where N is any natural or non-natural nucleotide. In some embodiments, the sgRNA comprises SEQ ID NO:74. In certain embodiments, the sgRNA comprises 2'O-methyl modification of the first three residues at its 5' end, with phosphorothioate linkages between residues 1-2, 2-3, and 3-4 of the RNA.

**Template Nucleic Acid**

The compositions and methods disclosed herein may include a template nucleic acid. The template may be used to alter or insert a nucleic acid sequence at or near a target site for a Cas nuclease. In some embodiments, the methods comprise introducing a template to the cell. In some embodiments, a single template may be provided. In other embodiments, two or more templates may be provided such that editing may occur at two or more target sites. For example, different templates may be provided to edit a single gene in a cell, or two different genes in a cell.

In some embodiments, the template may be used in homologous recombination. In some embodiments, the homologous recombination may result in the integration of the template sequence or a portion of the template sequence into the target nucleic acid molecule. In other embodiments, the template may be used in homology-directed repair, which involves DNA strand invasion at the site of the cleavage in the nucleic acid. In some embodiments, the homology-directed repair may result in including the template sequence in the edited target nucleic acid molecule. In yet other embodiments, the template may be used in gene editing mediated by non-homologous end joining. In some embodiments, the template sequence has no similarity to the nucleic acid sequence near the cleavage site. In some embodiments, the template or a portion of the template sequence is incorporated. In some embodiments, the template includes flanking inverted terminal repeat (ITR) sequences.

In some embodiments, the template may comprise a first homology arm and a second homology arm (also called a first and second nucleotide sequence) that are complementary to sequences located upstream and downstream of the cleavage site, respectively. Where a template contains two homology arms, each arm can be the same length or different lengths, and the sequence between the homology arms can be substantially similar or identical to the target sequence between the homology arms, or it can be entirely unrelated. In some embodiments, the degree of complementarity or percent identity between the first nucleotide sequence on the template and the sequence upstream of the cleavage site, and between the second nucleotide sequence on the template and the sequence downstream of the cleavage site, may permit homologous recombination, such as, *e.g.,* high-fidelity homologous recombination, between the template and the target nucleic acid molecule. In some embodiments, the degree of complementarity may be about 50%, 55%, 60%, 65%, 70%, 75%, 80%, 85%, 90%, 95%, 97%, 98%, 99%, or 100%. In some embodiments, the degree of complementarity may be about 95%, 97%, 98%, 99%, or 100%. In some embodiments, the degree of complementarity may be at least 98%, 99%, or 100%. In some embodiments, the degree of complementarity may be 100%. In some embodiments, the percent identity may be about 50%, 55%, 60%, 65%, 70%, 75%, 80%, 85%, 90%, 95%, 97%, 98%, 99%, or 100%. In some embodiments, the percent identity may be about 95%, 97%, 98%, 99%, or 100%. In some embodiments, the percent identity may be at least 98%, 99%, or 100%. In some embodiments, the percent identity may be 100%.

In some embodiments, the template sequence may correspond to, comprise, or consist of an endogenous sequence of a target cell. It may also or alternatively correspond to, comprise, or consist of an exogenous sequence of a target cell. As used herein, the term "endogenous sequence" refers to a sequence that is native to the cell. The term "exogenous sequence" refers to a sequence that is not native to a cell, or a sequence whose native location in the genome of the cell is in a different location. In some embodiments, the endogenous sequence may be a genomic sequence of the cell. In some embodiments, the endogenous sequence may be a chromosomal or extrachromosomal sequence. In some embodiments, the endogenous sequence may be a plasmid sequence of the cell. In some embodiments, the template sequence may be substantially identical to a portion of the endogenous sequence in a cell at or near the cleavage site, but comprise at least one nucleotide change. In some embodiments, editing the cleaved target nucleic acid molecule with the template may result in a mutation comprising an insertion, deletion, or substitution of one or more nucleotides of the target

nucleic acid molecule. In some embodiments, the mutation may result in one or more amino acid changes in a protein expressed from a gene comprising the target sequence. In some embodiments, the mutation may result in one or more nucleotide changes in an RNA expressed from the target gene. In some embodiments, the mutation may alter the expression level of the target gene. In some embodiments, the mutation may result in increased or decreased expression of the target gene. In some embodiments, the mutation may result in gene knock-down. In some embodiments, the mutation may result in gene knock-out. In some embodiments, the mutation may result in restored gene function. In some embodiments, editing of the cleaved target nucleic acid molecule with the template may result in a change in an exon sequence, an intron sequence, a regulatory sequence, a transcriptional control sequence, a translational control sequence, a splicing site, or a non-coding sequence of the target nucleic acid molecule, such as DNA.

In other embodiments, the template sequence may comprise an exogenous sequence. In some embodiments, the exogenous sequence may comprise a protein or RNA coding sequence operably linked to an exogenous promoter sequence such that, upon integration of the exogenous sequence into the target nucleic acid molecule, the cell is capable of expressing the protein or RNA encoded by the integrated sequence. In other embodiments, upon integration of the exogenous sequence into the target nucleic acid molecule, the expression of the integrated sequence may be regulated by an endogenous promoter sequence. In some embodiments, the exogenous sequence may provide a cDNA sequence encoding a protein or a portion of the protein. In yet other embodiments, the exogenous sequence may comprise or consist of an exon sequence, an intron sequence, a regulatory sequence, a transcriptional control sequence, a translational control sequence, a splicing site, or a non-coding sequence. In some embodiments, the integration of the exogenous sequence may result in restored gene function. In some embodiments, the integration of the exogenous sequence may result in a gene knock-in. In some embodiments, the integration of the exogenous sequence may result in a gene knock-out.

The template may be of any suitable length. In some embodiments, the template may comprise 10, 15, 20, 25, 50, 75, 100, 150, 200, 500, 1000, 1500, 2000, 2500, 3000, 3500, 4000, 4500, 5000, 5500, 6000, or more nucleotides in length. The template may be a single-stranded nucleic acid. The template can be double-stranded or partially double-stranded nucleic acid. In certain embodiments, the single stranded template is 20, 30, 40, 50, 75, 100, 125, 150, 175, or 200 nucleotides in length. In some embodiments, the template may comprise a nucleotide sequence that is complementary to a portion of the target nucleic acid molecule comprising the target sequence (*i.e.,* a "homology arm"). In some embodiments, the template may comprise a homology arm that is complementary to the sequence located upstream or downstream of the cleavage site on the target nucleic acid molecule.

In some embodiments, the template contains ssDNA or dsDNA containing flanking invert-terminal repeat (ITR) sequences. In some embodiments, the template is provided as a vector, plasmid, minicircle, nanocircle, or PCR product.

**Purification of Nucleic Acids**

In some embodiments, the nucleic acid is purified. In some embodiments, the nucleic acid is purified using a precipation method (*e.g.,* LiCl precipitation, alcohol precipitation, or an equivalent method, *e.g.,* as described herein). In some embodiments, the nucleic acid is purified using a chromatography-based method, such as an HPLC-based method or an equivalent method (*e.g.,* as described herein). In some embodiments, the nucleic is purified using both a precipitation method (*e.g.,* LiCl precipitation) and an HPLC-based method.

Target Sequences

In some embodiments, a CRISPR/Cas system of the present disclosure may be directed to and cleave a target sequence on a target nucleic acid molecule. For example, the target sequence may be recognized and cleaved by the Cas nuclease. In certain embodiments, a target sequence for a Cas nuclease is located near the nuclease's cognate PAM sequence. In some embodiments, a Class 2 Cas nuclease may be directed by a gRNA to a target sequence of a target nucleic acid molecule, where the gRNA hybridizes with and the Class 2 Cas protein cleaves the target sequence. In some embodiments, the guide RNA hybridizes with and a Class 2 Cas nuclease cleaves the target sequence adjacent to or comprising its cognate PAM. In some embodiments, the target sequence may be complementary to the targeting sequence of the guide RNA. In some embodiments, the degree of complementarity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. In some embodiments, the percent identity between a targeting sequence of a guide RNA and the portion of the corresponding target sequence that hybridizes to the guide RNA may be about 50%, 55%, 60%, 65%, 70%, 75%, 80%, 85%, 90%, 95%, 97%, 98%, 99%, or 100%. In some embodiments, the homology region of the target is adjacent to a cognate PAM sequence. In some embodiments, the target sequence may comprise a sequence 100% complementary with the targeting sequence of the guide RNA. In other embodiments, the target sequence may comprise at least one mismatch, deletion, or insertion, as compared to the targeting sequence of the guide RNA.

The length of the target sequence may depend on the nuclease system used. For example, the targeting sequence of a guide RNA for a CRISPR/Cas system may comprise 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more than 50 nucleotides in length and the target sequence is a corresponding length, optionally adjacent to a PAM sequence. In some embodiments, the target sequence may comprise 15-24 nucleotides in length. In some embodiments, the target sequence may comprise 17-21 nucleotides in length. In some embodiments, the target sequence may comprise 20 nucleotides in length. When nickases are used, the target sequence may comprise a pair of target sequences recognized by a pair of nickases that cleave opposite strands of the DNA molecule. In some embodiments, the target sequence may comprise a pair of target sequences recognized by a pair of nickases that cleave the same strands of the DNA molecule. In some embodiments, the target sequence may comprise a part of target sequences recognized by one or more Cas nucleases.

The target nucleic acid molecule may be any DNA or RNA molecule that is endogenous or exogenous to a cell. In some embodiments, the target nucleic acid molecule may be an episomal DNA, a plasmid, a genomic DNA, viral genome, mitochondrial DNA, or chromosomal DNA from a cell or in the cell. In some embodiments, the target sequence of the target nucleic acid molecule may be a genomic sequence from a cell or in a cell, including a human cell.

In further embodiments, the target sequence may be a viral sequence. In further embodiments, the target sequence may be a pathogen sequence. In yet other embodiments, the target sequence may be a synthesized sequence. In further embodiments, the target sequence may be a chromosomal sequence. In certain embodiments, the target sequence may comprise a translocation junction, *e.g.,* a translocation associated with a cancer. In some embodiments, the target sequence may be on a eukaryotic chromosome, such as a human chromosome. In certain embodiments, the target sequence is a liver-specific sequence, in that it is expressed in liver cells.

In some embodiments, the target sequence may be located in a coding sequence of a gene, an intron sequence of a gene, a regulatory sequence, a transcriptional control sequence of a gene, a translational control sequence of a gene, a splicing site or a non-coding sequence between genes. In some embodiments, the gene may be a protein coding gene. In other embodiments, the gene may be a non-coding RNA gene. In some embodiments, the target sequence may comprise all or a portion of a disease-associated gene. In some embodiments, the target sequence may be located in a non-genic functional site in the genome, for example a site that controls aspects of chromatin organization, such as a scaffold site or locus control region.

In embodiments involving a Cas nuclease, such as a Class 2 Cas nuclease, the target sequence may be adjacent to a protospacer adjacent motif ("PAM"). In some embodiments, the PAM may be adjacent to or within 1, 2, 3, or 4, nucleotides of the 3' end of the target sequence. The length and the sequence of the PAM may depend on the Cas protein used. For example, the PAM may be selected from a consensus or a particular PAM sequence for a specific Cas9 protein or Cas9 ortholog, including those disclosed in Figure 1 of Ran et al., Nature, 520: 186-191 (2015), and Figure S5 of Zetsche 2015, the relevant disclosure of each of which is incorporated herein by reference. In some embodiments, the PAM may be 2, 3, 4, 5, 6, 7, 8, 9, or 10 nucleotides in length. Non-limiting exemplary PAM sequences include NGG, NGGNG, NG, NAAAAN, NNAAAAW, NNNNACA, GNNNCNNA, TTN, and NNNNGATT (wherein N is defined as any nucleotide, and W is defined as either A or T). In some embodiments, the PAM sequence may be NGG. In some embodiments, the PAM sequence may be NGGNG. In some embodiments, the PAM sequence may be TTN. In some embodiments, the PAM sequence may be NNAAAAW.

## Lipid Formulation

Disclosed herein are various embodiments of LNP formulations for biologically active agents, such as RNAs, including CRISPR/Cas cargoes. Such LNP formulations include an "amine lipid" or a "biodegradable lipid", along with a helper lipid, a neutral lipid, and a stealth lipid, wherein the stealth lipid is a PEG lipid. By "lipid nanoparticle" is meant a particle that comprises a plurality of (*i.e.* more than one) lipid molecules physically associated with each other by intermolecular forces.

### Amine Lipids

LNP compositions for the delivery of biologically active agents comprise an "amine lipid", which is defined as Lipid A or its equivalents, including acetal analogs of Lipid A. In the methods of the present disclosure, the amine lipid is Lipid A or a C4-C12 acetal analog of Lipid A.

In some embodiments, the amine lipid is Lipid A, which is (9Z,12Z)-3-((4,4-bis(octyloxy)butanoyl) oxy)-2-((((3-(diethylamino)propoxy)carbonyl)oxy)methyl)propyl octadeca-9,12-dienoate, also called 3-((4,4-bis(octyloxy)butanoyl)oxy)-2-((((3-(diethylamino)propoxy)carbonyl)oxy)methyl)propyl (9Z,12Z)-octadeca-9,12-dienoate. Lipid A is depicted as:

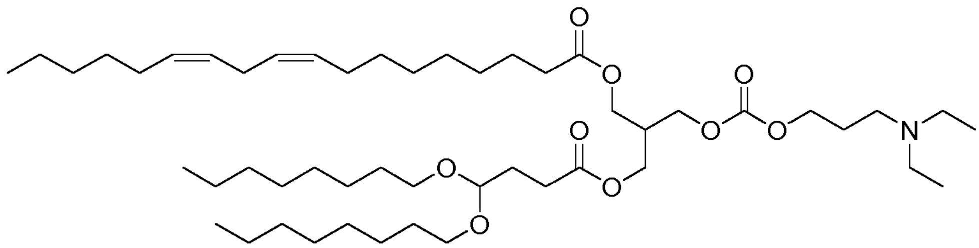

.

Lipid A may be synthesized according to WO2015/095340 (*e.g.,* pp. 84-86).

In certain embodimeints, the amine lipid is a C4-C12 acetal analog of Lipid A. In some embodiments, the acetal analog is a C5-C12 acetal analog. In additional embodiments, the acetal analog is a C5-C10 acetal analog. In further embodiments, the acetal analog is chosen from a C4, C5, C6, C7, C9, C10, C11, and C12 acetal analog.

Amine lipids and other "biodegradable lipids" suitable for use in the LNPs described herein are biodegradable *in vivo.* The amine lipids have low toxicity (*e.g.,* are tolerated in animal models without adverse effect in amounts of greater than or equal to 10 mg/kg). In certain embodiments, LNPs comprising an amine lipid include those where at least 75% of the amine lipid is cleared from the plasma within 8, 10, 12, 24, or 48 hours, or 3, 4, 5, 6, 7, or 10 days. In certain embodiments, LNPs comprising an amine lipid include those where at least 50% of the mRNA or gRNA is cleared from the plasma within 8, 10, 12, 24, or 48 hours, or 3, 4, 5, 6, 7, or 10 days. In certain embodiments, LNPs comprising an amine lipid include those where at least 50% of the LNP is cleared from the plasma within 8, 10, 12, 24, or 48 hours, or 3, 4, 5, 6, 7, or 10 days, for example by measuring a lipid (e.g. an amine lipid), RNA (e.g. mRNA), or other component. In certain embodiments, lipid-encapsulated versus free lipid, RNA, or nucleic acid component of the LNP is measured.

Biodegradable lipids include, for example the biodegradable lipids of WO/2017/173054, WO2015/095340, and WO2014/136086.

Lipid clearance may be measured as described in literature. *See* Maier, M.A., et al. Biodegradable Lipids Enabling Rapidly Eliminated Lipid Nanoparticles for Systemic Delivery of RNAi Therapeutics. Mol. Ther. 2013, 21(8), 1570-78 ("*Maier*"). For example, in *Maier,* LNP-siRNA systems containing luciferases-targeting siRNA were administered to six- to eight-week old male C57Bl/6 mice at 0.3 mg/kg by intravenous bolus injection *via* the lateral tail vein. Blood, liver, and spleen samples were collected at 0.083, 0.25, 0.5, 1, 2, 4, 8, 24, 48, 96, and 168 hours post-dose. Mice were perfused with saline before tissue collection and blood samples were processed to obtain plasma. All samples were processed and analyzed by LC-MS. Further, *Maier* describes a procedure for assessing toxicity after administration of LNP-siRNA formulations. For example, a luciferase-targeting siRNA was administered at 0, 1, 3, 5, and 10 mg/kg (5 animals/group) via single intravenous bolus injection at a dose volume of 5 mL/kg to male Sprague-Dawley rats. After 24 hours, about 1 mL of blood was obtained from the jugular vein of conscious animals and the serum was isolated. At 72 hours post-dose, all animals were euthanized for necropsy. Assessments of clinical signs, body weight, serum chemistry, organ weights and histopathology were performed. Although *Maier* describes methods for assessing siRNA-LNP formulations, these methods may be applied to assess clearance, pharmacokinetics, and toxicity of administration of LNP compositions of the present disclosure.

The lipids can lead to an increased clearance rate. In some embodiments, the clearance rate is a lipid clearance rate, for example the rate at which alipid is cleared from the blood, serum, or plasma. In some embodiments, the clearance rate is an RNA clearance rate, for example the rate at which an mRNA or a gRNA is cleared from the blood, serum, or plasma. In some embodiments, the clearance rate is the rate at which LNP is cleared from the blood, serum, or plasma. In some embodiments, the clearance rate is the rate at which LNP is cleared from a tissue, such as liver tissue or spleen tissue. In certain embodiments, a high rate of clearance rate leads to a safety profile with no substantial adverse effects. The amine lipids and biodegradable lipids may reduce LNP accumulation in circulation and in tissues. In some embodiments, a reduction in LNP accumulation in circulation and in tissues leads to a safety profile with no substantial adverse effects.

Lipids may be ionizable depending upon the pH of the medium they are in. For example, in a slightly acidic medium, the lipid, such as an amine lipid, may be protonated and thus bear a positive charge. Conversely, in a slightly basic medium, such as, for example, blood where pH is approximately 7.35, the lipid, such as an amine lipid, may not be protonated and thus bear no charge.

The ability of a lipid to bear a charge is related to its intrinsic pKa. In some embodiments, the amine lipids of the present disclosure may each, independently, have a pKa in the range of from about 5.1 to about 7.4. In some embodiments, the bioavailable lipids of the present disclosure may each, independently, have a pKa in the range of from about 5.1 to about 7.4. For example, the amine lipids of the present disclosure may each, independently, have a pKa in the range of from about 5.8 to about 6.5. Lipids with a pKa ranging from about 5.1 to about 7.4 are effective for delivery of cargo in vivo, e.g. to the liver. Further, it has been found that lipids with a pKa ranging from about 5.3 to about 6.4 are effective for delivery in vivo, e.g. to tumors. *See, e.g.,* WO2014/136086.

## Additional Lipids

"Neutral lipids" suitable for use in a lipid composition of the disclosure include, for example, a variety of neutral, uncharged or zwitterionic lipids. Examples of neutral phospholipids suitable for use in the present disclosure include, but are not limited to, 5-heptadecylbenzene-1,3-diol (resorcinol), dipalmitoylphosphatidylcholine (DPPC), distearoylphosphatidylcholine (DSPC), pohsphocholine (DOPC), dimyristoylphosphatidylcholine (DMPC), phosphatidylcholine (PLPC), 1,2-distearoyl-sn-glycero-3-phosphocholine (DAPC), phosphatidylethanolamine (PE), egg phosphatidylcholine (EPC), dilauryloylphosphatidylcholine (DLPC), dimyristoylphosphatidylcholine (DMPC), 1-myristoyl-2-palmitoyl phosphatidylcholine (MPPC), 1-palmitoyl-2-myristoyl phosphatidylcholine (PMPC), 1-palmitoyl-2-stearoyl phosphatidylcholine (PSPC), 1,2-diarachidoyl-sn-glycero-3-phosphocholine (DBPC), 1-stearoyl-2-palmitoyl phosphatidylcholine (SPPC), 1,2-dieicosenoyl-sn-glycero-3-phosphocholine (DEPC), palmitoyloleoyl phosphatidylcholine (POPC), lysophosphatidyl choline, dioleoyl phosphatidylethanolamine (DOPE), dilinoleoylphosphatidylcholine distearoylphosphatidylethanolamine (DSPE), dimyristoyl phosphatidylethanolamine (DMPE), dipalmitoyl phosphatidylethanolamine (DPPE), palmitoyloleoyl phosphatidylethanolamine (POPE), lysophosphatidylethanolamine and combinations thereof. In one embodiment, the neutral phospholipid may be selected from the group consisting of distearoylphosphatidylcholine (DSPC) and dimyristoyl phosphatidyl ethanolamine (DMPE). In another embodiment, the neutral phospholipid may be distearoylphosphatidylcholine (DSPC).

"Helper lipids" include steroids, sterols, and alkyl resorcinols. Helper lipids suitable for use in the present disclosure include, but are not limited to, cholesterol, 5-heptadecylresorcinol, and cholesterol hemisuccinate. In one embodiment, the helper lipid may be cholesterol. In one embodiment, the helper lipid may be cholesterol hemisuccinate.

"Stealth lipids" are lipids that alter the length of time the nanoparticles can exist *in vivo* (*e.g.,* in the blood). Stealth lipids may assist in the formulation process by, for example, reducing particle aggregation and controlling particle size. Stealth lipids used herein may modulate pharmacokinetic properties of the LNP. Stealth lipids suitable for use in a lipid composition of the disclosure includestealth lipids having a hydrophilic head group linked to a lipid moiety. Stealth lipids suitable for use in a lipid composition of the present disclosure and information about the biochemistry of such lipids can be found in Romberg et al., Pharmaceutical Research, Vol. 25, No. 1, 2008, pg. 55-71 and Hoekstra et al., Biochimica et Biophysica Acta 1660 (2004) 41-52. Additional suitable PEG lipids are disclosed, *e.g.,* in WO 2006/007712.

In the lipid compositions of the disclosure, the hydrophilic head group of stealth lipid comprises a polymer moiety selected from polymers based on PEG. Stealth lipids suitable for the lipid compositions of the disclosure comprise a lipid moiety. In the lipid compositions of the disclosure, the stealth lipid is a PEG lipid.

The stealth lipid comprises a polymer moiety based on PEG (sometimes referred to as poly(ethylene oxide)).

The PEG lipid comprises a polymer moiety based on PEG (sometimes referred to as poly(ethylene oxide)).

The PEG lipid further comprises a lipid moiety. In some embodiments, the lipid moiety may be derived from diacylglycerol or diacylglycamide, including those comprising a dialkylglycerol or dialkylglycamide group having alkyl chain length independently comprising from about C4 to about C40 saturated or unsaturated carbon atoms, wherein the chain may comprise one or more functional groups such as, for example, an amide or ester. In some embodiments, the alkyl chail length comprises about C10 to C20. The dialkylglycerol or dialkylglycamide group can further comprise one or more substituted alkyl groups. The chain lengths may be symmetrical or assymetric.

Unless otherwise indicated, the term "PEG" as used herein means any polyethylene glycol or other polyalkylene ether polymer. In one embodiment, PEG is an optionally substituted linear or branched polymer of ethylene glycol or ethylene oxide. In one embodiment, PEG is unsubstituted. In one embodiment, the PEG is substituted, e.g., by one or more alkyl, alkoxy, acyl, hydroxy, or aryl groups. In one embodiment, the term includes PEG copolymers such as PEG-polyurethane or PEG-polypropylene (see, *e.g.,* J. Milton Harris, Poly(ethylene glycol) chemistry: biotechnical and biomedical applications (1992)); in another embodiment, the term does not include PEG copolymers. In one embodiment, the PEG has a molecular weight of from about 130 to about 50,000, in a sub-embodiment, about 150 to about 30,000, in a sub-embodiment, about 150 to about 20,000, in a sub-embodiment about 150 to about 15,000, in a sub-embodiment, about 150 to about 10,000, in a sub-embodiment, about 150 to about 6,000, in a sub-embodiment, about 150 to about 5,000, in a sub-embodiment, about 150 to about 4,000, in a sub-embodiment, about 150 to about 3,000, in a sub-embodiment, about 300 to about 3,000, in a sub-embodiment, about 1,000 to about 3,000, and in a sub-embodiment, about 1,500 to about 2,500.

In certain embodiments, the PEG (e.g., conjugated to a lipid moiety or lipid, such as a stealth lipid), is a "PEG-2K," also termed "PEG 2000," which has an average molecular weight of about 2,000 daltons. PEG-2K is represented herein by the following formula (I), wherein n is 45, meaning that the number averaged degree of polymerization comprises about 45 subunits

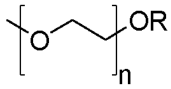

(I)

.

However, other PEG embodiments known in the art may be used, including, e.g., those where the number-averaged degree of polymerization comprises about 23 subunits (n=23), and/or 68 subunits (n=68). In some embodiments, n may range from about 30 to about 60. In some embodiments, n may range from about 35 to about 55. In some embodiments, n may range from about 40 to about 50. In some embodiments, n may range from about 42 to about 48. In some embodiments, n may be 45. In some embodiments, R may be selected from H, substituted alkyl, and unsubstituted alkyl. In some embodiments, R may be unsubstituted alkyl. In some embodiments, R may be methyl.

In any of the embodiments described herein, the PEG lipid may be selected from PEG-dilauroylglycerol, PEG-dimyristoylglycerol (PEG-DMG) (catalog # GM-020 from NOF, Tokyo, Japan), PEG-dipalmitoylglycerol, PEG-distearoylglycerol (PEG-DSPE) (catalog # DSPE-020CN, NOF, Tokyo, Japan), PEG-dilaurylglycamide, PEG-dimyristylglycamide, PEG-dipalmitoylglycamide, and PEG-distearoylglycamide, PEG-cholesterol (1-[8'-(Cholest-5-en-3[beta]-oxy)carboxamido-3',6'-dioxaoctanyl]carbamoyl-[omega]-methyl-poly(ethylene glycol), PEG-DMB (3,4-ditetradecoxylbenzyl-[omega]-methyl-poly(ethylene glycol)ether), 1,2-dimyristoyl-sn-glycero-3-phosphoethanolamine-N-[methoxy(polyethylene glycol)-2000] (PEG2k-DMG) (cat. #880150P from Avanti Polar Lipids, Alabaster, Alabama, USA), 1,2-distearoyl-sn-glycero-3-phosphoethanolamine-N-[methoxy(polyethylene glycol)-2000] (PEG2k-DSPE) (cat. #880120C from Avanti Polar Lipids, Alabaster, Alabama, USA), 1,2-distearoyl-sn-glycerol, methoxypolyethylene glycol (PEG2k-DSG; GS-020, NOF Tokyo, Japan), poly(ethylene glycol)-2000-dimethacrylate (PEG2k-DMA), and 1,2-distearyloxypropyl-3-amine-N-[methoxy(polyethylene glycol)-2000] (PEG2k-DSA). In one embodiment, the PEG lipid may be PEG2k-DMG. In some embodiments, the PEG lipid may be PEG2k-DSG. In one embodiment, the PEG lipid may be PEG2k-DSPE. In one embodiment, the PEG lipid may be PEG2k-DMA. In one embodiment, the PEG lipid may be PEG2k-C-DMA. In one embodiment, the PEG lipid may be compound S027, disclosed in WO2016/010840 (paragraphs [00240] to [00244]). In one embodiment, the PEG lipid may be PEG2k-DSA. In one embodiment, the PEG lipid may be PEG2k-C11. In some embodiments, the PEG lipid may be PEG2k-C14. In some embodiments, the PEG lipid may be PEG2k-C16. In some embodiments, the PEG lipid may be PEG2k-C18.

## LNP Formulations

The LNP contains (i) an amine lipid, (ii) a neutral lipid, (iii) a helper lipid, and (iv) a stealth lipid, wherein the stealth lipid is a PEG lipid.

The LNP may contain (i) an amine lipid for encapsulation and for endosomal escape, (ii) a neutral lipid for stabilization, (iii) a helper lipid, also for stabilization, and (iv) a stealth lipid, wherein the stealth lipid is a PEG lipid. The LNP may contain an amine lipid and a neutral lipid, a helper lipid, also for stabilization, and a stealth lipid, wherein the stealth lipid is a PEG lipid.

The LNP composition comprises an RNA component, wherein the RNA component is or comprises an mRNA. In some embodiments, the LNP composition may comprise an RNA component that includes one or more of an RNA-guided DNA-binding agent, a Cas nuclease mRNA, a Class 2 Cas nuclease mRNA, a Cas9 mRNA, and a gRNA. In some embodiments, an LNP composition may include a Class 2 Cas nuclease and a gRNA as the RNA component. The LNP composition comprises the RNA component, wherein the RNA component is or comprises an mRNA, an amine lipid, a helper lipid, a neutral lipid, and a stealth lipid, wherein the stealth lipid is a PEG lipid. In certain LNP compositions, the helper lipid is cholesterol. In other compositions, the neutral lipid is DSPC. In additional embodiments, the stealth lipid is PEG2k-DMG or PEG2k-C11. In certain embodiments, the LNP composition comprises Lipid A or a C4-C12 acetal analog of Lipid A; a helper lipid; a neutral lipid; a stealth lipid, wherein the stealth lipid is a PEG lipid; and a guide RNA. In certain compositions, the amine lipid is Lipid A. In certain compositions, the amine lipid is Lipid A or a C4-C12 acetal analog thereof; the helper lipid is cholesterol; the neutral lipid is DSPC; and the stealth lipid is PEG2k-DMG.

In certain embodiments, lipid compositions are described according to the respective molar ratios of the component lipids in the formulation. Embodiments of the present disclosure provide lipid compositions described according to the respective molar ratios of the component lipids in the formulation. In one embodiment, the mol-% of the amine lipid may be from about 30 mol-% to about 60 mol-%. In one embodiment, the mol-% of the amine lipid may be from about 40 mol-% to about 60 mol-%. In one embodiment, the mol-% of the amine lipid may be from about 45 mol-% to about 60 mol-%. In one embodiment, the mol-% of the amine lipid may be from about 50 mol-% to about 60 mol-%. In one embodiment, the mol-% of the amine lipid may be from about 55 mol-% to about 60 mol-%. In one embodiment, the mol-% of the amine lipid may be from about 50 mol-% to about 55 mol-%. In one embodiment, the mol-% of the amine lipid may be about 50 mol-%. In one embodiment, the mol-% of the amine lipid may be about 55 mol-%. In some embodiments, the amine lipid mol-% of the LNP batch will be ±30%, ±25%, ±20%, ±15%, ±10%, ±5%, or ±2.5% of the target mol-%. In some embodiments, the amine lipid mol-% of the LNP batch will be ±4 mol-%, ±3 mol-%, ±2 mol-%, ±1.5 mol-%, ±1 mol-%, ±0.5 mol-%, or ±0.25 mol-% of the target mol-%. All mol-% numbers are given as a fraction of the lipid component of the LNP compositions. In certain embodiments, LNP inter-lot variability of the amine lipid mol-% will be less than 15%, less than 10% or less than 5%.

In one embodiment, the mol-% of the neutral lipid may be from about 5 mol-% to about 15 mol-%. In one embodiment, the mol-% of the neutral lipid may be from about 7 mol-% to about 12 mol-%. In one embodiment, the mol-% of the neutral lipid may be about 9 mol-%. In some embodiments, the neutral lipid mol-% of the LNP batch will be ±30%, ±25%, ±20%, ±15%, ±10%, ±5%, or ±2.5% of the target neutral lipid mol-%. In certain embodiments, LNP inter-lot variability will be less than 15%, less than 10% or less than 5%.

In one embodiment, the mol-% of the helper lipid may be from about 20 mol-% to about 60 mol-%. In one embodiment, the mol-% of the helper lipid may be from about 25 mol-% to about 55 mol-%. In one embodiment, the mol-% of the helper lipid may be from about 25 mol-% to about 50 mol-%. In one embodiment, the mol-% of the helper lipid may be from about 25 mol-% to about 40 mol-%. In one embodiment, the mol-% of the helper lipid may be from about 30 mol-% to about 50 mol-%. In one embodiment, the mol-% of the helper lipid may be from about 30 mol-% to about 40 mol-%. In one embodiment, the mol-% of the helper lipid is adjusted based on amine lipid, neutral lipid, and PEG lipid concentrations to bring the lipid component to 100 mol-%. In some embodiments, the helper mol-% of the LNP batch will be ±30%, ±25%, ±20%, ±15%, ±10%, ±5%, or ±2.5% of the target mol-%. In certain embodiments, LNP inter-lot variability will be less than 15%, less than 10% or less than 5%.

In one embodiment, the mol-% of the PEG lipid may be from about 1 mol-% to about 10 mol-%. In one embodiment, the mol-% of the PEG lipid may be from about 2 mol-% to about 10 mol-%. In one embodiment, the mol-% of the PEG lipid may be from about 2 mol-% to about 8 mol-%. In one embodiment, the mol-% of the PEG lipid may be from about 2 mol-% to about 4 mol-%. In one embodiment, the mol-% of the PEG lipid may be from about 2.5 mol-% to about 4 mol-%. In one embodiment, the mol-% of the PEG lipid may be about 3 mol-%. In one embodiment, the mol-% of the PEG lipid may be about 2.5 mol-%. In some embodiments, the PEG lipid mol-% of the LNP batch will be ±30%, ±25%, ±20%, ±15%, ±10%, ±5%, or ±2.5% of the target PEG lipid mol-%. In certain embodiments, LNP inter-lot variability will be less than 15%, less than 10% or less than 5%.

In certain embodiments, the cargo includes an mRNA encoding an RNA-guided DNA-binding agent (e.g. a Cas nuclease, a Class 2 Cas nuclease, or Cas9), and a gRNA or a nucleic acid encoding a gRNA, or a combination of mRNA and gRNA. The LNP composition comprises Lipid A or a C4-C12 acetal analog of Lipid A. In some aspects, the amine lipid is Lipid A. In some aspects, the amine lipid is a C4-C12 acetal analog of Lipid A. The LNP composition comprises an amine lipid, a neutral lipid, a helper lipid, and a PEG lipid. In certain embodiments, the helper lipid is cholesterol. In certain embodiments, the neutral lipid is DSPC. In specific embodiments, PEG lipid is PEG2k-DMG. In some embodiments, an LNP composition may comprise a Lipid A, a helper lipid, a neutral lipid, and a PEG lipid. In some embodiments, an LNP composition comprises an amine lipid, DSPC, cholesterol, and a PEG lipid. In some embodiments, the LNP composition comprises a PEG lipid comprising DMG. The amine lipid is selected from Lipid A, and a C4-C12 acetal analog of Lipid A. In additional embodiments, an LNP composition comprises Lipid A, cholesterol, DSPC, and PEG2k-DMG.

Embodiments of the present disclosure also provide lipid compositions described according to the molar ratio between the positively charged amine groups of the amine lipid (N) and the negatively charged phosphate groups (P) of the nucleic acid to be encapsulated. This may be mathematically represented by the equation N/P. In some embodiments, an LNP composition may comprise a lipid component that comprises an amine lipid, a helper lipid, a neutral lipid, and a helper lipid; and a nucleic acid component, wherein the N/P ratio is about 3 to 10. In some embodiments, an LNP composition may comprise a lipid component that comprises an amine lipid, a helper lipid, a neutral lipid, and a PEG lipid; and an RNA component, wherein the N/P ratio is about 3 to 10. In one embodiment, the N/P ratio may about 5-7. In one embodiment, the N/P ratio may about 4.5-8. In one embodiment, the N/P ratio may about 6. In one embodiment, the N/P ratio may be 6 ±1. In one embodiment, the N/P ratio may about 6 ± 0.5. In some embodiments, the N/P ratio will be ±30%, ±25%, ±20%, ±15%, ±10%, ±5%, or ±2.5% of the target N/P ratio. In certain embodiments, LNP inter-lot variability will be less than 15%, less than 10% or less than 5%.

The RNA component comprises an mRNA, such as an mRNA encoding a Cas nuclease. In one embodiment, RNA component may comprise a Cas9 mRNA. In some compositions comprising an mRNA encoding a Cas nuclease, the LNP further comprises a gRNA nucleic acid, such as a gRNA. In some embodiments, the RNA component comprises a Cas nuclease mRNA and a gRNA. In some embodiments, the RNA component comprises a Class 2 Cas nucleast mRNA and a gRNA.

In certain embodiments, an LNP composition may comprise an mRNA encoding a Cas nuclease such as a Class 2 Cas nuclease, an amine lipid, a helper lipid, a neutral lipid, and a PEG lipid. In certain LNP compositions comprising an mRNA encoding a Cas nuclease such as a Class 2 Cas nuclease, the helper lipid is cholesterol. In other compositions comprising an mRNA encoding a Cas nuclease such as a Class 2 Cas nuclease, the neutral lipid is DSPC. In additional embodiments comprising an mRNA encoding a Cas nuclease such as a Class 2 Cas nuclease, the PEG lipid is PEG2k-DMG or PEG2k-C11. In specific compositions comprising an mRNA encoding a Cas nuclease such as a Class 2 Cas nuclease, the amine lipid is selected from Lipid A and a C4-C12 acetal analog of Lipid A.

In some embodiments, an LNP composition may comprise a gRNA. In certain embodiments, an LNP composition may comprise an amine lipid, a gRNA, a helper lipid, a neutral lipid, and a PEG lipid. In certain LNP compositions comprising a gRNA, the helper lipid is cholesterol. In some compositions comprising a gRNA, the neutral lipid is DSPC. In additional embodiments comprising a gRNA, the PEG lipid is PEG2k-DMG or PEG2k-C11. The amine lipid is selected from Lipid A and a C4-C12 acetal analog of Lipid A.

In one embodiment, an LNP composition may comprise an sgRNA. In one embodiment, an LNP composition may comprise a Cas9 sgRNA. In one embodiment, an LNP composition may comprise a Cpf1 sgRNA. In some compositions comprising an sgRNA, the LNP includes an amine lipid, a helper lipid, a neutral lipid, and a PEG lipid. In certain compositions comprising an sgRNA, the helper lipid is cholesterol. In other compositions comprising an sgRNA, the neutral lipid is DSPC. In additional embodiments comprising an sgRNA, the PEG lipid is PEG2k-DMG or PEG2k-C11. The amine lipid is selected from Lipid A and C4-C12 acetal analogs of Lipid A.

In certain embodiments, an LNP composition comprises an mRNA encoding a Cas nuclease and a gRNA, which may be an sgRNA. In one embodiment, an LNP composition may comprise an amine lipid, an mRNA encoding a Cas nuclease, a gRNA, a helper lipid, a neutral lipid, and a PEG lipid. In certain compositions comprising an mRNA encoding a Cas nuclease and a gRNA, the helper lipid is cholesterol. In some compositions comprising an mRNA encoding a Cas nuclease and a gRNA, the neutral lipid is DSPC. In additional embodiments comprising an mRNA encoding a Cas nuclease and a gRNA, the PEG lipid is PEG2k-DMG or PEG2k-C11. The amine lipid is selected from Lipid A and C4-C12 acetal analogs of Lipid A.

In certain embodiments, the LNP compositions include a Cas nuclease mRNA, such as a Class 2 Cas mRNA and at least one gRNA. In certain embodiments, the LNP composition includes a ratio of gRNA to Cas nuclease mRNA, such as Class 2 Cas nuclease mRNA from about 25:1 to about 1:25. In certain embodiments, the LNP formulation includes a ratio of gRNA to Cas nuclease mRNA, such as Class 2 Cas nuclease mRNA from about 10:1 to about 1:10. In certain embodiments, the LNP formulation includes a ratio of gRNA to Cas nuclease mRNA, such as Class 2 Cas nuclease mRNA from about 8:1 to about 1:8. As measured herein, the ratios are by weight. In some embodiments, the LNP formulation includes a ratio of gRNA to Cas nuclease mRNA, such as Class 2 Cas mRNA from about 5:1 to about 1:5. In some embodiments, ratio range is about 3:1 to 1:3, about 2:1 to 1:2, about 5:1 to 1:2, about 5:1 to 1:1, about 3:1 to 1:2, about 3:1 to 1:1, about 3:1, about 2:1 to 1:1. In some embodiments, the gRNA to mRNA ratio is about 3:1 or about 2:1 In some embodiments the ratio of gRNA to Cas nuclease mRNA, such as Class 2 Cas nuclease is about 1:1. The ratio may be about 25:1, 10:1, 5:1, 3:1, 1:1, 1:3, 1:5, 1:10, or 1:25.

The LNP compositions disclosed herein may include a template nucleic acid. The template nucleic acid may be co-formulated with an mRNA encoding a Cas nuclease, such as a Class 2 Cas nuclease mRNA. In some embodiments, the template nucleic acid may be co-formulated with a guide RNA. In some embodiments, the template nucleic acid may be co-formulated with both an mRNA encoding a Cas nuclease and a guide RNA. In some embodiments, the template nucleic acid may be formulated separately from an mRNA encoding a Cas nuclease or a guide RNA. The template nucleic acid may be delivered with, or separately from the LNP compositions. In some embodiments, the template nucleic acid may be single- or double-stranded, depending on the desired repair mechanism. The template may have regions of homology to the target DNA, or to sequences adjacent to the target DNA.

In some embodiments, LNPs are formed by mixing an aqueous RNA solution with an organic solvent-based lipid solution, e.g., 100% ethanol. Suitable solutions or solvents include or may contain: water, PBS, Tris buffer, NaCl, citrate buffer, ethanol, chloroform, diethylether, cyclohexane, tetrahydrofuran, methanol, isopropanol. A pharmaceutically acceptable buffer, e.g., for *in vivo* administration of LNPs, may be used. In certain embodiments, a buffer is used to maintain the pH of the composition comprising LNPs at or above pH 6.5. In certain embodiments, a buffer is used to maintain the pH of the composition comprising LNPs at or above pH 7.0. In certain embodiments, the composition has a pH ranging from about 7.2 to about 7.7. In additional embodiments, the composition has a pH ranging from about 7.3 to about 7.7 or ranging from about 7.4 to about 7.6. In further embodiments, the composition has a pH of about 7.2, 7.3, 7.4, 7.5, 7.6, or 7.7. The pH of a composition may be measured with a micro pH probe. In certain embodiments, a cryoprotectant is included in the composition. Non-limiting examples of cryoprotectants include sucrose, trehalose, glycerol, DMSO, and ethylene glycol. Exemplary compositions may include up to 10% cryoprotectant, such as, for example, sucrose. In certain embodiments, the LNP composition may include about 1, 2, 3, 4, 5, 6, 7, 8, 9, or 10% cryoprotectant. In certain embodiments, the LNP composition may include about 1, 2, 3, 4, 5, 6, 7, 8, 9, or 10% sucrose. In some embodiments, the LNP composition may include a buffer. In some embodiments, the buffer may comprise a phosphate buffer (PBS), a Tris buffer, a citrate buffer, and mixtures thereof. In certain exemplary embodiments, the buffer comprises NaCl. In certain emboidments, NaCl is omitted. Exemplary amounts of NaCl may range from about 20 mM to about 45 mM. Exemplary amounts of NaCl may range from about 40 mM to about 50 mM. In some embodiments, the amount of NaCl is about 45 mM. In some embodiments, the buffer is a Tris buffer. Exemplary amounts of Tris may range from about 20 mM to about 60 mM. Exemplary amounts of Tris may range from about 40 mM to about 60 mM. In some embodiments, the amount of Tris is about 50 mM. In some embodiments, the buffer comprises NaCl and Tris. Certain exemplary embodiments of the LNP compositions contain 5% sucrose and 45 mM NaCl in Tris buffer. In other exemplary embodiments, compositions contain

sucrose in an amount of about 5% w/v, about 45 mM NaCl, and about 50 mM Tris at pH 7.5. The salt, buffer, and cryoprotectant amounts may be varied such that the osmolality of the overall formulation is maintained. For example, the final osmolality may be maintained at less than 450 mOsm/L. In further embodiments, the osmolality is between 350 and 250 mOsm/L. Certain embodiments have a final osmolality of 300 +/- 20 mOsm/L.

In some embodiments, microfluidic mixing, T-mixing, or cross-mixing is used. In certain aspects, flow rates, junction size, junction geometry, junction shape, tube diameter, solutions, and/or RNA and lipid concentrations may be varied. LNPs or LNP compositions may be concentrated or purified, *e.g.,* via dialysis, tangential flow filtration, or chromatography. The LNPs may be stored as a suspension, an emulsion, or a lyophilized powder, for example. In some embodiments, an LNP composition is stored at 2-8° C, in certain aspects, the LNP compositions are stored at room temperature. In additional embodiments, an LNP composition is stored frozen, for example at -20° C or -80° C. In other embodiments, an LNP composition is stored at a temperature ranging from about 0° C to about -80° C. Frozen LNP compositions may be thawed before use, for example on ice, at 4° C, at room temperature, or at 25° C. Frozen LNP compositions may be maintained at various temperatures, for example on ice, at 4° C, at room temperature, at 25° C, or at 37° C.

**Methods of Engineering Stem Cells, e.g., HSPCs; Engineered Stem Cells, e.g., HSPCs**

The LNP compositions disclosed herein may be used in methods for engineering stem cells, e.g., HSPCs, e.g. by CRISPR/Cas system gene editing *in vitro.* In some embodiments, the genetically engineered cell population is a CD34+ cell population. In some embodiments, a method of producing a genetically engineered HSPC or CD34+ cell population *in vitro* is provided, the method comprising (a) preincubating a serum factor with an LNP composition for delivering a Cas nuclease mRNA and a gRNA; (b) contacting the HSPC or CD34+ cell population with the preincubated LNP composition *in vitro;* and (c) culturing the HSPC or CD34+ cell population *in vitro,* thereby producing a genetically engineered HSPC. In some embodiments, the methods involve contacting an HSPC or CD34+ cell with an LNP composition described herein according to the delivery methods described herein.

In some embodiments, engineered stem cells, e.g., HSPCs, are provided, for example, an engineered HSPC or HSPC population. Such engineered cells are produced according to the methods described herein. In some embodiments, the engineered HSPC resides within a tissue or organ, e.g., bone marrow, blood, or other tissue within a subject, e.g. after transplantation of an engineered HSPC.

In some of the methods and cells described herein, a cell comprises a modification, for example an insertion or deletion ("indel") or substitution of nucleotides in a target sequence. In some embodiments, the modification comprises an insertion of 1, 2, 3, 4 or 5 or more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification comprises an indel which results in a frameshift mutation in a target sequence. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

In some embodiments, a population of cells comprising engineered cells is provided, for example a population of cells comprising cells engineered according to the methods described herein. In some embodiments, the population comprises engineered cells cultured *in vitro.* In some embodiments, the population resides within a tissue or organ, e.g., a liver within a subject. In some embodiments, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% or more of the cells within the population is engineered. In certain embodiments, a method disclosed herein results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% editing efficiency (or "percent editing"), defined by detetion of indels. In other embodiments, a method disclosed herein, results in at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90% or at least 95% DNA modification efficiency, defined by detecting a change in sequence, whether by insertion, deletion, substitution or otherwise. In certain embodiments, a method disclosed herein results in an editing efficiency level or a DNA modification efficiency level of between about 5% to about 100%, about 10% to about 50%, about 20 to about 100%, about 20 to about 80%, about 40 to about 100%, or about 40 to about 80% in a cell population.

In some of the methods and cells described herein, cells within the population comprise a modification, e.g., an indel or substitution at a target sequence. In some embodiments, the modification comprises an insertion of 1, 2, 3, 4 or 5 or

more nucleotides in a target sequence. In some embodiments, the modification comprises an insertion of either 1 or 2 nucleotides in a target sequence. In other embodiments, the modification comprises a deletion of 1, 2, 3, 4, 5, 6, 7, 8, 9, 10, 15, 20 or 25 or more nucleotides in a target sequence. In some embodiments, the modification comprises a deletion of either 1 or 2 nucleotides in a target sequence. In some embodiments, the modification results in a frameshift mutation in a target sequence. In some embodiments, the modification comprises an indel which results in a frameshift mutation in a target sequence. In some embodiments, at least 80%, at least 85%, at least 90%, at least 91%, at least 92%, at least 93%, at least 94%, at least 95%, at least 96%, at least 97%, at least 98%, or at least 99% or more of the engineered cells in the population comprise a frameshift mutation. In some embodiments, the modification comprises a substitution of 1, 2, 3, 4, 5, 6, 7, 8, 9, 10, 15, 20 or 25 or more nucleotides in a target sequence. In some embodiments, the modification comprises a substitution of either 1 or 2 nucleotides in a target sequence. In some embodiments, the modification comprises one or more of an insertion, deletion, or substitution of nucleotides resulting from the incorporation of a template nucleic acid, for example any of the template nucleic acids described herein.

**Methods of Gene Editing**

The methods disclosed herein may be used for gene editing in a stem cell, an HSPC, or HSPC population *in vitro.* In one embodiment, one or more LNP compositions described herein may be administered to a stem cell, an HSPC, or an HSPC population. In one embodiment, one or more LNP compositions described herein may contact a stem cell, an HSPC, and HSC, or an HPC. In one embodiment, a genetically engineered cell may be produced by contacting a cell with an LNP composition according to the methods described herein. In some methods of gene editing, the HSPC or HSPC population is maintained in culture. In some methods of gene editing, the HSPC or HSPC population is transplanted into a patient. In some embodiments, the genetically engineered HSPC resides within a tissue or organ, e.g., bone marrow, blood, or other tissue within a patient, e.g. after transplantation of an engineered HSPC.

In some embodiments, the method comprises a stem cell, an HSPC, or HSPC population that s autologous with respect to a patient to be administered the cell. In some embodiments, the method comprises an HSPC or HSPC population that is allogeneic with respprecedingect to a patient to be administered said cell.

In various embodiments, the methods described herein achieve CRISPR-Cas gene editing in the stem cell, HSPC, or HSPC population. In some embodiments, the methods further comprise detecting gene editing in the HSPC or HSPC population. In some embodiments, the gene editing is measured as percent editing. In some embodiments, the gene editings is measured as percent DNA modification. The methods may achieve at least 40, 50, 60, 70, 80, 90, or 95% editing. The methods may achieve at least 40, 50, 60, 70, 80, 90, or 95% DNA modification.

In one embodiment, an LNP composition comprising an mRNA encoding a Class 2 Cas nuclease and a gRNA may be administered to a stem cell, an HSPC, or an HSPC population. In additional embodiments, a template nucleic acid is also introduced to the cell. In certain instances, an LNP composition comprising a Class 2 Cas nuclease and an sgRNA may be administered to a cell.

In one embodiment, the LNP compositions may be used to edit a gene in a stem cell, an HSPC, or HSPC population resulting in a gene knockout. In an embodiment, the LNP compositions may be used to edit a gene in an HSPC or HSPC population resulting in gene knockdown, e.g. in the population of cells. The knockdown or knockout may be detected by measuring target protein levels. The knockdown or knockout may be detected by detecting the target DNA. In another embodiment, the LNP compositions may be used to edit a gene in an HSPC or HSPC population resulting in a gene correction. In a further embodiment, the LNP compositions may be used to edit a cell resulting in gene insertion.

The LNP compositions may be administered as a formulation in association with one or more pharmaceutically acceptable excipients. The term "excipient" includes any ingredient other than the compound(s) of the disclosure, the other lipid component(s) and the biologically active agent. An excipient may impart either a functional (*e.g.* drug release rate controlling) and/or a non-functional (*e.g.* processing aid or diluent) characteristic to the formulations. The choice of excipient will to a large extent depend on factors such as the particular mode of administration, the effect of the excipient on the stem cell or HSPC culture, and on solubility and stability, and the nature of the dosage form.

Where the formulation is aqueous, excipients such as sugars (including but not restricted to glucose, mannitol, sorbitol, *etc.)* salts, carbohydrates and buffering agents (preferably to a pH of from 3 to 9), but, for some applications, they may be more suitably formulated with a sterile non-aqueous solution or as a dried form to be used in conjunction with a suitable vehicle such as sterile, pyrogen-free water (WFI).

While the invention is described in conjunction with the illustrated embodiments, it is understood that they are not intended to limit the invention to those embodiments. On the contrary, the invention is intended to cover all alternatives, modifications, and equivalents, including equivalents of specific features, which may be included within the invention as defined by the appended claims.

Both the foregoing general description and detailed description, as well as the following examples, are exemplary and explanatory only and are not restrictive of the teachings. The section headings used herein are for organizational purposes only and are not to be construed as limiting the desired subject matter in any way. In the event that any literature

incorporated by reference contradicts any term defined in this specification, this specification controls. All ranges given in the application encompass the endpoints unless stated otherwise.

It should be noted that, as used in this application, the singular form "a", "an" and "the" include plural references unless the context clearly dictates otherwise. Thus, for example, reference to "a composition" includes a plurality of compositions and reference to "a cell" includes a plurality of cells and the like. The use of "or" is inclusive and means "and/or" unless stated otherwise.

Numeric ranges are inclusive of the numbers defining the range. Measured and measureable values are understood to be approximate, taking into account significant digits and the error associated with the measurement. The term "about" or "approximately" means an acceptable error for a particular value as determined by one of ordinary skill in the art, which depends in part on how the value is measured or determined. The use of a modifier such as "about" before a range or before a list of values, modifies each endpoint of the range or each value in the list. For example, "about 50-55" encompasses "about 50 to about 55". Also, the use of "comprise", "comprises", "comprising", "contain", "contains", "containing", "include", "includes", and "including" is not limiting.

Unless specifically noted in the above specification, embodiments in the specification that recite "comprising" various components are also contemplated as "consisting of" or "consisting essentially of" the recited components; embodiments in the specification that recite "consisting of" various components are also contemplated as "comprising" or "consisting essentially of" the recited components; embodiments in the specification that recite "about" various components are also contemplated as "at" the recited components; and embodiments in the specification that recite "consisting essentially of" various components are also contemplated as "consisting of" or "comprising" the recited components (this interchangeability does not apply to the use of these terms in the claims).

EXAMPLES

**Example 1 - Methods**

***Cell Culture***

Cryopreserved human CD34+ bone marrow cells were obtained from AllCells (cat. no. ABM017F) or StemCell Technologies (cat. no. 70008). After thawing and washing twice in 20ml StemSpan SFEM (Stem Cell technologies, cat. no. 09650), cells were cultured for 48 hours in StemSpan SFEM (StemCell Technologies, cat. no. 09650) containing thrombopoietin (TPO, 50ng/ml, StemCell Technologies, cat. no. 02922), human Flt3 ligand (Flt3l, 50ng/ml, StemCell Technologies, cat. no. 78137.2), human interleukin-6 (Il-6, 50ng/ml, StemCell Technologies, cat. no. 78148.2), human stem cell factor (SCF, 50ng/ml, StemCell technologies, cat. no. 78155.2), and StemRegenin-1 (SR1, 0.75uM), as well as Penicillin/Streptomycin (P/S, 100U/ml Penicillin and 100ug/ml Streptomycin, Life Technologies, cat. no. 15140122).

***Lipid Nanoparticle ("LNP") Formulation***

The LNPs were formulated by dissolving lipid nanoparticle components in 100% ethanol with the following molar ratios: 45 mol-% (12.7 mM) lipid amine (Lipid A); 44 mol-% (12.4 mM) helper lipid (e.g., cholesterol); 9 mol-% (2.53 mM) neutral lipid (*e.g.,* DSPC); and 2 mol-% (.563 mM) PEG lipid (*e.g.,* PEG2k-DMG or PEG2k-C11), except as otherwise specified below. The N/P ratio (mol of lipid amine to mol of RNA) was 4.5. The ID numbers for LNP formulations are as follows: LNP522, LNP525 (GFP mRNA) and LNP670, LNP926 (B2M single guide, Cas9 mRNA) and LNP899 (AAVS1 single guide, Cas9 mRNA). The RNA cargos were dissolved in 50 mM acetate buffer, pH 4.5 or 25 mM sodium citrate, 100 mM NaCl, pH 5.0, resulting in a concentration of RNA cargo of approximately 0.45 mg/mL.

The LNPs were formed by microfluidic mixing of the lipid and RNA solutions using a Precision Nanosystems NanoAssemblrTM Benchtop Instrument, according to the manufacturer's protocol. A 2:1 ratio of aqueous to organic solvent was maintained during mixing using differential flow rates. After mixing, the LNPs were collected and diluted in either phosphate buffered saline, pH 7.4 (PBS) or 50 mM tris, pH 7.5 (Tris) (approximately 1:1) to reduce ethanol content prior to further processing. Final buffer exchange was completed by dialysis into PBS or Tris (100-fold excess of sample volume), overnight at 4°C under gentle stirring using a 10 kDa Slide-a-Lyzer™ G2 Dialysis Cassette (ThermoFisher Scientific). Tris processed formulations were diluted 1:1 into 100 mM tris, 90 mM saline, 5% (w/v) sucrose, pH 7.5 (2x TSS). Alternatively, LNPs were collected post-mixing, diluted in water, held at room temperature for 1 hour, and diluted a second time 1:1 with water. The final buffer exchange into TSS was completed with PD-10 desalting columns (GE). If required, formulations by either processing method were concentrated by centrifugation with Amicon 100 kDa centrifugal filters (Millipore). The resulting mixture was then filtered using a 0.2 μm sterile filter. The resulting filtrate was stored at 2-8 °C if final buffer was PBS or -80 °C if final buffer was TSS.

### *In Vitro Transcription ("IVT") of Nuclease mRNA and Single Guide RNA (sgRNA)*

Capped and polyadenylated Cas9 mRNA was generated by *in vitro* transcription using a linearized plasmid DNA template and T7 RNA polymerase. Plasmid DNA containing a T7 promoter and a 100 residue poly(A/T) region was linearized by incubating at 37 °C for 2 hours with XbaI with the following conditions: 200 ng/μL plasmid, 2 U/μL XbaI (NEB), and 1x reaction buffer. The XbaI was inactivated by heating the reaction at 65 °C for 20 min. The linearized plasmid was purified from enzyme and buffer salts using a silica maxi spin column (Epoch Life Sciences) and analyzed by agarose gel to confirm linearization. The IVT reaction to generate Cas9 modified mRNA was incubated at 37 °C for 4 hours in the following conditions: 50 ng/μL linearized plasmid; 2 mM each of GTP, ATP, CTP, and N1-methyl pseudo-UTP (Trilink); 10 mM ARCA (Trilink); 5 U/μL T7 RNA polymerase (NEB); 1 U/μL Murine RNase inhibitor (NEB); 0.004 U/μL Inorganic *E. coli* pyrophosphatase (NEB); and 1x reaction buffer. After the 4 hour incubation, TURBO DNase (ThermoFisher) was added to a final concentration of 0.01 U/μL, and the reaction was incubated for an additional 30 minutes to remove the DNA template. The Cas9 mRNA was purified using a LiCl precipitation method.

For all methods, the transcript concentration was determined by measuring the light absorbance at 260 nm (Nanodrop), and the transcript was analyzed by capillary electrophoresis by Bioanlayzer (Agilent). SgRNAs were chemically synthesized.

### *LNP Transfection of Human CD34+ Bone Marrow Cells*

LNPs containing either GFP mRNA or Cas9 mRNA and single guide targeting beta2-microgobulin (B2M) were added in various concentrations ranging from 50.0ng to 800.0ng to 30,000 human CD34+ bone marrow cells in a total volume of 100.0ul. The sequence of the sgRNA, which targets the GGCCACGGAGCGAGACATCT B2M target sequence (SEQ ID NO:75), is: mG*mG*mC*CACGGAGCGAGACAUCUGUUUUAGAmGmCmUmAmGmAmAm AmUmAmGm-CAAGUUAAAAUAAGGCUAGUCCGUUAUCAmAmCmUmUmGm AmAmAmAmAmGmUmGmGmCmAmCmCmG-mAmGmUmCmGmGmUmGmCmU* mU*mU*mU (SEQ ID NO:76). In this nucleic acid sequence, A, U, G, and C denote adenine, uracil, cytosine, and guanine, respectively; m" indicates 2'-O-Methyl nucleotides; and "*" indicates phosphorothioate bonds.

For species-specific serum studies (Triple S studies), LNPs were incubated in 6.0% serum from M. musculus (BioreclamationIVT, cat. no. MSESRM, lot no. MSE245821), M. fascicularis (BioreclamationIVT, cat. no. CYNSRM, lot no. CYN197451), and H. sapiens (Sigma, pooled, H4522-20ml, lot no. SLBR7629V; BioreclamationIVT, cat. no. HMSRM, lot no. BRH1278638; BioreclamationIVT, cat. no. HMSRM, lot no. BRH1227947) at 37°C for 5 minutes prior to cell transfection. Human recombinant apolipoprotein E3 (ApoE3, R&D Systems, cat. no. 4144-AE) was used in a range of concentrations (01.ug/ml, 1.0ug/ml, 10.0ug/ml, and 50.0ug/ml) in recommended buffer under the same incubation conditions as described above.

### *Flow Cytometry Read-Out of LNP Transfected Human CD34+ Bone Marrow cells*

Cells were collected for antibody staining 24 hours post LNP-GFP transfection or 5 days post LNP-B2M transfection. After washing cells in sample medium (PBS + 2% FBS + 2mM EDTA), cells were blocked with Human TruStain FcX (Biolegend, cat. no. 422302) at room temperature (RT) for 5min.

We stained cells with the following antibodies and labels as shown in Table 2.

Table 2. Antibodies and labels.

| Antibody | Fluorophore | Manufacturer | Catalog number |
|---|---|---|---|
| hCD34 | Brilliant Violet 421™ (BV421) | Biolegend | 343610 |
| hCD38 | R-phycoerythrin (PE) | Biolegend | 356604 |
| hCD90 | PE-Cy™7 (PE-cyanine dye tandem fluorophore) | Biolegend | 328124 |
| hCD45RA | Alexa Fluor® 700 (AF700) | Biolegend | 304120 |
| hB2M | Allophycocyanin (APC) | Biolegend | 316312 |
| 7-AAD | n/a | Biolegend | 420404 |

Cells were run on a Beckman Coulter CytoflexS and analyzed using the FlowJo software package.

Cell survival was assessed with 7-AAD staining. Normalization for living cells based on 7-AAD intercalation in GC-rich DNA regions and subsequent detection in flow cytometry assays. Cell survival was calculated using the following

formula:

[(Sample $1_{number}$ of cell events/$Sample1_{number}$ of bead $_{events}$)*$Sample1_{total}$ number of beads $_{added}$]/Mean{[($Control1-_{number\ of\ cell\ events}$/$Control1_{number\ of\ bead}$ events) * $Control1_{total}$ number of beads added], [($Control2_{number\ of\ cell\ events}$/ $Control1_{number\ of\ bead}$ events)* $Control2_{total\ number\ of\ beads\ added}$], ..., ControlN}

In this formula, "sample" is defined as any population of human CD34+ HSPCs that, during the course of the experiment, received treatment with either LNPs, mRNA, gRNA, or any combination of the former and "control" defined aa any population of human CD34+ HSPCs that, during the course of the experiment, did not receive any treatment with LNPs, mRNA, gRNA, or any combination of the former.

***Next-Generation Sequencing ("NGS") and Analysis for Cleavage Efficiency***

To quantitatively determine the efficiency of editing at the target location in the genome, deep sequencing was utilized to identify the presence of insertions and deletions introduced by gene editing.

Cells were collected on day 5 post tranfection and DNA extracted using the PureLink Genomic DNA Mini Kit (ThermoFisher Scientific, cat. no. K182002). Primers for B2M target locus containing the Illumina P5 and P7 adapter sequences were used to amplify genomic site of interest in a standard PCR reaction.

PCR primers were designed around the B2M target site and the genomic area of interest was amplified. Samples were submitted for sample preparation (Illumina MiSeq v2 Reagent Kit, 300 cycles, cat. no. 15033624) and sequencing run on an Illumina MiSeq instrument. Editing frequency at target locus of interest was analyzed using a bespoke pipeline. In brief, additional PCR was performed according to the manufacturer's protocols (Illumina) to add the necessary chemistry for sequencing. The amplicons were sequenced on an Illumina MiSeq instrument. The reads were aligned to the human reference genome (e.g., hg38) after eliminating those having low quality scores. The resulting files containing the reads were mapped to the reference genome (BAM files), where reads that overlapped the target region of interest were selected and the number of wild type reads versus the number of reads which contain an insertion, substitution, or deletion was calculated.

The editing percentage (e.g., the "editing efficiency" or "percent editing") is provided as the total number of sequence reads with insertions or deletions over the total number of sequence reads, including wild type.

Formulation Analytics

LNP formulations are analyzed for average particle size, polydispersity (pdi), total RNA content and encapsulation efficiency of RNA. Average particle size and polydispersity are measured by dynamic light scattering (DLS) using a Malvern Zetasizer DLS instrument. LNP samples are diluted 30X in PBS prior to being measured by DLS. Z-average diameter which is an intensity based measurement of average particle size was reported along with number average diameter and pdi.

A fluorescence-based assay (Ribogreen®, ThermoFisher Scientific) is used to determine total RNA concentration and free RNA. Encapsulation efficiency is calclulated as (Total RNA - Free RNA)/Total RNA. LNP samples are diluted appropriately with 1x TE buffer containing 0.2% Triton-X 100 to determine total RNA or 1x TE buffer to determine free RNA. Standard curves are prepared by utilizing the starting RNA solution used to make the formulations and diluted in 1x TE buffer +/-0.2% Triton-X 100.Diluted RiboGreen® dye (100X in 1xTE buffer, according to the manufacturer's instructions) is then added to each of the standards and samples and allowed to incubate for 10 minutes at room temperature, in the absence of light. A SpectraMax M5 Microplate Reader (Molecular Devices) is used to read the samples with excitation, auto cutoff and emission wavelengths set to 488 nm, 515 nm, and 525 nm respectively. Total RNA and free RNA are determined from the appropriate standard curves. Encapsulation efficiency is calclulated as (Total RNA - Free RNA)/Total RNA. The same procedure may be used for determining the encapsulation efficiency of a DNA-based cargo component. For single-strand DNA Oligreen Dye may be used, and for double-strand DNA, Picogreen Dye.

**Example 2** - **Delivery of GFP to CD34+ bone marrow cells**

LNPs were formulated as described in Example 1 with GFP mRNA in a final buffer of PBS and added to 30,000 human CD34+ bone marrow cells in a total volume of 100.0ul, providing 0, 50.0ng, 100.0ng, and 200.0ng of GFP mRNA in various reactions. Prior to administration to the cells, LNPs were pre-incubated with serum at 6% (v/v) from M. musculus (BioreclamationIVT, cat. no. MSESRM, lot no. MSE245821) at 37°C for 5 minutes. Cells were cultured as described in Example 1.

GFP+ cells were quantitated 24 hours after LNP addition to human CD34+ cells by flow cytometry. The population

of GFP+ cells was determined in FITC channel (excitation max 490, emission max 525, laser line 488) relative to a GFP- control (labelled "control" in Fig. 1). The percentage of GFP+ cells in all live human CD34+ bone marrow cells 24 hours post LNP-mediated GFP mRNA delivery is depicted in Fig. 1. The LNP compositions are as follows, Fig. 1(A): 45% Lipid A, 44% cholesterol, 9% DSPC, 2% PEG; Fig. 1(B): 45% Lipid A, 45% cholesterol, 9% DSPC, 1% PEG. Biological sample size n=3.

The LNP compositions demonstrate dose dependent delivery of mRNA to CD34+ bone marrow cells *in vitro.*

**Example 3 - Preincubation of LNPs Facilitates Delivery**

Tests demonstrate that LNPs require incubation with 6% mouse serum (v/v) prior to transfection in order to efficiently deliver GFP mRNA to human $CD34^+$ bone marrow cells. Cells were cultured and transfected with LNP compositions as described in Example 2 with the following modifications:

Fig. 2A shows the percentage of $GFP^+$ cells in all live cells of human $CD34^+$ bone marrow samples with LNP application on day 0 immediately post thaw of a cryopreserved cell vial. LNPs with 50.0 ng, 100.0 ng, or 200.0 ng GFP mRNA were added to the cells with and without serum incubation prior to transfection.

Fig. 2B shows the percentage of $GFP^+$ cells in all live cells of human $CD34^+$ bone marrow samples with LNP application on day 2 post thaw of a cryopreserved cell vial. LNPs with 50.0 ng, 100.0 ng, or 200.0 ng of GFP mRNA were added to cells with and without serum incubation prior to transfection. Biological sample size n=3.

**Example 4 - Delivery of Cas9 and Guide RNAs via LNPs; Gene Editing in CD34+ Bone Marrow Cells**

LNPs were formulated as described in Example 1 with sgRNA (G529) and Cas9 mRNA as described in Example 1 and at a 1:1 weight ratio in a final buffer of TSS. The LNP composition was 45% Lipid A, 44% cholesterol, 9% DSPC, 2% PEG with an N/P ratio of 4.5.

Using the LNP delivery methods to transfect human $CD34^+$ bone marrow cells, a Cas9 mRNA and B2M sgRNA was efficiently delivered to the cells using pre-incubation with increasing percentages (v/v) of either M. musculus or M. fascicularis serum. Active Cas9-sgRNA complexes are delivered by LNPs pre-incubated with various sera.

Fig. 3A depicts FACS analysis of transfected cells, showing the percentage of B2M negative cells after application of LNPs (at 400.0 ng Cas9 mRNA and sgRNA (1:1 by weight)) incubated with either mouse serum ("Mouse-S") at 6%, 30%, and 60% (v/v) or non-human primate serum ("Cyno-S") at 6%, 30%, and 60% (v/v). LNPs without serum pre-incubation ("LNP only") and cells without treatment ("Ctrl") serve do not show efficient delivery (measuring B2M expression knock-down). Pre-incubation with mouse or primate serum facilitates efficient knock-down of B2M expression in $CD34^+$ bone marrow cells.

Fig. 3B depicts editing frequency on the genomic level as determined by NGS for human $CD34^+$ bone marrow cells transfected with LNPs (at 400.0 ng Cas9 mRNA and sgRNA (1:1 by weight)) incubated with either mouse serum at 6%, 30%, and 60% (v/v) or non-human primate serum at 6%, 30%, and 60% (v/v). As in Fig. 3(A), LNP application without serum pre-incubation and cells without treatment do not show efficient delivery (measuring % editing). Insertions ("In", light grey) and deletions ("Del", black) are graphed on the Y axis, showing greater than about 60%, greater than about 70%, greater than about 80% and greater than about 90% editing efficiency in the $CD34^+$ cells. "LNP only" and "Cntrl" samples do not display detectable levels of indels at the B2M locus. Biological sample size n=3.

**Example 5 - Preincubation with Isolated Serum Factor ApoE3**

To investigate whether the serum pre-incubation step could be substituted by recombinant protein, LNPs, as described in Example 4, delivering Cas9 and the B2M sgRNA were pre-incubated with human recombinant Apolipoprotein E3 (ApoE3), mouse serum, or non-human primate serum during the LNP incubation step prior to cell transfection.

In Fig. 4A, the percentages of B2M negative cells after application of LNPs (at 400 ng Cas9 mRNA and sgRNA (1:1)) incubated with either mouse serum ("mouse-S") at 6% (v/v), non-human primate serum ("cyno-S") at 6% (v/v), or ApoE3 at 0.1 μg/ml, 1.0 μg/ml, 10.0 μg/ml, and 50.0 μg/ml are shown. Human $CD34^+$ bone marrow cells without treatment serve as negative control ("Ctrl"). ApoE3 shows a dose-dependent increase in delivery to the CD34+ cells, and it can be used in the pre-incubation step.

Similarly, gene editing shows a dose-dependent response to ApoE3. Fig. 4B depicts percentage of editing of the B2M target as determined by NGS for human $CD34^+$ bone marrow cells transfected with 400.0 ng LNPs incubated with either mouse serum at 6% (v/v), non-human primate serum at 6% (v/v), or ApoE3 at 0.1 μg/ml, 1.0 μg/ml, 10.0 μg/ml, and 50.0 μg/ml. Human $CD34^+$ bone marrow cells without treatment serve as the negative control, which does not display detectable levels of indels at the B2M locus. Biological sample size n=3.

**Example 6 - Preincubation with Serum Factors**

This experiment tested LNP pre-incubation with a variety of different apolipoproteins, showing *in-vitro* LNP uptake with ApoE isoforms, measured as level of B2M knockdown and editing frequency in an HSPC population. Prior to transfection, LNPs (ID LNP926) were incubated at 37C for 5 minutes with either 6% M. fascicularis serum (v/v) or the following apolipoproteins at various concentrations: recombinant human ApoA-I (Millipore Sigma, cat # SRP4693), ApoB from human plasma (Millipore Sigma, cat # A5353), ApoC-I from human plasma (Millipore Sigma, cat # A7785), human recombinant ApoE2 (Millipore Sigma, cat # SRP4760), human recombinant ApoE3 (Millipore Sigma, cat # SRP4696), human recombinant ApoE4 (Millipore Sigma, cat # A3234). LNPs were added to human CD34+ bone marrow cells at a concentration of 200ng total RNA cargo (1:1 w/w ratio of Cas9 mRNA and single guide). B2M expression on protein level was determined by flow cytometry using the same antibodies as described above on day 5 post transfection. Data analysis was performed using FlowJo software package. Data represent one biological sample (N=1), mean +/- SD of technical duplicates.

Fig. 5 shows B2M knockdown in a population of CD34+ HSPCs after transfection of cyno serum, ApoE2, ApoE3, and ApoE4 pre-incubated LNPs. No treatment, no pre-incubation, and pre-incubation with ApoA-I, ApoB, and ApoC-I did not result in B2M knockdown. In this experiment, LNP pre-incubation with ApoE2 showed less B2M knockdown compared to the other two ApoE isoforms.

**Example 7 - Time course of LNP exposure**

This experiment tested duration of LNP exposure for its impact on viability and editing rates. LNP899, delivering Cas9 mRNA and G562 targeting AAVS1, was preincubated at 37C for about 5 minutes with non-human primate serum at 6% (v/v). LNPs were added to human CD34+ bone marrow cells at a concentration of 300ng total RNA cargo (1:1 w/w ratio of Cas9 mRNA and single guide). At 2 hour, 6 hour or 24 hours post transfection, cells were centrifuged and resuspended in fresh media without LNP. Cell viability was assessed at 3 days and 8 days using CountBright™ Absolute Counting Beads (Invitrogen, Cat. C36950) measured on a CytoFLEXS flow cytometer (Beckman Coulter). Editing was measured by NGS as described in Example 1. Table 3 and Fig. 6B shows editing frequency at 8 days after transduction. Table 3 and Fig. 6A shows cell viability at 3 days and 8 days after transduction.

Table 3 - Viability and editing at varying LNP exposure

| LNP exposure | Viability 3d (Cells/ml) | SD Viability 3d | Viability 8d (Cells/ml) | SD Viability 8d | Indel Freq | SD Indels |
|---|---|---|---|---|---|---|
| Negative Control | 640,341 | 31,810 | 4,453,259 | 965,751 | 0.00 | 0.00 |
| 2h | 583,279 | 44,284 | 3,400,929 | 525,282 | 0.60 | 0.00 |
| 6h | 515,561 | 74,690 | 2,762,987 | 375,265 | 0.88 | 0.01 |
| 24h | 447,350 | 56,802 | 2,435,103 | 240,977 | 0.97 | 0.00 |

## BRIEF DESCRIPTION OF DISCLOSED SEQUENCES

| SEQ ID NO | Description |
|---|---|
| 1 | DNA coding sequence of Cas9 using the thymidine analog of the minimal uridine codons listed in Table 1, with start and stop codons |
| 2 | DNA coding sequence of Cas9 using codons with generally high expression in humans |
| 3 | Amino acid sequence of Cas9 with one nuclear localization signal (1xNLS) as the C-terminal 7 amino acids |
| 4 | Cas9 mRNA ORF using minimal uridine codons as listed in Table 1, with start and stop codons |
| 5 | Cas9 mRNA ORF using codons with generally high expression in humans, with start and stop codons |
| 10 | Cas9 mRNA coding sequence using minimal uridine codons as listed in Table 1 (no start or stop codons; suitable for inclusion in fusion protein coding sequence) |

(continued)

| SEQ ID NO | Description |
|---|---|
| 13 | Amino acid sequence of Cas9 (without NLS) |
| 14 | Cas9 mRNA ORF encoding SEQ ID NO: 13 using minimal uridine codons as listed in Table 1, with start and stop codons |
| 15 | Cas9 coding sequence encoding SEQ ID NO: 13 using minimal uridine codons as listed in Table 1 (no start or stop codons; suitable for inclusion in fusion protein coding sequence) |
| 16 | Amino acid sequence of Cas9 nickase (without NLS) |
| 17 | Cas9 nickase mRNA ORF encoding SEQ ID NO: 16 using minimal uridine codons as listed in Table 1, with start and stop codons |
| 18 | Cas9 nickase coding sequence encoding SEQ ID NO: 16 using minimal uridine codons as listed in Table 1 (no start or stop codons; suitable for inclusion in fusion protein coding sequence) |
| 19 | Amino acid sequence of dCas9 (without NLS) |
| 20 | dCas9 mRNA ORF encoding SEQ ID NO: 13 using minimal uridine codons as listed in Table 1, with start and stop codons |
| 21 | dCas9 coding sequence encoding SEQ ID NO: 13 using minimal uridine codons as listed in Table 1 (no start or stop codons; suitable for inclusion in fusion protein coding sequence) |
| 22 | Amino acid sequence of Cas9 with two nuclear localization signals (2xNLS) as the C-terminal amino acids |
| 23 | Cas9 mRNA ORF encoding SEQ ID NO: 13 using minimal uridine codons as listed in Table 1, with start and stop codons |
| 24 | Cas9 coding sequence encoding SEQ ID NO: 13 using minimal uridine codons as listed in Table 1 (no start or stop codons; suitable for inclusion in fusion protein coding sequence) |
| 25 | Amino acid sequence of Cas9 nickase with two nuclear localization signals as the C-terminal amino acids |
| 26 | Cas9 nickase mRNA ORF encoding SEQ ID NO: 16 using minimal uridine codons as listed in Table 1, with start and stop codons |
| 27 | Cas9 nickase coding sequence encoding SEQ ID NO: 16 using minimal uridine codons as listed in Table 1 (no start or stop codons; suitable for inclusion in fusion protein coding sequence) |
| 28 | Amino acid sequence of dCas9 with two nuclear localization signals as the C-terminal amino acids |
| 29 | dCas9 mRNA ORF encoding SEQ ID NO: 13 using minimal uridine codons as listed in Table 1, with start and stop codons |
| 30 | dCas9 coding sequence encoding SEQ ID NO: 13 using minimal uridine codons as listed in Table 1 (no start or stop codons; suitable for inclusion in fusion protein coding sequence) |
| 31 | T7 Promoter |
| 32 | Human beta-globin 5' UTR |
| 33 | Human beta-globin 3' UTR |
| 34 | Human alpha-globin 5' UTR |
| 35 | Human alpha-globin 3' UTR |
| 36 | *Xenopus laevis* beta-globin 5' UTR |
| 37 | *Xenopus laevis* beta-globin 3' UTR |
| 38 | Bovine Growth Hormone 5' UTR |
| 39 | Bovine Growth Hormone 3' UTR |
| 40 | Mus musculus hemoglobin alpha, adult chain 1 (Hba-a1), 3'UTR |
| 41 | HSD17B4 5' UTR |

(continued)

| SEQ ID NO | Description |
|---|---|
| 42 | G282 single guide RNA targeting the mouse TTR gene |
| 43 | Cas9 transcript with 5' UTR of HSD, ORF corresponding to SEQ ID NO: 4, Kozak sequence, and 3' UTR of ALB |
| 45 | Alternative Cas9 ORF with 19.36% U content |
| 46 | Cas9 transcript with 5' UTR of HSD, ORF corresponding to SEQ ID NO: 45, Kozak sequence, and 3' UTR of ALB |
| 47 | Cas9 transcript with 5' UTR of HSD, ORF corresponding to SEQ ID NO: 45, and 3' UTR of ALB |
| 48 | Cas9 transcript comprising Cas9 ORF using codons with generally high expression in humans |
| 49 | Cas9 transcript comprising Kozak sequence with Cas9 ORF using codons with generally high expression in humans |
| 50 | Cas9 ORF with splice junctions removed; 12.75% U content |
| 51 | Cas9 transcript with 5' UTR of HSD, ORF corresponding to SEQ ID NO: 50, Kozak sequence, and 3' UTR of ALB |
| 52 | Cas9 ORF with minimal uridine codons frequently used in humans in general; 12.75% U content |
| 53 | Cas9 transcript with 5' UTR of HSD, ORF corresponding to SEQ ID NO: 52, Kozak sequence, and 3' UTR of ALB |
| 54 | Cas9 ORF with minimal uridine codons infrequently used in humans in general; 12.75% U content |
| 55 | Cas9 transcript with 5' UTR of HSD, ORF corresponding to SEQ ID NO: 54, Kozak sequence, and 3' UTR of ALB |
| 63 | poly-A 100 sequence |
| 64 | G209 single guide RNA targeting the mouse TTR gene |
| 65 | ORF encoding Neisseria meningitidis Cas9 using minimal uridine codons as listed in Table 1, with start and stop codons |
| 66 | ORF encoding Neisseria meningitidis Cas9 using minimal uridine codons as listed in Table 1 (no start or stop codons; suitable for inclusion in fusion protein coding sequence) |
| 67 | Transcript comprising SEQ ID NO: 65 (encoding Neisseria meningitidis Cas9) |
| 68 | Amino acid sequence of Neisseria meningitidis Cas9 |
| 69 | G390 single guide RNA targeting the rat TTR gene |
| 70 | G502 single guide RNA targeting the cynomolgus monkey TTR gene |
| 71 | G509 single guide RNA targeting the cynomolgus monkey TTR gene |
| 72 | G534 single guide RNA targeting the rat TTR gene |

See the Sequence Table below for the sequences themselves. Transcript sequences generally include GGG as the first three nucleotides for use with ARCA or AGG as the first three nucleotides for use with CleanCap™. Accordingly, the first three nucleotides can be modified for use with other capping approaches, such as Vaccinia capping enzyme. Promoters and poly-A sequences are not included in the transcript sequences. A promoter such as a T7 promoter (SEQ ID NO: 31) and a poly-A sequence such as SEQ ID NO: 63 can be appended to the disclosed transcript sequences at the 5' and 3' ends, respectively. Most nucleotide sequences are provided as DNA but can be readily converted to RNA by changing Ts to Us.

**Sequence Table**

The following sequence table provides a listing of sequences disclosed herein. It is understood that if a DNA sequence (comprising Ts) is referenced with respect to an RNA, then Ts should be replaced with Us (which may be modified or unmodified depending on the context), and vice versa.

| Description | Sequence | SEQ ID No. |
|---|---|---|
| **Cas9 DNA coding sequence 2** | ATGGACAAGAAGTACAGCATCGGACTGGACATCGGAACAAACAGCGTCGGAT GGGCAGTCATCACAGACGAATACAAGGTCCCGAGCAAGAAGTTCAAGGTCCT GGGAAACACAGACAGACACAGCATCAAGAAGAACCTGATCGGAGCACTGCTG TTCGACAGCGGAGAAACAGCAGAAGCAACAAGACTGAAGAGAACAGCAAGAA GAAGATACACAAGAAGAAAGAACAGAATCTGCTACCTGCAGGAAATCTTCAG CAACGAAATGGCAAAGGTCGACGACAGCTTCTTCCACAGACTGGAAGAAAGC TTCCTGGTCGAAGAAGACAAGAAGCACGAAAGACACCCGATCTTCGGAAACA TCGTCGACGAAGTCGCATACCACGAAAAGTACCCGACAATCTACCACCTGAG AAAGAAGCTGGTCGACAGCACAGACAAGGCAGACCTGAGACTGATCTACCTG GCACTGGCACACATGATCAAGTTCAGAGGACACTTCCTGATCGAAGGAGACC TGAACCCGGACAACAGCGACGTCGACAAGCTGTTCATCCAGCTGGTCCAGAC ATACAACCAGCTGTTCGAAGAAAACCCGATCAACGCAAGCGGAGTCGACGCA AAGGCAATCCTGAGCGCAAGACTGAGCAAGAGCAGAAGACTGGAAAACCTGA TCGCACAGCTGCCGGGAGAAAAGAAGAACGGACTGTTCGGAAACCTGATCGC ACTGAGCCTGGGACTGACACCGAACTTCAAGAGCAACTTCGACCTGGCAGAA GACGCAAAGCTGCAGCTGAGCAAGGACACATACGACGACGACCTGGACAACC TGCTGGCACAGATCGGAGACCAGTACGCAGACCTGTTCCTGGCAGCAAAGAA | 1 |

(continued)

| Description | Sequence | SEQ ID No. |
|---|---|---|
| | CCTGAGCGACGCAATCCTGCTGAGCGACATCCTGAGAGTCAACACAGAAATC<br>ACAAAGGCACCGCTGAGCGCAAGCATGATCAAGAGATACGACGAACACCACC<br>AGGACCTGACACTGCTGAAGGCACTGGTCAGACAGCAGCTGCCGGAAAAGTA<br>CAAGGAAATCTTCTTCGACCAGAGCAAGAACGGATACGCAGGATACATCGAC<br>GGAGGAGCAAGCCAGGAAGAATTCTACAAGTTCATCAAGCCGATCCTGGAAA<br>AGATGGACGGAACAGAAGAACTGCTGGTCAAGCTGAACAGAGAAGACCTGCT<br>GAGAAAGCAGAGAACATTCGACAACGGAAGCATCCCGCACCAGATCCACCTG<br>GGAGAACTGCACGCAATCCTGAGAAGACAGGAAGACTTCTACCCGTTCCTGA<br>AGGACAACAGAGAAAAGATCGAAAAGATCCTGACATTCAGAATCCCGTACTA<br>CGTCGGACCGCTGGCAAGAGGAAACAGCAGATTCGCATGGATGACAAGAAAG<br>AGCGAAGAAACAATCACACCGTGGAACTTCGAAGAAGTCGTCGACAAGGGAG<br>CAAGCGCACAGAGCTTCATCGAAAGAATGACAAACTTCGACAAGAACCTGCC<br>GAACGAAAAGGTCCTGCCGAAGCACAGCCTGCTGTACGAATACTTCACAGTC<br>TACAACGAACTGACAAAGGTCAAGTACGTCACAGAAGGAATGAGAAAGCCGG<br>CATTCCTGAGCGGAGAACAGAAGAAGGCAATCGTCGACCTGCTGTTCAAGAC<br>AAACAGAAAGGTCACAGTCAAGCAGCTGAAGGAAGACTACTTCAAGAAGATC<br>GAATGCTTCGACAGCGTCGAAATCAGCGGAGTCGAAGACAGATTCAACGCAA<br>GCCTGGGAACATACCACGACCTGCTGAAGATCATCAAGGACAAGGACTTCCT<br>GGACAACGAAGAAAACGAAGACATCCTGGAAGACATCGTCCTGACACTGACA<br>CTGTTCGAAGACAGAGAAATGATCGAAGAAAGACTGAAGACATACGCACACC<br>TGTTCGACGACAAGGTCATGAAGCAGCTGAAGAGAAGAAGATACACAGGATG<br>GGGAAGACTGAGCAGAAAGCTGATCAACGGAATCAGAGACAAGCAGAGCGGA<br>AAGACAATCCTGGACTTCCTGAAGAGCGACGGATTCGCAAACAGAAACTTCA<br>TGCAGCTGATCCACGACGACAGCCTGACATTCAAGGAAGACATCCAGAAGGC<br>ACAGGTCAGCGGACAGGGAGACAGCCTGCACGAACACATCGCAAACCTGGCA<br>GGAAGCCCGGCAATCAAGAAGGGAATCCTGCAGACAGTCAAGGTCGTCGACG<br>AACTGGTCAAGGTCATGGGAAGACACAAGCCGGAAAACATCGTCATCGAAAT<br>GGCAAGAGAAAACCAGACAACACAGAAGGGACAGAAGAACAGCAGAGAAAGA<br>ATGAAGAGAATCGAAGAAGGAATCAAGGAACTGGGAAGCCAGATCCTGAAGG<br>AACACCCGGTCGAAAACACACAGCTGCAGAACGAAAAGCTGTACCTGTACTA<br>CCTGCAGAACGGAAGAGACATGTACGTCGACCAGGAACTGGACATCAACAGA<br>CTGAGCGACTACGACGTCGACCACATCGTCCCGCAGAGCTTCCTGAAGGACG<br>ACAGCATCGACAACAAGGTCCTGACAAGAAGCGACAAGAACAGAGGAAAGAG<br>CGACAACGTCCCGAGCGAAGAAGTCGTCAAGAAGATGAAGAACTACTGGAGA<br>CAGCTGCTGAACGCAAAGCTGATCACACAGAGAAAGTTCGACAACCTGACAA<br>AGGCAGAGAGAGGAGGACTGAGCGAACTGGACAAGGCAGGATTCATCAAGAG<br>ACAGCTGGTCGAAACAAGACAGATCACAAAGCACGTCGCACAGATCCTGGAC<br>AGCAGAATGAACACAAAGTACGACGAAAACGACAAGCTGATCAGAGAAGTCA<br>AGGTCATCACACTGAAGAGCAAGCTGGTCAGCGACTTCAGAAAGGACTTCCA<br>GTTCTACAAGGTCAGAGAAATCAACAACTACCACCACGCACACGACGCATAC<br>CTGAACGCAGTCGTCGGAACAGCACTGATCAAGAAGTACCCGAAGCTGGAAA<br>GCGAATTCGTCTACGGAGACTACAAGGTCTACGACGTCAGAAAGATGATCGC<br>AAAGAGCGAACAGGAAATCGGAAAGGCAACAGCAAAGTACTTCTTCTACAGC<br>AACATCATGAACTTCTTCAAGACAGAAATCACACTGGCAAACGGAGAAATCA<br>GAAAGAGACCGCTGATCGAAACAAACGGAGAAACAGGAGAAATCGTCTGGGA<br>CAAGGGAAGAGACTTCGCAACAGTCAGAAAGGTCCTGAGCATGCCGCAGGTC<br>AACATCGTCAAGAAGACAGAAGTCCAGACAGGAGGATTCAGCAAGGAAAGCA<br>TCCTGCCGAAGAGAAACAGCGACAAGCTGATCGCAAGAAAGAAGGACTGGGA<br>CCCGAAGAAGTACGGAGGATTCGACAGCCCGACAGTCGCATACAGCGTCCTG<br>GTCGTCGCAAAGGTCGAAAAGGGAAAGAGCAAGAAGCTGAAGAGCGTCAAGG<br>AACTGCTGGGAATCACAATCATGGAAAGAAGCAGCTTCGAAAAGAACCCGAT<br>CGACTTCCTGGAAGCAAAGGGATACAAGGAAGTCAAGAAGGACCTGATCATC<br>AAGCTGCCGAAGTACAGCCTGTTCGAACTGGAAAACGGAAGAAAGAGAATGC<br>TGGCAAGCGCAGGAGAACTGCAGAAGGGAAACGAACTGGCACTGCCGAGCAA<br>GTACGTCAACTTCCTGTACCTGGCAAGCCACTACGAAAAGCTGAAGGGAAGC<br>CCGGAAGACAACGAACAGAAGCAGCTGTTCGTCGAACAGCACAAGCACTACC<br>TGGACGAAATCATCGAACAGATCAGCGAATTCAGCAAGAGAGTCATCCTGGC<br>AGACGCAAACCTGGACAAGGTCCTGAGCGCATACAACAAGCACAGAGACAAG<br>CCGATCAGAGAACAGGCAGAAAACATCATCCACCTGTTCACACTGACAAACC<br>TGGGAGCACCGGCAGCATTCAAGTACTTCGACACAACAATCGACAGAAAGAG | |

(continued)

| Description | Sequence | SEQ ID No. |
|---|---|---|
| | ATACACAAGCACAAAGGAAGTCCTGGACGCAACACTGATCCACCAGAGCATCACAGGACTGTACGAAACAAGAATCGACCTGAGCCAGCTGGGAGGAGACGGAGGAGGAAGCCCGAAGAAGAAGAGAAAGGTCTAG | |

(continued)

| Description | Sequence | SEQ ID No. |
|---|---|---|
| **Cas9 DNA coding sequence 1** | ATGGATAAGAAGTACTCAATCGGGCTGGATATCGGAACTAATTCCGTGGGTT<br>GGGCAGTGATCACGGATGAATACAAAGTGCCGTCCAAGAAGTTCAAGGTCCT<br>GGGGAACACCGATAGACACAGCATCAAGAAAAATCTCATCGGAGCCCTGCTG<br>TTTGACTCCGGCGAAACCGCAGAAGCGACCCGGCTCAAACGTACCGCGAGGC<br>GACGCTACACCCGGCGGAAGAATCGCATCTGCTATCTGCAAGAGATCTTTTC<br>GAACGAAATGGCAAAGGTCGACGACAGCTTCTTCCACCGCCTGGAAGAATCT<br>TTCCTGGTGGAGGAGGACAAGAAGCATGAACGGCATCCTATCTTTGGAAACA<br>TCGTCGACGAAGTGGCGTACCACGAAAAGTACCCGACCATCTACCATCTGCG<br>GAAGAAGTTGGTTGACTCAACTGACAAGGCCGACCTCAGATTGATCTACTTG<br>GCCCTCGCCCATATGATCAAATTCCGCGGACACTTCCTGATCGAAGGCGATC<br>TGAACCCTGATAACTCCGACGTGGATAAGCTTTTCATTCAACTGGTGCAGAC<br>CTACAACCAACTGTTCGAAGAAAACCCAATCAATGCTAGCGGCGTCGATGCC<br>AAGGCCATCCTGTCCGCCCGGCTGTCGAAGTCGCGGCGCCTCGAAAACCTGA<br>TCGCACAGCTGCCGGGAGAGAAAAAGAACGGACTTTTCGGCAACTTGATCGC<br>TCTCTCACTGGGACTCACTCCCAATTTCAAGTCCAATTTTGACCTGGCCGAG<br>GACGCGAAGCTGCAACTCTCAAAGGACACCTACGACGACGACTTGGACAATT<br>TGCTGGCACAAATTGGCGATCAGTACGCGGATCTGTTCCTTGCCGCTAAGAA<br>CCTTTCGGACGCAATCTTGCTGTCCGATATCCTGCGCGTGAACACCGAAATA<br>ACCAAAGCGCCGCTTAGCGCCTCGATGATTAAGCGGTACGACGAGCATCACC<br>AGGATCTCACGCTGCTCAAAGCGCTCGTGAGACAGCAACTGCCTGAAAAGTA<br>CAAGGAGATCTTCTTCGACCAGTCCAAGAATGGGTACGCAGGGTACATCGAT<br>GGAGGCGCTAGCCAGGAAGAGTTCTATAAGTTCATCAAGCCAATCCTGGAAA<br>AGATGGACGGAACCGAAGAACTGCTGGTCAAGCTGAACAGGGAGGATCTGCT<br>CCGGAAACAGAGAACCTTTGACAACGGATCCATTCCCCACCAGATCCATCTG<br>GGTGAGCTGCACGCCATCTTGCGGCGCCAGGAGGACTTTTACCCATTCCTCA<br>AGGACAACCGGGAAAAGATCGAGAAAATTCTGACGTTCCGCATCCCGTATTA<br>CGTGGGCCCACTGGCGCGCGGCAATTCGCGCTTCGCGTGGATGACTAGAAAA<br>TCAGAGGAAACCATCACTCCTTGGAATTTCGAGGAAGTTGTGGATAAGGGAG<br>CTTCGGCACAAAGCTTCATCGAACGAATGACCAACTTCGACAAGAATCTCCC<br>AAACGAGAAGGTGCTTCCTAAGCACAGCCTCCTTTACGAATACTTCACTGTC<br>TACAACGAACTGACTAAAGTGAAATACGTTACTGAAGGAATGAGGAAGCCGG<br>CCTTTCTGTCCGGAGAACAGAAGAAAGCAATTGTCGATCTGCTGTTCAAGAC<br>CAACCGCAAGGTGACCGTCAAGCAGCTTAAAGAGGACTACTTCAAGAAGATC<br>GAGTGTTTCGACTCAGTGGAAATCAGCGGGGTGGAGGACAGATTCAACGCTT<br>CGCTGGGAACCTATCATGATCTCCTGAAGATCATCAAGGACAAGGACTTCCT<br>TGACAACGAGGAGAACGAGGACATCCTGGAAGATATCGTCCTGACCTTGACC<br>CTTTTCGAGGATCGCGAGATGATCGAGGAGAGGCTTAAGACCTACGCTCATC<br>TCTTCGACGATAAGGTCATGAAACAACTCAAGCGCCGCCGGTACACTGGTTG<br>GGGCCGCCTCTCCCGCAAGCTGATCAACGGTATTCGCGATAAACAGAGCGGT<br>AAAACTATCCTGGATTTCCTCAAATCGGATGGCTTCGCTAATCGTAACTTCA<br>TGCAATTGATCCACGACGACAGCCTGACCTTTAAGGAGGACATCCAAAAAGC<br>ACAAGTGTCCGGACAGGGAGACTCACTCCATGAACACATCGCGAATCTGGCC<br>GGTTCGCCGGCGATTAAGAAGGGAATTCTGCAAACTGTGAAGGTGGTCGACG<br>AGCTGGTGAAGGTCATGGGACGGCACAAACCGGAGAATATCGTGATTGAAAT<br>GGCCCGAGAAAACCAGACTACCCAGAAGGGCCAGAAAAACTCCCGCGAAAGG<br>ATGAAGCGGATCGAAGAAGGAATCAAGGAGCTGGGCAGCCAGATCCTGAAAG<br>AGCACCCGGTGGAAAACACGCAGCTGCAGAACGAGAAGCTCTACCTGTACTA<br>TTTGCAAAATGGACGGGACATGTACGTGGACCAAGAGCTGGACATCAATCGG<br>TTGTCTGATTACGACGTGGACCACATCGTTCCACAGTCCTTTCTGAAGGATG<br>ACTCGATCGATAACAAGGTGTTGACTCGCAGCGACAAGAACAGAGGGAAGTC<br>AGATAATGTGCCATCGGAGGAGGTCGTGAAGAAGATGAAGAATTACTGGCGG<br>CAGCTCCTGAATGCGAAGCTGATTACCCAGAGAAAGTTTGACAATCTCACTA<br>AAGCCGAGCGCGGCGGACTCTCAGAGCTGGATAAGGCTGGATTCATCAAACG<br>GCAGCTGGTCGAGACTCGGCAGATTACCAAGCACGTGGCGCAGATCTTGGAC<br>TCCCGCATGAACACTAAATACGACGAGAACGATAAGCTCATCCGGGAAGTGA<br>AGGTGATTACCCTGAAAAGCAAACTTGTGTCGGACTTTCGGAAGGACTTTCA | 2 |

(continued)

| Description | Sequence | SEQ ID No. |
|---|---|---|
| | GTTTTACAAAGTGAGAGAAATCAACAACTACCATCACGCGCATGACGCATAC<br>CTCAACGCTGTGGTCGGTACCGCCCTGATCAAAAAGTACCCTAAACTTGAAT<br>CGGAGTTTGTGTACGGAGACTACAAGGTCTACGACGTGAGGAAGATGATAGC<br>CAAGTCCGAACAGGAAATCGGGAAAGCAACTGCGAAATACTTCTTTTACTCA<br>AACATCATGAACTTTTTCAAGACTGAAATTACGCTGGCCAATGGAGAAATCA<br>GGAAGAGGCCACTGATCGAAACTAACGGAGAAACGGGCGAAATCGTGTGGGA<br>CAAGGGCAGGGACTTCGCAACTGTTCGCAAAGTGCTCTCTATGCCGCAAGTC<br>AATATTGTGAAGAAAACCGAAGTGCAAACCGGCGGATTTTCAAAGGAATCGA<br>TCCTCCCAAAGAGAAATAGCGACAAGCTCATTGCACGCAAGAAAGACTGGGA<br>CCCGAAGAAGTACGGAGGATTCGATTCGCCGACTGTCGCATACTCCGTCCTC<br>GTGGTGGCCAAGGTGGAGAAGGGAAAGAGCAAAAAGCTCAAATCCGTCAAAG<br>AGCTGCTGGGGATTACCATCATGGAACGATCCTCGTTCGAGAAGAACCCGAT<br>TGATTTCCTCGAGGCGAAGGGTTACAAGGAGGTGAAGAAGGATCTGATCATC<br>AAACTCCCCAAGTACTCACTGTTCGAACTGGAAAATGGTCGGAAGCGCATGC<br>TGGCTTCGGCCGGAGAACTCCAAAAAGGAAATGAGCTGGCCTTGCCTAGCAA<br>GTACGTCAACTTCCTCTATCTTGCTTCGCACTACGAAAAACTCAAAGGGTCA<br>CCGGAAGATAACGAACAGAAGCAGCTTTTCGTGGAGCAGCACAAGCATTATC<br>TGGATGAAATCATCGAACAAATCTCCGAGTTTTCAAAGCGCGTGATCCTCGC<br>CGACGCCAACCTCGACAAAGTCCTGTCGGCCTACAATAAGCATAGAGATAAG<br>CCGATCAGAGAACAGGCCGAGAACATTATCCACTTGTTCACCCTGACTAACC<br>TGGGAGCCCCAGCCGCCTTCAAGTACTTCGATACTACTATCGATCGCAAAAG<br>ATACACGTCCACCAAGGAAGTTCTGGACGCGACCCTGATCCACCAAAGCATC<br>ACTGGACTCTACGAAACTAGGATCGATCTGTCGCAGCTGGGTGGCGATGGCG<br>GTGGATCTCCGAAAAAGAAGAGAAAGGTGTAATGA | |
| **Cas9 amino acid sequence** | MDKKYSIGLDIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT<br>LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDHIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGDGGGSPKKKRKV | **3** |

(continued)

| Description | Sequence | SEQ ID No. |
|---|---|---|
| **Cas9 mRNA open reading frame (ORF) 2** | AUGGACAAGAAGUACAGCAUCGGACUGGACAUCGGAACAAACAGCGUCGGAU GGGCAGUCAUCACAGACGAAUACAAGGUCCCGAGCAAGAAGUUCAAGGUCCU GGGAAACACAGACAGACACAGCAUCAAGAAGAACCUGAUCGGAGCACUGCUG UUCGACAGCGGAGAAACAGCAGAAGCAACAAGACUGAAGAGAACAGCAAGAA GAAGAUACACAAGAAGAAAGAACAGAAUCUGCUACCUGCAGGAAAUCUUCAG | 4 |

(continued)

| Description | Sequence | SEQ ID No. |
| --- | --- | --- |
| | CAACGAAAUGGCAAAGGUCGACGACAGCUUCUUCCACAGACUGGAAGAAAGC<br>UUCCUGGUCGAAGAAGACAAGAAGCACGAAAGACACCCGAUCUUCGGAAACA<br>UCGUCGACGAAGUCGCAUACCACGAAAAGUACCCGACAAUCUACCACCUGAG<br>AAAGAAGCUGGUCGACAGCACAGACAAGGCAGACCUGAGACUGAUCUACCUG<br>GCACUGGCACACAUGAUCAAGUUCAGAGGACACUUCCUGAUCGAAGGAGACC<br>UGAACCCGGACAACAGCGACGUCGACAAGCUGUUCAUCCAGCUGGUCCAGAC<br>AUACAACCAGCUGUUCGAAGAAAACCCGAUCAACGCAAGCGGAGUCGACGCA<br>AAGGCAAUCCUGAGCGCAAGACUGAGCAAGAGCAGAAGACUGGAAAACCUGA<br>UCGCACAGCUGCCGGGAGAAAAGAAGAACGGACUGUUCGGAAACCUGAUCGC<br>ACUGAGCCUGGGACUGACACCGAACUUCAAGAGCAACUUCGACCUGGCAGAA<br>GACGCAAAGCUGCAGCUGAGCAAGGACACAUACGACGACGACCUGGACAACC<br>UGCUGGCACAGAUCGGAGACCAGUACGCAGACCUGUUCCUGGCAGCAAAGAA<br>CCUGAGCGACGCAAUCCUGCUGAGCGACAUCCUGAGAGUCAACACAGAAAUC<br>ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC<br>GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACCACAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA<br>GCGAAUUCGUCUACGGAGACUACAAGGUCUACGACGUCAGAAAGAUGAUCGC<br>AAAGAGCGAACAGGAAAUCGGAAAGGCAACAGCAAAGUACUUCUUCUACAGC<br>AACAUCAUGAACUUCUUCAAGACAGAAAUCACACUGGCAAACGGAGAAAUCA<br>GAAAGAGACCGCUGAUCGAAACAAACGGAGAAACAGGAGAAAUCGUCUGGGA<br>CAAGGGAAGAGACUUCGCAACAGUCAGAAAGGUCCUGAGCAUGCCGCAGGUC<br>AACAUCGUCAAGAAGACAGAAGUCCAGACAGGAGGAUUCAGCAAGGAAAGCA<br>UCCUGCCGAAGAGAAACAGCGACAAGCUGAUCGCAAGAAAGAAGGACUGGGA | |

(continued)

| Description | Sequence | SEQ ID No. |
|---|---|---|
| | CCCGAAGAAGUACGGAGGAUUCGACAGCCCGACAGUCGCAUACAGCGUCCUG GUCGUCGCAAAGGUCGAAAAGGGAAAGAGCAAGAAGCUGAAGAGCGUCAAGG AACUGCUGGGAAUCACAAUCAUGGAAAGAAGCAGCUUCGAAAAGAACCCGAU CGACUUCCUGGAAGCAAAGGGAUACAAGGAAGUCAAGAAGGACCUGAUCAUC AAGCUGCCGAAGUACAGCCUGUUCGAACUGGAAAACGGAAGAAAGAGAAUGC UGGCAAGCGCAGGAGAACUGCAGAAGGGAAACGAACUGGCACUGCCGAGCAA GUACGUCAACUUCCUGUACCUGGCAAGCCACUACGAAAAGCUGAAGGGAAGC CCGGAAGACAACGAACAGAAGCAGCUGUUCGUCGAACAGCACAAGCACUACC UGGACGAAAUCAUCGAACAGAUCAGCGAAUUCAGCAAGAGAGUCAUCCUGGC AGACGCAAACCUGGACAAGGUCCUGAGCGCAUACAACAAGCACAGAGACAAG CCGAUCAGAGAACAGGCAGAAAACAUCAUCCACCUGUUCACACUGACAAACC UGGGAGCACCGGCAGCAUUCAAGUACUUCGACACAACAAUCGACAGAAAGAG AUACACAAGCACAAAGGAAGUCCUGGACGCAACACUGAUCCACCAGAGCAUC ACAGGACUGUACGAAACAAGAAUCGACCUGAGCCAGCUGGGAGGAGACGGAG GAGGAAGCCCGAAGAAGAAGAGAAAGGUCUAG | |

(continued)

| Description | Sequence | SEQ ID No. |
|---|---|---|
| **Cas9 mRNA ORF 1** | AUGGAUAAGAAGUACUCAAUCGGGCUGGAUAUCGGAACUAAUUCCGUGGGUUGGGCAGUGAUCACGGAUGAAUACAAAGUGCCGUCCAAGAAGUUCAAGGUCCUGGGGAACACCGAUAGACACAGCAUCAAGAAAAAUCUCAUCGGAGCCCUGCUGUUUGACUCCGGCGAAACCGCAGAAGCGACCCGGCUCAAACGUACCGCGAGGCGACGCUACACCCGGCGGAAGAAUCGCAUCUGCUAUCUGCAAGAGAUCUUUUCGAACGAAAUGGCAAAGGUCGACGACAGCUUCUUCCACCGCCUGGAAGAAUCUUUCCUGGUGGAGGAGGACAAGAAGCAUGAACGGCAUCCUAUCUUUGGAAACAUCGUCGACGAAGUGGCGUACCACGAAAAGUACCCGACCAUCUACCAUCUGCGGAAGAAGUUGGUUGACUCAACUGACAAGGCCGACCUCAGAUUGAUCUACUUGGCCCUCGCCCAUAUGAUCAAAUUCCGCGGACACUUCCUGAUCGAAGGCGAUCUGAACCCUGAUAACUCCGACGUGGAUAAGCUUUUCAUUCAACUGGUGCAGACCUACAACCAACUGUUCGAAGAAAACCCAAUCAAUGCUAGCGGCGUCGAUGCCAAGGCCAUCCUGUCCGCCCGGCUGUCGAAGUCGCGGCGCCUCGAAAACCUGAUCGCACAGCUGCCGGGAGAGAAAAAGAACGGACUUUUCGGCAACUUGAUCGCUCUCUCACUGGGACUCACUCCCAAUUUCAAGUCCAAUUUUGACCUGGCCGAGGACGCGAAGCUGCAACUCUCAAAGGACACCUACGACGACGACUUGGACAAUUUGCUGGCACAAAUUGGCGAUCAGUACGCGGAUCUGUUCCUUGCCGCUAAGAACCUUUCGGACGCAAUCUUGCUGUCCGAUAUCCUGCGCGUGAACACCGAAAUAACCAAAGCGCCGCUUAGCGCCUCGAUGAUUAAGCGGUACGACGAGCAUCACCAGGAUCUCACGCUGCUCAAAGCGCUCGUGAGACAGCAACUGCCUGAAAAGUACAAGGAGAUCUUCUUCGACCAGUCCAAGAAUGGGUACGCAGGGUACAUCGAUGGAGGCGCUAGCCAGGAAGAGUUCUAUAAGUUCAUCAAGCCAAUCCUGGAAAAGAUGGACGGAACCGAAGAACUGCUGGUCAAGCUGAACAGGGAGGAUCUGCUCCGGAAACAGAGAACCUUUGACAACGGAUCCAUUCCCCACCAGAUCCAUCUGGGUGAGCUGCACGCCAUCUUGCGGCGCCAGGAGGACUUUUACCCAUUCCUCAAGGACAACCGGGAAAAGAUCGAGAAAAUUCUGACGUUCCGCAUCCCGUAUUACGUGGGCCCACUGGCGCGCGGCAAUUCGCGCUUCGCGUGGAUGACUAGAAAAUCAGAGGAAACCAUCACUCCUUGGAAUUUCGAGGAAGUUGUGGAUAAGGGAGCUUCGGCACAAAGCUUCAUCGAACGAAUGACCAACUUCGACAAGAAUCUCCCAAACGAGAAGGUGCUUCCUAAGCACAGCCUCCUUUACGAAUACUUCACUGUCUACAACGAACUGACUAAAGUGAAAUACGUUACUGAAGGAAUGAGGAAGCCGGCCUUUCUGUCCGGAGAACAGAAGAAAGCAAUUGUCGAUCUGCUGUUCAAGACCAACCGCAAGGUGACCGUCAAGCAGCUUAAAGAGGACUACUUCAAGAAGAUCGAGUGUUUCGACUCAGUGGAAAUCAGCGGGGUGGAGGACAGAUUCAACGCUUCGCUGGGAACCUAUCAUGAUCUCCUGAAGAUCAUCAAGGACAAGGACUUCCUUGACAACGAGGAGAACGAGGACAUCCUGGAAGAUAUCGUCCUGACCUUGACCCUUUUCGAGGAUCGCGAGAUGAUCGAGGAGAGGCUUAAGACCUACGCUCAUCUCUUCGACGAUAAGGUCAUGAAACAACUCAAGCGCCGCCGGUACACUGGUUGGGGCCGCCUCUCCCGCAAGCUGAUCAACGGUAUUCGCGAUAAACAGAGCGGUAAAACUAUCCUGGAUUUCCUCAAAUCGGAUGGCUUCGCUAAUCGUAACUUCAUGCAAUUGAUCCACGACGACAGCCUGACCUUUAAGGAGGACAUCCAAAAAGCACAAGUGUCCGGACAGGGAGACUCACUCCAUGAACACAUCGCGAAUCUGGCCGGUUCGCCGGCGAUUAAGAAGGGAAUUCUGCAAACUGUGAAGGUGGUCGACG | 5 |

(continued)

| Description | Sequence | SEQ ID No. |
|---|---|---|
| | AGCUGGUGAAGGUCAUGGGACGGCACAAACCGGAGAAUAUCGUGAUUGAAAU GGCCCGAGAAAACCAGACUACCCAGAAGGGCCAGAAAAACUCCCGCGAAAGG AUGAAGCGGAUCGAAGAAGGAAUCAAGGAGCUGGGCAGCCAGAUCCUGAAAG AGCACCCGGUGGAAAACACGCAGCUGCAGAACGAGAAGCUCUACCUGUACUA UUUGCAAAAUGGACGGGACAUGUACGUGGACCAAGAGCUGGACAUCAAUCGG UUGUCUGAUUACGACGUGGACCACAUCGUUCCACAGUCCUUUCUGAAGGAUG ACUCGAUCGAUAACAAGGUGUUGACUCGCAGCGACAAGAACAGAGGGAAGUC AGAUAAUGUGCCAUCGGAGGAGGUCGUGAAGAAGAUGAAGAAUUACUGGCGG CAGCUCCUGAAUGCGAAGCUGAUUACCCAGAGAAAGUUUGACAAUCUCACUA AAGCCGAGCGCGGCGGACUCUCAGAGCUGGAUAAGGCUGGAUUCAUCAAACG GCAGCUGGUCGAGACUCGGCAGAUUACCAAGCACGUGGCGCAGAUCUUGGAC UCCCGCAUGAACACUAAAUACGACGAGAACGAUAAGCUCAUCCGGGAAGUGA AGGUGAUUACCCUGAAAAGCAAACUUGUGUCGGACUUUCGGAAGGACUUUCA GUUUUACAAAGUGAGAGAAAUCAACAACUACCAUCACGCGCAUGACGCAUAC CUCAACGCUGUGGUCGGUACCGCCCUGAUCAAAAAGUACCCUAAACUUGAAU CGGAGUUUGUGUACGGAGACUACAAGGUCUACGACGUGAGGAAGAUGAUAGC CAAGUCCGAACAGGAAAUCGGGAAAGCAACUGCGAAAUACUUCUUUUACUCA AACAUCAUGAACUUUUUCAAGACUGAAAUUACGCUGGCCAAUGGAGAAAUCA GGAAGAGGCCACUGAUCGAAACUAACGGAGAAACGGGCGAAAUCGUGUGGGA CAAGGGCAGGGACUUCGCAACUGUUCGCAAAGUGCUCUCUAUGCCGCAAGUC AAUAUUGUGAAGAAAACCGAAGUGCAAACCGGCGGAUUUUCAAAGGAAUCGA UCCUCCCAAAGAGAAAUAGCGACAAGCUCAUUGCACGCAAGAAAGACUGGGA CCCGAAGAAGUACGGAGGAUUCGAUUCGCCGACUGUCGCAUACUCCGUCCUC GUGGUGGCCAAGGUGGAGAAGGGAAAGAGCAAAAAGCUCAAAUCCGUCAAAG AGCUGCUGGGGAUUACCAUCAUGGAACGAUCCUCGUUCGAGAAGAACCCGAU UGAUUUCCUCGAGGCGAAGGGUUACAAGGAGGUGAAGAAGGAUCUGAUCAUC AAACUCCCCAAGUACUCACUGUUCGAACUGGAAAAUGGUCGGAAGCGCAUGC UGGCUUCGGCCGGAGAACUCCAAAAAGGAAAUGAGCUGGCCUUGCCUAGCAA GUACGUCAACUUCCUCUAUCUUGCUUCGCACUACGAAAAACUCAAAGGGUCA CCGGAAGAUAACGAACAGAAGCAGCUUUUCGUGGAGCAGCACAAGCAUUAUC UGGAUGAAAUCAUCGAACAAAUCUCCGAGUUUUCAAAGCGCGUGAUCCUCGC CGACGCCAACCUCGACAAAGUCCUGUCGGCCUACAAUAAGCAUAGAGAUAAG CCGAUCAGAGAACAGGCCGAGAACAUUAUCCACUUGUUCACCCUGACUAACC UGGGAGCCCCAGCCGCCUUCAAGUACUUCGAUACUACUAUCGAUCGCAAAAG AUACACGUCCACCAAGGAAGUUCUGGACGCGACCCUGAUCCACCAAAGCAUC ACUGGACUCUACGAAACUAGGAUCGAUCUGUCGCAGCUGGGUGGCGAUGGCG GUGGAUCUCCGAAAAAGAAGAGAAAGGUGUAAUGA | |

(continued)

| Description | Sequence | SEQ ID No. |
|---|---|---|
| **Cas9 bare coding sequence** | GACAAGAAGUACAGCAUCGGACUGGACAUCGGAACAAACAGCGUCGGAUGGG CAGUCAUCACAGACGAAUACAAGGUCCCGAGCAAGAAGUUCAAGGUCCUGGG AAACACAGACAGACACAGCAUCAAGAAGAACCUGAUCGGAGCACUGCUGUUC GACAGCGGAGAAACAGCAGAAGCAACAAGACUGAAGAGAACAGCAAGAAGAA GAUACACAAGAAGAAAGAACAGAAUCUGCUACCUGCAGGAAAUCUUCAGCAA CGAAAUGGCAAAGGUCGACGACAGCUUCUUCCACAGACUGGAAGAAAGCUUC CUGGUCGAAGAAGACAAGAAGCACGAAAGACACCCGAUCUUCGGAAACAUCG UCGACGAAGUCGCAUACCACGAAAAGUACCCGACAAUCUACCACCUGAGAAA GAAGCUGGUCGACAGCACAGACAAGGCAGACCUGAGACUGAUCUACCUGGCA CUGGCACACAUGAUCAAGUUCAGAGGACACUUCCUGAUCGAAGGAGACCUGA ACCCGGACAACAGCGACGUCGACAAGCUGUUCAUCCAGCUGGUCCAGACAUA CAACCAGCUGUUCGAAGAAAACCCGAUCAACGCAAGCGGAGUCGACGCAAAG GCAAUCCUGAGCGCAAGACUGAGCAAGAGCAGAAGACUGGAAAACCUGAUCG CACAGCUGCCGGGAGAAAAGAAGAACGGACUGUUCGGAAACCUGAUCGCACU GAGCCUGGGACUGACACCGAACUUCAAGAGCAACUUCGACCUGGCAGAAGAC GCAAAGCUGCAGCUGAGCAAGGACACAUACGACGACGACCUGGACAACCUGC UGGCACAGAUCGGAGACCAGUACGCAGACCUGUUCCUGGCAGCAAAGAACCU GAGCGACGCAAUCCUGCUGAGCGACAUCCUGAGAGUCAACACAGAAAUCACA AAGGCACCGCUGAGCGCAAGCAUGAUCAAGAGAUACGACGAACACCACCAGG ACCUGACACUGCUGAAGGCACUGGUCAGACAGCAGCUGCCGGAAAAGUACAA GGAAAUCUUCUUCGACCAGAGCAAGAACGGAUACGCAGGAUACAUCGACGGA | 10 |

(continued)

| Description | Sequence | SEQ ID No. |
|---|---|---|
| | GGAGCAAGCCAGGAAGAAUUCUACAAGUUCAUCAAGCCGAUCCUGGAAAAGA UGGACGGAACAGAAGAACUGCUGGUCAAGCUGAACAGAGAAGACCUGCUGAG AAAGCAGAGAACAUUCGACAACGGAAGCAUCCCGCACCAGAUCCACCUGGGA GAACUGCACGCAAUCCUGAGAAGACAGGAAGACUUCUACCCGUUCCUGAAGG ACAACAGAGAAAAGAUCGAAAAGAUCCUGACAUUCAGAAUCCCGUACUACGU CGGACCGCUGGCAAGAGGAAACAGCAGAUUCGCAUGGAUGACAAGAAAGAGC GAAGAAACAAUCACACCGUGGAACUUCGAAGAAGUCGUCGACAAGGGAGCAA GCGCACAGAGCUUCAUCGAAAGAAUGACAAACUUCGACAAGAACCUGCCGAA CGAAAAGGUCCUGCCGAAGCACAGCCUGCUGUACGAAUACUUCACAGUCUAC AACGAACUGACAAAGGUCAAGUACGUCACAGAAGGAAUGAGAAAGCCGGCAU UCCUGAGCGGAGAACAGAAGAAGGCAAUCGUCGACCUGCUGUUCAAGACAAA CAGAAAGGUCACAGUCAAGCAGCUGAAGGAAGACUACUUCAAGAAGAUCGAA UGCUUCGACAGCGUCGAAAUCAGCGGAGUCGAAGACAGAUUCAACGCAAGCC UGGGAACAUACCACGACCUGCUGAAGAUCAUCAAGGACAAGGACUUCCUGGA CAACGAAGAAAACGAAGACAUCCUGGAAGACAUCGUCCUGACACUGACACUG UUCGAAGACAGAGAAAUGAUCGAAGAAAGACUGAAGACAUACGCACACCUGU UCGACGACAAGGUCAUGAAGCAGCUGAAGAGAAGAAGAUACACAGGAUGGGG AAGACUGAGCAGAAAGCUGAUCAACGGAAUCAGAGACAAGCAGAGCGGAAAG ACAAUCCUGGACUUCCUGAAGAGCGACGGAUUCGCAAACAGAAACUUCAUGC AGCUGAUCCACGACGACAGCCUGACAUUCAAGGAAGACAUCCAGAAGGCACA GGUCAGCGGACAGGGAGACAGCCUGCACGAACACAUCGCAAACCUGGCAGGA AGCCCGGCAAUCAAGAAGGGAAUCCUGCAGACAGUCAAGGUCGUCGACGAAC UGGUCAAGGUCAUGGGAAGACACAAGCCGGAAAACAUCGUCAUCGAAAUGGC AAGAGAAAACCAGACAACACAGAAGGGACAGAAGAACAGCAGAGAAAGAAUG AAGAGAAUCGAAGAAGGAAUCAAGGAACUGGGAAGCCAGAUCCUGAAGGAAC ACCCGGUCGAAAACACACAGCUGCAGAACGAAAAGCUGUACCUGUACUACCU GCAGAACGGAAGAGACAUGUACGUCGACCAGGAACUGGACAUCAACAGACUG AGCGACUACGACGUCGACCACAUCGUCCCGCAGAGCUUCCUGAAGGACGACA GCAUCGACAACAAGGUCCUGACAAGAAGCGACAAGAACAGAGGAAAGAGCGA CAACGUCCCGAGCGAAGAAGUCGUCAAGAAGAUGAAGAACUACUGGAGACAG CUGCUGAACGCAAAGCUGAUCACACAGAGAAAGUUCGACAACCUGACAAAGG CAGAGAGAGGAGGACUGAGCGAACUGGACAAGGCAGGAUUCAUCAAGAGACA GCUGGUCGAAACAAGACAGAUCACAAAGCACGUCGCACAGAUCCUGGACAGC AGAAUGAACACAAAGUACGACGAAAACGACAAGCUGAUCAGAGAAGUCAAGG UCAUCACACUGAAGAGCAAGCUGGUCAGCGACUUCAGAAAGGACUUCCAGUU CUACAAGGUCAGAGAAAUCAACAACUACCACCACGCACACGACGCAUACCUG AACGCAGUCGUCGGAACAGCACUGAUCAAGAAGUACCCGAAGCUGGAAAGCG AAUUCGUCUACGGAGACUACAAGGUCUACGACGUCAGAAAGAUGAUCGCAAA GAGCGAACAGGAAAUCGGAAAGGCAACAGCAAAGUACUUCUUCUACAGCAAC AUCAUGAACUUCUUCAAGACAGAAAUCACACUGGCAAACGGAGAAAUCAGAA AGAGACCGCUGAUCGAAACAAACGGAGAAACAGGAGAAAUCGUCUGGGACAA GGGAAGAGACUUCGCAACAGUCAGAAAGGUCCUGAGCAUGCCGCAGGUCAAC AUCGUCAAGAAGACAGAAGUCCAGACAGGAGGAUUCAGCAAGGAAAGCAUCC UGCCGAAGAGAAACAGCGACAAGCUGAUCGCAAGAAAGAAGGACUGGGACCC GAAGAAGUACGGAGGAUUCGACAGCCCGACAGUCGCAUACAGCGUCCUGGUC GUCGCAAAGGUCGAAAAGGGAAAGAGCAAGAAGCUGAAGAGCGUCAAGGAAC UGCUGGGAAUCACAAUCAUGGAAAGAAGCAGCUUCGAAAAGAACCCGAUCGA CUUCCUGGAAGCAAAGGGAUACAAGGAAGUCAAGAAGGACCUGAUCAUCAAG CUGCCGAAGUACAGCCUGUUCGAACUGGAAAACGGAAGAAAGAGAAUGCUGG CAAGCGCAGGAGAACUGCAGAAGGGAAACGAACUGGCACUGCCGAGCAAGUA CGUCAACUUCCUGUACCUGGCAAGCCACUACGAAAAGCUGAAGGGAAGCCCG GAAGACAACGAACAGAAGCAGCUGUUCGUCGAACAGCACAAGCACUACCUGG ACGAAAUCAUCGAACAGAUCAGCGAAUUCAGCAAGAGAGUCAUCCUGGCAGA CGCAAACCUGGACAAGGUCCUGAGCGCAUACAACAAGCACAGAGACAAGCCG AUCAGAGAACAGGCAGAAAACAUCAUCCACCUGUUCACACUGACAAACCUGG GAGCACCGGCAGCAUUCAAGUACUUCGACACAACAAUCGACAGAAAGAGAUA CACAAGCACAAAGGAAGUCCUGGACGCAACACUGAUCCACCAGAGCAUCACA GGACUGUACGAAACAAGAAUCGACCUGAGCCAGCUGGGAGGAGACGGAGGAG GAAGCCCGAAGAAGAAGAGAAAGGUC | |

(continued)

| Description | Sequence | SEQ ID No. |
|---|---|---|
| | | |
| **Amino acid sequence of Cas9 (without NLS)** | MDKKYSIGLDIGTNSVGWAVITDEYKVPSKKFKVLGNTDRHSIKKNLIGALL FDSGETAEATRLKRTARRRYTRRKNRICYLQEIFSNEMAKVDDSFFHRLEES FLVEEDKKHERHPIFGNIVDEVAYHEKYPTIYHLRKKLVDSTDKADLRLIYL ALAHMIKFRGHFLIEGDLNPDNSDVDKLFIQLVQTYNQLFEENPINASGVDA KAILSARLSKSRRLENLIAQLPGEKKNGLFGNLIALSLGLTPNFKSNFDLAE DAKLQLSKDTYDDDLDNLLAQIGDQYADLFLAAKNLSDAILLSDILRVNTEI TKAPLSASMIKRYDEHHQDLTLLKALVRQQLPEKYKEIFFDQSKNGYAGYID GGASQEEFYKFIKPILEKMDGTEELLVKLNREDLLRKQRTFDNGSIPHQIHL GELHAILRRQEDFYPFLKDNREKIEKILTFRIPYYVGPLARGNSRFAWMTRK SEETITPWNFEEVVDKGASAQSFIERMTNFDKNLPNEKVLPKHSLLYEYFTV YNELTKVKYVTEGMRKPAFLSGEQKKAIVDLLFKTNRKVTVKQLKEDYFKKI ECFDSVEISGVEDRFNASLGTYHDLLKIIKDKDFLDNEENEDILEDIVLTLT LFEDREMIEERLKTYAHLFDDKVMKQLKRRRYTGWGRLSRKLINGIRDKQSG KTILDFLKSDGFANRNFMQLIHDDSLTFKEDIQKAQVSGQGDSLHEHIANLA GSPAIKKGILQTVKVVDELVKVMGRHKPENIVIEMARENQTTQKGQKNSRER MKRIEEGIKELGSQILKEHPVENTQLQNEKLYLYYLQNGRDMYVDQELDINR LSDYDVDHIVPQSFLKDDSIDNKVLTRSDKNRGKSDNVPSEEVVKKMKNYWR QLLNAKLITQRKFDNLTKAERGGLSELDKAGFIKRQLVETRQITKHVAQILD SRMNTKYDENDKLIREVKVITLKSKLVSDFRKDFQFYKVREINNYHHAHDAY LNAVVGTALIKKYPKLESEFVYGDYKVYDVRKMIAKSEQEIGKATAKYFFYS NIMNFFKTEITLANGEIRKRPLIETNGETGEIVWDKGRDFATVRKVLSMPQV NIVKKTEVQTGGFSKESILPKRNSDKLIARKKDWDPKKYGGFDSPTVAYSVL VVAKVEKGKSKKLKSVKELLGITIMERSSFEKNPIDFLEAKGYKEVKKDLII KLPKYSLFELENGRKRMLASAGELQKGNELALPSKYVNFLYLASHYEKLKGS PEDNEQKQLFVEQHKHYLDEIIEQISEFSKRVILADANLDKVLSAYNKHRDK PIREQAENIIHLFTLTNLGAPAAFKYFDTTIDRKRYTSTKEVLDATLIHQSI TGLYETRIDLSQLGGD | **13** |

(continued)

| Description | Sequence | SEQ ID No. |
|---|---|---|
| **Cas9 mRNA ORF encoding SEQ ID NO: 13 using minimal uridine codons as listed in Table 1, with start and stop codons** | AUGGACAAGAAGUACAGCAUCGGACUGGACAUCGGAACAAACAGCGUCGGAU<br>GGGCAGUCAUCACAGACGAAUACAAGGUCCCGAGCAAGAAGUUCAAGGUCCU<br>GGGAAACACAGACAGACACAGCAUCAAGAAGAACCUGAUCGGAGCACUGCUG<br>UUCGACAGCGGAGAAACAGCAGAAGCAACAAGACUGAAGAGAACAGCAAGAA<br>GAAGAUACACAAGAAGAAAGAACAGAAUCUGCUACCUGCAGGAAAUCUUCAG<br>CAACGAAAUGGCAAAGGUCGACGACAGCUUCUUCCACAGACUGGAAGAAAGC<br>UUCCUGGUCGAAGAAGACAAGAAGCACGAAAGACACCCGAUCUUCGGAAACA<br>UCGUCGACGAAGUCGCAUACCACGAAAAGUACCCGACAAUCUACCACCUGAG<br>AAAGAAGCUGGUCGACAGCACAGACAAGGCAGACCUGAGACUGAUCUACCUG<br>GCACUGGCACACAUGAUCAAGUUCAGAGGACACUUCCUGAUCGAAGGAGACC<br>UGAACCCGGACAACAGCGACGUCGACAAGCUGUUCAUCCAGCUGGUCCAGAC<br>AUACAACCAGCUGUUCGAAGAAAACCCGAUCAACGCAAGCGGAGUCGACGCA<br>AAGGCAAUCCUGAGCGCAAGACUGAGCAAGAGCAGAAGACUGGAAAACCUGA<br>UCGCACAGCUGCCGGGAGAAAAGAAGAACGGACUGUUCGGAAACCUGAUCGC<br>ACUGAGCCUGGGACUGACACCGAACUUCAAGAGCAACUUCGACCUGGCAGAA<br>GACGCAAAGCUGCAGCUGAGCAAGGACACAUACGACGACGACCUGGACAACC<br>UGCUGGCACAGAUCGGAGACCAGUACGCAGACCUGUUCCUGGCAGCAAAGAA<br>CCUGAGCGACGCAAUCCUGCUGAGCGACAUCCUGAGAGUCAACACAGAAAUC<br>ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC | **14** |

(continued)

| Description | Sequence | SEQ ID No. |
|---|---|---|
| | GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACCACAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA<br>GCGAAUUCGUCUACGGAGACUACAAGGUCUACGACGUCAGAAAGAUGAUCGC<br>AAAGAGCGAACAGGAAAUCGGAAAGGCAACAGCAAAGUACUUCUUCUACAGC<br>AACAUCAUGAACUUCUUCAAGACAGAAAUCACACUGGCAAACGGAGAAAUCA<br>GAAAGAGACCGCUGAUCGAAACAAACGGAGAAACAGGAGAAAUCGUCUGGGA<br>CAAGGGAAGAGACUUCGCAACAGUCAGAAAGGUCCUGAGCAUGCCGCAGGUC<br>AACAUCGUCAAGAAGACAGAAGUCCAGACAGGAGGAUUCAGCAAGGAAAGCA<br>UCCUGCCGAAGAGAAACAGCGACAAGCUGAUCGCAAGAAAGAAGGACUGGGA<br>CCCGAAGAAGUACGGAGGAUUCGACAGCCCGACAGUCGCAUACAGCGUCCUG<br>GUCGUCGCAAAGGUCGAAAAGGGAAAGAGCAAGAAGCUGAAGAGCGUCAAGG<br>AACUGCUGGGAAUCACAAUCAUGGAAAGAAGCAGCUUCGAAAAGAACCCGAU<br>CGACUUCCUGGAAGCAAAGGGAUACAAGGAAGUCAAGAAGGACCUGAUCAUC<br>AAGCUGCCGAAGUACAGCCUGUUCGAACUGGAAAACGGAAGAAAGAGAAUGC<br>UGGCAAGCGCAGGAGAACUGCAGAAGGGAAACGAACUGGCACUGCCGAGCAA<br>GUACGUCAACUUCCUGUACCUGGCAAGCCACUACGAAAAGCUGAAGGGAAGC<br>CCGGAAGACAACGAACAGAAGCAGCUGUUCGUCGAACAGCACAAGCACUACC<br>UGGACGAAAUCAUCGAACAGAUCAGCGAAUUCAGCAAGAGAGUCAUCCUGGC<br>AGACGCAAACCUGGACAAGGUCCUGAGCGCAUACAACAAGCACAGAGACAAG<br>CCGAUCAGAGAACAGGCAGAAAACAUCAUCCACCUGUUCACACUGACAAACC<br>UGGGAGCACCGGCAGCAUUCAAGUACUUCGACACAACAAUCGACAGAAAGAG<br>AUACACAAGCACAAAGGAAGUCCUGGACGCAACACUGAUCCACCAGAGCAUC<br>ACAGGACUGUACGAAACAAGAAUCGACCUGAGCCAGCUGGGAGGAGACUAG | |

(continued)

| Description | Sequence | SEQ ID No. |
|---|---|---|
| **Cas9 coding sequence encoding SEQ ID NO: 13 using minimal uridine codons as** | GACAAGAAGUACAGCAUCGGACUGGACAUCGGAACAAACAGCGUCGGAUGGG<br>CAGUCAUCACAGACGAAUACAAGGUCCCGAGCAAGAAGUUCAAGGUCCUGGG<br>AAACACAGACAGACACAGCAUCAAGAAGAACCUGAUCGGAGCACUGCUGUUC<br>GACAGCGGAGAAACAGCAGAAGCAACAAGACUGAAGAGAACAGCAAGAAGAA<br>GAUACACAAGAAGAAAGAACAGAAUCUGCUACCUGCAGGAAAUCUUCAGCAA<br>CGAAAUGGCAAAGGUCGACGACAGCUUCUUCCACAGACUGGAAGAAAGCUUC<br>CUGGUCGAAGAAGACAAGAAGCACGAAAGACACCCGAUCUUCGGAAACAUCG<br>UCGACGAAGUCGCAUACCACGAAAAGUACCCGACAAUCUACCACCUGAGAAA | **15** |

(continued)

| Description | Sequence | SEQ ID No. |
|---|---|---|
| listed in Table 1 (no start or stop codons; suitable for inclusion in fusion protein coding sequence) | GAAGCUGGUCGACAGCACAGACAAGGCAGACCUGAGACUGAUCUACCUGGCA<br>CUGGCACACAUGAUCAAGUUCAGAGGACACUUCCUGAUCGAAGGAGACCUGA<br>ACCCGGACAACAGCGACGUCGACAAGCUGUUCAUCCAGCUGGUCCAGACAUA<br>CAACCAGCUGUUCGAAGAAAACCCGAUCAACGCAAGCGGAGUCGACGCAAAG<br>GCAAUCCUGAGCGCAAGACUGAGCAAGAGCAGAAGACUGGAAAACCUGAUCG<br>CACAGCUGCCGGGAGAAAAGAAGAACGGACUGUUCGGAAACCUGAUCGCACU<br>GAGCCUGGGACUGACACCGAACUUCAAGAGCAACUUCGACCUGGCAGAAGAC<br>GCAAAGCUGCAGCUGAGCAAGGACACAUACGACGACGACCUGGACAACCUGC<br>UGGCACAGAUCGGAGACCAGUACGCAGACCUGUUCCUGGCAGCAAAGAACCU<br>GAGCGACGCAAUCCUGCUGAGCGACAUCCUGAGAGUCAACACAGAAAUCACA<br>AAGGCACCGCUGAGCGCAAGCAUGAUCAAGAGAUACGACGAACACCACCAGG<br>ACCUGACACUGCUGAAGGCACUGGUCAGACAGCAGCUGCCGGAAAAGUACAA<br>GGAAAUCUUCUUCGACCAGAGCAAGAACGGAUACGCAGGAUACAUCGACGGA<br>GGAGCAAGCCAGGAAGAAUUCUACAAGUUCAUCAAGCCGAUCCUGGAAAAGA<br>UGGACGGAACAGAAGAACUGCUGGUCAAGCUGAACAGAGAAGACCUGCUGAG<br>AAAGCAGAGAACAUUCGACAACGGAAGCAUCCCGCACCAGAUCCACCUGGGA<br>GAACUGCACGCAAUCCUGAGAAGACAGGAAGACUUCUACCCGUUCCUGAAGG<br>ACAACAGAGAAAAGAUCGAAAAGAUCCUGACAUUCAGAAUCCCGUACUACGU<br>CGGACCGCUGGCAAGAGGAAACAGCAGAUUCGCAUGGAUGACAAGAAAGAGC<br>GAAGAAACAAUCACACCGUGGAACUUCGAAGAAGUCGUCGACAAGGGAGCAA<br>GCGCACAGAGCUUCAUCGAAAGAAUGACAAACUUCGACAAGAACCUGCCGAA<br>CGAAAAGGUCCUGCCGAAGCACAGCCUGCUGUACGAAUACUUCACAGUCUAC<br>AACGAACUGACAAAGGUCAAGUACGUCACAGAAGGAAUGAGAAAGCCGGCAU<br>UCCUGAGCGGAGAACAGAAGAAGGCAAUCGUCGACCUGCUGUUCAAGACAAA<br>CAGAAAGGUCACAGUCAAGCAGCUGAAGGAAGACUACUUCAAGAAGAUCGAA<br>UGCUUCGACAGCGUCGAAAUCAGCGGAGUCGAAGACAGAUUCAACGCAAGCC<br>UGGGAACAUACCACGACCUGCUGAAGAUCAUCAAGGACAAGGACUUCCUGGA<br>CAACGAAGAAAACGAAGACAUCCUGGAAGACAUCGUCCUGACACUGACACUG<br>UUCGAAGACAGAGAAAUGAUCGAAGAAAGACUGAAGACAUACGCACACCUGU<br>UCGACGACAAGGUCAUGAAGCAGCUGAAGAGAAGAAGAUACACAGGAUGGGG<br>AAGACUGAGCAGAAAGCUGAUCAACGGAAUCAGAGACAAGCAGAGCGGAAAG<br>ACAAUCCUGGACUUCCUGAAGAGCGACGGAUUCGCAAACAGAAACUUCAUGC<br>AGCUGAUCCACGACGACAGCCUGACAUUCAAGGAAGACAUCCAGAAGGCACA<br>GGUCAGCGGACAGGGAGACAGCCUGCACGAACACAUCGCAAACCUGGCAGGA<br>AGCCCGGCAAUCAAGAAGGGAAUCCUGCAGACAGUCAAGGUCGUCGACGAAC<br>UGGUCAAGGUCAUGGGAAGACACAAGCCGGAAAACAUCGUCAUCGAAAUGGC<br>AAGAGAAAACCAGACAACACAGAAGGGACAGAAGAACAGCAGAGAAAGAAUG<br>AAGAGAAUCGAAGAAGGAAUCAAGGAACUGGGAAGCCAGAUCCUGAAGGAAC<br>ACCCGGUCGAAAACACACAGCUGCAGAACGAAAAGCUGUACCUGUACUACCU<br>GCAGAACGGAAGAGACAUGUACGUCGACCAGGAACUGGACAUCAACAGACUG<br>AGCGACUACGACGUCGACCACAUCGUCCCGCAGAGCUUCCUGAAGGACGACA<br>GCAUCGACAACAAGGUCCUGACAAGAAGCGACAAGAACAGAGGAAAGAGCGA<br>CAACGUCCCGAGCGAAGAAGUCGUCAAGAAGAUGAAGAACUACUGGAGACAG<br>CUGCUGAACGCAAAGCUGAUCACACAGAGAAAGUUCGACAACCUGACAAAGG<br>CAGAGAGAGGAGGACUGAGCGAACUGGACAAGGCAGGAUUCAUCAAGAGACA<br>GCUGGUCGAAACAAGACAGAUCACAAAGCACGUCGCACAGAUCCUGGACAGC<br>AGAAUGAACACAAAGUACGACGAAAACGACAAGCUGAUCAGAGAAGUCAAGG<br>UCAUCACACUGAAGAGCAAGCUGGUCAGCGACUUCAGAAAGGACUUCCAGUU<br>CUACAAGGUCAGAGAAAUCAACAACUACCACCACGCACACGACGCAUACCUG<br>AACGCAGUCGUCGGAACAGCACUGAUCAAGAAGUACCCGAAGCUGGAAAGCG<br>AAUUCGUCUACGGAGACUACAAGGUCUACGACGUCAGAAAGAUGAUCGCAAA<br>GAGCGAACAGGAAAUCGGAAAGGCAACAGCAAAGUACUUCUUCUACAGCAAC<br>AUCAUGAACUUCUUCAAGACAGAAAUCACACUGGCAAACGGAGAAAUCAGAA<br>AGAGACCGCUGAUCGAAACAAACGGAGAAACAGGAGAAAUCGUCUGGGACAA<br>GGGAAGAGACUUCGCAACAGUCAGAAAGGUCCUGAGCAUGCCGCAGGUCAAC<br>AUCGUCAAGAAGACAGAAGUCCAGACAGGAGGAUUCAGCAAGGAAAGCAUCC<br>UGCCGAAGAGAAACAGCGACAAGCUGAUCGCAAGAAAGAAGGACUGGGACCC<br>GAAGAAGUACGGAGGAUUCGACAGCCCGACAGUCGCAUACAGCGUCCUGGUC<br>GUCGCAAAGGUCGAAAAGGGAAAGAGCAAGAAGCUGAAGAGCGUCAAGGAAC<br>UGCUGGGAAUCACAAUCAUGGAAAGAAGCAGCUUCGAAAAGAACCCGAUCGA | |

(continued)

| Description | Sequence | SEQ ID No. |
|---|---|---|
| | CUUCCUGGAAGCAAAGGGAUACAAGGAAGUCAAGAAGGACCUGAUCAUCAAG<br>CUGCCGAAGUACAGCCUGUUCGAACUGGAAAACGGAAGAAAGAGAAUGCUGG<br>CAAGCGCAGGAGAACUGCAGAAGGGAAACGAACUGGCACUGCCGAGCAAGUA<br>CGUCAACUUCCUGUACCUGGCAAGCCACUACGAAAAGCUGAAGGGAAGCCCG<br>GAAGACAACGAACAGAAGCAGCUGUUCGUCGAACAGCACAAGCACUACCUGG<br>ACGAAAUCAUCGAACAGAUCAGCGAAUUCAGCAAGAGAGUCAUCCUGGCAGA<br>CGCAAACCUGGACAAGGUCCUGAGCGCAUACAACAAGCACAGAGACAAGCCG<br>AUCAGAGAACAGGCAGAAAACAUCAUCCACCUGUUCACACUGACAAACCUGG<br>GAGCACCGGCAGCAUUCAAGUACUUCGACACAACAAUCGACAGAAAGAGAUA<br>CACAAGCACAAAGGAAGUCCUGGACGCAACACUGAUCCACCAGAGCAUCACA<br>GGACUGUACGAAACAAGAAUCGACCUGAGCCAGCUGGGAGGAGAC | |
| **Amino acid sequence of Cas9 nickase (without NLS)** | MDKKYSIGLAIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT<br>LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDHIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGD | **16** |

(continued)

| Description | Sequence | SEQ ID No. |
|---|---|---|
| **Cas9 nickase mRNA ORF encoding SEQ ID NO: 16 using minimal uridine codons as listed in Table 1, with start and stop codons** | AUGGACAAGAAGUACAGCAUCGGACUGGCAAUCGGAACAAACAGCGUCGGAU GGGCAGUCAUCACAGACGAAUACAAGGUCCCGAGCAAGAAGUUCAAGGUCCU GGGAAACACAGACAGACACAGCAUCAAGAAGAACCUGAUCGGAGCACUGCUG UUCGACAGCGGAGAAACAGCAGAAGCAACAAGACUGAAGAGAACAGCAAGAA GAAGAUACACAAGAAGAAAGAACAGAAUCUGCUACCUGCAGGAAAUCUUCAG CAACGAAAUGGCAAAGGUCGACGACAGCUUCUUCCACAGACUGGAAGAAAGC UUCCUGGUCGAAGAAGACAAGAAGCACGAAAGACACCCGAUCUUCGGAAACA UCGUCGACGAAGUCGCAUACCACGAAAAGUACCCGACAAUCUACCACCUGAG AAAGAAGCUGGUCGACAGCACAGACAAGGCAGACCUGAGACUGAUCUACCUG GCACUGGCACACAUGAUCAAGUUCAGAGGACACUUCCUGAUCGAAGGAGACC UGAACCCGGACAACAGCGACGUCGACAAGCUGUUCAUCCAGCUGGUCCAGAC AUACAACCAGCUGUUCGAAGAAAACCCGAUCAACGCAAGCGGAGUCGACGCA AAGGCAAUCCUGAGCGCAAGACUGAGCAAGAGCAGAAGACUGGAAAACCUGA UCGCACAGCUGCCGGGAGAAAAGAAGAACGGACUGUUCGGAAACCUGAUCGC ACUGAGCCUGGGACUGACACCGAACUUCAAGAGCAACUUCGACCUGGCAGAA GACGCAAAGCUGCAGCUGAGCAAGGACACAUACGACGACGACCUGGACAACC UGCUGGCACAGAUCGGAGACCAGUACGCAGACCUGUUCCUGGCAGCAAAGAA CCUGAGCGACGCAAUCCUGCUGAGCGACAUCCUGAGAGUCAACACAGAAAUC | **17** |

(continued)

| Description | Sequence | SEQ ID No. |
|---|---|---|
| | ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC<br>GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACCACAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA<br>GCGAAUUCGUCUACGGAGACUACAAGGUCUACGACGUCAGAAAGAUGAUCGC<br>AAAGAGCGAACAGGAAAUCGGAAAGGCAACAGCAAAGUACUUCUUCUACAGC<br>AACAUCAUGAACUUCUUCAAGACAGAAAUCACACUGGCAAACGGAGAAAUCA<br>GAAAGAGACCGCUGAUCGAAACAAACGGAGAAACAGGAGAAAUCGUCUGGGA<br>CAAGGGAAGAGACUUCGCAACAGUCAGAAAGGUCCUGAGCAUGCCGCAGGUC<br>AACAUCGUCAAGAAGACAGAAGUCCAGACAGGAGGAUUCAGCAAGGAAAGCA<br>UCCUGCCGAAGAGAAACAGCGACAAGCUGAUCGCAAGAAAGAAGGACUGGGA<br>CCCGAAGAAGUACGGAGGAUUCGACAGCCCGACAGUCGCAUACAGCGUCCUG<br>GUCGUCGCAAAGGUCGAAAAGGGAAAGAGCAAGAAGCUGAAGAGCGUCAAGG<br>AACUGCUGGGAAUCACAAUCAUGGAAAGAAGCAGCUUCGAAAAGAACCCGAU<br>CGACUUCCUGGAAGCAAAGGGAUACAAGGAAGUCAAGAAGGACCUGAUCAUC<br>AAGCUGCCGAAGUACAGCCUGUUCGAACUGGAAAACGGAAGAAAGAGAAUGC<br>UGGCAAGCGCAGGAGAACUGCAGAAGGGAAACGAACUGGCACUGCCGAGCAA<br>GUACGUCAACUUCCUGUACCUGGCAAGCCACUACGAAAAGCUGAAGGGAAGC<br>CCGGAAGACAACGAACAGAAGCAGCUGUUCGUCGAACAGCACAAGCACUACC<br>UGGACGAAAUCAUCGAACAGAUCAGCGAAUUCAGCAAGAGAGUCAUCCUGGC<br>AGACGCAAACCUGGACAAGGUCCUGAGCGCAUACAACAAGCACAGAGACAAG<br>CCGAUCAGAGAACAGGCAGAAAACAUCAUCCACCUGUUCACACUGACAAACC<br>UGGGAGCACCGGCAGCAUUCAAGUACUUCGACACAACAAUCGACAGAAAGAG | |

(continued)

| Description | Sequence | SEQ ID No. |
|---|---|---|
| | AUACACAAGCACAAAGGAAGUCCUGGACGCAACACUGAUCCACCAGAGCAUC ACAGGACUGUACGAAACAAGAAUCGACCUGAGCCAGCUGGGAGGAGACUAG | |

(continued)

| Description | Sequence | SEQ ID No. |
|---|---|---|
| **Cas9 nickase coding sequence encoding SEQ ID NO: 16 using minimal uridine codons as listed in Table 1 (no start or stop codons; suitable for inclusion in fusion protein coding sequence)** | GACAAGAAGUACAGCAUCGGACUGGCAAUCGGAACAAACAGCGUCGGAUGGG<br>CAGUCAUCACAGACGAAUACAAGGUCCCGAGCAAGAAGUUCAAGGUCCUGGG<br>AAACACAGACAGACACAGCAUCAAGAAGAACCUGAUCGGAGCACUGCUGUUC<br>GACAGCGGAGAAACAGCAGAAGCAACAAGACUGAAGAGAACAGCAAGAAGAA<br>GAUACACAAGAAGAAAGAACAGAAUCUGCUACCUGCAGGAAAUCUUCAGCAA<br>CGAAAUGGCAAAGGUCGACGACAGCUUCUUCCACAGACUGGAAGAAAGCUUC<br>CUGGUCGAAGAAGACAAGAAGCACGAAAGACACCCGAUCUUCGGAAACAUCG<br>UCGACGAAGUCGCAUACCACGAAAAGUACCCGACAAUCUACCACCUGAGAAA<br>GAAGCUGGUCGACAGCACAGACAAGGCAGACCUGAGACUGAUCUACCUGGCA<br>CUGGCACACAUGAUCAAGUUCAGAGGACACUUCCUGAUCGAAGGAGACCUGA<br>ACCCGGACAACAGCGACGUCGACAAGCUGUUCAUCCAGCUGGUCCAGACAUA<br>CAACCAGCUGUUCGAAGAAAACCCGAUCAACGCAAGCGGAGUCGACGCAAAG<br>GCAAUCCUGAGCGCAAGACUGAGCAAGAGCAGAAGACUGGAAAACCUGAUCG<br>CACAGCUGCCGGGAGAAAAGAAGAACGGACUGUUCGGAAACCUGAUCGCACU<br>GAGCCUGGGACUGACACCGAACUUCAAGAGCAACUUCGACCUGGCAGAAGAC<br>GCAAAGCUGCAGCUGAGCAAGGACACAUACGACGACGACCUGGACAACCUGC<br>UGGCACAGAUCGGAGACCAGUACGCAGACCUGUUCCUGGCAGCAAAGAACCU<br>GAGCGACGCAAUCCUGCUGAGCGACAUCCUGAGAGUCAACACAGAAAUCACA<br>AAGGCACCGCUGAGCGCAAGCAUGAUCAAGAGAUACGACGAACACCACCAGG<br>ACCUGACACUGCUGAAGGCACUGGUCAGACAGCAGCUGCCGGAAAAGUACAA<br>GGAAAUCUUCUUCGACCAGAGCAAGAACGGAUACGCAGGAUACAUCGACGGA<br>GGAGCAAGCCAGGAAGAAUUCUACAAGUUCAUCAAGCCGAUCCUGGAAAAGA<br>UGGACGGAACAGAAGAACUGCUGGUCAAGCUGAACAGAGAAGACCUGCUGAG<br>AAAGCAGAGAACAUUCGACAACGGAAGCAUCCCGCACCAGAUCCACCUGGGA<br>GAACUGCACGCAAUCCUGAGAAGACAGGAAGACUUCUACCCGUUCCUGAAGG<br>ACAACAGAGAAAAGAUCGAAAAGAUCCUGACAUUCAGAAUCCCGUACUACGU<br>CGGACCGCUGGCAAGAGGAAACAGCAGAUUCGCAUGGAUGACAAGAAAGAGC<br>GAAGAAACAAUCACACCGUGGAACUUCGAAGAAGUCGUCGACAAGGGAGCAA<br>GCGCACAGAGCUUCAUCGAAAGAAUGACAAACUUCGACAAGAACCUGCCGAA<br>CGAAAAGGUCCUGCCGAAGCACAGCCUGCUGUACGAAUACUUCACAGUCUAC<br>AACGAACUGACAAAGGUCAAGUACGUCACAGAAGGAAUGAGAAAGCCGGCAU<br>UCCUGAGCGGAGAACAGAAGAAGGCAAUCGUCGACCUGCUGUUCAAGACAAA<br>CAGAAAGGUCACAGUCAAGCAGCUGAAGGAAGACUACUUCAAGAAGAUCGAA<br>UGCUUCGACAGCGUCGAAAUCAGCGGAGUCGAAGACAGAUUCAACGCAAGCC<br>UGGGAACAUACCACGACCUGCUGAAGAUCAUCAAGGACAAGGACUUCCUGGA<br>CAACGAAGAAAACGAAGACAUCCUGGAAGACAUCGUCCUGACACUGACACUG<br>UUCGAAGACAGAGAAAUGAUCGAAGAAAGACUGAAGACAUACGCACACCUGU<br>UCGACGACAAGGUCAUGAAGCAGCUGAAGAGAAGAAGAUACACAGGAUGGGG<br>AAGACUGAGCAGAAAGCUGAUCAACGGAAUCAGAGACAAGCAGAGCGGAAAG<br>ACAAUCCUGGACUUCCUGAAGAGCGACGGAUUCGCAAACAGAAACUUCAUGC<br>AGCUGAUCCACGACGACAGCCUGACAUUCAAGGAAGACAUCCAGAAGGCACA<br>GGUCAGCGGACAGGGAGACAGCCUGCACGAACACAUCGCAAACCUGGCAGGA<br>AGCCCGGCAAUCAAGAAGGGAAUCCUGCAGACAGUCAAGGUCGUCGACGAAC<br>UGGUCAAGGUCAUGGGAAGACACAAGCCGGAAAACAUCGUCAUCGAAAUGGC<br>AAGAGAAAACCAGACAACACAGAAGGGACAGAAGAACAGCAGAGAAAGAAUG<br>AAGAGAAUCGAAGAAGGAAUCAAGGAACUGGGAAGCCAGAUCCUGAAGGAAC<br>ACCCGGUCGAAAACACACAGCUGCAGAACGAAAAGCUGUACCUGUACUACCU<br>GCAGAACGGAAGAGACAUGUACGUCGACCAGGAACUGGACAUCAACAGACUG<br>AGCGACUACGACGUCGACCACAUCGUCCCGCAGAGCUUCCUGAAGGACGACA<br>GCAUCGACAACAAGGUCCUGACAAGAAGCGACAAGAACAGAGGAAAGAGCGA<br>CAACGUCCCGAGCGAAGAAGUCGUCAAGAAGAUGAAGAACUACUGGAGACAG<br>CUGCUGAACGCAAAGCUGAUCACACAGAGAAAGUUCGACAACCUGACAAAGG<br>CAGAGAGAGGAGGACUGAGCGAACUGGACAAGGCAGGAUUCAUCAAGAGACA<br>GCUGGUCGAAACAAGACAGAUCACAAAGCACGUCGCACAGAUCCUGGACAGC<br>AGAAUGAACACAAAGUACGACGAAAACGACAAGCUGAUCAGAGAAGUCAAGG<br>UCAUCACACUGAAGAGCAAGCUGGUCAGCGACUUCAGAAAGGACUUCCAGUU<br>CUACAAGGUCAGAGAAAUCAACAACUACCACCACGCACACGACGCAUACCUG | **18** |

(continued)

| Description | Sequence | SEQ ID No. |
|---|---|---|
| | AACGCAGUCGUCGGAACAGCACUGAUCAAGAAGUACCCGAAGCUGGAAAGCG<br>AAUUCGUCUACGGAGACUACAAGGUCUACGACGUCAGAAAGAUGAUCGCAAA<br>GAGCGAACAGGAAAUCGGAAAGGCAACAGCAAAGUACUUCUUCUACAGCAAC<br>AUCAUGAACUUCUUCAAGACAGAAAUCACACUGGCAAACGGAGAAAUCAGAA<br>AGAGACCGCUGAUCGAAACAAACGGAGAAACAGGAGAAAUCGUCUGGGACAA<br>GGGAAGAGACUUCGCAACAGUCAGAAAGGUCCUGAGCAUGCCGCAGGUCAAC<br>AUCGUCAAGAAGACAGAAGUCCAGACAGGAGGAUUCAGCAAGGAAAGCAUCC<br>UGCCGAAGAGAAACAGCGACAAGCUGAUCGCAAGAAAGAAGGACUGGGACCC<br>GAAGAAGUACGGAGGAUUCGACAGCCCGACAGUCGCAUACAGCGUCCUGGUC<br>GUCGCAAAGGUCGAAAAGGGAAAGAGCAAGAAGCUGAAGAGCGUCAAGGAAC<br>UGCUGGGAAUCACAAUCAUGGAAAGAAGCAGCUUCGAAAAGAACCCGAUCGA<br>CUUCCUGGAAGCAAAGGGAUACAAGGAAGUCAAGAAGGACCUGAUCAUCAAG<br>CUGCCGAAGUACAGCCUGUUCGAACUGGAAAACGGAAGAAAGAGAAUGCUGG<br>CAAGCGCAGGAGAACUGCAGAAGGGAAACGAACUGGCACUGCCGAGCAAGUA<br>CGUCAACUUCCUGUACCUGGCAAGCCACUACGAAAAGCUGAAGGGAAGCCCG<br>GAAGACAACGAACAGAAGCAGCUGUUCGUCGAACAGCACAAGCACUACCUGG<br>ACGAAAUCAUCGAACAGAUCAGCGAAUUCAGCAAGAGAGUCAUCCUGGCAGA<br>CGCAAACCUGGACAAGGUCCUGAGCGCAUACAACAAGCACAGAGACAAGCCG<br>AUCAGAGAACAGGCAGAAAACAUCAUCCACCUGUUCACACUGACAAACCUGG<br>GAGCACCGGCAGCAUUCAAGUACUUCGACACAACAAUCGACAGAAAGAGAUA<br>CACAAGCACAAAGGAAGUCCUGGACGCAACACUGAUCCACCAGAGCAUCACA<br>GGACUGUACGAAACAAGAAUCGACCUGAGCCAGCUGGGAGGAGAC | |
| **Amino acid sequence of dCas9 (without NLS)** | MDKKYSIGLAIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT<br>LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDAIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGD | **19** |

(continued)

| Description | Sequence | SEQ ID No. |
|---|---|---|
| **dCas9 mRNA ORF encoding SEQ ID NO: 19 using minimal uridine codons as listed in** | AUGGACAAGAAGUACAGCAUCGGACUGGCAAUCGGAACAAACAGCGUCGGAU GGGCAGUCAUCACAGACGAAUACAAGGUCCCGAGCAAGAAGUUCAAGGUCCU GGGAAACACAGACAGACACAGCAUCAAGAAGAACCUGAUCGGAGCACUGCUG UUCGACAGCGGAGAAACAGCAGAAGCAACAAGACUGAAGAGAACAGCAAGAA GAAGAUACACAAGAAGAAAGAACAGAAUCUGCUACCUGCAGGAAAUCUUCAG CAACGAAAUGGCAAAGGUCGACGACAGCUUCUUCCACAGACUGGAAGAAAGC UUCCUGGUCGAAGAAGACAAGAAGCACGAAAGACACCCGAUCUUCGGAAACA UCGUCGACGAAGUCGCAUACCACGAAAAGUACCCGACAAUCUACCACCUGAG | **20** |

(continued)

| Description | Sequence | SEQ ID No. |
|---|---|---|
| **Table 1, with start and stop codons** | AAAGAAGCUGGUCGACAGCACAGACAAGGCAGACCUGAGACUGAUCUACCUG<br>GCACUGGCACACAUGAUCAAGUUCAGAGGACACUUCCUGAUCGAAGGAGACC<br>UGAACCCGGACAACAGCGACGUCGACAAGCUGUUCAUCCAGCUGGUCCAGAC<br>AUACAACCAGCUGUUCGAAGAAAACCCGAUCAACGCAAGCGGAGUCGACGCA<br>AAGGCAAUCCUGAGCGCAAGACUGAGCAAGAGCAGAAGACUGGAAAACCUGA<br>UCGCACAGCUGCCGGGAGAAAAGAAGAACGGACUGUUCGGAAACCUGAUCGC<br>ACUGAGCCUGGGACUGACACCGAACUUCAAGAGCAACUUCGACCUGGCAGAA<br>GACGCAAAGCUGCAGCUGAGCAAGGACACAUACGACGACGACCUGGACAACC<br>UGCUGGCACAGAUCGGAGACCAGUACGCAGACCUGUUCCUGGCAGCAAAGAA<br>CCUGAGCGACGCAAUCCUGCUGAGCGACAUCCUGAGAGUCAACACAGAAAUC<br>ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC<br>GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACGCAAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA<br>GCGAAUUCGUCUACGGAGACUACAAGGUCUACGACGUCAGAAAGAUGAUCGC<br>AAAGAGCGAACAGGAAAUCGGAAAGGCAACAGCAAAGUACUUCUUCUACAGC<br>AACAUCAUGAACUUCUUCAAGACAGAAAUCACACUGGCAAACGGAGAAAUCA<br>GAAAGAGACCGCUGAUCGAAACAAACGGAGAAACAGGAGAAAUCGUCUGGGA<br>CAAGGGAAGAGACUUCGCAACAGUCAGAAAGGUCCUGAGCAUGCCGCAGGUC<br>AACAUCGUCAAGAAGACAGAAGUCCAGACAGGAGGAUUCAGCAAGGAAAGCA<br>UCCUGCCGAAGAGAAACAGCGACAAGCUGAUCGCAAGAAAGAAGGACUGGGA<br>CCCGAAGAAGUACGGAGGAUUCGACAGCCCGACAGUCGCAUACAGCGUCCUG<br>GUCGUCGCAAAGGUCGAAAAGGGAAAGAGCAAGAAGCUGAAGAGCGUCAAGG<br>AACUGCUGGGAAUCACAAUCAUGGAAAGAAGCAGCUUCGAAAAGAACCCGAU | |

(continued)

| Description | Sequence | SEQ ID No. |
|---|---|---|
| | CGACUUCCUGGAAGCAAAGGGAUACAAGGAAGUCAAGAAGGACCUGAUCAUC AAGCUGCCGAAGUACAGCCUGUUCGAACUGGAAAACGGAAGAAAGAGAAUGC UGGCAAGCGCAGGAGAACUGCAGAAGGGAAACGAACUGGCACUGCCGAGCAA GUACGUCAACUUCCUGUACCUGGCAAGCCACUACGAAAAGCUGAAGGGAAGC CCGGAAGACAACGAACAGAAGCAGCUGUUCGUCGAACAGCACAAGCACUACC UGGACGAAAUCAUCGAACAGAUCAGCGAAUUCAGCAAGAGAGUCAUCCUGGC AGACGCAAACCUGGACAAGGUCCUGAGCGCAUACAACAAGCACAGAGACAAG CCGAUCAGAGAACAGGCAGAAAACAUCAUCCACCUGUUCACACUGACAAACC UGGGAGCACCGGCAGCAUUCAAGUACUUCGACACAACAAUCGACAGAAAGAG AUACACAAGCACAAAGGAAGUCCUGGACGCAACACUGAUCCACCAGAGCAUC ACAGGACUGUACGAAACAAGAAUCGACCUGAGCCAGCUGGGAGGAGACUAG | |

(continued)

| Description | Sequence | SEQ ID No. |
|---|---|---|
| **dCas9 coding sequence encoding SEQ ID NO: 19 using minimal uridine codons as listed in Table 1 (no start or stop codons; suitable for inclusion in fusion protein coding sequence)** | GACAAGAAGUACAGCAUCGGACUGGCAAUCGGAACAAACAGCGUCGGAUGGG<br>CAGUCAUCACAGACGAAUACAAGGUCCCGAGCAAGAAGUUCAAGGUCCUGGG<br>AAACACAGACAGACACAGCAUCAAGAAGAACCUGAUCGGAGCACUGCUGUUC<br>GACAGCGGAGAAACAGCAGAAGCAACAAGACUGAAGAGAACAGCAAGAAGAA<br>GAUACACAAGAAGAAAGAACAGAAUCUGCUACCUGCAGGAAAUCUUCAGCAA<br>CGAAAUGGCAAAGGUCGACGACAGCUUCUUCCACAGACUGGAAGAAAGCUUC<br>CUGGUCGAAGAAGACAAGAAGCACGAAAGACACCCGAUCUUCGGAAACAUCG<br>UCGACGAAGUCGCAUACCACGAAAAGUACCCGACAAUCUACCACCUGAGAAA<br>GAAGCUGGUCGACAGCACAGACAAGGCAGACCUGAGACUGAUCUACCUGGCA<br>CUGGCACACAUGAUCAAGUUCAGAGGACACUUCCUGAUCGAAGGAGACCUGA<br>ACCCGGACAACAGCGACGUCGACAAGCUGUUCAUCCAGCUGGUCCAGACAUA<br>CAACCAGCUGUUCGAAGAAAACCCGAUCAACGCAAGCGGAGUCGACGCAAAG<br>GCAAUCCUGAGCGCAAGACUGAGCAAGAGCAGAAGACUGGAAAACCUGAUCG<br>CACAGCUGCCGGGAGAAAAGAAGAACGGACUGUUCGGAAACCUGAUCGCACU<br>GAGCCUGGGACUGACACCGAACUUCAAGAGCAACUUCGACCUGGCAGAAGAC<br>GCAAAGCUGCAGCUGAGCAAGGACACAUACGACGACGACCUGGACAACCUGC<br>UGGCACAGAUCGGAGACCAGUACGCAGACCUGUUCCUGGCAGCAAAGAACCU<br>GAGCGACGCAAUCCUGCUGAGCGACAUCCUGAGAGUCAACACAGAAAUCACA<br>AAGGCACCGCUGAGCGCAAGCAUGAUCAAGAGAUACGACGAACACCACCAGG<br>ACCUGACACUGCUGAAGGCACUGGUCAGACAGCAGCUGCCGGAAAAGUACAA<br>GGAAAUCUUCUUCGACCAGAGCAAGAACGGAUACGCAGGAUACAUCGACGGA<br>GGAGCAAGCCAGGAAGAAUUCUACAAGUUCAUCAAGCCGAUCCUGGAAAAGA<br>UGGACGGAACAGAAGAACUGCUGGUCAAGCUGAACAGAGAAGACCUGCUGAG<br>AAAGCAGAGAACAUUCGACAACGGAAGCAUCCCGCACCAGAUCCACCUGGGA<br>GAACUGCACGCAAUCCUGAGAAGACAGGAAGACUUCUACCCGUUCCUGAAGG<br>ACAACAGAGAAAAGAUCGAAAAGAUCCUGACAUUCAGAAUCCCGUACUACGU<br>CGGACCGCUGGCAAGAGGAAACAGCAGAUUCGCAUGGAUGACAAGAAAGAGC<br>GAAGAAACAAUCACACCGUGGAACUUCGAAGAAGUCGUCGACAAGGGAGCAA<br>GCGCACAGAGCUUCAUCGAAAGAAUGACAAACUUCGACAAGAACCUGCCGAA<br>CGAAAAGGUCCUGCCGAAGCACAGCCUGCUGUACGAAUACUUCACAGUCUAC<br>AACGAACUGACAAAGGUCAAGUACGUCACAGAAGGAAUGAGAAAGCCGGCAU<br>UCCUGAGCGGAGAACAGAAGAAGGCAAUCGUCGACCUGCUGUUCAAGACAAA<br>CAGAAAGGUCACAGUCAAGCAGCUGAAGGAAGACUACUUCAAGAAGAUCGAA<br>UGCUUCGACAGCGUCGAAAUCAGCGGAGUCGAAGACAGAUUCAACGCAAGCC<br>UGGGAACAUACCACGACCUGCUGAAGAUCAUCAAGGACAAGGACUUCCUGGA<br>CAACGAAGAAAACGAAGACAUCCUGGAAGACAUCGUCCUGACACUGACACUG<br>UUCGAAGACAGAGAAAUGAUCGAAGAAAGACUGAAGACAUACGCACACCUGU<br>UCGACGACAAGGUCAUGAAGCAGCUGAAGAGAAGAAGAUACACAGGAUGGGG<br>AAGACUGAGCAGAAAGCUGAUCAACGGAAUCAGAGACAAGCAGAGCGGAAAG<br>ACAAUCCUGGACUUCCUGAAGAGCGACGGAUUCGCAAACAGAAACUUCAUGC<br>AGCUGAUCCACGACGACAGCCUGACAUUCAAGGAAGACAUCCAGAAGGCACA<br>GGUCAGCGGACAGGGAGACAGCCUGCACGAACACAUCGCAAACCUGGCAGGA<br>AGCCCGGCAAUCAAGAAGGGAAUCCUGCAGACAGUCAAGGUCGUCGACGAAC<br>UGGUCAAGGUCAUGGGAAGACACAAGCCGGAAAACAUCGUCAUCGAAAUGGC<br>AAGAGAAAACCAGACAACACAGAAGGGACAGAAGAACAGCAGAGAAAGAAUG<br>AAGAGAAUCGAAGAAGGAAUCAAGGAACUGGGAAGCCAGAUCCUGAAGGAAC<br>ACCCGGUCGAAAACACACAGCUGCAGAACGAAAAGCUGUACCUGUACUACCU<br>GCAGAACGGAAGAGACAUGUACGUCGACCAGGAACUGGACAUCAACAGACUG | **21** |

(continued)

| Description | Sequence | SEQ ID No. |
|---|---|---|
| | AGCGACUACGACGUCGACGCAAUCGUCCCGCAGAGCUUCCUGAAGGACGACA GCAUCGACAACAAGGUCCUGACAAGAAGCGACAAGAACAGAGGAAAGAGCGA CAACGUCCCGAGCGAAGAAGUCGUCAAGAAGAUGAAGAACUACUGGAGACAG CUGCUGAACGCAAAGCUGAUCACACAGAGAAAGUUCGACAACCUGACAAAGG CAGAGAGAGGAGGACUGAGCGAACUGGACAAGGCAGGAUUCAUCAAGAGACA GCUGGUCGAAACAAGACAGAUCACAAAGCACGUCGCACAGAUCCUGGACAGC AGAAUGAACACAAAGUACGACGAAAACGACAAGCUGAUCAGAGAAGUCAAGG UCAUCACACUGAAGAGCAAGCUGGUCAGCGACUUCAGAAAGGACUUCCAGUU CUACAAGGUCAGAGAAAUCAACAACUACCACCACGCACACGACGCAUACCUG AACGCAGUCGUCGGAACAGCACUGAUCAAGAAGUACCCGAAGCUGGAAAGCG AAUUCGUCUACGGAGACUACAAGGUCUACGACGUCAGAAAGAUGAUCGCAAA GAGCGAACAGGAAAUCGGAAAGGCAACAGCAAAGUACUUCUUCUACAGCAAC AUCAUGAACUUCUUCAAGACAGAAAUCACACUGGCAAACGGAGAAAUCAGAA AGAGACCGCUGAUCGAAACAAACGGAGAAACAGGAGAAAUCGUCUGGGACAA GGGAAGAGACUUCGCAACAGUCAGAAAGGUCCUGAGCAUGCCGCAGGUCAAC AUCGUCAAGAAGACAGAAGUCCAGACAGGAGGAUUCAGCAAGGAAAGCAUCC UGCCGAAGAGAAACAGCGACAAGCUGAUCGCAAGAAAGAAGGACUGGGACCC GAAGAAGUACGGAGGAUUCGACAGCCCGACAGUCGCAUACAGCGUCCUGGUC GUCGCAAAGGUCGAAAAGGGAAAGAGCAAGAAGCUGAAGAGCGUCAAGGAAC UGCUGGGAAUCACAAUCAUGGAAAGAAGCAGCUUCGAAAAGAACCCGAUCGA CUUCCUGGAAGCAAAGGGAUACAAGGAAGUCAAGAAGGACCUGAUCAUCAAG CUGCCGAAGUACAGCCUGUUCGAACUGGAAAACGGAAGAAAGAGAAUGCUGG CAAGCGCAGGAGAACUGCAGAAGGGAAACGAACUGGCACUGCCGAGCAAGUA CGUCAACUUCCUGUACCUGGCAAGCCACUACGAAAAGCUGAAGGGAAGCCCG GAAGACAACGAACAGAAGCAGCUGUUCGUCGAACAGCACAAGCACUACCUGG ACGAAAUCAUCGAACAGAUCAGCGAAUUCAGCAAGAGAGUCAUCCUGGCAGA CGCAAACCUGGACAAGGUCCUGAGCGCAUACAACAAGCACAGAGACAAGCCG AUCAGAGAACAGGCAGAAAACAUCAUCCACCUGUUCACACUGACAAACCUGG GAGCACCGGCAGCAUUCAAGUACUUCGACACAACAAUCGACAGAAAGAGAUA CACAAGCACAAAGGAAGUCCUGGACGCAACACUGAUCCACCAGAGCAUCACA GGACUGUACGAAACAAGAAUCGACCUGAGCCAGCUGGGAGGAGACGGAGGAG GAAGC | |

(continued)

| Description | Sequence | SEQ ID No. |
|---|---|---|
| **Amino acid sequence of Cas9 with two nuclear localization signals as the C-terminal amino acids** | MDKKYSIGLDIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT<br>LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDHIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGD GSGSPKKKRKVDGSPKKKRKVDSG | 22 |
| | | |

(continued)

| Description | Sequence | SEQ ID No. |
|---|---|---|
| Cas9 mRNA ORF encoding SEQ ID NO: 22 using minimal uridine codons as listed in Table 1, with start and stop codons | AUGGACAAGAAGUACAGCAUCGGACUGGACAUCGGAACAAACAGCGUCGGAU<br>GGGCAGUCAUCACAGACGAAUACAAGGUCCCGAGCAAGAAGUUCAAGGUCCU<br>GGGAAACACAGACAGACACAGCAUCAAGAAGAACCUGAUCGGAGCACUGCUG<br>UUCGACAGCGGAGAAACAGCAGAAGCAACAAGACUGAAGAGAACAGCAAGAA<br>GAAGAUACACAAGAAGAAAGAACAGAAUCUGCUACCUGCAGGAAAUCUUCAG<br>CAACGAAAUGGCAAAGGUCGACGACAGCUUCUUCCACAGACUGGAAGAAAGC<br>UUCCUGGUCGAAGAAGACAAGAAGCACGAAAGACACCCGAUCUUCGGAAACA<br>UCGUCGACGAAGUCGCAUACCACGAAAAGUACCCGACAAUCUACCACCUGAG<br>AAAGAAGCUGGUCGACAGCACAGACAAGGCAGACCUGAGACUGAUCUACCUG<br>GCACUGGCACACAUGAUCAAGUUCAGAGGACACUUCCUGAUCGAAGGAGACC<br>UGAACCCGGACAACAGCGACGUCGACAAGCUGUUCAUCCAGCUGGUCCAGAC<br>AUACAACCAGCUGUUCGAAGAAAACCCGAUCAACGCAAGCGGAGUCGACGCA<br>AAGGCAAUCCUGAGCGCAAGACUGAGCAAGAGCAGAAGACUGGAAAACCUGA<br>UCGCACAGCUGCCGGGAGAAAAGAAGAACGGACUGUUCGGAAACCUGAUCGC<br>ACUGAGCCUGGGACUGACACCGAACUUCAAGAGCAACUUCGACCUGGCAGAA<br>GACGCAAAGCUGCAGCUGAGCAAGGACACAUACGACGACGACCUGGACAACC<br>UGCUGGCACAGAUCGGAGACCAGUACGCAGACCUGUUCCUGGCAGCAAAGAA<br>CCUGAGCGACGCAAUCCUGCUGAGCGACAUCCUGAGAGUCAACACAGAAAUC<br>ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC<br>GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACCACAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA | 23 |

(continued)

| Description | Sequence | SEQ ID No. |
|---|---|---|
| | GCGAAUUCGUCUACGGAGACUACAAGGUCUACGACGUCAGAAAGAUGAUCGC AAAGAGCGAACAGGAAAUCGGAAAGGCAACAGCAAAGUACUUCUUCUACAGC AACAUCAUGAACUUCUUCAAGACAGAAAUCACACUGGCAAACGGAGAAAUCA GAAAGAGACCGCUGAUCGAAACAAACGGAGAAACAGGAGAAAUCGUCUGGGA CAAGGGAAGAGACUUCGCAACAGUCAGAAAGGUCCUGAGCAUGCCGCAGGUC AACAUCGUCAAGAAGACAGAAGUCCAGACAGGAGGAUUCAGCAAGGAAAGCA UCCUGCCGAAGAGAAACAGCGACAAGCUGAUCGCAAGAAAGAAGGACUGGGA CCCGAAGAAGUACGGAGGAUUCGACAGCCCGACAGUCGCAUACAGCGUCCUG GUCGUCGCAAAGGUCGAAAAGGGAAAGAGCAAGAAGCUGAAGAGCGUCAAGG AACUGCUGGGAAUCACAAUCAUGGAAAGAAGCAGCUUCGAAAAGAACCCGAU CGACUUCCUGGAAGCAAAGGGAUACAAGGAAGUCAAGAAGGACCUGAUCAUC AAGCUGCCGAAGUACAGCCUGUUCGAACUGGAAAACGGAAGAAAGAGAAUGC UGGCAAGCGCAGGAGAACUGCAGAAGGGAAACGAACUGGCACUGCCGAGCAA GUACGUCAACUUCCUGUACCUGGCAAGCCACUACGAAAAGCUGAAGGGAAGC CCGGAAGACAACGAACAGAAGCAGCUGUUCGUCGAACAGCACAAGCACUACC UGGACGAAAUCAUCGAACAGAUCAGCGAAUUCAGCAAGAGAGUCAUCCUGGC AGACGCAAACCUGGACAAGGUCCUGAGCGCAUACAACAAGCACAGAGACAAG CCGAUCAGAGAACAGGCAGAAAACAUCAUCCACCUGUUCACACUGACAAACC UGGGAGCACCGGCAGCAUUCAAGUACUUCGACACAACAAUCGACAGAAAGAG AUACACAAGCACAAAGGAAGUCCUGGACGCAACACUGAUCCACCAGAGCAUC ACAGGACUGUACGAAACAAGAAUCGACCUGAGCCAGCUGGGAGGAGACGGAA GCGGAAGCCCGAAGAAGAAGAGAAAGGUCGACGGAAGCCCGAAGAAGAAGAG AAAGGUCGACAGCGGAUAG | |

(continued)

| Description | Sequence | SEQ ID No. |
|---|---|---|
| **Cas9 coding sequence encoding SEQ ID NO: 23 using minimal uridine codons as listed in Table 1 (no start or stop codons; suitable for inclusion in fusion protein coding sequence)** | GACAAGAAGUACAGCAUCGGACUGGACAUCGGAACAAACAGCGUCGGAUGGG CAGUCAUCACAGACGAAUACAAGGUCCCGAGCAAGAAGUUCAAGGUCCUGGG AAACACAGACAGACACAGCAUCAAGAAGAACCUGAUCGGAGCACUGCUGUUC GACAGCGGAGAAACAGCAGAAGCAACAAGACUGAAGAGAACAGCAAGAAGAA GAUACACAAGAAGAAAGAACAGAAUCUGCUACCUGCAGGAAAUCUUCAGCAA CGAAAUGGCAAAGGUCGACGACAGCUUCUUCCACAGACUGGAAGAAAGCUUC CUGGUCGAAGAAGACAAGAAGCACGAAAGACACCCGAUCUUCGGAAACAUCG UCGACGAAGUCGCAUACCACGAAAAGUACCCGACAAUCUACCACCUGAGAAA GAAGCUGGUCGACAGCACAGACAAGGCAGACCUGAGACUGAUCUACCUGGCA CUGGCACACAUGAUCAAGUUCAGAGGACACUUCCUGAUCGAAGGAGACCUGA ACCCGGACAACAGCGACGUCGACAAGCUGUUCAUCCAGCUGGUCCAGACAUA CAACCAGCUGUUCGAAGAAAACCCGAUCAACGCAAGCGGAGUCGACGCAAAG GCAAUCCUGAGCGCAAGACUGAGCAAGAGCAGAAGACUGGAAAACCUGAUCG CACAGCUGCCGGGAGAAAAGAAGAACGGACUGUUCGGAAACCUGAUCGCACU GAGCCUGGGACUGACACCGAACUUCAAGAGCAACUUCGACCUGGCAGAAGAC GCAAAGCUGCAGCUGAGCAAGGACACAUACGACGACGACCUGGACAACCUGC UGGCACAGAUCGGAGACCAGUACGCAGACCUGUUCCUGGCAGCAAAGAACCU GAGCGACGCAAUCCUGCUGAGCGACAUCCUGAGAGUCAACACAGAAAUCACA AAGGCACCGCUGAGCGCAAGCAUGAUCAAGAGAUACGACGAACACCACCAGG ACCUGACACUGCUGAAGGCACUGGUCAGACAGCAGCUGCCGGAAAAGUACAA GGAAAUCUUCUUCGACCAGAGCAAGAACGGAUACGCAGGAUACAUCGACGGA GGAGCAAGCCAGGAAGAAUUCUACAAGUUCAUCAAGCCGAUCCUGGAAAAGA UGGACGGAACAGAAGAACUGCUGGUCAAGCUGAACAGAGAAGACCUGCUGAG AAAGCAGAGAACAUUCGACAACGGAAGCAUCCCGCACCAGAUCCACCUGGGA GAACUGCACGCAAUCCUGAGAAGACAGGAAGACUUCUACCCGUUCCUGAAGG ACAACAGAGAAAAGAUCGAAAAGAUCCUGACAUUCAGAAUCCCGUACUACGU CGGACCGCUGGCAAGAGGAAACAGCAGAUUCGCAUGGAUGACAAGAAAGAGC GAAGAAACAAUCACACCGUGGAACUUCGAAGAAGUCGUCGACAAGGGAGCAA GCGCACAGAGCUUCAUCGAAAGAAUGACAAACUUCGACAAGAACCUGCCGAA CGAAAAGGUCCUGCCGAAGCACAGCCUGCUGUACGAAUACUUCACAGUCUAC AACGAACUGACAAAGGUCAAGUACGUCACAGAAGGAAUGAGAAAGCCGGCAU UCCUGAGCGGAGAACAGAAGAAGGCAAUCGUCGACCUGCUGUUCAAGACAAA CAGAAAGGUCACAGUCAAGCAGCUGAAGGAAGACUACUUCAAGAAGAUCGAA UGCUUCGACAGCGUCGAAAUCAGCGGAGUCGAAGACAGAUUCAACGCAAGCC UGGGAACAUACCACGACCUGCUGAAGAUCAUCAAGGACAAGGACUUCCUGGA | **24** |

(continued)

| Description | Sequence | SEQ ID No. |
|---|---|---|
| | CAACGAAGAAAACGAAGACAUCCUGGAAGACAUCGUCCUGACACUGACACUG<br>UUCGAAGACAGAGAAAUGAUCGAAGAAAGACUGAAGACAUACGCACACCUGU<br>UCGACGACAAGGUCAUGAAGCAGCUGAAGAGAAGAAGAUACACAGGAUGGGG<br>AAGACUGAGCAGAAAGCUGAUCAACGGAAUCAGAGACAAGCAGAGCGGAAAG<br>ACAAUCCUGGACUUCCUGAAGAGCGACGGAUUCGCAAACAGAAACUUCAUGC<br>AGCUGAUCCACGACGACAGCCUGACAUUCAAGGAAGACAUCCAGAAGGCACA<br>GGUCAGCGGACAGGGAGACAGCCUGCACGAACACAUCGCAAACCUGGCAGGA<br>AGCCCGGCAAUCAAGAAGGGAAUCCUGCAGACAGUCAAGGUCGUCGACGAAC<br>UGGUCAAGGUCAUGGGAAGACACAAGCCGGAAAACAUCGUCAUCGAAAUGGC<br>AAGAGAAAACCAGACAACACAGAAGGGACAGAAGAACAGCAGAGAAAGAAUG<br>AAGAGAAUCGAAGAAGGAAUCAAGGAACUGGGAAGCCAGAUCCUGAAGGAAC<br>ACCCGGUCGAAAACACACAGCUGCAGAACGAAAAGCUGUACCUGUACUACCU<br>GCAGAACGGAAGAGACAUGUACGUCGACCAGGAACUGGACAUCAACAGACUG<br>AGCGACUACGACGUCGACCACAUCGUCCCGCAGAGCUUCCUGAAGGACGACA<br>GCAUCGACAACAAGGUCCUGACAAGAAGCGACAAGAACAGAGGAAAGAGCGA<br>CAACGUCCCGAGCGAAGAAGUCGUCAAGAAGAUGAAGAACUACUGGAGACAG<br>CUGCUGAACGCAAAGCUGAUCACACAGAGAAAGUUCGACAACCUGACAAAGG<br>CAGAGAGAGGAGGACUGAGCGAACUGGACAAGGCAGGAUUCAUCAAGAGACA<br>GCUGGUCGAAACAAGACAGAUCACAAAGCACGUCGCACAGAUCCUGGACAGC<br>AGAAUGAACACAAAGUACGACGAAAACGACAAGCUGAUCAGAGAAGUCAAGG<br>UCAUCACACUGAAGAGCAAGCUGGUCAGCGACUUCAGAAAGGACUUCCAGUU<br>CUACAAGGUCAGAGAAAUCAACAACUACCACCACGCACACGACGCAUACCUG<br>AACGCAGUCGUCGGAACAGCACUGAUCAAGAAGUACCCGAAGCUGGAAAGCG<br>AAUUCGUCUACGGAGACUACAAGGUCUACGACGUCAGAAAGAUGAUCGCAAA<br>GAGCGAACAGGAAAUCGGAAAGGCAACAGCAAAGUACUUCUUCUACAGCAAC<br>AUCAUGAACUUCUUCAAGACAGAAAUCACACUGGCAAACGGAGAAAUCAGAA<br>AGAGACCGCUGAUCGAAACAAACGGAGAAACAGGAGAAAUCGUCUGGGACAA<br>GGGAAGAGACUUCGCAACAGUCAGAAAGGUCCUGAGCAUGCCGCAGGUCAAC<br>AUCGUCAAGAAGACAGAAGUCCAGACAGGAGGAUUCAGCAAGGAAAGCAUCC<br>UGCCGAAGAGAAACAGCGACAAGCUGAUCGCAAGAAAGAAGGACUGGGACCC<br>GAAGAAGUACGGAGGAUUCGACAGCCCGACAGUCGCAUACAGCGUCCUGGUC<br>GUCGCAAAGGUCGAAAAGGGAAAGAGCAAGAAGCUGAAGAGCGUCAAGGAAC<br>UGCUGGGAAUCACAAUCAUGGAAAGAAGCAGCUUCGAAAAGAACCCGAUCGA<br>CUUCCUGGAAGCAAAGGGAUACAAGGAAGUCAAGAAGGACCUGAUCAUCAAG<br>CUGCCGAAGUACAGCCUGUUCGAACUGGAAAACGGAAGAAAGAGAAUGCUGG<br>CAAGCGCAGGAGAACUGCAGAAGGGAAACGAACUGGCACUGCCGAGCAAGUA<br>CGUCAACUUCCUGUACCUGGCAAGCCACUACGAAAAGCUGAAGGGAAGCCCG<br>GAAGACAACGAACAGAAGCAGCUGUUCGUCGAACAGCACAAGCACUACCUGG<br>ACGAAAUCAUCGAACAGAUCAGCGAAUUCAGCAAGAGAGUCAUCCUGGCAGA<br>CGCAAACCUGGACAAGGUCCUGAGCGCAUACAACAAGCACAGAGACAAGCCG<br>AUCAGAGAACAGGCAGAAAACAUCAUCCACCUGUUCACACUGACAAACCUGG<br>GAGCACCGGCAGCAUUCAAGUACUUCGACACAACAAUCGACAGAAAGAGAUA<br>CACAAGCACAAAGGAAGUCCUGGACGCAACACUGAUCCACCAGAGCAUCACA<br>GGACUGUACGAAACAAGAAUCGACCUGAGCCAGCUGGGAGGAGACGGAAGCG<br>GAAGCCCGAAGAAGAAGAGAAAGGUCGACGGAAGCCCGAAGAAGAAGAGAAA<br>GGUCGACAGCGGA | |

(continued)

| Description | Sequence | SEQ ID No. |
|---|---|---|
| **Amino acid sequence of Cas9 nickase with two nuclear localization signals as the C-terminal amino acids** | MDKKYSIGLAIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT | 25 |
| | LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDHIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGDGSGSPKKKRKVDGSPKKKRKVDSG | |

(continued)

| Description | Sequence | SEQ ID No. |
|---|---|---|
| **Cas9 nickase mRNA ORF encoding SEQ ID NO: 25 using minimal uridine codons as listed in Table 1, with start and stop codons** | AUGGACAAGAAGUACAGCAUCGGACUGGCAAUCGGAACAAACAGCGUCGGAU<br>GGGCAGUCAUCACAGACGAAUACAAGGUCCCGAGCAAGAAGUUCAAGGUCCU<br>GGGAAACACAGACAGACACAGCAUCAAGAAGAACCUGAUCGGAGCACUGCUG<br>UUCGACAGCGGAGAAACAGCAGAAGCAACAAGACUGAAGAGAACAGCAAGAA<br>GAAGAUACACAAGAAGAAAGAACAGAAUCUGCUACCUGCAGGAAAUCUUCAG<br>CAACGAAAUGGCAAAGGUCGACGACAGCUUCUUCCACAGACUGGAAGAAAGC<br>UUCCUGGUCGAAGAAGACAAGAAGCACGAAAGACACCCGAUCUUCGGAAACA<br>UCGUCGACGAAGUCGCAUACCACGAAAAGUACCCGACAAUCUACCACCUGAG<br>AAAGAAGCUGGUCGACAGCACAGACAAGGCAGACCUGAGACUGAUCUACCUG<br>GCACUGGCACACAUGAUCAAGUUCAGAGGACACUUCCUGAUCGAAGGAGACC<br>UGAACCCGGACAACAGCGACGUCGACAAGCUGUUCAUCCAGCUGGUCCAGAC<br>AUACAACCAGCUGUUCGAAGAAAACCCGAUCAACGCAAGCGGAGUCGACGCA<br>AAGGCAAUCCUGAGCGCAAGACUGAGCAAGAGCAGAAGACUGGAAAACCUGA<br>UCGCACAGCUGCCGGGAGAAAAGAAGAACGGACUGUUCGGAAACCUGAUCGC<br>ACUGAGCCUGGGACUGACACCGAACUUCAAGAGCAACUUCGACCUGGCAGAA<br>GACGCAAAGCUGCAGCUGAGCAAGGACACAUACGACGACGACCUGGACAACC<br>UGCUGGCACAGAUCGGAGACCAGUACGCAGACCUGUUCCUGGCAGCAAAGAA<br>CCUGAGCGACGCAAUCCUGCUGAGCGACAUCCUGAGAGUCAACACAGAAAUC<br>ACAAAGGCACCGCUGAGCGCAAGCAUGAUCAAGAGAUACGACGAACACCACC<br>AGGACCUGACACUGCUGAAGGCACUGGUCAGACAGCAGCUGCCGGAAAAGUA<br>CAAGGAAAUCUUCUUCGACCAGAGCAAGAACGGAUACGCAGGAUACAUCGAC<br>GGAGGAGCAAGCCAGGAAGAAUUCUACAAGUUCAUCAAGCCGAUCCUGGAAA<br>AGAUGGACGGAACAGAAGAACUGCUGGUCAAGCUGAACAGAGAAGACCUGCU<br>GAGAAAGCAGAGAACAUUCGACAACGGAAGCAUCCCGCACCAGAUCCACCUG<br>GGAGAACUGCACGCAAUCCUGAGAAGACAGGAAGACUUCUACCCGUUCCUGA<br>AGGACAACAGAGAAAAGAUCGAAAAGAUCCUGACAUUCAGAAUCCCGUACUA<br>CGUCGGACCGCUGGCAAGAGGAAACAGCAGAUUCGCAUGGAUGACAAGAAAG<br>AGCGAAGAAACAAUCACACCGUGGAACUUCGAAGAAGUCGUCGACAAGGGAG<br>CAAGCGCACAGAGCUUCAUCGAAAGAAUGACAAACUUCGACAAGAACCUGCC<br>GAACGAAAAGGUCCUGCCGAAGCACAGCCUGCUGUACGAAUACUUCACAGUC<br>UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG | 26 |

(continued)

| Description | Sequence | SEQ ID No. |
|---|---|---|
| | AACUGGUCAAGGUCAUGGGAAGACACAAGCCGGAAAACAUCGUCAUCGAAAUGGCAAGAGAAAACCAGACAACACAGAAGGGACAGAAGAACAGCAGAGAAAGAAUGAAGAGAAUCGAAGAAGGAAUCAAGGAACUGGGAAGCCAGAUCCUGAAGGAACACCCGGUCGAAAACACACAGCUGCAGAACGAAAAGCUGUACCUGUACUACCUGCAGAACGGAAGAGACAUGUACGUCGACCAGGAACUGGACAUCAACAGACUGAGCGACUACGACGUCGACCACAUCGUCCCGCAGAGCUUCCUGAAGGACGACAGCAUCGACAACAAGGUCCUGACAAGAAGCGACAAGAACAGAGGAAAGAGCGACAACGUCCCGAGCGAAGAAGUCGUCAAGAAGAUGAAGAACUACUGGAGACAGCUGCUGAACGCAAAGCUGAUCACACAGAGAAAGUUCGACAACCUGACAAAGGCAGAGAGAGGAGGACUGAGCGAACUGGACAAGGCAGGAUUCAUCAAGAGACAGCUGGUCGAAACAAGACAGAUCACAAAGCACGUCGCACAGAUCCUGGACAGCAGAAUGAACACAAAGUACGACGAAAACGACAAGCUGAUCAGAGAAGUCAAGGUCAUCACACUGAAGAGCAAGCUGGUCAGCGACUUCAGAAAGGACUUCCAGUUCUACAAGGUCAGAGAAAUCAACAACUACCACCACGCACACGACGCAUACCUGAACGCAGUCGUCGGAACAGCACUGAUCAAGAAGUACCCGAAGCUGGAAAGCGAAUUCGUCUACGGAGACUACAAGGUCUACGACGUCAGAAAGAUGAUCGCAAAGAGCGAACAGGAAAUCGGAAAGGCAACAGCAAAGUACUUCUUCUACAGCAACAUCAUGAACUUCUUCAAGACAGAAAUCACACUGGCAAACGGAGAAAUCAGAAAGAGACCGCUGAUCGAAACAAACGGAGAAACAGGAGAAAUCGUCUGGGACAAGGGAAGAGACUUCGCAACAGUCAGAAAGGUCCUGAGCAUGCCGCAGGUCAACAUCGUCAAGAAGACAGAAGUCCAGACAGGAGGAUUCAGCAAGGAAAGCAUCCUGCCGAAGAGAAACAGCGACAAGCUGAUCGCAAGAAAGAAGGACUGGGACCCGAAGAAGUACGGAGGAUUCGACAGCCCGACAGUCGCAUACAGCGUCCUGGUCGUCGCAAAGGUCGAAAAGGGAAAGAGCAAGAAGCUGAAGAGCGUCAAGGAACUGCUGGGAAUCACAAUCAUGGAAAGAAGCAGCUUCGAAAAGAACCCGAUCGACUUCCUGGAAGCAAAGGGAUACAAGGAAGUCAAGAAGGACCUGAUCAUCAAGCUGCCGAAGUACAGCCUGUUCGAACUGGAAAACGGAAGAAAGAGAAUGCUGGCAAGCGCAGGAGAACUGCAGAAGGGAAACGAACUGGCACUGCCGAGCAAGUACGUCAACUUCCUGUACCUGGCAAGCCACUACGAAAAGCUGAAGGGAAGCCCGGAAGACAACGAACAGAAGCAGCUGUUCGUCGAACAGCACAAGCACUACCUGGACGAAAUCAUCGAACAGAUCAGCGAAUUCAGCAAGAGAGUCAUCCUGGCAGACGCAAACCUGGACAAGGUCCUGAGCGCAUACAACAAGCACAGAGACAAGCCGAUCAGAGAACAGGCAGAAAACAUCAUCCACCUGUUCACACUGACAAACCUGGGAGCACCGGCAGCAUUCAAGUACUUCGACACAACAAUCGACAGAAAGAGAUACACAAGCACAAAGGAAGUCCUGGACGCAACACUGAUCCACCAGAGCAUCACAGGACUGUACGAAACAAGAAUCGACCUGAGCCAGCUGGGAGGAGACGGAAGCGGAAGCCCGAAGAAGAAGAGAAAGGUCGACGGAAGCCCGAAGAAGAAGAGAAAGGUCGACAGCGGAUAG | |

(continued)

| Description | Sequence | SEQ ID No. |
|---|---|---|
| **Cas9 nickase coding sequence encoding SEQ ID NO: 25 using minimal uridine codons as listed in Table 1 (no start or stop codons; suitable for inclusion in fusion protein coding sequence)** | GACAAGAAGUACAGCAUCGGACUGGCAAUCGGAACAAACAGCGUCGGAUGGG CAGUCAUCACAGACGAAUACAAGGUCCCGAGCAAGAAGUUCAAGGUCCUGGG AAACACAGACAGACACAGCAUCAAGAAGAACCUGAUCGGAGCACUGCUGUUC GACAGCGGAGAAACAGCAGAAGCAACAAGACUGAAGAGAACAGCAAGAAGAA GAUACACAAGAAGAAAGAACAGAAUCUGCUACCUGCAGGAAAUCUUCAGCAA CGAAAUGGCAAAGGUCGACGACAGCUUCUUCCACAGACUGGAAGAAAGCUUC CUGGUCGAAGAAGACAAGAAGCACGAAAGACACCCGAUCUUCGGAAACAUCG UCGACGAAGUCGCAUACCACGAAAAGUACCCGACAAUCUACCACCUGAGAAA GAAGCUGGUCGACAGCACAGACAAGGCAGACCUGAGACUGAUCUACCUGGCA CUGGCACACAUGAUCAAGUUCAGAGGACACUUCCUGAUCGAAGGAGACCUGA ACCCGGACAACAGCGACGUCGACAAGCUGUUCAUCCAGCUGGUCCAGACAUA CAACCAGCUGUUCGAAGAAAACCCGAUCAACGCAAGCGGAGUCGACGCAAAG GCAAUCCUGAGCGCAAGACUGAGCAAGAGCAGAAGACUGGAAAACCUGAUCG CACAGCUGCCGGGAGAAAAGAAGAACGGACUGUUCGGAAACCUGAUCGCACU GAGCCUGGGACUGACACCGAACUUCAAGAGCAACUUCGACCUGGCAGAAGAC GCAAAGCUGCAGCUGAGCAAGGACACAUACGACGACGACCUGGACAACCUGC UGGCACAGAUCGGAGACCAGUACGCAGACCUGUUCCUGGCAGCAAAGAACCU GAGCGACGCAAUCCUGCUGAGCGACAUCCUGAGAGUCAACACAGAAAUCACA AAGGCACCGCUGAGCGCAAGCAUGAUCAAGAGAUACGACGAACACCACCAGG ACCUGACACUGCUGAAGGCACUGGUCAGACAGCAGCUGCCGGAAAAGUACAA GGAAAUCUUCUUCGACCAGAGCAAGAACGGAUACGCAGGAUACAUCGACGGA | **27** |

(continued)

| Description | Sequence | SEQ ID No. |
|---|---|---|
| | GGAGCAAGCCAGGAAGAAUUCUACAAGUUCAUCAAGCCGAUCCUGGAAAAGA UGGACGGAACAGAAGAACUGCUGGUCAAGCUGAACAGAGAAGACCUGCUGAG AAAGCAGAGAACAUUCGACAACGGAAGCAUCCCGCACCAGAUCCACCUGGGA GAACUGCACGCAAUCCUGAGAAGACAGGAAGACUUCUACCCGUUCCUGAAGG ACAACAGAGAAAAGAUCGAAAAGAUCCUGACAUUCAGAAUCCCGUACUACGU CGGACCGCUGGCAAGAGGAAACAGCAGAUUCGCAUGGAUGACAAGAAAGAGC GAAGAAACAAUCACACCGUGGAACUUCGAAGAAGUCGUCGACAAGGGAGCAA GCGCACAGAGCUUCAUCGAAAGAAUGACAAACUUCGACAAGAACCUGCCGAA CGAAAAGGUCCUGCCGAAGCACAGCCUGCUGUACGAAUACUUCACAGUCUAC AACGAACUGACAAAGGUCAAGUACGUCACAGAAGGAAUGAGAAAGCCGGCAU UCCUGAGCGGAGAACAGAAGAAGGCAAUCGUCGACCUGCUGUUCAAGACAAA CAGAAAGGUCACAGUCAAGCAGCUGAAGGAAGACUACUUCAAGAAGAUCGAA UGCUUCGACAGCGUCGAAAUCAGCGGAGUCGAAGACAGAUUCAACGCAAGCC UGGGAACAUACCACGACCUGCUGAAGAUCAUCAAGGACAAGGACUUCCUGGA CAACGAAGAAAACGAAGACAUCCUGGAAGACAUCGUCCUGACACUGACACUG UUCGAAGACAGAGAAAUGAUCGAAGAAAGACUGAAGACAUACGCACACCUGU UCGACGACAAGGUCAUGAAGCAGCUGAAGAGAAGAAGAUACACAGGAUGGGG AAGACUGAGCAGAAAGCUGAUCAACGGAAUCAGAGACAAGCAGAGCGGAAAG ACAAUCCUGGACUUCCUGAAGAGCGACGGAUUCGCAAACAGAAACUUCAUGC AGCUGAUCCACGACGACAGCCUGACAUUCAAGGAAGACAUCCAGAAGGCACA GGUCAGCGGACAGGGAGACAGCCUGCACGAACACAUCGCAAACCUGGCAGGA AGCCCGGCAAUCAAGAAGGGAAUCCUGCAGACAGUCAAGGUCGUCGACGAAC UGGUCAAGGUCAUGGGAAGACACAAGCCGGAAAACAUCGUCAUCGAAAUGGC AAGAGAAAACCAGACAACACAGAAGGGACAGAAGAACAGCAGAGAAAGAAUG AAGAGAAUCGAAGAAGGAAUCAAGGAACUGGGAAGCCAGAUCCUGAAGGAAC ACCCGGUCGAAAACACACAGCUGCAGAACGAAAAGCUGUACCUGUACUACCU GCAGAACGGAAGAGACAUGUACGUCGACCAGGAACUGGACAUCAACAGACUG AGCGACUACGACGUCGACCACAUCGUCCCGCAGAGCUUCCUGAAGGACGACA GCAUCGACAACAAGGUCCUGACAAGAAGCGACAAGAACAGAGGAAAGAGCGA CAACGUCCCGAGCGAAGAAGUCGUCAAGAAGAUGAAGAACUACUGGAGACAG CUGCUGAACGCAAAGCUGAUCACACAGAGAAAGUUCGACAACCUGACAAAGG CAGAGAGAGGAGGACUGAGCGAACUGGACAAGGCAGGAUUCAUCAAGAGACA GCUGGUCGAAACAAGACAGAUCACAAAGCACGUCGCACAGAUCCUGGACAGC AGAAUGAACACAAAGUACGACGAAAACGACAAGCUGAUCAGAGAAGUCAAGG UCAUCACACUGAAGAGCAAGCUGGUCAGCGACUUCAGAAAGGACUUCCAGUU CUACAAGGUCAGAGAAAUCAACAACUACCACCACGCACACGACGCAUACCUG AACGCAGUCGUCGGAACAGCACUGAUCAAGAAGUACCCGAAGCUGGAAAGCG AAUUCGUCUACGGAGACUACAAGGUCUACGACGUCAGAAAGAUGAUCGCAAA GAGCGAACAGGAAAUCGGAAAGGCAACAGCAAAGUACUUCUUCUACAGCAAC AUCAUGAACUUCUUCAAGACAGAAAUCACACUGGCAAACGGAGAAAUCAGAA AGAGACCGCUGAUCGAAACAAACGGAGAAACAGGAGAAAUCGUCUGGGACAA GGGAAGAGACUUCGCAACAGUCAGAAAGGUCCUGAGCAUGCCGCAGGUCAAC AUCGUCAAGAAGACAGAAGUCCAGACAGGAGGAUUCAGCAAGGAAAGCAUCC UGCCGAAGAGAAACAGCGACAAGCUGAUCGCAAGAAAGAAGGACUGGGACCC GAAGAAGUACGGAGGAUUCGACAGCCCGACAGUCGCAUACAGCGUCCUGGUC GUCGCAAAGGUCGAAAAGGGAAAGAGCAAGAAGCUGAAGAGCGUCAAGGAAC UGCUGGGAAUCACAAUCAUGGAAAGAAGCAGCUUCGAAAAGAACCCGAUCGA CUUCCUGGAAGCAAAGGGAUACAAGGAAGUCAAGAAGGACCUGAUCAUCAAG CUGCCGAAGUACAGCCUGUUCGAACUGGAAAACGGAAGAAAGAGAAUGCUGG CAAGCGCAGGAGAACUGCAGAAGGGAAACGAACUGGCACUGCCGAGCAAGUA CGUCAACUUCCUGUACCUGGCAAGCCACUACGAAAAGCUGAAGGGAAGCCCG GAAGACAACGAACAGAAGCAGCUGUUCGUCGAACAGCACAAGCACUACCUGG ACGAAAUCAUCGAACAGAUCAGCGAAUUCAGCAAGAGAGUCAUCCUGGCAGA CGCAAACCUGGACAAGGUCCUGAGCGCAUACAACAAGCACAGAGACAAGCCG AUCAGAGAACAGGCAGAAAACAUCAUCCACCUGUUCACACUGACAAACCUGG GAGCACCGGCAGCAUUCAAGUACUUCGACACAACAAUCGACAGAAAGAGAUA CACAAGCACAAAGGAAGUCCUGGACGCAACACUGAUCCACCAGAGCAUCACA GGACUGUACGAAACAAGAAUCGACCUGAGCCAGCUGGGAGGAGAC GGAAGCGGAAGCCCGAAGAAGAAGAGAAAGGUCGACGGAAGCCCGAAGAAGA AGAGAAAGGUCGACAGCGGA | |

(continued)

| Description | Sequence | SEQ ID No. |
|---|---|---|
| **Amino acid sequence of dCas9 with two nuclear localization signals as the C-terminal amino acids** | MDKKYSIGLAIGTNSVGWAVITDEYKVPSKKFKVLGNTDRHSIKKNLIGALL<br>FDSGETAEATRLKRTARRRYTRRKNRICYLQEIFSNEMAKVDDSFFHRLEES<br>FLVEEDKKHERHPIFGNIVDEVAYHEKYPTIYHLRKKLVDSTDKADLRLIYL<br>ALAHMIKFRGHFLIEGDLNPDNSDVDKLFIQLVQTYNQLFEENPINASGVDA<br>KAILSARLSKSRRLENLIAQLPGEKKNGLFGNLIALSLGLTPNFKSNFDLAE<br>DAKLQLSKDTYDDDLDNLLAQIGDQYADLFLAAKNLSDAILLSDILRVNTEI<br>TKAPLSASMIKRYDEHHQDLTLLKALVRQQLPEKYKEIFFDQSKNGYAGYID<br>GGASQEEFYKFIKPILEKMDGTEELLVKLNREDLLRKQRTFDNGSIPHQIHL<br>GELHAILRRQEDFYPFLKDNREKIEKILTFRIPYYVGPLARGNSRFAWMTRK<br>SEETITPWNFEEVVDKGASAQSFIERMTNFDKNLPNEKVLPKHSLLYEYFTV<br>YNELTKVKYVTEGMRKPAFLSGEQKKAIVDLLFKTNRKVTVKQLKEDYFKKI<br>ECFDSVEISGVEDRFNASLGTYHDLLKIIKDKDFLDNEENEDILEDIVLTLT<br>LFEDREMIEERLKTYAHLFDDKVMKQLKRRRYTGWGRLSRKLINGIRDKQSG<br>KTILDFLKSDGFANRNFMQLIHDDSLTFKEDIQKAQVSGQGDSLHEHIANLA<br>GSPAIKKGILQTVKVVDELVKVMGRHKPENIVIEMARENQTTQKGQKNSRER<br>MKRIEEGIKELGSQILKEHPVENTQLQNEKLYLYYLQNGRDMYVDQELDINR<br>LSDYDVDAIVPQSFLKDDSIDNKVLTRSDKNRGKSDNVPSEEVVKKMKNYWR<br>QLLNAKLITQRKFDNLTKAERGGLSELDKAGFIKRQLVETRQITKHVAQILD<br>SRMNTKYDENDKLIREVKVITLKSKLVSDFRKDFQFYKVREINNYHHAHDAY<br>LNAVVGTALIKKYPKLESEFVYGDYKVYDVRKMIAKSEQEIGKATAKYFFYS<br>NIMNFFKTEITLANGEIRKRPLIETNGETGEIVWDKGRDFATVRKVLSMPQV<br>NIVKKTEVQTGGFSKESILPKRNSDKLIARKKDWDPKKYGGFDSPTVAYSVL<br>VVAKVEKGKSKKLKSVKELLGITIMERSSFEKNPIDFLEAKGYKEVKKDLII<br>KLPKYSLFELENGRKRMLASAGELQKGNELALPSKYVNFLYLASHYEKLKGS<br>PEDNEQKQLFVEQHKHYLDEIIEQISEFSKRVILADANLDKVLSAYNKHRDK<br>PIREQAENIIHLFTLTNLGAPAAFKYFDTTIDRKRYTSTKEVLDATLIHQSI<br>TGLYETRIDLSQLGGDGSGSPKKKRKVDGSPKKKRKVDSG | 28 |

(continued)

| Description | Sequence | SEQ ID No. |
|---|---|---|
| **dCas9 mRNA ORF encoding SEQ ID NO: 28 using minimal uridine codons as listed in Table 1, with start and stop codons** | AUGGACAAGAAGUACAGCAUCGGACUGGCAAUCGGAACAAACAGCGUCGGAU GGGCAGUCAUCACAGACGAAUACAAGGUCCCGAGCAAGAAGUUCAAGGUCCU GGGAAACACAGACAGACACAGCAUCAAGAAGAACCUGAUCGGAGCACUGCUG UUCGACAGCGGAGAAACAGCAGAAGCAACAAGACUGAAGAGAACAGCAAGAA GAAGAUACACAAGAAGAAAGAACAGAAUCUGCUACCUGCAGGAAAUCUUCAG CAACGAAAUGGCAAAGGUCGACGACAGCUUCUUCCACAGACUGGAAGAAAGC UUCCUGGUCGAAGAAGACAAGAAGCACGAAAGACACCCGAUCUUCGGAAACA UCGUCGACGAAGUCGCAUACCACGAAAAGUACCCGACAAUCUACCACCUGAG AAAGAAGCUGGUCGACAGCACAGACAAGGCAGACCUGAGACUGAUCUACCUG GCACUGGCACACAUGAUCAAGUUCAGAGGACACUUCCUGAUCGAAGGAGACC UGAACCCGGACAACAGCGACGUCGACAAGCUGUUCAUCCAGCUGGUCCAGAC AUACAACCAGCUGUUCGAAGAAAACCCGAUCAACGCAAGCGGAGUCGACGCA AAGGCAAUCCUGAGCGCAAGACUGAGCAAGAGCAGAAGACUGGAAAACCUGA UCGCACAGCUGCCGGGAGAAAAGAAGAACGGACUGUUCGGAAACCUGAUCGC ACUGAGCCUGGGACUGACACCGAACUUCAAGAGCAACUUCGACCUGGCAGAA GACGCAAAGCUGCAGCUGAGCAAGGACACAUACGACGACGACCUGGACAACC UGCUGGCACAGAUCGGAGACCAGUACGCAGACCUGUUCCUGGCAGCAAAGAA CCUGAGCGACGCAAUCCUGCUGAGCGACAUCCUGAGAGUCAACACAGAAAUC ACAAAGGCACCGCUGAGCGCAAGCAUGAUCAAGAGAUACGACGAACACCACC AGGACCUGACACUGCUGAAGGCACUGGUCAGACAGCAGCUGCCGGAAAAGUA CAAGGAAAUCUUCUUCGACCAGAGCAAGAACGGAUACGCAGGAUACAUCGAC GGAGGAGCAAGCCAGGAAGAAUUCUACAAGUUCAUCAAGCCGAUCCUGGAAA AGAUGGACGGAACAGAAGAACUGCUGGUCAAGCUGAACAGAGAAGACCUGCU GAGAAAGCAGAGAACAUUCGACAACGGAAGCAUCCCGCACCAGAUCCACCUG GGAGAACUGCACGCAAUCCUGAGAAGACAGGAAGACUUCUACCCGUUCCUGA AGGACAACAGAGAAAAGAUCGAAAAGAUCCUGACAUUCAGAAUCCCGUACUA CGUCGGACCGCUGGCAAGAGGAAACAGCAGAUUCGCAUGGAUGACAAGAAAG AGCGAAGAAACAAUCACACCGUGGAACUUCGAAGAAGUCGUCGACAAGGGAG CAAGCGCACAGAGCUUCAUCGAAAGAAUGACAAACUUCGACAAGAACCUGCC GAACGAAAAGGUCCUGCCGAAGCACAGCCUGCUGUACGAAUACUUCACAGUC | **29** |

(continued)

| Description | Sequence | SEQ ID No. |
| --- | --- | --- |
| | UACAACGAACUGACAAAGGUCAAGUACGUCACAGAAGGAAUGAGAAAGCCGG<br>CAUUCCUGAGCGGAGAACAGAAGAAGGCAAUCGUCGACCUGCUGUUCAAGAC<br>AAACAGAAAGGUCACAGUCAAGCAGCUGAAGGAAGACUACUUCAAGAAGAUC<br>GAAUGCUUCGACAGCGUCGAAAUCAGCGGAGUCGAAGACAGAUUCAACGCAA<br>GCCUGGGAACAUACCACGACCUGCUGAAGAUCAUCAAGGACAAGGACUUCCU<br>GGACAACGAAGAAAACGAAGACAUCCUGGAAGACAUCGUCCUGACACUGACA<br>CUGUUCGAAGACAGAGAAAUGAUCGAAGAAAGACUGAAGACAUACGCACACC<br>UGUUCGACGACAAGGUCAUGAAGCAGCUGAAGAGAAGAAGAUACACAGGAUG<br>GGGAAGACUGAGCAGAAAGCUGAUCAACGGAAUCAGAGACAAGCAGAGCGGA<br>AAGACAAUCCUGGACUUCCUGAAGAGCGACGGAUUCGCAAACAGAAACUUCA<br>UGCAGCUGAUCCACGACGACAGCCUGACAUUCAAGGAAGACAUCCAGAAGGC<br>ACAGGUCAGCGGACAGGGAGACAGCCUGCACGAACACAUCGCAAACCUGGCA<br>GGAAGCCCGGCAAUCAAGAAGGGAAUCCUGCAGACAGUCAAGGUCGUCGACG<br>AACUGGUCAAGGUCAUGGGAAGACACAAGCCGGAAAACAUCGUCAUCGAAAU<br>GGCAAGAGAAAACCAGACAACACAGAAGGGACAGAAGAACAGCAGAGAAAGA<br>AUGAAGAGAAUCGAAGAAGGAAUCAAGGAACUGGGAAGCCAGAUCCUGAAGG<br>AACACCCGGUCGAAAACACACAGCUGCAGAACGAAAAGCUGUACCUGUACUA<br>CCUGCAGAACGGAAGAGACAUGUACGUCGACCAGGAACUGGACAUCAACAGA<br>CUGAGCGACUACGACGUCGACGCAAUCGUCCCGCAGAGCUUCCUGAAGGACG<br>ACAGCAUCGACAACAAGGUCCUGACAAGAAGCGACAAGAACAGAGGAAAGAG<br>CGACAACGUCCCGAGCGAAGAAGUCGUCAAGAAGAUGAAGAACUACUGGAGA<br>CAGCUGCUGAACGCAAAGCUGAUCACACAGAGAAAGUUCGACAACCUGACAA<br>AGGCAGAGAGAGGAGGACUGAGCGAACUGGACAAGGCAGGAUUCAUCAAGAG<br>ACAGCUGGUCGAAACAAGACAGAUCACAAAGCACGUCGCACAGAUCCUGGAC<br>AGCAGAAUGAACACAAAGUACGACGAAAACGACAAGCUGAUCAGAGAAGUCA<br>AGGUCAUCACACUGAAGAGCAAGCUGGUCAGCGACUUCAGAAAGGACUUCCA<br>GUUCUACAAGGUCAGAGAAAUCAACAACUACCACCACGCACACGACGCAUAC<br>CUGAACGCAGUCGUCGGAACAGCACUGAUCAAGAAGUACCCGAAGCUGGAAA<br>GCGAAUUCGUCUACGGAGACUACAAGGUCUACGACGUCAGAAAGAUGAUCGC<br>AAAGAGCGAACAGGAAAUCGGAAAGGCAACAGCAAAGUACUUCUUCUACAGC<br>AACAUCAUGAACUUCUUCAAGACAGAAAUCACACUGGCAAACGGAGAAAUCA<br>GAAAGAGACCGCUGAUCGAAACAAACGGAGAAACAGGAGAAAUCGUCUGGGA<br>CAAGGGAAGAGACUUCGCAACAGUCAGAAAGGUCCUGAGCAUGCCGCAGGUC<br>AACAUCGUCAAGAAGACAGAAGUCCAGACAGGAGGAUUCAGCAAGGAAAGCA<br>UCCUGCCGAAGAGAAACAGCGACAAGCUGAUCGCAAGAAAGAAGGACUGGGA<br>CCCGAAGAAGUACGGAGGAUUCGACAGCCCGACAGUCGCAUACAGCGUCCUG<br>GUCGUCGCAAAGGUCGAAAAGGGAAAGAGCAAGAAGCUGAAGAGCGUCAAGG<br>AACUGCUGGGAAUCACAAUCAUGGAAAGAAGCAGCUUCGAAAAGAACCCGAU<br>CGACUUCCUGGAAGCAAAGGGAUACAAGGAAGUCAAGAAGGACCUGAUCAUC<br>AAGCUGCCGAAGUACAGCCUGUUCGAACUGGAAAACGGAAGAAAGAGAAUGC<br>UGGCAAGCGCAGGAGAACUGCAGAAGGGAAACGAACUGGCACUGCCGAGCAA<br>GUACGUCAACUUCCUGUACCUGGCAAGCCACUACGAAAAGCUGAAGGGAAGC<br>CCGGAAGACAACGAACAGAAGCAGCUGUUCGUCGAACAGCACAAGCACUACC<br>UGGACGAAAUCAUCGAACAGAUCAGCGAAUUCAGCAAGAGAGUCAUCCUGGC<br>AGACGCAAACCUGGACAAGGUCCUGAGCGCAUACAACAAGCACAGAGACAAG<br>CCGAUCAGAGAACAGGCAGAAAACAUCAUCCACCUGUUCACACUGACAAACC<br>UGGGAGCACCGGCAGCAUUCAAGUACUUCGACACAACAAUCGACAGAAAGAG<br>AUACACAAGCACAAAGGAAGUCCUGGACGCAACACUGAUCCACCAGAGCAUC<br>ACAGGACUGUACGAAACAAGAAUCGACCUGAGCCAGCUGGGAGGAGAC<br>GGAAGCGGAAGCCCGAAGAAGAAGAGAAAGGUCGACGGAAGCCCGAAGAAGA<br>AGAGAAAGGUCGACAGCGGAUAG | |

(continued)

| Description | Sequence | SEQ ID No. |
|---|---|---|
| **dCas9 coding sequence encoding SEQ ID NO: 28 using minimal uridine codons as** | GACAAGAAGUACAGCAUCGGACUGGCAAUCGGAACAAACAGCGUCGGAUGGG<br>CAGUCAUCACAGACGAAUACAAGGUCCCGAGCAAGAAGUUCAAGGUCCUGGG<br>AAACACAGACAGACACAGCAUCAAGAAGAACCUGAUCGGAGCACUGCUGUUC<br>GACAGCGGAGAAACAGCAGAAGCAACAAGACUGAAGAGAACAGCAAGAAGAA<br>GAUACACAAGAAGAAAGAACAGAAUCUGCUACCUGCAGGAAAUCUUCAGCAA<br>CGAAAUGGCAAAGGUCGACGACAGCUUCUUCCACAGACUGGAAGAAAGCUUC<br>CUGGUCGAAGAAGACAAGAAGCACGAAAGACACCCGAUCUUCGGAAACAUCG<br>UCGACGAAGUCGCAUACCACGAAAAGUACCCGACAAUCUACCACCUGAGAAA | **30** |

(continued)

| Description | Sequence | SEQ ID No. |
|---|---|---|
| listed in Table 1 (no start or stop codons; suitable for inclusion in fusion protein coding sequence) | GAAGCUGGUCGACAGCACAGACAAGGCAGACCUGAGACUGAUCUACCUGGCA CUGGCACACAUGAUCAAGUUCAGAGGACACUUCCUGAUCGAAGGAGACCUGA ACCCGGACAACAGCGACGUCGACAAGCUGUUCAUCCAGCUGGUCCAGACAUA CAACCAGCUGUUCGAAGAAAACCCGAUCAACGCAAGCGGAGUCGACGCAAAG GCAAUCCUGAGCGCAAGACUGAGCAAGAGCAGAAGACUGGAAAACCUGAUCG CACAGCUGCCGGGAGAAAAGAAGAACGGACUGUUCGGAAACCUGAUCGCACU GAGCCUGGGACUGACACCGAACUUCAAGAGCAACUUCGACCUGGCAGAAGAC GCAAAGCUGCAGCUGAGCAAGGACACAUACGACGACGACCUGGACAACCUGC UGGCACAGAUCGGAGACCAGUACGCAGACCUGUUCCUGGCAGCAAAGAACCU GAGCGACGCAAUCCUGCUGAGCGACAUCCUGAGAGUCAACACAGAAAUCACA AAGGCACCGCUGAGCGCAAGCAUGAUCAAGAGAUACGACGAACACCACCAGG ACCUGACACUGCUGAAGGCACUGGUCAGACAGCAGCUGCCGGAAAAGUACAA GGAAAUCUUCUUCGACCAGAGCAAGAACGGAUACGCAGGAUACAUCGACGGA GGAGCAAGCCAGGAAGAAUUCUACAAGUUCAUCAAGCCGAUCCUGGAAAAGA UGGACGGAACAGAAGAACUGCUGGUCAAGCUGAACAGAGAAGACCUGCUGAG AAAGCAGAGAACAUUCGACAACGGAAGCAUCCCGCACCAGAUCCACCUGGGA GAACUGCACGCAAUCCUGAGAAGACAGGAAGACUUCUACCCGUUCCUGAAGG ACAACAGAGAAAAGAUCGAAAAGAUCCUGACAUUCAGAAUCCCGUACUACGU CGGACCGCUGGCAAGAGGAAACAGCAGAUUCGCAUGGAUGACAAGAAAGAGC GAAGAAACAAUCACACCGUGGAACUUCGAAGAAGUCGUCGACAAGGGAGCAA GCGCACAGAGCUUCAUCGAAAGAAUGACAAACUUCGACAAGAACCUGCCGAA CGAAAAGGUCCUGCCGAAGCACAGCCUGCUGUACGAAUACUUCACAGUCUAC AACGAACUGACAAAGGUCAAGUACGUCACAGAAGGAAUGAGAAAGCCGGCAU UCCUGAGCGGAGAACAGAAGAAGGCAAUCGUCGACCUGCUGUUCAAGACAAA CAGAAAGGUCACAGUCAAGCAGCUGAAGGAAGACUACUUCAAGAAGAUCGAA UGCUUCGACAGCGUCGAAAUCAGCGGAGUCGAAGACAGAUUCAACGCAAGCC UGGGAACAUACCACGACCUGCUGAAGAUCAUCAAGGACAAGGACUUCCUGGA CAACGAAGAAAACGAAGACAUCCUGGAAGACAUCGUCCUGACACUGACACUG UUCGAAGACAGAGAAAUGAUCGAAGAAAGACUGAAGACAUACGCACACCUGU UCGACGACAAGGUCAUGAAGCAGCUGAAGAGAAGAAGAUACACAGGAUGGGG AAGACUGAGCAGAAAGCUGAUCAACGGAAUCAGAGACAAGCAGAGCGGAAAG ACAAUCCUGGACUUCCUGAAGAGCGACGGAUUCGCAAACAGAAACUUCAUGC AGCUGAUCCACGACGACAGCCUGACAUUCAAGGAAGACAUCCAGAAGGCACA GGUCAGCGGACAGGGAGACAGCCUGCACGAACACAUCGCAAACCUGGCAGGA AGCCCGGCAAUCAAGAAGGGAAUCCUGCAGACAGUCAAGGUCGUCGACGAAC UGGUCAAGGUCAUGGGAAGACACAAGCCGGAAAACAUCGUCAUCGAAAUGGC AAGAGAAAACCAGACAACACAGAAGGGACAGAAGAACAGCAGAGAAAGAAUG AAGAGAAUCGAAGAAGGAAUCAAGGAACUGGGAAGCCAGAUCCUGAAGGAAC ACCCGGUCGAAAACACACAGCUGCAGAACGAAAAGCUGUACCUGUACUACCU GCAGAACGGAAGAGACAUGUACGUCGACCAGGAACUGGACAUCAACAGACUG AGCGACUACGACGUCGACGCAAUCGUCCCGCAGAGCUUCCUGAAGGACGACA GCAUCGACAACAAGGUCCUGACAAGAAGCGACAAGAACAGAGGAAAGAGCGA CAACGUCCCGAGCGAAGAAGUCGUCAAGAAGAUGAAGAACUACUGGAGACAG CUGCUGAACGCAAAGCUGAUCACACAGAGAAAGUUCGACAACCUGACAAAGG CAGAGAGAGGAGGACUGAGCGAACUGGACAAGGCAGGAUUCAUCAAGAGACA GCUGGUCGAAACAAGACAGAUCACAAAGCACGUCGCACAGAUCCUGGACAGC AGAAUGAACACAAAGUACGACGAAAACGACAAGCUGAUCAGAGAAGUCAAGG UCAUCACACUGAAGAGCAAGCUGGUCAGCGACUUCAGAAAGGACUUCCAGUU CUACAAGGUCAGAGAAAUCAACAACUACCACCACGCACACGACGCAUACCUG AACGCAGUCGUCGGAACAGCACUGAUCAAGAAGUACCCGAAGCUGGAAAGCG AAUUCGUCUACGGAGACUACAAGGUCUACGACGUCAGAAAGAUGAUCGCAAA GAGCGAACAGGAAAUCGGAAAGGCAACAGCAAAGUACUUCUUCUACAGCAAC AUCAUGAACUUCUUCAAGACAGAAAUCACACUGGCAAACGGAGAAAUCAGAA AGAGACCGCUGAUCGAAACAAACGGAGAAACAGGAGAAAUCGUCUGGGACAA GGGAAGAGACUUCGCAACAGUCAGAAAGGUCCUGAGCAUGCCGCAGGUCAAC AUCGUCAAGAAGACAGAAGUCCAGACAGGAGGAUUCAGCAAGGAAAGCAUCC UGCCGAAGAGAAACAGCGACAAGCUGAUCGCAAGAAAGAAGGACUGGGACCC GAAGAAGUACGGAGGAUUCGACAGCCCGACAGUCGCAUACAGCGUCCUGGUC GUCGCAAAGGUCGAAAAGGGAAAGAGCAAGAAGCUGAAGAGCGUCAAGGAAC UGCUGGGAAUCACAAUCAUGGAAAGAAGCAGCUUCGAAAAGAACCCGAUCGA | |

(continued)

| Description | Sequence | SEQ ID No. |
|---|---|---|
| | CUUCCUGGAAGCAAAGGGAUACAAGGAAGUCAAGAAGGACCUGAUCAUCAAG CUGCCGAAGUACAGCCUGUUCGAACUGGAAAACGGAAGAAAGAGAAUGCUGG CAAGCGCAGGAGAACUGCAGAAGGGAAACGAACUGGCACUGCCGAGCAAGUA CGUCAACUUCCUGUACCUGGCAAGCCACUACGAAAAGCUGAAGGGAAGCCCG GAAGACAACGAACAGAAGCAGCUGUUCGUCGAACAGCACAAGCACUACCUGG ACGAAAUCAUCGAACAGAUCAGCGAAUUCAGCAAGAGAGUCAUCCUGGCAGA CGCAAACCUGGACAAGGUCCUGAGCGCAUACAACAAGCACAGAGACAAGCCG AUCAGAGAACAGGCAGAAAACAUCAUCCACCUGUUCACACUGACAAACCUGG GAGCACCGGCAGCAUUCAAGUACUUCGACACAACAAUCGACAGAAAGAGAUA CACAAGCACAAAGGAAGUCCUGGACGCAACACUGAUCCACCAGAGCAUCACA GGACUGUACGAAACAAGAAUCGACCUGAGCCAGCUGGGAGGAGAC GGAAGCGGAAGCCCGAAGAAGAAGAGAAAGGUCGACGGAAGCCCGAAGAAGA AGAGAAAGGUCGACAGCGGA | |
| **T7 promoter** | TAATACGACTCACTATA | **31** |
| **Human beta-globin 5' UTR** | ACATTTGCTTCTGACACAACTGTGTTCACTAGCAACCTCAAACAGACACC | **32** |
| **Human beta-globin 3' UTR** | GCTCGCTTTCTTGCTGTCCAATTTCTATTAAAGGTTCCTTTGTTCCCTAAGT CCAACTACTAAACTGGGGGATATTATGAAGGGCCTTGAGCATCTGGATTCTG CCTAATAAAAAACATTTATTTTCATTGC | **33** |
| **Human alpha-globin 5' UTR** | CATAAACCCTGGCGCGCTCGCGGCCCGGCACTCTTCTGGTCCCCACAGACTC AGAGAGAACCCACC | **34** |
| **Human alpha-globin 3' UTR** | GCTGGAGCCTCGGTGGCCATGCTTCTTGCCCCTTGGGCCTCCCCCCAGCCCC TCCTCCCCTTCCTGCACCCGTACCCCCGTGGTCTTTGAATAAAGTCTGAGTG GGCGGC | **35** |
| ***Xenopus laevis* beta-globin 5' UTR** | AAGCTCAGAATAAACGCTCAACTTTGGCC | **36** |
| ***Xenopus laevis* beta-globin 3' UTR** | ACCAGCCTCAAGAACACCCGAATGGAGTCTCTAAGCTACATAATACCAACTT ACACTTTACAAAATGTTGTCCCCCAAAATGTAGCCATTCGTATCTGCTCCTA ATAAAAAGAAAGTTTCTTCACATTCT | **37** |
| **Bovine Growth Hormone 5' UTR** | CAGGGTCCTGTGGACAGCTCACCAGCT | **38** |
| **Bovine Growth Hormone 3' UTR** | TTGCCAGCCATCTGTTGTTTGCCCCTCCCCCGTGCCTTCCTTGACCCTGGAA GGTGCCACTCCCACTGTCCTTTCCTAATAAAATGAGGAAATTGCATCGCA | **39** |
| **Mus musculus hemoglobin alpha, adult chain 1 (Hba-a1), 3'UTR** | GCTGCCTTCTGCGGGGCTTGCCTTCTGGCCATGCCCTTCTTCTCTCCCTTGC ACCTGTACCTCTTGGTCTTTGAATAAAGCCTGAGTAGGAAG | **40** |
| **HSD17B4 5' UTR** | TCCCGCAGTCGGCGTCCAGCGGCTCTGCTTGTTCGTGTGTGTGTCGTTGCAG GCCTTATTC | **41** |
| **G282 guide RNA** | mU*mU*mA*CAGCCACGUCUACAGCAGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **42** |
| **targeting TTR** | | |

(continued)

| Description | Sequence | SEQ ID No. |
|---|---|---|
| Cas9 transcript with 5' UTR of HSD, ORF corresponding to SEQ ID NO: 4, Kozak sequence, and 3' UTR of ALB | GGGTCCCGCAGTCGGCGTCCAGCGGCTCTGCTTGTTCGTGTGTGTGTCGTTG CAGGCCTTATTCGGATCCGCCACCATGGACAAGAAGTACAGCATCGGACTGG ACATCGGAACAAACAGCGTCGGATGGGCAGTCATCACAGACGAATACAAGGT CCCGAGCAAGAAGTTCAAGGTCCTGGGAAACACAGACAGACACAGCATCAAG AAGAACCTGATCGGAGCACTGCTGTTCGACAGCGGAGAAACAGCAGAAGCAA CAAGACTGAAGAGAACAGCAAGAAGAAGATACACAAGAAGAAAGAACAGAAT CTGCTACCTGCAGGAAATCTTCAGCAACGAAATGGCAAAGGTCGACGACAGC TTCTTCCACAGACTGGAAGAAAGCTTCCTGGTCGAAGAAGACAAGAAGCACG AAAGACACCCGATCTTCGGAAACATCGTCGACGAAGTCGCATACCACGAAAA GTACCCGACAATCTACCACCTGAGAAAGAAGCTGGTCGACAGCACAGACAAG GCAGACCTGAGACTGATCTACCTGGCACTGGCACACATGATCAAGTTCAGAG GACACTTCCTGATCGAAGGAGACCTGAACCCGGACAACAGCGACGTCGACAA GCTGTTCATCCAGCTGGTCCAGACATACAACCAGCTGTTCGAAGAAAACCCG ATCAACGCAAGCGGAGTCGACGCAAAGGCAATCCTGAGCGCAAGACTGAGCA AGAGCAGAAGACTGGAAAACCTGATCGCACAGCTGCCGGGAGAAAAGAAGAA CGGACTGTTCGGAAACCTGATCGCACTGAGCCTGGGACTGACACCGAACTTC AAGAGCAACTTCGACCTGGCAGAAGACGCAAAGCTGCAGCTGAGCAAGGACA CATACGACGACGACCTGGACAACCTGCTGGCACAGATCGGAGACCAGTACGC AGACCTGTTCCTGGCAGCAAAGAACCTGAGCGACGCAATCCTGCTGAGCGAC ATCCTGAGAGTCAACACAGAAATCACAAAGGCACCGCTGAGCGCAAGCATGA TCAAGAGATACGACGAACACCACCAGGACCTGACACTGCTGAAGGCACTGGT CAGACAGCAGCTGCCGGAAAAGTACAAGGAAATCTTCTTCGACCAGAGCAAG AACGGATACGCAGGATACATCGACGGAGGAGCAAGCCAGGAAGAATTCTACA AGTTCATCAAGCCGATCCTGGAAAAGATGGACGGAACAGAAGAACTGCTGGT CAAGCTGAACAGAGAAGACCTGCTGAGAAAGCAGAGAACATTCGACAACGGA AGCATCCCGCACCAGATCCACCTGGGAGAACTGCACGCAATCCTGAGAAGAC AGGAAGACTTCTACCCGTTCCTGAAGGACAACAGAGAAAAGATCGAAAAGAT CCTGACATTCAGAATCCCGTACTACGTCGGACCGCTGGCAAGAGGAAACAGC AGATTCGCATGGATGACAAGAAAGAGCGAAGAAACAATCACACCGTGGAACT TCGAAGAAGTCGTCGACAAGGGAGCAAGCGCACAGAGCTTCATCGAAAGAAT GACAAACTTCGACAAGAACCTGCCGAACGAAAAGGTCCTGCCGAAGCACAGC CTGCTGTACGAATACTTCACAGTCTACAACGAACTGACAAAGGTCAAGTACG TCACAGAAGGAATGAGAAAGCCGGCATTCCTGAGCGGAGAACAGAAGAAGGC AATCGTCGACCTGCTGTTCAAGACAAACAGAAAGGTCACAGTCAAGCAGCTG AAGGAAGACTACTTCAAGAAGATCGAATGCTTCGACAGCGTCGAAATCAGCG GAGTCGAAGACAGATTCAACGCAAGCCTGGGAACATACCACGACCTGCTGAA GATCATCAAGGACAAGGACTTCCTGGACAACGAAGAAAACGAAGACATCCTG GAAGACATCGTCCTGACACTGACACTGTTCGAAGACAGAGAAATGATCGAAG AAAGACTGAAGACATACGCACACCTGTTCGACGACAAGGTCATGAAGCAGCT GAAGAGAAGAAGATACACAGGATGGGGAAGACTGAGCAGAAAGCTGATCAAC GGAATCAGAGACAAGCAGAGCGGAAAGACAATCCTGGACTTCCTGAAGAGCG ACGGATTCGCAAACAGAAACTTCATGCAGCTGATCCACGACGACAGCCTGAC ATTCAAGGAAGACATCCAGAAGGCACAGGTCAGCGGACAGGGAGACAGCCTG CACGAACACATCGCAAACCTGGCAGGAAGCCCGGCAATCAAGAAGGGAATCC TGCAGACAGTCAAGGTCGTCGACGAACTGGTCAAGGTCATGGGAAGACACAA GCCGGAAAACATCGTCATCGAAATGGCAAGAGAAAACCAGACAACACAGAAG GGACAGAAGAACAGCAGAGAAAGAATGAAGAGAATCGAAGAAGGAATCAAGG AACTGGGAAGCCAGATCCTGAAGGAACACCCGGTCGAAAACACACAGCTGCA GAACGAAAAGCTGTACCTGTACTACCTGCAGAACGGAAGAGACATGTACGTC GACCAGGAACTGGACATCAACAGACTGAGCGACTACGACGTCGACCACATCG TCCCGCAGAGCTTCCTGAAGGACGACAGCATCGACAACAAGGTCCTGACAAG AAGCGACAAGAACAGAGGAAAGAGCGACAACGTCCCGAGCGAAGAAGTCGTC AAGAAGATGAAGAACTACTGGAGACAGCTGCTGAACGCAAAGCTGATCACAC AGAGAAAGTTCGACAACCTGACAAAGGCAGAGAGAGGAGGACTGAGCGAACT GGACAAGGCAGGATTCATCAAGAGACAGCTGGTCGAAACAAGACAGATCACA AAGCACGTCGCACAGATCCTGGACAGCAGAATGAACACAAAGTACGACGAAA ACGACAAGCTGATCAGAGAAGTCAAGGTCATCACACTGAAGAGCAAGCTGGT | 43 |

(continued)

| Description | Sequence | SEQ ID No. |
|---|---|---|
| | CAGCGACTTCAGAAAGGACTTCCAGTTCTACAAGGTCAGAGAAATCAACAAC TACCACCACGCACACGACGCATACCTGAACGCAGTCGTCGGAACAGCACTGA TCAAGAAGTACCCGAAGCTGGAAAGCGAATTCGTCTACGGAGACTACAAGGT CTACGACGTCAGAAAGATGATCGCAAAGAGCGAACAGGAAATCGGAAAGGCA ACAGCAAAGTACTTCTTCTACAGCAACATCATGAACTTCTTCAAGACAGAAA TCACACTGGCAAACGGAGAAATCAGAAAGAGACCGCTGATCGAAACAAACGG AGAAACAGGAGAAATCGTCTGGGACAAGGGAAGAGACTTCGCAACAGTCAGA AAGGTCCTGAGCATGCCGCAGGTCAACATCGTCAAGAAGACAGAAGTCCAGA CAGGAGGATTCAGCAAGGAAAGCATCCTGCCGAAGAGAAACAGCGACAAGCT GATCGCAAGAAAGAAGGACTGGGACCCGAAGAAGTACGGAGGATTCGACAGC CCGACAGTCGCATACAGCGTCCTGGTCGTCGCAAAGGTCGAAAAGGGAAAGA GCAAGAAGCTGAAGAGCGTCAAGGAACTGCTGGGAATCACAATCATGGAAAG AAGCAGCTTCGAAAAGAACCCGATCGACTTCCTGGAAGCAAAGGGATACAAG GAAGTCAAGAAGGACCTGATCATCAAGCTGCCGAAGTACAGCCTGTTCGAAC TGGAAAACGGAAGAAAGAGAATGCTGGCAAGCGCAGGAGAACTGCAGAAGGG AAACGAACTGGCACTGCCGAGCAAGTACGTCAACTTCCTGTACCTGGCAAGC CACTACGAAAAGCTGAAGGGAAGCCCGGAAGACAACGAACAGAAGCAGCTGT TCGTCGAACAGCACAAGCACTACCTGGACGAAATCATCGAACAGATCAGCGA ATTCAGCAAGAGAGTCATCCTGGCAGACGCAAACCTGGACAAGGTCCTGAGC GCATACAACAAGCACAGAGACAAGCCGATCAGAGAACAGGCAGAAAACATCA TCCACCTGTTCACACTGACAAACCTGGGAGCACCGGCAGCATTCAAGTACTT CGACACAACAATCGACAGAAAGAGATACACAAGCACAAAGGAAGTCCTGGAC GCAACACTGATCCACCAGAGCATCACAGGACTGTACGAAACAAGAATCGACC TGAGCCAGCTGGGAGGAGACGGAGGAGGAAGCCCGAAGAAGAAGAGAAAGGT CTAGCTAGCCATCACATTTAAAAGCATCTCAGCCTACCATGAGAATAAGAGA AAGAAAATGAAGATCAATAGCTTATTCATCTCTTTTTCTTTTTCGTTGGTGT AAAGCCAACACCCTGTCTAAAAAACATAAATTTCTTTAATCATTTTGCCTCT TTTCTCTGTGCTTCAATTAATAAAAAATGGAAAGAACCTCGAG | |

(continued)

| Description | Sequence | SEQ ID No. |
|---|---|---|
| Alternative Cas9 ORF with 19.36% U content | ATGGATAAGAAGTACTCGATCGGGCTGGATATCGGAACTAATTCCGTGGGTT GGGCAGTGATCACGGATGAATACAAAGTGCCGTCCAAGAAGTTCAAGGTCCT GGGGAACACCGATAGACACAGCATCAAGAAGAATCTCATCGGAGCCCTGCTG TTTGACTCCGGCGAAACCGCAGAAGCGACCCGGCTCAAACGTACCGCGAGGC GACGCTACACCCGGCGGAAGAATCGCATCTGCTATCTGCAAGAAATCTTTTC GAACGAAATGGCAAAGGTGGACGACAGCTTCTTCCACCGCCTGGAAGAATCT TTCCTGGTGGAGGAGGACAAGAAGCATGAACGGCATCCTATCTTTGGAAACA TCGTGGACGAAGTGGCGTACCACGAAAAGTACCCGACCATCTACCATCTGCG GAAGAAGTTGGTTGACTCAACTGACAAGGCCGACCTCAGATTGATCTACTTG GCCCTCGCCCATATGATCAAATTCCGCGGACACTTCCTGATCGAAGGCGATC TGAACCCTGATAACTCCGACGTGGATAAGCTGTTCATTCAACTGGTGCAGAC CTACAACCAACTGTTCGAAGAAAACCCAATCAATGCCAGCGGCGTCGATGCC AAGGCCATCCTGTCCGCCCGGCTGTCGAAGTCGCGGCGCCTCGAAAACCTGA TCGCACAGCTGCCGGGAGAGAAGAAGAACGGACTTTTCGGCAACTTGATCGC TCTCTCACTGGGACTCACTCCCAATTTCAAGTCCAATTTTGACCTGGCCGAG GACGCGAAGCTGCAACTCTCAAAGGACACCTACGACGACGACTTGGACAATT TGCTGGCACAAATTGGCGATCAGTACGCGGATCTGTTCCTTGCCGCTAAGAA CCTTTCGGACGCAATCTTGCTGTCCGATATCCTGCGCGTGAACACCGAAATA ACCAAAGCGCCGCTTAGCGCCTCGATGATTAAGCGGTACGACGAGCATCACC AGGATCTCACGCTGCTCAAAGCGCTCGTGAGACAGCAACTGCCTGAAAAGTA CAAGGAGATTTTCTTCGACCAGTCCAAGAATGGGTACGCAGGGTACATCGAT GGAGGCGCCAGCCAGGAAGAGTTCTATAAGTTCATCAAGCCAATCCTGGAAA AGATGGACGGAACCGAAGAACTGCTGGTCAAGCTGAACAGGGAGGATCTGCT CCGCAAACAGAGAACCTTTGACAACGGAAGCATTCCACACCAGATCCATCTG GGTGAGCTGCACGCCATCTTGCGGCGCCAGGAGGACTTTTACCCATTCCTCA AGGACAACCGGGAAAAGATCGAGAAAATTCTGACGTTCCGCATCCCGTATTA CGTGGGCCCACTGGCGCGCGGCAATTCGCGCTTCGCGTGGATGACTAGAAAA TCAGAGGAAACCATCACTCCTTGGAATTTCGAGGAAGTTGTGGATAAGGGAG CTTCGGCACAATCCTTCATCGAACGAATGACCAACTTCGACAAGAATCTCCC AAACGAGAAGGTGCTTCCTAAGCACAGCCTCCTTTACGAATACTTCACTGTC | 45 |

(continued)

| Description | Sequence | SEQ ID No. |
|---|---|---|
| | TACAACGAACTGACTAAAGTGAAATACGTTACTGAAGGAATGAGGAAGCCGGCCTTTCTGAGCGGAGAACAGAAGAAAGCGATTGTCGATCTGCTGTTCAAGACCAACCGCAAGGTGACCGTCAAGCAGCTTAAAGAGGACTACTTCAAGAAGATCGAGTGTTTCGACTCAGTGGAAATCAGCGGAGTGGAGGACAGATTCAACGCTTCGCTGGGAACCTATCATGATCTCCTGAAGATCATCAAGGACAAGGACTTCCTTGACAACGAGGAGAACGAGGACATCCTGGAAGATATCGTCCTGACCTTGACCCTTTTCGAGGATCGCGAGATGATCGAGGAGAGGCTTAAGACCTACGCTCATCTCTTCGACGATAAGGTCATGAAACAACTCAAGCGCCGCCGGTACACTGGTTGGGGCCGCCTCTCCCGCAAGCTGATCAACGGTATTCGCGATAAACAGAGCGGTAAAACTATCCTGGATTTCCTCAAATCGGATGGCTTCGCTAATCGTAACTTCATGCAGTTGATCCACGACGACAGCCTGACCTTTAAGGAGGACATCCAGAAAGCACAAGTGAGCGGACAGGGAGACTCACTCCATGAACACATCGCGAATCTGGCCGGTTCGCCGGCGATTAAGAAGGGAATCCTGCAAACTGTGAAGGTGGTGGACGAGCTGGTGAAGGTCATGGGACGGCACAAACCGGAGAATATCGTGATTGAAATGGCCCGAGAAAACCAGACTACCCAGAAGGGCCAGAAGAACTCCCGCGAAAGGATGAAGCGGATCGAAGAAGGAATCAAGGAGCTGGGCAGCCAGATCCTGAAAGAGCACCCGGTGGAAAACACGCAGCTGCAGAACGAGAAGCTCTACCTGTACTATTTGCAAAATGGACGGGACATGTACGTGGACCAAGAGCTGGACATCAATCGGTTGTCTGATTACGACGTGGACCACATCGTTCCACAGTCCTTTCTGAAGGATGACTCCATCGATAACAAGGTGTTGACTCGCAGCGACAAGAACAGAGGGAAGTCAGATAATGTGCCATCGGAGGAGGTCGTGAAGAAGATGAAGAATTACTGGCGGCAGCTCCTGAATGCGAAGCTGATTACCCAGAGAAAGTTTGACAATCTCACTAAAGCCGAGCGCGGCGGACTCTCAGAGCTGGATAAGGCTGGATTCATCAAACGGCAGCTGGTCGAGACTCGGCAGATTACCAAGCACGTGGCGCAGATCCTGGACTCCCGCATGAACACTAAATACGACGAGAACGATAAGCTCATCCGGGAAGTGAAGGTGATTACCCTGAAAAGCAAACTTGTGTCGGACTTTCGGAAGGACTTTCAGTTTTACAAAGTGAGAGAAATCAACAACTACCATCACGCGCATGACGCATACCTCAACGCTGTGGTCGGCACCGCCCTGATCAAGAAGTACCCTAAACTTGAATCGGAGTTTGTGTACGGAGACTACAAGGTCTACGACGTGAGGAAGATGATAGCCAAGTCCGAACAGGAAATCGGGAAAGCAACTGCGAAATACTTCTTTTACTCAAACATCATGAACTTCTTCAAGACTGAAATTACGCTGGCCAATGGAGAAATCAGGAAGAGGCCACTGATCGAAACTAACGGAGAAACGGGCGAAATCGTGTGGGACAAGGGCAGGGACTTCGCAACTGTTCGCAAAGTGCTCTCTATGCCGCAAGTCAATATTGTGAAGAAAACCGAAGTGCAAACCGGCGGATTTTCAAAGGAATCGATCCTCCCAAAGAGAAATAGCGACAAGCTCATTGCACGCAAGAAAGACTGGGACCCGAAGAAGTACGGAGGATTCGATTCGCCGACTGTCGCATACTCCGTCCTCGTGGTGGCCAAGGTGGAGAAGGGAAAGAGCAAGAAGCTCAAATCCGTCAAAGAGCTGCTGGGGATTACCATCATGGAACGATCCTCGTTCGAGAAGAACCCGATTGATTTCCTGGAGGCGAAGGGTTACAAGGAGGTGAAGAAGGATCTGATCATCAAACTGCCCAAGTACTCACTGTTCGAACTGGAAAATGGTCGGAAGCGCATGCTGGCTTCGGCCGGAGAACTCCAGAAAGGAAATGAGCTGGCCTTGCCTAGCAAGTACGTCAACTTCCTCTATCTTGCTTCGCACTACGAGAAACTCAAAGGGTCACCGGAAGATAACGAACAGAAGCAGCTTTTCGTGGAGCAGCACAAGCATTATCTGGATGAAATCATCGAACAAATCTCCGAGTTTTCAAAGCGCGTGATCCTCGCCGACGCCAACCTCGACAAAGTCCTGTCGGCCTACAATAAGCATAGAGATAAGCCGATCAGAGAACAGGCCGAGAACATTATCCACTTGTTCACCCTGACTAACCTGGGAGCTCCAGCCGCCTTCAAGTACTTCGATACTACTATCGACCGCAAAAGATACACGTCCACCAAGGAAGTTCTGGACGCGACCCTGATCCACCAAAGCATCACTGGACTCTACGAAACTAGGATCGATCTGTCGCAGCTGGGTGGCGATGGTGGCGGTGGATCCTACCCATACGACGTGCCTGACTACGCCTCCGGAGGTGGTGGCCCCAAGAAGAAACGGAAGGTGTGATAG | |

(continued)

| Description | Sequence | SEQ ID No. |
|---|---|---|
| **Cas9 transcript with 5' UTR of HSD, ORF corresponding to SEQ ID NO: 45,** | GGGTCCCGCAGTCGGCGTCCAGCGGCTCTGCTTGTTCGTGTGTGTGTCGTTG<br>CAGGCCTTATTCGGATCTGCCACCATGGATAAGAAGTACTCGATCGGGCTGG<br>ATATCGGAACTAATTCCGTGGGTTGGGCAGTGATCACGGATGAATACAAAGT<br>GCCGTCCAAGAAGTTCAAGGTCCTGGGGAACACCGATAGACACAGCATCAAG<br>AAGAATCTCATCGGAGCCCTGCTGTTTGACTCCGGCGAAACCGCAGAAGCGA<br>CCCGGCTCAAACGTACCGCGAGGCGACGCTACACCCGGCGGAAGAATCGCAT<br>CTGCTATCTGCAAGAAATCTTTTCGAACGAAATGGCAAAGGTGGACGACAGC | **46** |

(continued)

| Description | Sequence | SEQ ID No. |
|---|---|---|
| **Kozak sequence, and 3' UTR of ALB** | TTCTTCCACCGCCTGGAAGAATCTTTCCTGGTGGAGGAGGACAAGAAGCATG<br>AACGGCATCCTATCTTTGGAAACATCGTGGACGAAGTGGCGTACCACGAAAA<br>GTACCCGACCATCTACCATCTGCGGAAGAAGTTGGTTGACTCAACTGACAAG<br>GCCGACCTCAGATTGATCTACTTGGCCCTCGCCCATATGATCAAATTCCGCG<br>GACACTTCCTGATCGAAGGCGATCTGAACCCTGATAACTCCGACGTGGATAA<br>GCTGTTCATTCAACTGGTGCAGACCTACAACCAACTGTTCGAAGAAAACCCA<br>ATCAATGCCAGCGGCGTCGATGCCAAGGCCATCCTGTCCGCCCGGCTGTCGA<br>AGTCGCGGCGCCTCGAAAACCTGATCGCACAGCTGCCGGGAGAGAAGAAGAA<br>CGGACTTTTCGGCAACTTGATCGCTCTCTCACTGGGACTCACTCCCAATTTC<br>AAGTCCAATTTTGACCTGGCCGAGGACGCGAAGCTGCAACTCTCAAAGGACA<br>CCTACGACGACGACTTGGACAATTTGCTGGCACAAATTGGCGATCAGTACGC<br>GGATCTGTTCCTTGCCGCTAAGAACCTTTCGGACGCAATCTTGCTGTCCGAT<br>ATCCTGCGCGTGAACACCGAAATAACCAAAGCGCCGCTTAGCGCCTCGATGA<br>TTAAGCGGTACGACGAGCATCACCAGGATCTCACGCTGCTCAAAGCGCTCGT<br>GAGACAGCAACTGCCTGAAAAGTACAAGGAGATTTTCTTCGACCAGTCCAAG<br>AATGGGTACGCAGGGTACATCGATGGAGGCGCCAGCCAGGAAGAGTTCTATA<br>AGTTCATCAAGCCAATCCTGGAAAAGATGGACGGAACCGAAGAACTGCTGGT<br>CAAGCTGAACAGGGAGGATCTGCTCCGCAAACAGAGAACCTTTGACAACGGA<br>AGCATTCCACACCAGATCCATCTGGGTGAGCTGCACGCCATCTTGCGGCGCC<br>AGGAGGACTTTTACCCATTCCTCAAGGACAACCGGGAAAAGATCGAGAAAAT<br>TCTGACGTTCCGCATCCCGTATTACGTGGGCCCACTGGCGCGCGGCAATTCG<br>CGCTTCGCGTGGATGACTAGAAAATCAGAGGAAACCATCACTCCTTGGAATT<br>TCGAGGAAGTTGTGGATAAGGGAGCTTCGGCACAATCCTTCATCGAACGAAT<br>GACCAACTTCGACAAGAATCTCCCAAACGAGAAGGTGCTTCCTAAGCACAGC<br>CTCCTTTACGAATACTTCACTGTCTACAACGAACTGACTAAAGTGAAATACG<br>TTACTGAAGGAATGAGGAAGCCGGCCTTTCTGAGCGGAGAACAGAAGAAAGC<br>GATTGTCGATCTGCTGTTCAAGACCAACCGCAAGGTGACCGTCAAGCAGCTT<br>AAAGAGGACTACTTCAAGAAGATCGAGTGTTTCGACTCAGTGGAAATCAGCG<br>GAGTGGAGGACAGATTCAACGCTTCGCTGGGAACCTATCATGATCTCCTGAA<br>GATCATCAAGGACAAGGACTTCCTTGACAACGAGGAGAACGAGGACATCCTG<br>GAAGATATCGTCCTGACCTTGACCCTTTTCGAGGATCGCGAGATGATCGAGG<br>AGAGGCTTAAGACCTACGCTCATCTCTTCGACGATAAGGTCATGAAACAACT<br>CAAGCGCCGCCGGTACACTGGTTGGGGCCGCCTCTCCCGCAAGCTGATCAAC<br>GGTATTCGCGATAAACAGAGCGGTAAAACTATCCTGGATTTCCTCAAATCGG<br>ATGGCTTCGCTAATCGTAACTTCATGCAGTTGATCCACGACGACAGCCTGAC<br>CTTTAAGGAGGACATCCAGAAAGCACAAGTGAGCGGACAGGGAGACTCACTC<br>CATGAACACATCGCGAATCTGGCCGGTTCGCCGGCGATTAAGAAGGGAATCC<br>TGCAAACTGTGAAGGTGGTGGACGAGCTGGTGAAGGTCATGGGACGGCACAA<br>ACCGGAGAATATCGTGATTGAAATGGCCCGAGAAAACCAGACTACCCAGAAG<br>GGCCAGAAGAACTCCCGCGAAAGGATGAAGCGGATCGAAGAAGGAATCAAGG<br>AGCTGGGCAGCCAGATCCTGAAAGAGCACCCGGTGGAAAACACGCAGCTGCA<br>GAACGAGAAGCTCTACCTGTACTATTTGCAAAATGGACGGGACATGTACGTG<br>GACCAAGAGCTGGACATCAATCGGTTGTCTGATTACGACGTGGACCACATCG<br>TTCCACAGTCCTTTCTGAAGGATGACTCCATCGATAACAAGGTGTTGACTCG<br>CAGCGACAAGAACAGAGGGAAGTCAGATAATGTGCCATCGGAGGAGGTCGTG<br>AAGAAGATGAAGAATTACTGGCGGCAGCTCCTGAATGCGAAGCTGATTACCC<br>AGAGAAAGTTTGACAATCTCACTAAAGCCGAGCGCGGCGGACTCTCAGAGCT<br>GGATAAGGCTGGATTCATCAAACGGCAGCTGGTCGAGACTCGGCAGATTACC<br>AAGCACGTGGCGCAGATCCTGGACTCCCGCATGAACACTAAATACGACGAGA<br>ACGATAAGCTCATCCGGGAAGTGAAGGTGATTACCCTGAAAAGCAAACTTGT<br>GTCGGACTTTCGGAAGGACTTTCAGTTTTACAAAGTGAGAGAAATCAACAAC<br>TACCATCACGCGCATGACGCATACCTCAACGCTGTGGTCGGCACCGCCCTGA<br>TCAAGAAGTACCCTAAACTTGAATCGGAGTTTGTGTACGGAGACTACAAGGT<br>CTACGACGTGAGGAAGATGATAGCCAAGTCCGAACAGGAAATCGGGAAAGCA<br>ACTGCGAAATACTTCTTTTACTCAAACATCATGAACTTCTTCAAGACTGAAA<br>TTACGCTGGCCAATGGAGAAATCAGGAAGAGGCCACTGATCGAAACTAACGG<br>AGAAACGGGCGAAATCGTGTGGGACAAGGGCAGGGACTTCGCAACTGTTCGC<br>AAAGTGCTCTCTATGCCGCAAGTCAATATTGTGAAGAAAACCGAAGTGCAAA<br>CCGGCGGATTTTCAAAGGAATCGATCCTCCCAAAGAGAAATAGCGACAAGCT<br>CATTGCACGCAAGAAAGACTGGGACCCGAAGAAGTACGGAGGATTCGATTCG | |

(continued)

| Description | Sequence | SEQ ID No. |
|---|---|---|
| | CCGACTGTCGCATACTCCGTCCTCGTGGTGGCCAAGGTGGAGAAGGGAAAGA GCAAGAAGCTCAAATCCGTCAAAGAGCTGCTGGGGATTACCATCATGGAACG ATCCTCGTTCGAGAAGAACCCGATTGATTTCCTGGAGGCGAAGGGTTACAAG GAGGTGAAGAAGGATCTGATCATCAAACTGCCCAAGTACTCACTGTTCGAAC TGGAAAATGGTCGGAAGCGCATGCTGGCTTCGGCCGGAGAACTCCAGAAAGG AAATGAGCTGGCCTTGCCTAGCAAGTACGTCAACTTCCTCTATCTTGCTTCG CACTACGAGAAACTCAAAGGGTCACCGGAAGATAACGAACAGAAGCAGCTTT TCGTGGAGCAGCACAAGCATTATCTGGATGAAATCATCGAACAAATCTCCGA GTTTTCAAAGCGCGTGATCCTCGCCGACGCCAACCTCGACAAAGTCCTGTCG GCCTACAATAAGCATAGAGATAAGCCGATCAGAGAACAGGCCGAGAACATTA TCCACTTGTTCACCCTGACTAACCTGGGAGCTCCAGCCGCCTTCAAGTACTT CGATACTACTATCGACCGCAAAAGATACACGTCCACCAAGGAAGTTCTGGAC GCGACCCTGATCCACCAAAGCATCACTGGACTCTACGAAACTAGGATCGATC TGTCGCAGCTGGGTGGCGATGGTGGCGGTGGATCCTACCCATACGACGTGCC TGACTACGCCTCCGGAGGTGGTGGCCCCAAGAAGAAACGGAAGGTGTGATAG CTAGCCATCACATTTAAAAGCATCTCAGCCTACCATGAGAATAAGAGAAAGA AAATGAAGATCAATAGCTTATTCATCTCTTTTTCTTTTTCGTTGGTGTAAAG CCAACACCCTGTCTAAAAAACATAAATTTCTTTAATCATTTTGCCTCTTTTC TCTGTGCTTCAATTAATAAAAAATGGAAAGAACCTCGAG | |

(continued)

| Description | Sequence | SEQ ID No. |
|---|---|---|
| **Cas9 transcript with 5' UTR of HSD, ORF corresponding to SEQ ID NO: 45, and 3' UTR of ALB** | GGGTCCCGCAGTCGGCGTCCAGCGGCTCTGCTTGTTCGTGTGTGTGTCGTTG<br>CAGGCCTTATTCGGATCTATGGATAAGAAGTACTCGATCGGGCTGGATATCG<br>GAACTAATTCCGTGGGTTGGCAGTGATCACGGATGAATACAAAGTGCCGTC<br>CAAGAAGTTCAAGGTCCTGGGGAACACCGATAGACACAGCATCAAGAAGAAT<br>CTCATCGGAGCCCTGCTGTTTGACTCCGGCGAAACCGCAGAAGCGACCCGGC<br>TCAAACGTACCGCGAGGCGACGCTACACCCGGCGGAAGAATCGCATCTGCTA<br>TCTGCAAGAAATCTTTTCGAACGAAATGGCAAAGGTGGACGACAGCTTCTTC<br>CACCGCCTGGAAGAATCTTTCCTGGTGGAGGAGGACAAGAAGCATGAACGGC<br>ATCCTATCTTTGGAAACATCGTGGACGAAGTGGCGTACCACGAAAAGTACCC<br>GACCATCTACCATCTGCGGAAGAAGTTGGTTGACTCAACTGACAAGGCCGAC<br>CTCAGATTGATCTACTTGGCCCTCGCCCATATGATCAAATTCCGCGGACACT<br>TCCTGATCGAAGGCGATCTGAACCCTGATAACTCCGACGTGGATAAGCTGTT<br>CATTCAACTGGTGCAGACCTACAACCAACTGTTCGAAGAAAACCCAATCAAT<br>GCCAGCGGCGTCGATGCCAAGGCCATCCTGTCCGCCCGGCTGTCGAAGTCGC<br>GGCGCCTCGAAAACCTGATCGCACAGCTGCCGGGAGAGAAGAAGAACGGACT<br>TTTCGGCAACTTGATCGCTCTCTCACTGGGACTCACTCCCAATTTCAAGTCC<br>AATTTTGACCTGGCCGAGGACGCGAAGCTGCAACTCTCAAAGGACACCTACG<br>ACGACGACTTGGACAATTTGCTGGCACAAATTGGCGATCAGTACGCGGATCT<br>GTTCCTTGCCGCTAAGAACCTTTCGGACGCAATCTTGCTGTCCGATATCCTG<br>CGCGTGAACACCGAAATAACCAAAGCGCCGCTTAGCGCCTCGATGATTAAGC<br>GGTACGACGAGCATCACCAGGATCTCACGCTGCTCAAAGCGCTCGTGAGACA<br>GCAACTGCCTGAAAAGTACAAGGAGATTTTCTTCGACCAGTCCAAGAATGGG<br>TACGCAGGGTACATCGATGGAGGCGCCAGCCAGGAAGAGTTCTATAAGTTCA<br>TCAAGCCAATCCTGGAAAAGATGGACGGAACCGAAGAACTGCTGGTCAAGCT<br>GAACAGGGAGGATCTGCTCCGCAAACAGAGAACCTTTGACAACGGAAGCATT<br>CCACACCAGATCCATCTGGGTGAGCTGCACGCCATCTTGCGGCGCCAGGAGG<br>ACTTTTACCCATTCCTCAAGGACAACCGGGAAAAGATCGAGAAAATTCTGAC<br>GTTCCGCATCCCGTATTACGTGGGCCCACTGGCGCGCGGCAATTCGCGCTTC<br>GCGTGGATGACTAGAAAATCAGAGGAAACCATCACTCCTTGGAATTTCGAGG<br>AAGTTGTGGATAAGGGAGCTTCGGCACAATCCTTCATCGAACGAATGACCAA<br>CTTCGACAAGAATCTCCCAAACGAGAAGGTGCTTCCTAAGCACAGCCTCCTT<br>TACGAATACTTCACTGTCTACAACGAACTGACTAAAGTGAAATACGTTACTG<br>AAGGAATGAGGAAGCCGGCCTTTCTGAGCGGAGAACAGAAGAAAGCGATTGT<br>CGATCTGCTGTTCAAGACCAACCGCAAGGTGACCGTCAAGCAGCTTAAAGAG<br>GACTACTTCAAGAAGATCGAGTGTTTCGACTCAGTGGAAATCAGCGGAGTGG<br>AGGACAGATTCAACGCTTCGCTGGGAACCTATCATGATCTCCTGAAGATCAT<br>CAAGGACAAGGACTTCCTTGACAACGAGGAGAACGAGGACATCCTGGAAGAT<br>ATCGTCCTGACCTTGACCCTTTTCGAGGATCGCGAGATGATCGAGGAGAGGC<br>TTAAGACCTACGCTCATCTCTTCGACGATAAGGTCATGAAACAACTCAAGCG | 47 |

(continued)

| Description | Sequence | SEQ ID No. |
|---|---|---|
| | CCGCCGGTACACTGGTTGGGGCCGCCTCTCCCGCAAGCTGATCAACGGTATT<br>CGCGATAAACAGAGCGGTAAAACTATCCTGGATTTCCTCAAATCGGATGGCT<br>TCGCTAATCGTAACTTCATGCAGTTGATCCACGACGACAGCCTGACCTTTAA<br>GGAGGACATCCAGAAAGCACAAGTGAGCGGACAGGGAGACTCACTCCATGAA<br>CACATCGCGAATCTGGCCGGTTCGCCGGCGATTAAGAAGGGAATCCTGCAAA<br>CTGTGAAGGTGGTGGACGAGCTGGTGAAGGTCATGGGACGGCACAAACCGGA<br>GAATATCGTGATTGAAATGGCCCGAGAAAACCAGACTACCCAGAAGGGCCAG<br>AAGAACTCCCGCGAAAGGATGAAGCGGATCGAAGAAGGAATCAAGGAGCTGG<br>GCAGCCAGATCCTGAAAGAGCACCCGGTGGAAAACACGCAGCTGCAGAACGA<br>GAAGCTCTACCTGTACTATTTGCAAAATGGACGGGACATGTACGTGGACCAA<br>GAGCTGGACATCAATCGGTTGTCTGATTACGACGTGGACCACATCGTTCCAC<br>AGTCCTTTCTGAAGGATGACTCCATCGATAACAAGGTGTTGACTCGCAGCGA<br>CAAGAACAGAGGGAAGTCAGATAATGTGCCATCGGAGGAGGTCGTGAAGAAG<br>ATGAAGAATTACTGGCGGCAGCTCCTGAATGCGAAGCTGATTACCCAGAGAA<br>AGTTTGACAATCTCACTAAAGCCGAGCGCGGCGGACTCTCAGAGCTGGATAA<br>GGCTGGATTCATCAAACGGCAGCTGGTCGAGACTCGGCAGATTACCAAGCAC<br>GTGGCGCAGATCCTGGACTCCCGCATGAACACTAAATACGACGAGAACGATA<br>AGCTCATCCGGGAAGTGAAGGTGATTACCCTGAAAAGCAAACTTGTGTCGGA<br>CTTTCGGAAGGACTTTCAGTTTTACAAAGTGAGAGAAATCAACAACTACCAT<br>CACGCGCATGACGCATACCTCAACGCTGTGGTCGGCACCGCCCTGATCAAGA<br>AGTACCCTAAACTTGAATCGGAGTTTGTGTACGGAGACTACAAGGTCTACGA<br>CGTGAGGAAGATGATAGCCAAGTCCGAACAGGAAATCGGGAAAGCAACTGCG<br>AAATACTTCTTTTACTCAAACATCATGAACTTCTTCAAGACTGAAATTACGC<br>TGGCCAATGGAGAAATCAGGAAGAGGCCACTGATCGAAACTAACGGAGAAAC<br>GGGCGAAATCGTGTGGGACAAGGGCAGGGACTTCGCAACTGTTCGCAAAGTG<br>CTCTCTATGCCGCAAGTCAATATTGTGAAGAAAACCGAAGTGCAAACCGGCG<br>GATTTTCAAAGGAATCGATCCTCCCAAAGAGAAATAGCGACAAGCTCATTGC<br>ACGCAAGAAAGACTGGGACCCGAAGAAGTACGGAGGATTCGATTCGCCGACT<br>GTCGCATACTCCGTCCTCGTGGTGGCCAAGGTGGAGAAGGGAAAGAGCAAGA<br>AGCTCAAATCCGTCAAAGAGCTGCTGGGGATTACCATCATGGAACGATCCTC<br>GTTCGAGAAGAACCCGATTGATTTCCTGGAGGCGAAGGGTTACAAGGAGGTG<br>AAGAAGGATCTGATCATCAAACTGCCCAAGTACTCACTGTTCGAACTGGAAA<br>ATGGTCGGAAGCGCATGCTGGCTTCGGCCGGAGAACTCCAGAAAGGAAATGA<br>GCTGGCCTTGCCTAGCAAGTACGTCAACTTCCTCTATCTTGCTTCGCACTAC<br>GAGAAACTCAAAGGGTCACCGGAAGATAACGAACAGAAGCAGCTTTTCGTGG<br>AGCAGCACAAGCATTATCTGGATGAAATCATCGAACAAATCTCCGAGTTTTC<br>AAAGCGCGTGATCCTCGCCGACGCCAACCTCGACAAAGTCCTGTCGGCCTAC<br>AATAAGCATAGAGATAAGCCGATCAGAGAACAGGCCGAGAACATTATCCACT<br>TGTTCACCCTGACTAACCTGGGAGCTCCAGCCGCCTTCAAGTACTTCGATAC<br>TACTATCGACCGCAAAAGATACACGTCCACCAAGGAAGTTCTGGACGCGACC<br>CTGATCCACCAAAGCATCACTGGACTCTACGAAACTAGGATCGATCTGTCGC<br>AGCTGGGTGGCGATGGTGGCGGTGGATCCTACCCATACGACGTGCCTGACTA<br>CGCCTCCGGAGGTGGTGGCCCCAAGAAGAAACGGAAGGTGTGATAGCTAGCC<br>ATCACATTTAAAAGCATCTCAGCCTACCATGAGAATAAGAGAAAGAAAATGA<br>AGATCAATAGCTTATTCATCTCTTTTTCTTTTTCGTTGGTGTAAAGCCAACA<br>CCCTGTCTAAAAAACATAAATTTCTTTAATCATTTTGCCTCTTTTCTCTGTG<br>CTTCAATTAATAAAAAATGGAAAGAACCTCGAG | |

(continued)

| Description | Sequence | SEQ ID No. |
|---|---|---|
| Cas9 transcript comprising Cas9 ORF using codons with generally high expression in humans | GGGTCCCGCAGTCGGCGTCCAGCGGCTCTGCTTGTTCGTGTGTGTGTCGTTG CAGGCCTTATTCGGATCCATGCCTAAGAAAAAGCGGAAGGTCGACGGGGATA AGAAGTACTCAATCGGGCTGGATATCGGAACTAATTCCGTGGGTTGGGCAGT GATCACGGATGAATACAAAGTGCCGTCCAAGAAGTTCAAGGTCCTGGGGAAC ACCGATAGACACAGCATCAAGAAAAATCTCATCGGAGCCCTGCTGTTTGACT CCGGCGAAACCGCAGAAGCGACCCGGCTCAAACGTACCGCGAGGCGACGCTA CACCCGGCGGAAGAATCGCATCTGCTATCTGCAAGAGATCTTTTCGAACGAA ATGGCAAAGGTCGACGACAGCTTCTTCCACCGCCTGGAAGAATCTTTCCTGG TGGAGGAGGACAAGAAGCATGAACGGCATCCTATCTTTGGAAACATCGTCGA CGAAGTGGCGTACCACGAAAAGTACCCGACCATCTACCATCTGCGGAAGAAG TTGGTTGACTCAACTGACAAGGCCGACCTCAGATTGATCTACTTGGCCCTCG | 48 |

(continued)

| Description | Sequence | SEQ ID No. |
|---|---|---|
| | CCCATATGATCAAATTCCGCGGACACTTCCTGATCGAAGGCGATCTGAACCC<br>TGATAACTCCGACGTGGATAAGCTTTTCATTCAACTGGTGCAGACCTACAAC<br>CAACTGTTCGAAGAAAACCCAATCAATGCTAGCGGCGTCGATGCCAAGGCCA<br>TCCTGTCCGCCCGGCTGTCGAAGTCGCGGCGCCTCGAAAACCTGATCGCACA<br>GCTGCCGGGAGAGAAAAAGAACGGACTTTTCGGCAACTTGATCGCTCTCTCA<br>CTGGGACTCACTCCCAATTTCAAGTCCAATTTTGACCTGGCCGAGGACGCGA<br>AGCTGCAACTCTCAAAGGACACCTACGACGACGACTTGGACAATTTGCTGGC<br>ACAAATTGGCGATCAGTACGCGGATCTGTTCCTTGCCGCTAAGAACCTTTCG<br>GACGCAATCTTGCTGTCCGATATCCTGCGCGTGAACACCGAAATAACCAAAG<br>CGCCGCTTAGCGCCTCGATGATTAAGCGGTACGACGAGCATCACCAGGATCT<br>CACGCTGCTCAAAGCGCTCGTGAGACAGCAACTGCCTGAAAAGTACAAGGAG<br>ATCTTCTTCGACCAGTCCAAGAATGGGTACGCAGGGTACATCGATGGAGGCG<br>CTAGCCAGGAAGAGTTCTATAAGTTCATCAAGCCAATCCTGGAAAAGATGGA<br>CGGAACCGAAGAACTGCTGGTCAAGCTGAACAGGGAGGATCTGCTCCGGAAA<br>CAGAGAACCTTTGACAACGGATCCATTCCCCACCAGATCCATCTGGGTGAGC<br>TGCACGCCATCTTGCGGCGCCAGGAGGACTTTTACCCATTCCTCAAGGACAA<br>CCGGGAAAAGATCGAGAAAATTCTGACGTTCCGCATCCCGTATTACGTGGGC<br>CCACTGGCGCGCGGCAATTCGCGCTTCGCGTGGATGACTAGAAAATCAGAGG<br>AAACCATCACTCCTTGGAATTTCGAGGAAGTTGTGGATAAGGGAGCTTCGGC<br>ACAAAGCTTCATCGAACGAATGACCAACTTCGACAAGAATCTCCCAAACGAG<br>AAGGTGCTTCCTAAGCACAGCCTCCTTTACGAATACTTCACTGTCTACAACG<br>AACTGACTAAAGTGAAATACGTTACTGAAGGAATGAGGAAGCCGGCCTTTCT<br>GTCCGGAGAACAGAAGAAAGCAATTGTCGATCTGCTGTTCAAGACCAACCGC<br>AAGGTGACCGTCAAGCAGCTTAAAGAGGACTACTTCAAGAAGATCGAGTGTT<br>TCGACTCAGTGGAAATCAGCGGGGTGGAGGACAGATTCAACGCTTCGCTGGG<br>AACCTATCATGATCTCCTGAAGATCATCAAGGACAAGGACTTCCTTGACAAC<br>GAGGAGAACGAGGACATCCTGGAAGATATCGTCCTGACCTTGACCCTTTTCG<br>AGGATCGCGAGATGATCGAGGAGAGGCTTAAGACCTACGCTCATCTCTTCGA<br>CGATAAGGTCATGAAACAACTCAAGCGCCGCCGGTACACTGGTTGGGGCCGC<br>CTCTCCCGCAAGCTGATCAACGGTATTCGCGATAAACAGAGCGGTAAAACTA<br>TCCTGGATTTCCTCAAATCGGATGGCTTCGCTAATCGTAACTTCATGCAATT<br>GATCCACGACGACAGCCTGACCTTTAAGGAGGACATCCAAAAAGCACAAGTG<br>TCCGGACAGGGAGACTCACTCCATGAACACATCGCGAATCTGGCCGGTTCGC<br>CGGCGATTAAGAAGGGAATTCTGCAAACTGTGAAGGTGGTCGACGAGCTGGT<br>GAAGGTCATGGGACGGCACAAACCGGAGAATATCGTGATTGAAATGGCCCGA<br>GAAAACCAGACTACCCAGAAGGGCCAGAAAAACTCCCGCGAAAGGATGAAGC<br>GGATCGAAGAAGGAATCAAGGAGCTGGGCAGCCAGATCCTGAAAGAGCACCC<br>GGTGGAAAACACGCAGCTGCAGAACGAGAAGCTCTACCTGTACTATTTGCAA<br>AATGGACGGGACATGTACGTGGACCAAGAGCTGGACATCAATCGGTTGTCTG<br>ATTACGACGTGGACCACATCGTTCCACAGTCCTTTCTGAAGGATGACTCGAT<br>CGATAACAAGGTGTTGACTCGCAGCGACAAGAACAGAGGGAAGTCAGATAAT<br>GTGCCATCGGAGGAGGTCGTGAAGAAGATGAAGAATTACTGGCGGCAGCTCC<br>TGAATGCGAAGCTGATTACCCAGAGAAAGTTTGACAATCTCACTAAAGCCGA<br>GCGCGGCGGACTCTCAGAGCTGGATAAGGCTGGATTCATCAAACGGCAGCTG<br>GTCGAGACTCGGCAGATTACCAAGCACGTGGCGCAGATCTTGGACTCCCGCA<br>TGAACACTAAATACGACGAGAACGATAAGCTCATCCGGGAAGTGAAGGTGAT<br>TACCCTGAAAAGCAAACTTGTGTCGGACTTTCGGAAGGACTTTCAGTTTTAC<br>AAAGTGAGAGAAATCAACAACTACCATCACGCGCATGACGCATACCTCAACG<br>CTGTGGTCGGTACCGCCCTGATCAAAAAGTACCCTAAACTTGAATCGGAGTT<br>TGTGTACGGAGACTACAAGGTCTACGACGTGAGGAAGATGATAGCCAAGTCC<br>GAACAGGAAATCGGGAAAGCAACTGCGAAATACTTCTTTTACTCAAACATCA<br>TGAACTTTTTCAAGACTGAAATTACGCTGGCCAATGGAGAAATCAGGAAGAG<br>GCCACTGATCGAAACTAACGGAGAAACGGGCGAAATCGTGTGGGACAAGGGC<br>AGGGACTTCGCAACTGTTCGCAAAGTGCTCTCTATGCCGCAAGTCAATATTG<br>TGAAGAAAACCGAAGTGCAAACCGGCGGATTTTCAAAGGAATCGATCCTCCC<br>AAAGAGAAATAGCGACAAGCTCATTGCACGCAAGAAAGACTGGGACCCGAAG<br>AAGTACGGAGGATTCGATTCGCCGACTGTCGCATACTCCGTCCTCGTGGTGG<br>CCAAGGTGGAGAAGGGAAAGAGCAAAAAGCTCAAATCCGTCAAAGAGCTGCT<br>GGGGATTACCATCATGGAACGATCCTCGTTCGAGAAGAACCCGATTGATTTC<br>CTCGAGGCGAAGGGTTACAAGGAGGTGAAGAAGGATCTGATCATCAAACTCC | |

(continued)

| Description | Sequence | SEQ ID No. |
|---|---|---|
| | CCAAGTACTCACTGTTCGAACTGGAAAATGGTCGGAAGCGCATGCTGGCTTC GGCCGGAGAACTCCAAAAAGGAAATGAGCTGGCCTTGCCTAGCAAGTACGTC AACTTCCTCTATCTTGCTTCGCACTACGAAAAACTCAAAGGGTCACCGGAAG ATAACGAACAGAAGCAGCTTTTCGTGGAGCAGCACAAGCATTATCTGGATGA AATCATCGAACAAATCTCCGAGTTTTCAAAGCGCGTGATCCTCGCCGACGCC AACCTCGACAAAGTCCTGTCGGCCTACAATAAGCATAGAGATAAGCCGATCA GAGAACAGGCCGAGAACATTATCCACTTGTTCACCCTGACTAACCTGGGAGC CCCAGCCGCCTTCAAGTACTTCGATACTACTATCGATCGCAAAAGATACACG TCCACCAAGGAAGTTCTGGACGCGACCCTGATCCACCAAAGCATCACTGGAC TCTACGAAACTAGGATCGATCTGTCGCAGCTGGGTGGCGATTGATAGTCTAG CCATCACATTTAAAAGCATCTCAGCCTACCATGAGAATAAGAGAAAGAAAAT GAAGATCAATAGCTTATTCATCTCTTTTTCTTTTTCGTTGGTGTAAAGCCAA CACCCTGTCTAAAAAACATAAATTTCTTTAATCATTTTGCCTCTTTTCTCTG TGCTTCAATTAATAAAAAATGGAAAGAACCTCGAG | |

(continued)

| Description | Sequence | SEQ ID No. |
|---|---|---|
| Cas9 transcript comprising Kozak sequence with Cas9 ORF using codons with generally high expression in humans | GGGTCCCGCAGTCGGCGTCCAGCGGCTCTGCTTGTTCGTGTGTGTGTCGTTG CAGGCCTTATTCGGATCCGCCACCATGCCTAAGAAAAAGCGGAAGGTCGACG GGGATAAGAAGTACTCAATCGGGCTGGATATCGGAACTAATTCCGTGGGTTG GGCAGTGATCACGGATGAATACAAAGTGCCGTCCAAGAAGTTCAAGGTCCTG GGGAACACCGATAGACACAGCATCAAGAAAAATCTCATCGGAGCCCTGCTGT TTGACTCCGGCGAAACCGCAGAAGCGACCCGGCTCAAACGTACCGCGAGGCG ACGCTACACCCGGCGGAAGAATCGCATCTGCTATCTGCAAGAGATCTTTTCG AACGAAATGGCAAAGGTCGACGACAGCTTCTTCCACCGCCTGGAAGAATCTT TCCTGGTGGAGGAGGACAAGAAGCATGAACGGCATCCTATCTTTGGAAACAT CGTCGACGAAGTGGCGTACCACGAAAAGTACCCGACCATCTACCATCTGCGG AAGAAGTTGGTTGACTCAACTGACAAGGCCGACCTCAGATTGATCTACTTGG CCCTCGCCCATATGATCAAATTCCGCGGACACTTCCTGATCGAAGGCGATCT GAACCCTGATAACTCCGACGTGGATAAGCTTTTCATTCAACTGGTGCAGACC TACAACCAACTGTTCGAAGAAAACCCAATCAATGCTAGCGGCGTCGATGCCA AGGCCATCCTGTCCGCCCGGCTGTCGAAGTCGCGGCGCCTCGAAAACCTGAT CGCACAGCTGCCGGGAGAGAAAAAGAACGGACTTTTCGGCAACTTGATCGCT CTCTCACTGGGACTCACTCCCAATTTCAAGTCCAATTTTGACCTGGCCGAGG ACGCGAAGCTGCAACTCTCAAAGGACACCTACGACGACGACTTGGACAATTT GCTGGCACAAATTGGCGATCAGTACGCGGATCTGTTCCTTGCCGCTAAGAAC CTTTCGGACGCAATCTTGCTGTCCGATATCCTGCGCGTGAACACCGAAATAA CCAAAGCGCCGCTTAGCGCCTCGATGATTAAGCGGTACGACGAGCATCACCA GGATCTCACGCTGCTCAAAGCGCTCGTGAGACAGCAACTGCCTGAAAAGTAC AAGGAGATCTTCTTCGACCAGTCCAAGAATGGGTACGCAGGGTACATCGATG GAGGCGCTAGCCAGGAAGAGTTCTATAAGTTCATCAAGCCAATCCTGGAAAA GATGGACGGAACCGAAGAACTGCTGGTCAAGCTGAACAGGGAGGATCTGCTC CGGAAACAGAGAACCTTTGACAACGGATCCATTCCCCACCAGATCCATCTGG GTGAGCTGCACGCCATCTTGCGGCGCCAGGAGGACTTTTACCCATTCCTCAA GGACAACCGGGAAAAGATCGAGAAAATTCTGACGTTCCGCATCCCGTATTAC GTGGGCCCACTGGCGCGCGGCAATTCGCGCTTCGCGTGGATGACTAGAAAAT CAGAGGAAACCATCACTCCTTGGAATTTCGAGGAAGTTGTGGATAAGGGAGC TTCGGCACAAAGCTTCATCGAACGAATGACCAACTTCGACAAGAATCTCCCA AACGAGAAGGTGCTTCCTAAGCACAGCCTCCTTTACGAATACTTCACTGTCT ACAACGAACTGACTAAAGTGAAATACGTTACTGAAGGAATGAGGAAGCCGGC CTTTCTGTCCGGAGAACAGAAGAAAGCAATTGTCGATCTGCTGTTCAAGACC AACCGCAAGGTGACCGTCAAGCAGCTTAAAGAGGACTACTTCAAGAAGATCG AGTGTTTCGACTCAGTGGAAATCAGCGGGGTGGAGGACAGATTCAACGCTTC GCTGGGAACCTATCATGATCTCCTGAAGATCATCAAGGACAAGGACTTCCTT GACAACGAGGAGAACGAGGACATCCTGGAAGATATCGTCCTGACCTTGACCC TTTTCGAGGATCGCGAGATGATCGAGGAGAGGCTTAAGACCTACGCTCATCT CTTCGACGATAAGGTCATGAAACAACTCAAGCGCCGCCGGTACACTGGTTGG GGCCGCCTCTCCCGCAAGCTGATCAACGGTATTCGCGATAAACAGAGCGGTA AAACTATCCTGGATTTCCTCAAATCGGATGGCTTCGCTAATCGTAACTTCAT GCAATTGATCCACGACGACAGCCTGACCTTTAAGGAGGACATCCAAAAAGCA CAAGTGTCCGGACAGGGAGACTCACTCCATGAACACATCGCGAATCTGGCCG | 49 |

(continued)

| Description | Sequence | SEQ ID No. |
|---|---|---|
| | GTTCGCCGGCGATTAAGAAGGGAATTCTGCAAACTGTGAAGGTGGTCGACGA GCTGGTGAAGGTCATGGGACGGCACAAACCGGAGAATATCGTGATTGAAATG GCCCGAGAAAACCAGACTACCCAGAAGGGCCAGAAAAACTCCCGCGAAAGGA TGAAGCGGATCGAAGAAGGAATCAAGGAGCTGGGCAGCCAGATCCTGAAAGA GCACCCGGTGGAAAACACGCAGCTGCAGAACGAGAAGCTCTACCTGTACTAT TTGCAAAATGGACGGGACATGTACGTGGACCAAGAGCTGGACATCAATCGGT TGTCTGATTACGACGTGGACCACATCGTTCCACAGTCCTTTCTGAAGGATGA CTCGATCGATAACAAGGTGTTGACTCGCAGCGACAAGAACAGAGGGAAGTCA GATAATGTGCCATCGGAGGAGGTCGTGAAGAAGATGAAGAATTACTGGCGGC AGCTCCTGAATGCGAAGCTGATTACCCAGAGAAAGTTTGACAATCTCACTAA AGCCGAGCGCGGCGGACTCTCAGAGCTGGATAAGGCTGGATTCATCAAACGG CAGCTGGTCGAGACTCGGCAGATTACCAAGCACGTGGCGCAGATCTTGGACT CCCGCATGAACACTAAATACGACGAGAACGATAAGCTCATCCGGGAAGTGAA GGTGATTACCCTGAAAAGCAAACTTGTGTCGGACTTTCGGAAGGACTTTCAG TTTTACAAAGTGAGAGAAATCAACAACTACCATCACGCGCATGACGCATACC TCAACGCTGTGGTCGGTACCGCCCTGATCAAAAAGTACCCTAAACTTGAATC GGAGTTTGTGTACGGAGACTACAAGGTCTACGACGTGAGGAAGATGATAGCC AAGTCCGAACAGGAAATCGGGAAAGCAACTGCGAAATACTTCTTTTACTCAA ACATCATGAACTTTTTCAAGACTGAAATTACGCTGGCCAATGGAGAAATCAG GAAGAGGCCACTGATCGAAACTAACGGAGAAACGGGCGAAATCGTGTGGGAC AAGGGCAGGGACTTCGCAACTGTTCGCAAAGTGCTCTCTATGCCGCAAGTCA ATATTGTGAAGAAAACCGAAGTGCAAACCGGCGGATTTTCAAAGGAATCGAT CCTCCCAAAGAGAAATAGCGACAAGCTCATTGCACGCAAGAAAGACTGGGAC CCGAAGAAGTACGGAGGATTCGATTCGCCGACTGTCGCATACTCCGTCCTCG TGGTGGCCAAGGTGGAGAAGGGAAAGAGCAAAAAGCTCAAATCCGTCAAAGA GCTGCTGGGGATTACCATCATGGAACGATCCTCGTTCGAGAAGAACCCGATT GATTTCCTCGAGGCGAAGGGTTACAAGGAGGTGAAGAAGGATCTGATCATCA AACTCCCCAAGTACTCACTGTTCGAACTGGAAAATGGTCGGAAGCGCATGCT GGCTTCGGCCGGAGAACTCCAAAAAGGAAATGAGCTGGCCTTGCCTAGCAAG TACGTCAACTTCCTCTATCTTGCTTCGCACTACGAAAAACTCAAAGGGTCAC CGGAAGATAACGAACAGAAGCAGCTTTTCGTGGAGCAGCACAAGCATTATCT GGATGAAATCATCGAACAAATCTCCGAGTTTTCAAAGCGCGTGATCCTCGCC GACGCCAACCTCGACAAAGTCCTGTCGGCCTACAATAAGCATAGAGATAAGC CGATCAGAGAACAGGCCGAGAACATTATCCACTTGTTCACCCTGACTAACCT GGGAGCCCCAGCCGCCTTCAAGTACTTCGATACTACTATCGATCGCAAAAGA TACACGTCCACCAAGGAAGTTCTGGACGCGACCCTGATCCACCAAAGCATCA CTGGACTCTACGAAACTAGGATCGATCTGTCGCAGCTGGGTGGCGATTGATA GTCTAGCCATCACATTTAAAAGCATCTCAGCCTACCATGAGAATAAGAGAAA GAAAATGAAGATCAATAGCTTATTCATCTCTTTTTCTTTTTCGTTGGTGTAA AGCCAACACCCTGTCTAAAAAACATAAATTTCTTTAATCATTTTGCCTCTTT TCTCTGTGCTTCAATTAATAAAAAATGGAAAGAACCTCGAG | |

(continued)

| Description | Sequence | SEQ ID No. |
|---|---|---|
| **Cas9 ORF with splice junctions removed; 12.75% U content** | ATGGACAAGAAGTACAGCATCGGACTGGACATCGGAACAAACAGCGTCGGAT GGGCAGTCATCACAGACGAATACAAGGTCCCGAGCAAGAAGTTCAAGGTCCT GGGAAACACAGACAGACACAGCATCAAGAAGAACCTGATCGGAGCACTGCTG TTCGACAGCGGAGAAACAGCAGAAGCAACAAGACTGAAGAGAACAGCAAGAA GAAGATACACAAGAAGAAAGAACAGAATCTGCTACCTGCAGGAAATCTTCAG CAACGAAATGGCAAAGGTCGACGACAGCTTCTTCCACcggCTGGAAGAAAGC TTCCTGGTCGAAGAAGACAAGAAGCACGAAAGACACCCGATCTTCGGAAACA TCGTCGACGAAGTCGCATACCACGAAAAGTACCCGACAATCTACCACCTGAG AAAGAAGCTGGTCGACAGCACAGACAAGGCAGACCTGAGACTGATCTACCTG GCACTGGCACACATGATCAAGTTCAGAGGACACTTCCTGATCGAAGGAGACC TGAACCCGGACAACAGCGACGTCGACAAGCTGTTCATCCAGCTGGTCCAGAC ATACAACCAGCTGTTCGAAGAAAACCCGATCAACGCAAGCGGAGTCGACGCA AAGGCAATCCTGAGCGCAAGACTGAGCAAGAGCAGAAGACTGGAAAACCTGA TCGCACAGCTGCCGGGAGAAAAGAAGAACGGACTGTTCGGAAACCTGATCGC ACTGAGCCTGGGACTGACACCGAACTTCAAGAGCAACTTCGACCTGGCAGAA GACGCAAAGCTGCAGCTGAGCAAGGACACATACGACGACGACCTGGACAACC TGCTGGCACAGATCGGAGACCAGTACGCAGACCTGTTCCTGGCAGCAAAGAA | **50** |

(continued)

| Description | Sequence | SEQ ID No. |
|---|---|---|
| | CCTGAGCGACGCAATCCTGCTGAGCGACATCCTGAGAGTCAACACAGAAATC<br>ACAAAGGCACCGCTGAGCGCAAGCATGATCAAGAGATACGACGAACACCACC<br>AGGACCTGACACTGCTGAAGGCACTGGTCAGACAGCAGCTGCCGGAAAAGTA<br>CAAGGAAATCTTCTTCGACCAGAGCAAGAACGGATACGCAGGATACATCGAC<br>GGAGGAGCAAGCCAGGAAGAATTCTACAAGTTCATCAAGCCGATCCTGGAAA<br>AGATGGACGGAACAGAAGAACTGCTGGTCAAGCTGAACAGAGAAGACCTGCT<br>GAGAAAGCAGAGAACATTCGACAACGGAAGCATCCCGCACCAGATCCACCTG<br>GGAGAACTGCACGCAATCCTGAGAAGACAGGAAGACTTCTACCCGTTCCTGA<br>AGGACAACAGAGAAAAGATCGAAAAGATCCTGACATTCAGAATCCCGTACTA<br>CGTCGGACCGCTGGCAAGAGGAAACAGCAGATTCGCATGGATGACAAGAAAG<br>AGCGAAGAAACAATCACACCGTGGAACTTCGAAGAAGTCGTCGACAAGGGAG<br>CAAGCGCACAGAGCTTCATCGAAAGAATGACAAACTTCGACAAGAACCTGCC<br>GAACGAAAAGGTCCTGCCGAAGCACAGCCTGCTGTACGAATACTTCACAGTC<br>TACAACGAACTGACAAAGGTCAAGTACGTCACAGAAGGAATGAGAAAGCCGG<br>CATTCCTGAGCGGAGAACAGAAGAAGGCAATCGTCGACCTGCTGTTCAAGAC<br>AAACAGAAAGGTCACAGTCAAGCAGCTGAAGGAAGACTACTTCAAGAAGATC<br>GAATGCTTCGACAGCGTCGAAATCAGCGGAGTCGAAGACAGATTCAACGCAA<br>GCCTGGGAACATACCACGACCTGCTGAAGATCATCAAGGACAAGGACTTCCT<br>GGACAACGAAGAAAACGAAGACATCCTGGAAGACATCGTCCTGACACTGACA<br>CTGTTCGAAGACAGAGAAATGATCGAAGAAAGACTGAAGACATACGCACACC<br>TGTTCGACGACAAGGTCATGAAGCAGCTGAAGAGAAGAAGATACACAGGATG<br>GGGAAGACTGAGCAGAAAGCTGATCAACGGAATCAGAGACAAGCAGAGCGGA<br>AAGACAATCCTGGACTTCCTGAAGAGCGACGGATTCGCAAACAGAAACTTCA<br>TGCAGCTGATCCACGACGACAGCCTGACATTCAAGGAAGACATCCAGAAGGC<br>ACAGGTCAGCGGACAGGGAGACAGCCTGCACGAACACATCGCAAACCTGGCA<br>GGAAGCCCGGCAATCAAGAAGGGAATCCTGCAGACAGTCAAGGTCGTCGACG<br>AACTGGTCAAGGTCATGGGAAGACACAAGCCGGAAAACATCGTCATCGAAAT<br>GGCAAGAGAAAACCAGACAACACAGAAGGGACAGAAGAACAGCAGAGAAAGA<br>ATGAAGAGAATCGAAGAAGGAATCAAGGAACTGGGAAGCCAGATCCTGAAGG<br>AACACCCGGTCGAAAACACACAGCTGCAGAACGAAAAGCTGTACCTGTACTA<br>CCTGCAaAACGGAAGAGACATGTACGTCGACCAGGAACTGGACATCAACAGA<br>CTGAGCGACTACGACGTCGACCACATCGTCCCGCAGAGCTTCCTGAAGGACG<br>ACAGCATCGACAACAAGGTCCTGACAAGAAGCGACAAGAACAGAGGAAAGAG<br>CGACAACGTCCCGAGCGAAGAAGTCGTCAAGAAGATGAAGAACTACTGGAGA<br>CAGCTGCTGAACGCAAAGCTGATCACACAGAGAAAGTTCGACAACCTGACAA<br>AGGCAGAGAGAGGAGGACTGAGCGAACTGGACAAGGCAGGATTCATCAAGAG<br>ACAGCTGGTCGAAACAAGACAGATCACAAAGCACGTCGCACAGATCCTGGAC<br>AGCAGAATGAACACAAAGTACGACGAAAACGACAAGCTGATCAGAGAAGTCA<br>AGGTCATCACACTGAAGAGCAAGCTGGTCAGCGACTTCAGAAAGGACTTCCA<br>GTTCTACAAGGTCAGAGAAATCAACAACTACCACCACGCACACGACGCATAC<br>CTGAACGCAGTCGTCGGAACAGCACTGATCAAGAAGTACCCGAAGCTGGAAA<br>GCGAATTCGTCTACGGAGACTACAAGGTCTACGACGTCAGAAAGATGATCGC<br>AAAGAGCGAACAGGAAATCGGAAAGGCAACAGCAAAGTACTTCTTCTACAGC<br>AACATCATGAACTTCTTCAAGACAGAAATCACACTGGCAAACGGAGAAATCA<br>GAAAGAGACCGCTGATCGAAACAAACGGAGAAACAGGAGAAATCGTCTGGGA<br>CAAGGGAAGAGACTTCGCAACAGTCAGAAAGGTCCTGAGCATGCCGCAGGTC<br>AACATCGTCAAGAAGACAGAAGTCCAGACAGGAGGATTCAGCAAGGAAAGCA<br>TCCTGCCGAAGAGAAACAGCGACAAGCTGATCGCAAGAAAGAAGGACTGGGA<br>CCCGAAGAAGTACGGAGGATTCGACAGCCCGACAGTCGCATACAGCGTCCTG<br>GTCGTCGCAAAGGTCGAAAAGGGAAAGAGCAAGAAGCTGAAGAGCGTCAAGG<br>AACTGCTGGGAATCACAATCATGGAAAGAAGCAGCTTCGAAAAGAACCCGAT<br>CGACTTCCTGGAAGCAAAGGGATACAAGGAAGTCAAGAAGGACCTGATCATC<br>AAGCTGCCGAAGTACAGCCTGTTCGAACTGGAAAACGGAAGAAAGAGAATGC<br>TGGCAAGCGCAGGAGAACTGCAGAAGGGAAACGAACTGGCACTGCCGAGCAA<br>GTACGTCAACTTCCTGTACCTGGCAAGCCACTACGAAAAGCTGAAGGGAAGC<br>CCGGAAGACAACGAACAGAAGCAGCTGTTCGTCGAACAGCACAAGCACTACC<br>TGGACGAAATCATCGAACAGATCAGCGAATTCAGCAAGAGAGTCATCCTGGC<br>AGACGCAAACCTGGACAAGGTCCTGAGCGCATACAACAAGCACAGAGACAAG<br>CCGATCAGAGAACAGGCAGAAAACATCATCCACCTGTTCACACTGACAAACC<br>TGGGAGCACCGGCAGCATTCAAGTACTTCGACACAACAATCGACAGAAAGAG | |

(continued)

| Description | Sequence | SEQ ID No. |
|---|---|---|
| | ATACACAAGCACAAAGGAAGTCCTGGACGCAACACTGATCCACCAGAGCATC ACAGGACTGTACGAAACAAGAATCGACCTGAGCCAGCTGGGAGGAGACGGAG GAGGAAGCCCGAAGAAGAAGAGAAAGGTCTAG | |

(continued)

| Description | Sequence | SEQ ID No. |
|---|---|---|
| Cas9 transcript with 5' UTR of HSD, ORF corresponding to SEQ ID NO: 50, Kozak sequence, and 3' UTR of ALB | GGGTCCCGCAGTCGGCGTCCAGCGGCTCTGCTTGTTCGTGTGTGTGTCGTTG<br>CAGGCCTTATTCGGATCCGCCACCATGGACAAGAAGTACAGCATCGGACTGG<br>ACATCGGAACAAACAGCGTCGGATGGGCAGTCATCACAGACGAATACAAGGT<br>CCCGAGCAAGAAGTTCAAGGTCCTGGGAAACACAGACAGACACAGCATCAAG<br>AAGAACCTGATCGGAGCACTGCTGTTCGACAGCGGAGAAACAGCAGAAGCAA<br>CAAGACTGAAGAGAACAGCAAGAAGAAGATACACAAGAAGAAAGAACAGAAT<br>CTGCTACCTGCAGGAAATCTTCAGCAACGAAATGGCAAAGGTCGACGACAGC<br>TTCTTCCACcggCTGGAAGAAAGCTTCCTGGTCGAAGAAGACAAGAAGCACG<br>AAAGACACCCGATCTTCGGAAACATCGTCGACGAAGTCGCATACCACGAAAA<br>GTACCCGACAATCTACCACCTGAGAAAGAAGCTGGTCGACAGCACAGACAAG<br>GCAGACCTGAGACTGATCTACCTGGCACTGGCACACATGATCAAGTTCAGAG<br>GACACTTCCTGATCGAAGGAGACCTGAACCCGGACAACAGCGACGTCGACAA<br>GCTGTTCATCCAGCTGGTCCAGACATACAACCAGCTGTTCGAAGAAAACCCG<br>ATCAACGCAAGCGGAGTCGACGCAAAGGCAATCCTGAGCGCAAGACTGAGCA<br>AGAGCAGAAGACTGGAAAACCTGATCGCACAGCTGCCGGGAGAAAAGAAGAA<br>CGGACTGTTCGGAAACCTGATCGCACTGAGCCTGGGACTGACACCGAACTTC<br>AAGAGCAACTTCGACCTGGCAGAAGACGCAAAGCTGCAGCTGAGCAAGGACA<br>CATACGACGACGACCTGGACAACCTGCTGGCACAGATCGGAGACCAGTACGC<br>AGACCTGTTCCTGGCAGCAAAGAACCTGAGCGACGCAATCCTGCTGAGCGAC<br>ATCCTGAGAGTCAACACAGAAATCACAAAGGCACCGCTGAGCGCAAGCATGA<br>TCAAGAGATACGACGAACACCACCAGGACCTGACACTGCTGAAGGCACTGGT<br>CAGACAGCAGCTGCCGGAAAAGTACAAGGAAATCTTCTTCGACCAGAGCAAG<br>AACGGATACGCAGGATACATCGACGGAGGAGCAAGCCAGGAAGAATTCTACA<br>AGTTCATCAAGCCGATCCTGGAAAAGATGGACGGAACAGAAGAACTGCTGGT<br>CAAGCTGAACAGAGAAGACCTGCTGAGAAAGCAGAGAACATTCGACAACGGA<br>AGCATCCCGCACCAGATCCACCTGGGAGAACTGCACGCAATCCTGAGAAGAC<br>AGGAAGACTTCTACCCGTTCCTGAAGGACAACAGAGAAAAGATCGAAAAGAT<br>CCTGACATTCAGAATCCCGTACTACGTCGGACCGCTGGCAAGAGGAAACAGC<br>AGATTCGCATGGATGACAAGAAAGAGCGAAGAAACAATCACACCGTGGAACT<br>TCGAAGAAGTCGTCGACAAGGGAGCAAGCGCACAGAGCTTCATCGAAAGAAT<br>GACAAACTTCGACAAGAACCTGCCGAACGAAAAGGTCCTGCCGAAGCACAGC<br>CTGCTGTACGAATACTTCACAGTCTACAACGAACTGACAAAGGTCAAGTACG<br>TCACAGAAGGAATGAGAAAGCCGGCATTCCTGAGCGGAGAACAGAAGAAGGC<br>AATCGTCGACCTGCTGTTCAAGACAAACAGAAAGGTCACAGTCAAGCAGCTG<br>AAGGAAGACTACTTCAAGAAGATCGAATGCTTCGACAGCGTCGAAATCAGCG<br>GAGTCGAAGACAGATTCAACGCAAGCCTGGGAACATACCACGACCTGCTGAA<br>GATCATCAAGGACAAGGACTTCCTGGACAACGAAGAAAACGAAGACATCCTG<br>GAAGACATCGTCCTGACACTGACACTGTTCGAAGACAGAGAAATGATCGAAG<br>AAAGACTGAAGACATACGCACACCTGTTCGACGACAAGGTCATGAAGCAGCT<br>GAAGAGAAGAAGATACACAGGATGGGGAAGACTGAGCAGAAAGCTGATCAAC<br>GGAATCAGAGACAAGCAGAGCGGAAAGACAATCCTGGACTTCCTGAAGAGCG<br>ACGGATTCGCAAACAGAAACTTCATGCAGCTGATCCACGACGACAGCCTGAC<br>ATTCAAGGAAGACATCCAGAAGGCACAGGTCAGCGGACAGGGAGACAGCCTG<br>CACGAACACATCGCAAACCTGGCAGGAAGCCCGGCAATCAAGAAGGGAATCC<br>TGCAGACAGTCAAGGTCGTCGACGAACTGGTCAAGGTCATGGGAAGACACAA<br>GCCGGAAAACATCGTCATCGAAATGGCAAGAGAAAACCAGACAACACAGAAG<br>GGACAGAAGAACAGCAGAGAAAGAATGAAGAGAATCGAAGAAGGAATCAAGG<br>AACTGGGAAGCCAGATCCTGAAGGAACACCCGGTCGAAAACACACAGCTGCA<br>GAACGAAAAGCTGTACCTGTACTACCTGCAaAACGGAAGAGACATGTACGTC<br>GACCAGGAACTGGACATCAACAGACTGAGCGACTACGACGTCGACCACATCG<br>TCCCGCAGAGCTTCCTGAAGGACGACAGCATCGACAACAAGGTCCTGACAAG<br>AAGCGACAAGAACAGAGGAAAGAGCGACAACGTCCCGAGCGAAGAAGTCGTC<br>AAGAAGATGAAGAACTACTGGAGACAGCTGCTGAACGCAAAGCTGATCACAC<br>AGAGAAAGTTCGACAACCTGACAAAGGCAGAGAGAGGAGGACTGAGCGAACT<br>GGACAAGGCAGGATTCATCAAGAGACAGCTGGTCGAAACAAGACAGATCACA | 51 |

(continued)

| Description | Sequence | SEQ ID No. |
|---|---|---|
| | AAGCACGTCGCACAGATCCTGGACAGCAGAATGAACACAAAGTACGACGAAA ACGACAAGCTGATCAGAGAAGTCAAGGTCATCACACTGAAGAGCAAGCTGGT CAGCGACTTCAGAAAGGACTTCCAGTTCTACAAGGTCAGAGAAATCAACAAC TACCACCACGCACACGACGCATACCTGAACGCAGTCGTCGGAACAGCACTGA TCAAGAAGTACCCGAAGCTGGAAAGCGAATTCGTCTACGGAGACTACAAGGT CTACGACGTCAGAAAGATGATCGCAAAGAGCGAACAGGAAATCGGAAAGGCA ACAGCAAAGTACTTCTTCTACAGCAACATCATGAACTTCTTCAAGACAGAAA TCACACTGGCAAACGGAGAAATCAGAAAGAGACCGCTGATCGAAACAAACGG AGAAACAGGAGAAATCGTCTGGGACAAGGGAAGAGACTTCGCAACAGTCAGA AAGGTCCTGAGCATGCCGCAGGTCAACATCGTCAAGAAGACAGAAGTCCAGA CAGGAGGATTCAGCAAGGAAAGCATCCTGCCGAAGAGAAACAGCGACAAGCT GATCGCAAGAAAGAAGGACTGGGACCCGAAGAAGTACGGAGGATTCGACAGC CCGACAGTCGCATACAGCGTCCTGGTCGTCGCAAAGGTCGAAAAGGGAAAGA GCAAGAAGCTGAAGAGCGTCAAGGAACTGCTGGGAATCACAATCATGGAAAG AAGCAGCTTCGAAAAGAACCCGATCGACTTCCTGGAAGCAAAGGGATACAAG GAAGTCAAGAAGGACCTGATCATCAAGCTGCCGAAGTACAGCCTGTTCGAAC TGGAAAACGGAAGAAAGAGAATGCTGGCAAGCGCAGGAGAACTGCAGAAGGG AAACGAACTGGCACTGCCGAGCAAGTACGTCAACTTCCTGTACCTGGCAAGC CACTACGAAAAGCTGAAGGGAAGCCCGGAAGACAACGAACAGAAGCAGCTGT TCGTCGAACAGCACAAGCACTACCTGGACGAAATCATCGAACAGATCAGCGA ATTCAGCAAGAGAGTCATCCTGGCAGACGCAAACCTGGACAAGGTCCTGAGC GCATACAACAAGCACAGAGACAAGCCGATCAGAGAACAGGCAGAAAACATCA TCCACCTGTTCACACTGACAAACCTGGGAGCACCGGCAGCATTCAAGTACTT CGACACAACAATCGACAGAAAGAGATACACAAGCACAAAGGAAGTCCTGGAC GCAACACTGATCCACCAGAGCATCACAGGACTGTACGAAACAAGAATCGACC TGAGCCAGCTGGGAGGAGACGGAGGAGGAAGCCCGAAGAAGAAGAGAAAGGT CTAGCTAGCCATCACATTTAAAAGCATCTCAGCCTACCATGAGAATAAGAGA AAGAAAATGAAGATCAATAGCTTATTCATCTCTTTTTCTTTTTCGTTGGTGT AAAGCCAACACCCTGTCTAAAAAACATAAATTTCTTTAATCATTTTGCCTCT TTTCTCTGTGCTTCAATTAATAAAAAATGGAAAGAACCTCGAG | |

(continued)

| Description | Sequence | SEQ ID No. |
|---|---|---|
| **Cas9 transcript with 5' UTR of HSD, ORF corresponding to SEQ ID NO: 52, Kozak sequence, and 3' UTR of ALB** | GGGTCCCGCAGTCGGCGTCCAGCGGCTCTGCTTGTTCGTGTGTGTGTCGTTG CAGGCCTTATTCGGATCCGCCACCATGGACAAGAAGTACAGCATCGGCCTGG ACATCGGCACCAACAGCGTGGGCTGGGCCGTGATCACCGACGAGTACAAGGT GCCCAGCAAGAAGTTCAAGGTGCTGGGCAACACCGACAGACACAGCATCAAG AAGAACCTGATCGGCGCCCTGCTGTTCGACAGCGGCGAGACCGCCGAGGCCA CCAGACTGAAGAGAACCGCCAGAAGAAGATACACCAGAAGAAAGAACAGAAT CTGCTACCTGCAGGAGATCTTCAGCAACGAGATGGCCAAGGTGGACGACAGC TTCTTCCACAGACTGGAGGAGAGCTTCCTGGTGGAGGAGGACAAGAAGCACG AGAGACACCCCATCTTCGGCAACATCGTGGACGAGGTGGCCTACCACGAGAA GTACCCCACCATCTACCACCTGAGAAAGAAGCTGGTGGACAGCACCGACAAG GCCGACCTGAGACTGATCTACCTGGCCCTGGCCCACATGATCAAGTTCAGAG GCCACTTCCTGATCGAGGGCGACCTGAACCCCGACAACAGCGACGTGGACAA GCTGTTCATCCAGCTGGTGCAGACCTACAACCAGCTGTTCGAGGAGAACCCC ATCAACGCCAGCGGCGTGGACGCCAAGGCCATCCTGAGCGCCAGACTGAGCA AGAGCAGAAGACTGGAGAACCTGATCGCCCAGCTGCCCGGCGAGAAGAAGAA CGGCCTGTTCGGCAACCTGATCGCCCTGAGCCTGGGCCTGACCCCCAACTTC AAGAGCAACTTCGACCTGGCCGAGGACGCCAAGCTGCAGCTGAGCAAGGACA CCTACGACGACGACCTGGACAACCTGCTGGCCCAGATCGGCGACCAGTACGC CGACCTGTTCCTGGCCGCCAAGAACCTGAGCGACGCCATCCTGCTGAGCGAC ATCCTGAGAGTGAACACCGAGATCACCAAGGCCCCCCTGAGCGCCAGCATGA TCAAGAGATACGACGAGCACCACCAGGACCTGACCCTGCTGAAGGCCCTGGT GAGACAGCAGCTGCCCGAGAAGTACAAGGAGATCTTCTTCGACCAGAGCAAG AACGGCTACGCCGGCTACATCGACGGCGGCGCCAGCCAGGAGGAGTTCTACA AGTTCATCAAGCCCATCCTGGAGAAGATGGACGGCACCGAGGAGCTGCTGGT GAAGCTGAACAGAGAGGACCTGCTGAGAAAGCAGAGAACCTTCGACAACGGC AGCATCCCCCACCAGATCCACCTGGGCGAGCTGCACGCCATCCTGAGAAGAC AGGAGGACTTCTACCCCTTCCTGAAGGACAACAGAGAGAAGATCGAGAAGAT CCTGACCTTCAGAATCCCCTACTACGTGGGCCCCCTGGCCAGAGGCAACAGC | **53** |

(continued)

| Description | Sequence | SEQ ID No. |
|---|---|---|
| | AGATTCGCCTGGATGACCAGAAAGAGCGAGGAGACCATCACCCCCTGGAACTTCGAGGAGGTGGTGGACAAGGGCGCCAGCGCCCAGAGCTTCATCGAGAGAATGACCAACTTCGACAAGAACCTGCCCAACGAGAAGGTGCTGCCCAAGCACAGCCTGCTGTACGAGTACTTCACCGTGTACAACGAGCTGACCAAGGTGAAGTACGTGACCGAGGGCATGAGAAAGCCCGCCTTCCTGAGCGGCGAGCAGAAGAAGGCCATCGTGGACCTGCTGTTCAAGACCAACAGAAAGGTGACCGTGAAGCAGCTGAAGGAGGACTACTTCAAGAAGATCGAGTGCTTCGACAGCGTGGAGATCAGCGGCGTGGAGGACAGATTCAACGCCAGCCTGGGCACCTACCACGACCTGCTGAAGATCATCAAGGACAAGGACTTCCTGGACAACGAGGAGAACGAGGACATCCTGGAGGACATCGTGCTGACCCTGACCCTGTTCGAGGACAGAGAGATGATCGAGGAGAGACTGAAGACCTACGCCCACCTGTTCGACGACAAGGTGATGAAGCAGCTGAAGAGAAGAAGATACACCGGCTGGGGCAGACTGAGCAGAAAGCTGATCAACGGCATCAGAGACAAGCAGAGCGGCAAGACCATCCTGGACTTCCTGAAGAGCGACGGCTTCGCCAACAGAAACTTCATGCAGCTGATCCACGACGACAGCCTGACCTTCAAGGAGGACATCCAGAAGGCCCAGGTGAGCGGCCAGGGCGACAGCCTGCACGAGCACATCGCCAACCTGGCCGGCAGCCCCGCCATCAAGAAGGGCATCCTGCAGACCGTGAAGGTGGTGGACGAGCTGGTGAAGGTGATGGGCAGACACAAGCCCGAGAACATCGTGATCGAGATGGCCAGAGAGAACCAGACCACCCAGAAGGGCCAGAAGAACAGCAGAGAGAGAATGAAGAGAATCGAGGAGGGCATCAAGGAGCTGGGCAGCCAGATCCTGAAGGAGCACCCCGTGGAGAACACCCAGCTGCAGAACGAGAAGCTGTACCTGTACTACCTGCAGAACGGCAGAGACATGTACGTGGACCAGGAGCTGGACATCAACAGACTGAGCGACTACGACGTGGACCACATCGTGCCCCAGAGCTTCCTGAAGGACGACAGCATCGACAACAAGGTGCTGACCAGAAGCGACAAGAACAGAGGCAAGAGCGACAACGTGCCCAGCGAGGAGGTGGTGAAGAAGATGAAGAACTACTGGAGACAGCTGCTGAACGCCAAGCTGATCACCCAGAGAAAGTTCGACAACCTGACCAAGGCCGAGAGAGGCGGCCTGAGCGAGCTGGACAAGGCCGGCTTCATCAAGAGACAGCTGGTGGAGACCAGACAGATCACCAAGCACGTGGCCCAGATCCTGGACAGCAGAATGAACACCAAGTACGACGAGAACGACAAGCTGATCAGAGAGGTGAAGGTGATCACCCTGAAGAGCAAGCTGGTGAGCGACTTCAGAAAGGACTTCCAGTTCTACAAGGTGAGAGAGATCAACAACTACCACCACGCCCACGACGCCTACCTGAACGCCGTGGTGGGCACCGCCCTGATCAAGAAGTACCCCAAGCTGGAGAGCGAGTTCGTGTACGGCGACTACAAGGTGTACGACGTGAGAAAGATGATCGCCAAGAGCGAGCAGGAGATCGGCAAGGCCACCGCCAAGTACTTCTTCTACAGCAACATCATGAACTTCTTCAAGACCGAGATCACCCTGGCCAACGGCGAGATCAGAAAGAGACCCCTGATCGAGACCAACGGCGAGACCGGCGAGATCGTGTGGGACAAGGGCAGAGACTTCGCCACCGTGAGAAAGGTGCTGAGCATGCCCCAGGTGAACATCGTGAAGAAGACCGAGGTGCAGACCGGCGGCTTCAGCAAGGAGAGCATCCTGCCCAAGAGAAACAGCGACAAGCTGATCGCCAGAAAGAAGGACTGGGACCCCAAGAAGTACGGCGGCTTCGACAGCCCCACCGTGGCCTACAGCGTGCTGGTGGTGGCCAAGGTGGAGAAGGGCAAGAGCAAGAAGCTGAAGAGCGTGAAGGAGCTGCTGGGCATCACCATCATGGAGAGAAGCAGCTTCGAGAAGAACCCCATCGACTTCCTGGAGGCCAAGGGCTACAAGGAGGTGAAGAAGGACCTGATCATCAAGCTGCCCAAGTACAGCCTGTTCGAGCTGGAGAACGGCAGAAAGAGAATGCTGGCCAGCGCCGGCGAGCTGCAGAAGGGCAACGAGCTGGCCCTGCCCAGCAAGTACGTGAACTTCCTGTACCTGGCCAGCCACTACGAGAAGCTGAAGGGCAGCCCCGAGGACAACGAGCAGAAGCAGCTGTTCGTGGAGCAGCACAAGCACTACCTGGACGAGATCATCGAGCAGATCAGCGAGTTCAGCAAGAGAGTGATCCTGGCCGACGCCAACCTGGACAAGGTGCTGAGCGCCTACAACAAGCACAGAGACAAGCCCATCAGAGAGCAGGCCGAGAACATCATCCACCTGTTCACCCTGACCAACCTGGGCGCCCCCGCCGCCTTCAAGTACTTCGACACCACCATCGACAGAAAGAGATACACCAGCACCAAGGAGGTGCTGGACGCCACCCTGATCCACCAGAGCATCACCGGCCTGTACGAGACCAGAATCGACCTGAGCCAGCTGGGCGGCGACGGCGGCGGCAGCCCCAAGAAGAAGAGAAAGGTGTGACTAGCCATCACATTTAAAAGCATCTCAGCCTACCATGAGAATAAGAGAAAGAAAATGAAGATCAATAGCTTATTCATCTCTTTTTCTTTTTCGTTGGTGTAAAGCCAACACCCTGTCTAAAAAACATAAATTTCTTTAATCATTTTGCCTCTTTTCTCTGTGCTTCAATTAATAAAAAATGGAAAGAACCTCGAG | |

(continued)

| Description | Sequence | SEQ ID No. |
|---|---|---|
| **Cas9 transcript with 5' UTR of HSD, ORF corresponding to SEQ ID NO: 54, Kozak sequence, and 3' UTR of ALB** | GGGTCCCGCAGTCGGCGTCCAGCGGCTCTGCTTGTTCGTGTGTGTGTCGTTG CAGGCCTTATTCGGATCCGCCACCATGGACAAAAAATACAGCATAGGGCTAG ACATAGGGACGAACAGCGTAGGGTGGGCGGTAATAACGGACGAATACAAAGT ACCGAGCAAAAAATTCAAAGTACTAGGGAACACGGACCGACACAGCATAAAA AAAAACCTAATAGGGGCGCTACTATTCGACAGCGGGGAAACGGCGGAAGCGA CGCGACTAAAACGAACGGCGCGACGACGATACACGCGACGAAAAAACCGAAT ATGCTACCTACAAGAAATATTCAGCAACGAAATGGCGAAAGTAGACGACAGC TTCTTCCACCGACTAGAAGAAAGCTTCCTAGTAGAAGAAGACAAAAAACACG AACGACACCCGATATTCGGGAACATAGTAGACGAAGTAGCGTACCACGAAAA ATACCCGACGATATACCACCTACGAAAAAAACTAGTAGACAGCACGGACAAA GCGGACCTACGACTAATATACCTAGCGCTAGCGCACATGATAAAATTCCGAG GGCACTTCCTAATAGAAGGGGACCTAAACCCGGACAACAGCGACGTAGACAA ACTATTCATACAACTAGTACAAACGTACAACCAACTATTCGAAGAAAACCCG ATAAACGCGAGCGGGGTAGACGCGAAAGCGATACTAAGCGCGCGACTAAGCA AAAGCCGACGACTAGAAAACCTAATAGCGCAACTACCGGGGGAAAAAAAAAA CGGGCTATTCGGGAACCTAATAGCGCTAAGCCTAGGGCTAACGCCGAACTTC AAAAGCAACTTCGACCTAGCGGAAGACGCGAAACTACAACTAAGCAAAGACA CGTACGACGACGACCTAGACAACCTACTAGCGCAAATAGGGGACCAATACGC GGACCTATTCCTAGCGGCGAAAAACCTAAGCGACGCGATACTACTAAGCGAC ATACTACGAGTAAACACGGAAATAACGAAAGCGCCGCTAAGCGCGAGCATGA TAAAACGATACGACGAACACCACCAAGACCTAACGCTACTAAAAGCGCTAGT ACGACAACAACTACCGGAAAAATACAAAGAAATATTCTTCGACCAAAGCAAA AACGGGTACGCGGGGTACATAGACGGGGGGGCGAGCCAAGAAGAATTCTACA AATTCATAAAACCGATACTAGAAAAAATGGACGGGACGGAAGAACTACTAGT AAAACTAAACCGAGAAGACCTACTACGAAAACAACGAACGTTCGACAACGGG AGCATACCGCACCAAATACACCTAGGGGAACTACACGCGATACTACGACGAC AAGAAGACTTCTACCCGTTCCTAAAAGACAACCGAGAAAAAATAGAAAAAAT ACTAACGTTCCGAATACCGTACTACGTAGGGCCGCTAGCGCGAGGGAACAGC CGATTCGCGTGGATGACGCGAAAAAGCGAAGAAACGATAACGCCGTGGAACT TCGAAGAAGTAGTAGACAAAGGGGCGAGCGCGCAAAGCTTCATAGAACGAAT GACGAACTTCGACAAAAACCTACCGAACGAAAAAGTACTACCGAAACACAGC CTACTATACGAATACTTCACGGTATACAACGAACTAACGAAAGTAAAATACG TAACGGAAGGGATGCGAAAACCGGCGTTCCTAAGCGGGGAACAAAAAAAAGC GATAGTAGACCTACTATTCAAAACGAACCGAAAAGTAACGGTAAAACAACTA AAAGAAGACTACTTCAAAAAAATAGAATGCTTCGACAGCGTAGAAATAAGCG GGGTAGAAGACCGATTCAACGCGAGCCTAGGGACGTACCACGACCTACTAAA AATAATAAAAGACAAAGACTTCCTAGACAACGAAGAAAACGAAGACATACTA GAAGACATAGTACTAACGCTAACGCTATTCGAAGACCGAGAAATGATAGAAG AACGACTAAAAACGTACGCGCACCTATTCGACGACAAAGTAATGAAACAACT AAAACGACGACGATACACGGGGTGGGGGCGACTAAGCCGAAAACTAATAAAC GGGATACGAGACAAACAAAGCGGGAAAACGATACTAGACTTCCTAAAAAGCG ACGGGTTCGCGAACCGAAACTTCATGCAACTAATACACGACGACAGCCTAAC GTTCAAAGAAGACATACAAAAAGCGCAAGTAAGCGGGCAAGGGGACAGCCTA CACGAACACATAGCGAACCTAGCGGGGAGCCCGGCGATAAAAAAAGGGATAC TACAAACGGTAAAAGTAGTAGACGAACTAGTAAAAGTAATGGGGCGACACAA ACCGGAAAACATAGTAATAGAAATGGCGCGAGAAAACCAAACGACGCAAAAA GGGCAAAAAAACAGCCGAGAACGAATGAAACGAATAGAAGAAGGGATAAAAG AACTAGGGAGCCAAATACTAAAAGAACACCCGGTAGAAAACACGCAACTACA AAACGAAAAACTATACCTATACTACCTACAAAACGGGCGAGACATGTACGTA GACCAAGAACTAGACATAAACCGACTAAGCGACTACGACGTAGACCACATAG TACCGCAAAGCTTCCTAAAAGACGACAGCATAGACAACAAAGTACTAACGCG AAGCGACAAAAACCGAGGGAAAAGCGACAACGTACCGAGCGAAGAAGTAGTA AAAAAAATGAAAAACTACTGGCGACAACTACTAAACGCGAAACTAATAACGC AACGAAAATTCGACAACCTAACGAAAGCGGAACGAGGGGGGCTAAGCGAACT AGACAAAGCGGGGTTCATAAAACGACAACTAGTAGAAACGCGACAAATAACG AAACACGTAGCGCAAATACTAGACAGCCGAATGAACACGAAATACGACGAAA ACGACAAACTAATACGAGAAGTAAAAGTAATAACGCTAAAAAGCAAACTAGT AAGCGACTTCCGAAAAGACTTCCAATTCTACAAAGTACGAGAAATAAACAAC TACCACCACGCGCACGACGCGTACCTAAACGCGGTAGTAGGGACGGCGCTAA | **55** |

(continued)

| Description | Sequence | SEQ ID No. |
|---|---|---|
| | TAAAAAAATACCCGAAACTAGAAAGCGAATTCGTATACGGGGACTACAAAGT<br>ATACGACGTACGAAAAATGATAGCGAAAAGCGAACAAGAAATAGGGAAAGCG<br>ACGGCGAAATACTTCTTCTACAGCAACATAATGAACTTCTTCAAAACGGAAA<br>TAACGCTAGCGAACGGGGAAATACGAAAACGACCGCTAATAGAAACGAACGG<br>GGAAACGGGGGAAATAGTATGGGACAAAGGGCGAGACTTCGCGACGGTACGA<br>AAAGTACTAAGCATGCCGCAAGTAAACATAGTAAAAAAAACGGAAGTACAAA<br>CGGGGGGGTTCAGCAAAGAAAGCATACTACCGAAACGAAACAGCGACAAACT<br>AATAGCGCGAAAAAAAGACTGGGACCCGAAAAAATACGGGGGGTTCGACAGC<br>CCGACGGTAGCGTACAGCGTACTAGTAGTAGCGAAAGTAGAAAAAGGGAAAA<br>GCAAAAAACTAAAAAGCGTAAAAGAACTACTAGGGATAACGATAATGGAACG<br>AAGCAGCTTCGAAAAAAACCCGATAGACTTCCTAGAAGCGAAAGGGTACAAA<br>GAAGTAAAAAAAGACCTAATAATAAAACTACCGAAATACAGCCTATTCGAAC<br>TAGAAAACGGGCGAAAACGAATGCTAGCGAGCGCGGGGGAACTACAAAAAGG<br>GAACGAACTAGCGCTACCGAGCAAATACGTAAACTTCCTATACCTAGCGAGC<br>CACTACGAAAAACTAAAAGGGAGCCCGGAAGACAACGAACAAAAACAACTAT<br>TCGTAGAACAACACAAACACTACCTAGACGAAATAATAGAACAAATAAGCGA<br>ATTCAGCAAACGAGTAATACTAGCGGACGCGAACCTAGACAAAGTACTAAGC<br>GCGTACAACAAACACCGAGACAAACCGATACGAGAACAAGCGGAAAACATAA<br>TACACCTATTCACGCTAACGAACCTAGGGGCGCCGGCGGCGTTCAAATACTT<br>CGACACGACGATAGACCGAAAACGATACACGAGCACGAAAGAAGTACTAGAC<br>GCGACGCTAATACACCAAAGCATAACGGGGCTATACGAAACGCGAATAGACC<br>TAAGCCAACTAGGGGGGGACGGGGGGGGGAGCCCGAAAAAAAAACGAAAAGT<br>ATGACTAGCCATCACATTTAAAAGCATCTCAGCCTACCATGAGAATAAGAGA<br>AAGAAAATGAAGATCAATAGCTTATTCATCTCTTTTTCTTTTTCGTTGGTGT<br>AAAGCCAACACCCTGTCTAAAAAACATAAATTTCTTTAATCATTTTGCCTCT<br>TTTCTCTGTGCTTCAATTAATAAAAAATGGAAAGAACCTCGAG | |
| **poly-A 100 sequence** | AAAAAAAAAAAAAAAAAAAAAAAAAAAAAAAAAAAAAAAAAAAAAAAAAAAA<br>AAAAAAAAAAAAAAAAAAAAAAAAAAAAAAAAAAAAAAAAAAAA | **63** |
| **G209 guide RNA** | mC*mC*mA*GUCCAGCGAGGCAAAGGGUUUUAGAGCUAGAAAUAGCAAGUUA<br>AAAUAAGGCUAGUCCGUUAUCAACUUGAAAAAGUGGCACCGAGUCGGUGCmU<br>*mU*mU*U | **64** |

(continued)

| Description | Sequence | SEQ ID No. |
|---|---|---|
| **ORF encoding Neisseria meningitidis Cas9** | ATGGCAGCATTCAAGCCGAACTCGATCAACTACATCCTGGGACTGGACATCG<br>GAATCGCATCGGTCGGATGGGCAATGGTCGAAATCGACGAAGAAGAAAACCC<br>GATCAGACTGATCGACCTGGGAGTCAGAGTCTTCGAAAGAGCAGAAGTCCCG<br>AAGACAGGAGACTCGCTGGCAATGGCAAGAAGACTGGCAAGATCGGTCAGAA<br>GACTGACAAGAAGAAGAGCACACAGACTGCTGAGAACAAGAAGACTGCTGAA<br>GAGAGAAGGAGTCCTGCAGGCAGCAAACTTCGACGAAAACGGACTGATCAAG<br>TCGCTGCCGAACACACCGTGGCAGCTGAGAGCAGCAGCACTGGACAGAAAGC<br>TGACACCGCTGGAATGGTCGGCAGTCCTGCTGCACCTGATCAAGCACAGAGG<br>ATACCTGTCGCAGAGAAAGAACGAAGGAGAAACAGCAGACAAGGAACTGGGA<br>GCACTGCTGAAGGGAGTCGCAGGAAACGCACACGCACTGCAGACAGGAGACT<br>TCAGAACACCGGCAGAACTGGCACTGAACAAGTTCGAAAAGGAATCGGGACA<br>CATCAGAAACCAGAGATCGGACTACTCGCACACATTCTCGAGAAAGGACCTG<br>CAGGCAGAACTGATCCTGCTGTTCGAAAAGCAGAAGGAATTCGGAAACCCGC<br>ACGTCTCGGGAGGACTGAAGGAAGGAATCGAAACACTGCTGATGACACAGAG<br>ACCGGCACTGTCGGGAGACGCAGTCCAGAAGATGCTGGGACACTGCACATTC<br>GAACCGGCAGAACCGAAGGCAGCAAAGAACACATACACAGCAGAAAGATTCA<br>TCTGGCTGACAAAGCTGAACAACCTGAGAATCCTGGAACAGGGATCGGAAAG<br>ACCGCTGACAGACACAGAAAGAGCAACACTGATGGACGAACCGTACAGAAAG<br>TCGAAGCTGACATACGCACAGGCAAGAAAGCTGCTGGGACTGGAAGACACAG<br>CATTCTTCAAGGGACTGAGATACGGAAAGGACAACGCAGAAGCATCGACACT<br>GATGGAAATGAAGGCATACCACGCAATCTCGAGAGCACTGGAAAAGGAAGGA<br>CTGAAGGACAAGAAGTCGCCGCTGAACCTGTCGCCGGAACTGCAGGACGAAA<br>TCGGAACAGCATTCTCGCTGTTCAAGACAGACGAAGACATCACAGGAAGACT<br>GAAGGACAGAATCCAGCCGGAAATCCTGGAAGCACTGCTGAAGCACATCTCG<br>TTCGACAAGTTCGTCCAGATCTCGCTGAAGGCACTGAGAAGAATCGTCCCGC<br>TGATGGAACAGGGAAAGAGATACGACGAAGCATGCGCAGAAATCTACGGAGA | **65** |

(continued)

| Description | Sequence | SEQ ID No. |
|---|---|---|
| | CCACTACGGAAAGAAGAACACAGAAGAAAAGATCTACCTGCCGCCGATCCCG GCAGACGAAATCAGAAACCCGGTCGTCCTGAGAGCACTGTCGCAGGCAAGAA AGGTCATCAACGGAGTCGTCAGAAGATACGGATCGCCGGCAAGAATCCACAT CGAAACAGCAAGAGAAGTCGGAAAGTCGTTCAAGGACAGAAAGGAAATCGAA AAGAGACAGGAAGAAAACAGAAAGGACAGAGAAAAGGCAGCAGCAAAGTTCA GAGAATACTTCCCGAACTTCGTCGGAGAACCGAAGTCGAAGGACATCCTGAA GCTGAGACTGTACGAACAGCAGCACGGAAAGTGCCTGTACTCGGGAAAGGAA ATCAACCTGGGAAGACTGAACGAAAAGGGATACGTCGAAATCGACCACGCAC TGCCGTTCTCGAGAACATGGGACGACTCGTTCAACAACAAGGTCCTGGTCCT GGGATCGGAAAACCAGAACAAGGGAAACCAGACACCGTACGAATACTTCAAC GGAAAGGACAACTCGAGAGAATGGCAGGAATTCAAGGCAAGAGTCGAAACAT CGAGATTCCCGAGATCGAAGAAGCAGAGAATCCTGCTGCAGAAGTTCGACGA AGACGGATTCAAGGAAAGAAACCTGAACGACACAAGATACGTCAACAGATTC CTGTGCCAGTTCGTCGCAGACAGAATGAGACTGACAGGAAAGGGAAAGAAGA GAGTCTTCGCATCGAACGGACAGATCACAAACCTGCTGAGAGGATTCTGGGG ACTGAGAAAGGTCAGAGCAGAAAACGACAGACACCACGCACTGGACGCAGTC GTCGTCGCATGCTCGACAGTCGCAATGCAGCAGAAGATCACAAGATTCGTCA GATACAAGGAAATGAACGCATTCGACGGAAAGACAATCGACAAGGAAACAGG AGAAGTCCTGCACCAGAAGACACACTTCCCGCAGCCGTGGGAATTCTTCGCA CAGGAAGTCATGATCAGAGTCTTCGGAAAGCCGGACGGAAAGCCGGAATTCG AAGAAGCAGACACACTGGAAAAGCTGAGAACACTGCTGGCAGAAAAGCTGTC GTCGAGACCGGAAGCAGTCCACGAATACGTCACACCGCTGTTCGTCTCGAGA GCACCGAACAGAAAGATGTCGGGACAGGGACACATGGAAACAGTCAAGTCGG CAAAGAGACTGGACGAAGGAGTCTCGGTCCTGAGAGTCCCGCTGACACAGCT GAAGCTGAAGGACCTGGAAAAGATGGTCAACAGAGAAAGAGAACCGAAGCTG TACGAAGCACTGAAGGCAAGACTGGAAGCACACAAGGACGACCCGGCAAAGG CATTCGCAGAACCGTTCTACAAGTACGACAAGGCAGGAAACAGAACACAGCA GGTCAAGGCAGTCAGAGTCGAACAGGTCCAGAAGACAGGAGTCTGGGTCAGA AACCACAACGGAATCGCAGACAACGCAACAATGGTCAGAGTAGACGTCTTCG AAAAGGGAGACAAGTACTACCTGGTCCCGATCTACTCGTGGCAGGTCGCAAA GGGAATCCTGCCGGACAGAGCAGTCGTCCAGGGAAAGGACGAAGAAGACTGG CAGCTGATCGACGACTCGTTCAACTTCAAGTTCTCGCTGCACCCGAACGACC TGGTCGAAGTCATCACAAAGAAGGCAAGAATGTTCGGATACTTCGCATCGTG CCACAGAGGAACAGGAAACATCAACATCAGAATCCACGACCTGGACCACAAG ATCGGAAAGAACGGAATCCTGGAAGGAATCGGAGTCAAGACAGCACTGTCGT TCCAGAAGTACCAGATCGACGAACTGGGAAAGGAAATCAGACCGTGCAGACT GAAGAAGAGACCGCCGGTCAGATCCGGAAAGAGAACAGCAGACGGATCGGAA TTCGAATCGCCGAAGAAGAAGAGAAAGGTCGAATGA | |

(continued)

| Description | Sequence | SEQ ID No. |
|---|---|---|
| **ORF encoding Neisseria meningitidis Cas9** (no start or stop codons; suitable for inclusion in fusion protein coding sequence) | GCAGCATTCAAGCCGAACTCGATCAACTACATCCTGGGACTGGACATCGGAA<br>TCGCATCGGTCGGATGGGCAATGGTCGAAATCGACGAAGAAGAAAACCCGAT<br>CAGACTGATCGACCTGGGAGTCAGAGTCTTCGAAAGAGCAGAAGTCCCGAAG<br>ACAGGAGACTCGCTGGCAATGGCAAGAAGACTGGCAAGATCGGTCAGAAGAC<br>TGACAAGAAGAAGAGCACACAGACTGCTGAGAACAAGAAGACTGCTGAAGAG<br>AGAAGGAGTCCTGCAGGCAGCAAACTTCGACGAAAACGGACTGATCAAGTCG<br>CTGCCGAACACACCGTGGCAGCTGAGAGCAGCAGCACTGGACAGAAAGCTGA<br>CACCGCTGGAATGGTCGGCAGTCCTGCTGCACCTGATCAAGCACAGAGGATA<br>CCTGTCGCAGAGAAAGAACGAAGGAGAAACAGCAGACAAGGAACTGGGAGCA<br>CTGCTGAAGGGAGTCGCAGGAAACGCACACGCACTGCAGACAGGAGACTTCA<br>GAACACCGGCAGAACTGGCACTGAACAAGTTCGAAAAGGAATCGGGACACAT<br>CAGAAACCAGAGATCGGACTACTCGCACACATTCTCGAGAAAGGACCTGCAG<br>GCAGAACTGATCCTGCTGTTCGAAAAGCAGAAGGAATTCGGAAACCCGCACG<br>TCTCGGGAGGACTGAAGGAAGGAATCGAAACACTGCTGATGACACAGAGACC<br>GGCACTGTCGGGAGACGCAGTCCAGAAGATGCTGGGACACTGCACATTCGAA<br>CCGGCAGAACCGAAGGCAGCAAAGAACACATACACAGCAGAAAGATTCATCT<br>GGCTGACAAAGCTGAACAACCTGAGAATCCTGGAACAGGGATCGGAAAGACC<br>GCTGACAGACACAGAAAGAGCAACACTGATGGACGAACCGTACAGAAAGTCG<br>AAGCTGACATACGCACAGGCAAGAAAGCTGCTGGGACTGGAAGACACAGCAT<br>TCTTCAAGGGACTGAGATACGGAAAGGACAACGCAGAAGCATCGACACTGAT<br>GGAAATGAAGGCATACCACGCAATCTCGAGAGCACTGGAAAAGGAAGGACTG | **66** |

(continued)

| Description | Sequence | SEQ ID No. |
|---|---|---|
| | AAGGACAAGAAGTCGCCGCTGAACCTGTCGCCGGAACTGCAGGACGAAATCG<br>GAACAGCATTCTCGCTGTTCAAGACAGACGAAGACATCACAGGAAGACTGAA<br>GGACAGAATCCAGCCGGAAATCCTGGAAGCACTGCTGAAGCACATCTCGTTC<br>GACAAGTTCGTCCAGATCTCGCTGAAGGCACTGAGAAGAATCGTCCCGCTGA<br>TGGAACAGGGAAAGAGATACGACGAAGCATGCGCAGAAATCTACGGAGACCA<br>CTACGGAAAGAAGAACACAGAAGAAAAGATCTACCTGCCGCCGATCCCGGCA<br>GACGAAATCAGAAACCCGGTCGTCCTGAGAGCACTGTCGCAGGCAAGAAAGG<br>TCATCAACGGAGTCGTCAGAAGATACGGATCGCCGGCAAGAATCCACATCGA<br>AACAGCAAGAGAAGTCGGAAAGTCGTTCAAGGACAGAAAGGAAATCGAAAAG<br>AGACAGGAAGAAAACAGAAAGGACAGAGAAAAGGCAGCAGCAAAGTTCAGAG<br>AATACTTCCCGAACTTCGTCGGAGAACCGAAGTCGAAGGACATCCTGAAGCT<br>GAGACTGTACGAACAGCAGCACGGAAAGTGCCTGTACTCGGGAAAGGAAATC<br>AACCTGGGAAGACTGAACGAAAAGGGATACGTCGAAATCGACCACGCACTGC<br>CGTTCTCGAGAACATGGGACGACTCGTTCAACAACAAGGTCCTGGTCCTGGG<br>ATCGGAAAACCAGAACAAGGGAAACCAGACACCGTACGAATACTTCAACGGA<br>AAGGACAACTCGAGAGAATGGCAGGAATTCAAGGCAAGAGTCGAAACATCGA<br>GATTCCCGAGATCGAAGAAGCAGAGAATCCTGCTGCAGAAGTTCGACGAAGA<br>CGGATTCAAGGAAAGAAACCTGAACGACACAAGATACGTCAACAGATTCCTG<br>TGCCAGTTCGTCGCAGACAGAATGAGACTGACAGGAAAGGGAAAGAAGAGAG<br>TCTTCGCATCGAACGGACAGATCACAAACCTGCTGAGAGGATTCTGGGGACT<br>GAGAAAGGTCAGAGCAGAAAACGACAGACACCACGCACTGGACGCAGTCGTC<br>GTCGCATGCTCGACAGTCGCAATGCAGCAGAAGATCACAAGATTCGTCAGAT<br>ACAAGGAAATGAACGCATTCGACGGAAAGACAATCGACAAGGAAACAGGAGA<br>AGTCCTGCACCAGAAGACACACTTCCCGCAGCCGTGGGAATTCTTCGCACAG<br>GAAGTCATGATCAGAGTCTTCGGAAAGCCGGACGGAAAGCCGGAATTCGAAG<br>AAGCAGACACACTGGAAAAGCTGAGAACACTGCTGGCAGAAAAGCTGTCGTC<br>GAGACCGGAAGCAGTCCACGAATACGTCACACCGCTGTTCGTCTCGAGAGCA<br>CCGAACAGAAAGATGTCGGGACAGGGACACATGGAAACAGTCAAGTCGGCAA<br>AGAGACTGGACGAAGGAGTCTCGGTCCTGAGAGTCCCGCTGACACAGCTGAA<br>GCTGAAGGACCTGGAAAAGATGGTCAACAGAGAAAGAGAACCGAAGCTGTAC<br>GAAGCACTGAAGGCAAGACTGGAAGCACACAAGGACGACCCGGCAAAGGCAT<br>TCGCAGAACCGTTCTACAAGTACGACAAGGCAGGAAACAGAACACAGCAGGT<br>CAAGGCAGTCAGAGTCGAACAGGTCCAGAAGACAGGAGTCTGGGTCAGAAAC<br>CACAACGGAATCGCAGACAACGCAACAATGGTCAGAGTAGACGTCTTCGAAA<br>AGGGAGACAAGTACTACCTGGTCCCGATCTACTCGTGGCAGGTCGCAAAGGG<br>AATCCTGCCGGACAGAGCAGTCGTCCAGGGAAAGGACGAAGAAGACTGGCAG<br>CTGATCGACGACTCGTTCAACTTCAAGTTCTCGCTGCACCCGAACGACCTGG<br>TCGAAGTCATCACAAAGAAGGCAAGAATGTTCGGATACTTCGCATCGTGCCA<br>CAGAGGAACAGGAAACATCAACATCAGAATCCACGACCTGGACCACAAGATC<br>GGAAAGAACGGAATCCTGGAAGGAATCGGAGTCAAGACAGCACTGTCGTTCC<br>AGAAGTACCAGATCGACGAACTGGGAAAGGAAATCAGACCGTGCAGACTGAA<br>GAAGAGACCGCCGGTCAGATCCGGAAAGAGAACAGCAGACGGATCGGAATTC<br>GAATCGCCGAAGAAGAAGAGAAAGGTCGAA | |

(continued)

| Description | Sequence | SEQ ID No. |
|---|---|---|
| **Transcript comprising SEQ ID NO: 65 (encoding Neisseria meningitidis Cas9)** | GGGAGACCCAAGCTGGCTAGCGTTTAAACTTAAGCTTGGATCCGCCACCATG GCAGCATTCAAGCCGAACTCGATCAACTACATCCTGGGACTGGACATCGGAA TCGCATCGGTCGGATGGGCAATGGTCGAAATCGACGAAGAAGAAAACCCGAT CAGACTGATCGACCTGGGAGTCAGAGTCTTCGAAAGAGCAGAAGTCCCGAAG ACAGGAGACTCGCTGGCAATGGCAAGAAGACTGGCAAGATCGGTCAGAAGAC TGACAAGAAGAAGAGCACACAGACTGCTGAGAACAAGAAGACTGCTGAAGAG AGAAGGAGTCCTGCAGGCAGCAAACTTCGACGAAAACGGACTGATCAAGTCG CTGCCGAACACACCGTGGCAGCTGAGAGCAGCAGCACTGGACAGAAAGCTGA CACCGCTGGAATGGTCGGCAGTCCTGCTGCACCTGATCAAGCACAGAGGATA CCTGTCGCAGAGAAAGAACGAAGGAGAAACAGCAGACAAGGAACTGGGAGCA CTGCTGAAGGGAGTCGCAGGAAACGCACACGCACTGCAGACAGGAGACTTCA GAACACCGGCAGAACTGGCACTGAACAAGTTCGAAAAGGAATCGGGACACAT CAGAAACCAGAGATCGGACTACTCGCACACATTCTCGAGAAAGGACCTGCAG GCAGAACTGATCCTGCTGTTCGAAAAGCAGAAGGAATTCGGAAACCCGCACG TCTCGGGAGGACTGAAGGAAGGAATCGAAACACTGCTGATGACACAGAGACC GGCACTGTCGGGAGACGCAGTCCAGAAGATGCTGGGACACTGCACATTCGAA | **67** |

(continued)

| Description | Sequence | SEQ ID No. |
|---|---|---|
| | CCGGCAGAACCGAAGGCAGCAAAGAACACATACACAGCAGAAAGATTCATCT<br>GGCTGACAAAGCTGAACAACCTGAGAATCCTGGAACAGGGATCGGAAAGACC<br>GCTGACAGACACAGAAAGAGCAACACTGATGGACGAACCGTACAGAAAGTCG<br>AAGCTGACATACGCACAGGCAAGAAAGCTGCTGGGACTGGAAGACACAGCAT<br>TCTTCAAGGGACTGAGATACGGAAAGGACAACGCAGAAGCATCGACACTGAT<br>GGAAATGAAGGCATACCACGCAATCTCGAGAGCACTGGAAAAGGAAGGACTG<br>AAGGACAAGAAGTCGCCGCTGAACCTGTCGCCGGAACTGCAGGACGAAATCG<br>GAACAGCATTCTCGCTGTTCAAGACAGACGAAGACATCACAGGAAGACTGAA<br>GGACAGAATCCAGCCGGAAATCCTGGAAGCACTGCTGAAGCACATCTCGTTC<br>GACAAGTTCGTCCAGATCTCGCTGAAGGCACTGAGAAGAATCGTCCCGCTGA<br>TGGAACAGGGAAAGAGATACGACGAAGCATGCGCAGAAATCTACGGAGACCA<br>CTACGGAAAGAAGAACACAGAAGAAAAGATCTACCTGCCGCCGATCCCGGCA<br>GACGAAATCAGAAACCCGGTCGTCCTGAGAGCACTGTCGCAGGCAAGAAAGG<br>TCATCAACGGAGTCGTCAGAAGATACGGATCGCCGGCAAGAATCCACATCGA<br>AACAGCAAGAGAAGTCGGAAAGTCGTTCAAGGACAGAAAGGAAATCGAAAAG<br>AGACAGGAAGAAAACAGAAAGGACAGAGAAAAGGCAGCAGCAAAGTTCAGAG<br>AATACTTCCCGAACTTCGTCGGAGAACCGAAGTCGAAGGACATCCTGAAGCT<br>GAGACTGTACGAACAGCAGCACGGAAAGTGCCTGTACTCGGGAAAGGAAATC<br>AACCTGGGAAGACTGAACGAAAAGGGATACGTCGAAATCGACCACGCACTGC<br>CGTTCTCGAGAACATGGGACGACTCGTTCAACAACAAGGTCCTGGTCCTGGG<br>ATCGGAAAACCAGAACAAGGGAAACCAGACACCGTACGAATACTTCAACGGA<br>AAGGACAACTCGAGAGAATGGCAGGAATTCAAGGCAAGAGTCGAAACATCGA<br>GATTCCCGAGATCGAAGAAGCAGAGAATCCTGCTGCAGAAGTTCGACGAAGA<br>CGGATTCAAGGAAAGAAACCTGAACGACACAAGATACGTCAACAGATTCCTG<br>TGCCAGTTCGTCGCAGACAGAATGAGACTGACAGGAAAGGGAAAGAAGAGAG<br>TCTTCGCATCGAACGGACAGATCACAAACCTGCTGAGAGGATTCTGGGGACT<br>GAGAAAGGTCAGAGCAGAAAACGACAGACACCACGCACTGGACGCAGTCGTC<br>GTCGCATGCTCGACAGTCGCAATGCAGCAGAAGATCACAAGATTCGTCAGAT<br>ACAAGGAAATGAACGCATTCGACGGAAAGACAATCGACAAGGAAACAGGAGA<br>AGTCCTGCACCAGAAGACACACTTCCCGCAGCCGTGGGAATTCTTCGCACAG<br>GAAGTCATGATCAGAGTCTTCGGAAAGCCGGACGGAAAGCCGGAATTCGAAG<br>AAGCAGACACACTGGAAAAGCTGAGAACACTGCTGGCAGAAAAGCTGTCGTC<br>GAGACCGGAAGCAGTCCACGAATACGTCACACCGCTGTTCGTCTCGAGAGCA<br>CCGAACAGAAAGATGTCGGGACAGGGACACATGGAAACAGTCAAGTCGGCAA<br>AGAGACTGGACGAAGGAGTCTCGGTCCTGAGAGTCCCGCTGACACAGCTGAA<br>GCTGAAGGACCTGGAAAAGATGGTCAACAGAGAAAGAGAACCGAAGCTGTAC<br>GAAGCACTGAAGGCAAGACTGGAAGCACACAAGGACGACCCGGCAAAGGCAT<br>TCGCAGAACCGTTCTACAAGTACGACAAGGCAGGAAACAGAACACAGCAGGT<br>CAAGGCAGTCAGAGTCGAACAGGTCCAGAAGACAGGAGTCTGGGTCAGAAAC<br>CACAACGGAATCGCAGACAACGCAACAATGGTCAGAGTAGACGTCTTCGAAA<br>AGGGAGACAAGTACTACCTGGTCCCGATCTACTCGTGGCAGGTCGCAAAGGG<br>AATCCTGCCGGACAGAGCAGTCGTCCAGGGAAAGGACGAAGAAGACTGGCAG<br>CTGATCGACGACTCGTTCAACTTCAAGTTCTCGCTGCACCCGAACGACCTGG<br>TCGAAGTCATCACAAAGAAGGCAAGAATGTTCGGATACTTCGCATCGTGCCA<br>CAGAGGAACAGGAAACATCAACATCAGAATCCACGACCTGGACCACAAGATC<br>GGAAAGAACGGAATCCTGGAAGGAATCGGAGTCAAGACAGCACTGTCGTTCC<br>AGAAGTACCAGATCGACGAACTGGGAAAGGAAATCAGACCGTGCAGACTGAA<br>GAAGAGACCGCCGGTCAGATCCGGAAAGAGAACAGCAGACGGATCGGAATTC<br>GAATCGCCGAAGAAGAAGAGAAAGGTCGAATGATAGCTAGCTCGAGTCTAGA<br>GGGCCCGTTTAAACCCGCTGATCAGCCTCGACTGTGCCTTCTAGTTGCCAGC<br>CATCTGTTGTTTGCCCCTCCCCCGTGCCTTCCTTGACCCTGGAAGGTGCCAC<br>TCCCACTGTCCTTTCCTAATAAAATGAGGAAATTGCATCGCATTGTCTGAGT<br>AGGTGTCATTCTATTCTGGGGGGTGGGGTGGGGCAGGACAGCAAGGGGGAGG<br>ATTGGGAAGACAATAGCAGGCATGCTGGGGATGCGGTGGGCTCTATGG | |

(continued)

| Description | Sequence | SEQ ID No. |
|---|---|---|
| Amino acid sequence of **Neisseria meningitidis Cas9** | MAAFKPNSINYILGLDIGIASVGWAMVEIDEEENPIRLIDLGVRVFERAEVP KTGDSLAMARRLARSVRRLTRRRAHRLLRTRRLLKREGVLQAANFDENGLIK SLPNTPWQLRAAALDRKLTPLEWSAVLLHLIKHRGYLSQRKNEGETADKELG ALLKGVAGNAHALQTGDFRTPAELALNKFEKESGHIRNQRSDYSHTFSRKDL QAELILLFEKQKEFGNPHVSGGLKEGIETLLMTQRPALSGDAVQKMLGHCTF | **68** |
| | EPAEPKAAKNTYTAERFIWLTKLNNLRILEQGSERPLTDTERATLMDEPYRK SKLTYAQARKLLGLEDTAFFKGLRYGKDNAEASTLMEMKAYHAISRALEKEG LKDKKSPLNLSPELQDEIGTAFSLFKTDEDITGRLKDRIQPEILEALLKHIS FDKFVQISLKALRRIVPLMEQGKRYDEACAEIYGDHYGKKNTEEKIYLPPIP ADEIRNPVVLRALSQARKVINGVVRRYGSPARIHIETAREVGKSFKDRKEIE KRQEENRKDREKAAAKFREYFPNFVGEPKSKDILKLRLYEQQHGKCLYSGKE INLGRLNEKGYVEIDHALPFSRTWDDSFNNKVLVLGSENQNKGNQTPYEYFN GKDNSREWQEFKARVETSRFPRSKKQRILLQKFDEDGFKERNLNDTRYVNRF LCQFVADRMRLTGKGKKRVFASNGQITNLLRGFWGLRKVRAENDRHHALDAV VVACSTVAMQQKITRFVRYKEMNAFDGKTIDKETGEVLHQKTHFPQPWEFFA QEVMIRVFGKPDGKPEFEEADTLEKLRTLLAEKLSSRPEAVHEYVTPLFVSR APNRKMSGQGHMETVKSAKRLDEGVSVLRVPLTQLKLKDLEKMVNREREPKL YEALKARLEAHKDDPAKAFAEPFYKYDKAGNRTQQVKAVRVEQVQKTGVWVR NHNGIADNATMVRVDVFEKGDKYYLVPIYSWQVAKGILPDRAVVQGKDEEDW QLIDDSFNFKFSLHPNDLVEVITKKARMFGYFASCHRGTGNINIRIHDLDHK IGKNGILEGIGVKTALSFQKYQIDELGKEIRPCRLKKRPPVRSGKRTADGSE FESPKKKRKVE | |
| **G390 guide RNA** | mG*mC*mC*GAGUCUGGAGAGCUGCAGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **69** |
| **G502 guide RNA** | mA*mC*mA*CAAAUACCAGUCCAGCGGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **70** |
| **G509 guide RNA** | mA*mA*mA*GUUCUAGAUGCCGUCCGGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **71** |
| **G534 guide RNA** | mA*mC*mG*CAAAUAUCAGUCCAGCGGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **72** |
| **sgRNA comprising modification pattern** | mN*mN*mN*NNNNNNNNNNNNNNNNNGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **74** |
| **G562 guide RNA** | mC*mC*mA*AUAUCAGGAGACUAGGAGUUUUAGAmGmCmUmAmGmAmAmAmU mAmGmCAAGUUAAAAUAAGGCUAGUCCGUUAUCAmAmCmUmUmGmAmAmAmA mAmGmUmGmGmCmAmCmCmGmAmGmUmCmGmGmUmGmCmU*mU*mU*mU | **75** |
| *** = PS linkage; m = 2'-O-Me nucleotide; N =** any natural or non-natural nucleotide | | |

**GFP sequence:**

TCGCGCGTTTCGGTGATGACGGTGAAAACCTCTGACACATGCAGCTCCCGG
AGACGGTCACAGCTTGTCTGTAAGCGGATGCCGGGAGCAGACAAGCCCGTC
AGGGCGCGTCAGCGGGTGTTGGCGGGTGTCGGGGCTGGCTTAACTATGCGG
CATCAGAGCAGATTGTACTGAGAGTGCACCATATGCGGTGTGAAATACCGC
ACAGATGCGTAAGGAGAAAATACCGCATCAGGCGCCATTCGCCATTCAGGC
TGCGCAACTGTTGGGAAGGGCGATCGGTGCGGGCCTCTTCGCTATTACGCCA
GCTGGCGAAAGGGGGATGTGCTGCAAGGCGATTAAGTTGGGTAACGCCAGG
GTTTTCCCAGTCACGACGTTGTAAAACGACGGCCAGTGAATTCTAATACGAC
TCACTATAGGGTCCCGCAGTCGGCGTCCAGCGGCTCTGCTTGTTCGTGTGTG
TGTCGTTGCAGGCCTTATTCGGATCCATGGTGAGCAAGGGCGAGGAGCTGTT
CACCGGGGTGGTGCCCATCCTGGTCGAGCTGGACGGCGACGTAAACGGCCA

CAAGTTCAGCGTGTCCGGCGAGGGCGAGGGCGATGCCACCTACGGCAAGCT
GACCCTGAAGTTCATCTGCACCACCGGCAAGCTGCCCGTGCCCTGGCCCACC
CTCGTGACCACCCTGACCTACGGCGTGCAGTGCTTCAGCCGCTACCCCGACC
ACATGAAGCAGCACGACTTCTTCAAGTCCGCCATGCCCGAAGGCTACGTCC
AGGAGCGCACCATCTTCTTCAAGGACGACGGCAACTACAAGACCCGCGCCG
AGGTGAAGTTCGAGGGCGACACCCTGGTGAACCGCATCGAGCTGAAGGGCA
TCGACTTCAAGGAGGACGGCAACATCCTGGGGCACAAGCTGGAGTACAACT
ACAACAGCCACAACGTCTATATCATGGCCGACAAGCAGAAGAACGGCATCA
AGGTGAACTTCAAGATCCGCCACAACATCGAGGACGGCAGCGTGCAGCTCG
CCGACCACTACCAGCAGAACACCCCCATCGGCGACGGCCCCGTGCTGCTGC
CCGACAACCACTACCTGAGCACCCAGTCCGCCCTGAGCAAAGACCCCAACG
AGAAGCGCGATCACATGGTCCTGCTGGAGTTCGTGACCGCCGCCGGGATCA
CTCTCGGCATGGACGAGCTGTACAAGTAATAGGAATTATGCAGTCTAGCCA
TCACATTTAAAAGCATCTCAGCCTACCATGAGAATAAGAGAAAGAAAATGA
AGATCAATAGCTTATTCATCTCTTTTTCTTTTTCGTTGGTGTAAAGCCAACAC
CCTGTCTAAAAAACATAAATTTCTTTAATCATTTTGCCTCTTTTCTCTGTGCT
TCAATTAATAAAAAATGGAAAGAACCTCGAGAAAAAAAAAAAAAAAAAAA
AAAAAAAAAAAAAAAAAAAAAAAAAAAAAAAAAAAAAAAAAAAAAAAAAA
AAAAAAAAAAAAAAAAAAAAAAAAAAAAAAAAAATCTAGACTTAAGCTTGAT
GAGCTCTAGCTTGGCGTAATCATGGTCATAGCTGTTTCCTGTGTGAAATTGT
TATCCGCTCACAATTCCACACAACATACGAGCCGGAAGCATAAAGTGTAAA
GCCTGGGGTGCCTAATGAGTGAGCTAACTCACATTAATTGCGTTGCGCTCAC
TGCCCGCTTTCCAGTCGGGAAACCTGTCGTGCCAGCTGCATTAATGAATCGG
CCAACGCGCGGGGAGAGGCGGTTTGCGTATTGGGCGCTCTTCCGCTTCCTCG
CTCACTGACTCGCTGCGCTCGGTCGTTCGGCTGCGGCGAGCGGTATCAGCTC
ACTCAAAGGCGGTAATACGGTTATCCACAGAATCAGGGGATAACGCAGGAA
AGAACATGTGAGCAAAAGGCCAGCAAAAGGCCAGGAACCGTAAAAAGGCC
GCGTTGCTGGCGTTTTTCCATAGGCTCCGCCCCCCTGACGAGCATCACAAAA
ATCGACGCTCAAGTCAGAGGTGGCGAAACCCGACAGGACTATAAAGATACC
AGGCGTTTCCCCCTGGAAGCTCCCTCGTGCGCTCTCCTGTTCCGACCCTGCC
GCTTACCGGATACCTGTCCGCCTTTCTCCCTTCGGGAAGCGTGGCGCTTTCTC
ATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGT
AACTATCGTCTTGAGTCCAACCCGGTAAGACACGACTTATCGCCACTGGCAG
CAGCCACTGGTAACAGGATTAGCAGAGCGAGGTATGTAGGCGGTGCTACAG
AGTTCTTGAAGTGGTGGCCTAACTACGGCTACACTAGAAGAACAGTATTTG
GTATCTGCGCTCTGCTGAAGCCAGTTACCTTCGGAAAAAGAGTTGGTAGCTC
TTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTT
TCTACGGGGTCTGACGCTCAGTGGAACGAAAACTCACGTTAAGGGATTTTG
GTCATGAGATTATCAAAAAGGATCTTCACCTAGATCCTTTTAAATTAAAAAT
GAAGTTTTAAATCAATCTAAAGTATATATGAGTAAACTTGGTCTGACAGTTA
CCAATGCTTAATCAGTGAGGCACCTATCTCAGCGATCTGTCTATTTCGTTCA
TCCATAGTTGCCTGACTCCCCGTCGTGTAGATAACTACGATACGGGAGGGCT
TACCATCTGGCCCCAGTGCTGCAATGATACCGCGAGACCCACGCTCACCGG
CTCCAGATTTATCAGCAATAAACCAGCCAGCCGGAAGGGCCGAGCGCAGAA
GTGGTCCTGCAACTTTATCCGCCTCCATCCAGTCTATTAATTGTTGCCGGGA
AGCTAGAGTAAGTAGTTCGCCAGTTAATAGTTTGCGCAACGTTGTTGCCATT
GCTACAGGCATCGTGGTGTCACGCTCGTCGTTTGGTATGGCTTCATTCAGCT

CCGGTTCCCAACGATCAAGGCGAGTTACATGATCCCCCATGTTGTGCAAAA
AAGCGGTTAGCTCCTTCGGTCCTCCGATCGTTGTCAGAAGTAAGTTGGCCGC
AGTGTTATCACTCATGGTTATGGCAGCACTGCATAATTCTCTTACTGTCATG
CCATCCGTAAGATGCTTTTCTGTGACTGGTGAGTACTCAACCAAGTCATTCT
GAGAATAGTGTATGCGGCGACCGAGTTGCTCTTGCCCGGCGTCAATACGGG
ATAATACCGCGCCACATAGCAGAACTTTAAAAGTGCTCATCATTGGAAAAC
GTTCTTCGGGGCGAAAACTCTCAAGGATCTTACCGCTGTTGAGATCCAGTTC
GATGTAACCCACTCGTGCACCCAACTGATCTTCAGCATCTTTTACTTTCACC
AGCGTTTCTGGGTGAGCAAAAACAGGAAGGCAAAATGCCGCAAAAAAGGG
AATAAGGGCGACACGGAAATGTTGAATACTCATACTCTTCCTTTTTCAATAT
TATTGAAGCATTTATCAGGGTTATTGTCTCATGAGCGGATACATATTTGAAT
GTATTTAGAAAAATAAACAAATAGGGGTTCCGCGCACATTTCCCCGAAAAG
TGCCACCTGACGTCTAAGAAACCATTATTATCATGACATTAACCTATAAAAA
TAGGCGTATCACGAGGCCCTTTCGTCG

## Claims

**1.** A method of delivering an mRNA to a stem cell or a stem cell population, the method comprising:

a. preincubating a serum factor, with an LNP composition comprising the mRNA, an amine lipid, a helper lipid, a neutral lipid, and a PEG lipid, wherein the amine lipid is Lipid A

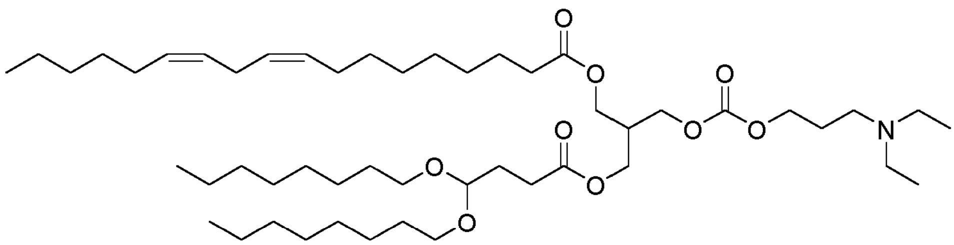

,

or a C4-C12 acetal analog of Lipid A;
b. contacting the stem cell or the stem cell population with the preincubated LNP composition *in vitro;* and
c. culturing the stem cell or the stem cell population *in vitro;*

thereby delivering the mRNA to the stem cell or the stem cell population.

**2.** The method of claim 1, wherein the stem cell is a hematopoietic stem and/or progenitor cell (HSPC) and the stem cell population is an HSPC population.

**3.** The method of claim 2, wherein the HSPC or HSPC population is CD34+, preferably CD34+CD90+.

**4.** The method of any one of claims 1-3, wherein the mRNA encodes a Cas nuclease.

**5.** The method of claim 4, wherein the LNP composition further comprises a gRNA.

**6.** The method of claim 4 or 5, wherein the Cas nuclease is a Class 2 Cas nuclease, such as Cas9 nuclease, an *S. pyogenes* Cas9, or a Cpf1 nuclease.

**7.** The method of claim 5 or 6, wherein the gRNA is a dual-guide RNA (dgRNA).

**8.** The method of claim 5 or 6, wherein the gRNA is a single-guide RNA (sgRNA).

**9.** The method of any one of claims 1-8, wherein post-transfection cell survival is at least 60%, such as at least 70%, at

least 80%, at least 90%, or at least 95%.

**10.** The method of any one of claims 1-9, wherein the LNP composition is preincubated with a serum, such as mammalian, mouse, primate, or human serum, or an isolated serum factor.

**11.** The method of claim 10, wherein the isolated serum factor is an ApoE, such as ApoE2, ApoE3, ApoE4, or a recombinant human protein.

**12.** The method of any one of claims 1-11, wherein the culturing step comprises contacting the cells with a stem cell expander.

**13.** The method of any one of claims 1-12, wherein the stem cell, HSPC, or HSPC population is a human cell or sample.

**14.** The method of any one of claims 1-13, wherein the LNP composition further comprises a template nucleic acid.

**15.** The method of any one of claims 1-14, wherein the LNP composition comprises: an RNA component and a lipid component, wherein the lipid component comprises an amine lipid, a neutral lipid, a helper lipid, and a stealth lipid; and wherein the N/P ratio is 1-10, wherein the N/P ratio is a ratio of positively charged amine groups of the amine lipid (N) and negatively charged phosphate groups (P) of the RNA component.

**16.** The method of claim 15, wherein the lipid component comprises:

40-60 mol-%, such as 50-60 mol-%, preferably 48-53 mol-%, amine lipid;
5-15 mol-%, such as 8-10 mol-%, neutral lipid, wherein the neutral lipid is preferably DSPC; and
1.5-10 mol-%, such as 2.5-4 mol-%, PEG lipid,
wherein the remainder of the lipid component is helper lipid, preferably cholesterol, and
wherein the N/P ratio of the LNP composition is 3-10, preferably 3-8 $\pm$ 0.2.

**17.** The method of any one of claims 1-16, wherein the mRNA is a modified mRNA.

**18.** The method of any one of claims 1-17, wherein:

the mRNA comprises an open reading frame encoding an RNA-guided DNA-binding agent, wherein the open reading frame has a uridine content ranging from its minimum uridine content to 150% of the minimum uridine content; or
the mRNA comprises an open reading frame encoding an RNA-guided DNA-binding agent, wherein the open reading frame has a uridine dinucleotide content ranging from its minimum uridine dinucleotide content to 150% of the minimum uridine dinucleotide content.

**19.** The method of any one of claims 1-18, wherein the mRNA comprises a sequence with at least 90% identity to any one of SEQ ID NO: 1, 4, 10, 14, 15, 17, 18, 20, 21, 23, 24, 26, 27, 29, 30, 50, 52, 54, 65, or 66, wherein the mRNA comprises an open reading frame encoding an RNA-guided DNA-binding agent.

**20.** The method of any one of claims 5-19, wherein the gRNA is a modified gRNA.

**21.** The method of claim 20, wherein the gRNA comprises a modification chosen from 2'-O-methyl (2'-O-Me) modified nucleotide, a phosphorothioate (PS) bond between nucleotides; and a 2'-fluoro (2'-F) modified nucleotide.

**22.** The method of claim 19 or 20, wherein the gRNA comprises a 5' end and a 3' end, wherein the gRNA comprises at least one modification selected from:

(a) a modification at one or more of the first five nucleotides at the 5' end;
(b) a modification at one or more of the last five nucleotides at the 3' end;
(c) PS bonds between the first four nucleotides at the 5' end;
(d) PS bonds between the last four nucleotides at the 3' end;
(e) 2'-O-Me modified nucleotides at the first three nucleotides at the 5' end; and
(f) 2'-O-Me modified nucleotides at the last three nucleotides at the 3' end.

**23.** The method of any one of claims 1-22, further comprising achieving CRISPR-Cas gene editing in the stem cell, HSPC, or HSPC population, wherein gene editing is achieved and measured as percent editing, and wherein the percent editing is at least 40%, at least 60%, at least 70%, at least 80%, at least 90%, or at least 95% of the total number of sequence reads.

**24.** The method of any one of claims 1-23, wherein the amine lipid is

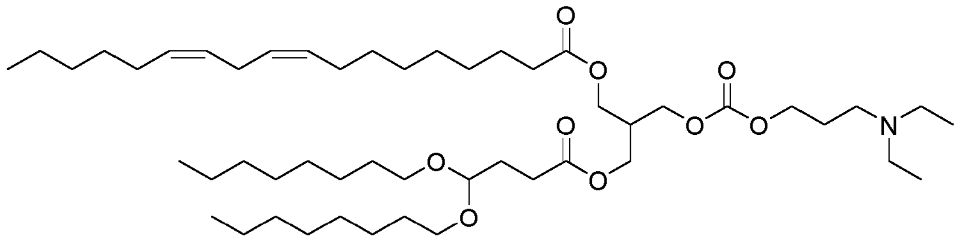

.

**Patentansprüche**

**1.** Verfahren zum Freisetzen einer mRNA an eine Stammzelle oder eine Stammzellpopulation, wobei das Verfahren Folgendes umfasst:

a. Vorinkubieren eines Serumfaktors mit einer LNP-Zusammensetzung, welche die mRNA, ein Aminlipid, ein Helferlipid, ein neutrales Lipid und ein PEG-Lipid umfasst, wobei das Aminlipid Lipid A

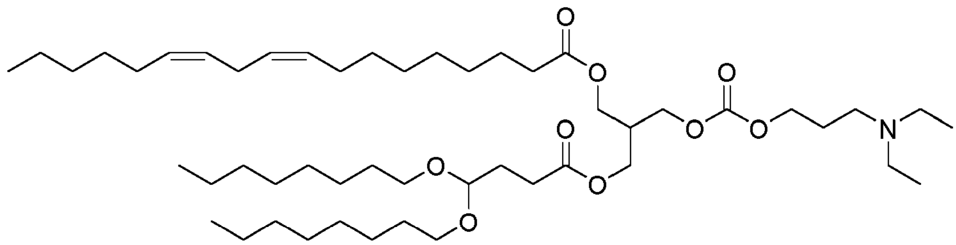

,

oder ein C4-C12-Acetalanalogon von Lipid A ist;
b. Kontaktieren der Stammzelle oder der Stammzellpopulation mit der vorinkubierten LNP-Zusammensetzung *in vitro;* und
c. Kultivieren der Stammzelle oder der Stammzellpopulation *in vitro;*

wodurch die mRNA an die Stammzelle oder die Stammzellpopulation freigesetzt wird.

**2.** Verfahren nach Anspruch 1, wobei die Stammzelle eine hämatopoetische Stamm- und/oder Vorläuferzelle (HSPC) ist und die Stammzellpopulation eine HSPC-Population ist.

**3.** Verfahren nach Anspruch 2, wobei die HSPC oder HSPC-Population CD34+, bevorzugt CD34+CD90+ ist.

**4.** Verfahren nach einem der Ansprüche 1-3, wobei die mRNA eine Cas-Nuklease codiert.

**5.** Verfahren nach Anspruch 4, wobei die LNP-Zusammensetzung ferner eine gRNA umfasst.

**6.** Verfahren nach Anspruch 4 oder 5, wobei die Cas-Nuklease eine Klasse-2-Cas-Nuklease, wie Cas9-Nuklease, eine *S.-pyogenes-Cas9* oder eine Cpfl-Nuklease ist.

**7.** Verfahren nach Anspruch 5 oder 6, wobei die gRNA eine Dual-Guide-RNA (dgRNA) ist.

**8.** Verfahren nach Anspruch 5 oder 6, wobei die gRNA eine Single-Guide-RNA (sgRNA) ist.

**9.** Verfahren nach einem der Ansprüche 1-8, wobei Post-Transfektions-Zellüberleben zumindest 60 % ist, wie zumindest 70 %, zumindest 80 %, zumindest 90 % oder zumindest 95 %.

**10.** Verfahren nach einem der Ansprüche 1-9, wobei die LNP-Zusammensetzung mit einem Serum, wie Säugetier-, Maus-, Primaten- oder menschlichem Serum, oder einem isolierten Serumfaktor vorinkubiert ist.

**11.** Verfahren nach Anspruch 10, wobei der isolierte Serumfaktor ein ApoE, wie ApoE2, ApoE3, ApoE4, oder ein rekombinantes menschliches Protein ist.

**12.** Verfahren nach einem der Ansprüche 1-11, wobei der Kultivierungsschritt Kontaktieren der Zellen mit einem Stammzellexpander umfasst.

**13.** Verfahren nach einem der Ansprüche 1-12, wobei die Stammzelle, HSPC oder HSPC-Population eine menschliche Zelle oder Probe ist.

**14.** Verfahren nach einem der Ansprüche 1-13, wobei die LNP-Zusammensetzung ferner eine Template-Nukleinsäure umfasst.

**15.** Verfahren nach einem der Ansprüche 1-14, wobei die LNP-Zusammensetzung Folgendes umfasst: eine RNA-Komponente und eine Lipidkomponente, wobei die Lipidkomponente ein Aminlipid, ein neutrales Lipid, ein Helferlipid und ein Stealth-Lipid umfasst; und wobei das N/P-Verhältnis 1-10 ist, wobei das N/P-Verhältnis ein Verhältnis von positiv geladenen Amingruppen des Aminlipids (N) und negativ geladenen Phosphatgruppen (P) der RNA-Komponente ist.

**16.** Verfahren nach Anspruch 15, wobei die Lipidkomponente Folgendes umfasst:

40-60 Mol-%, wie 50-60 Mol-%, bevorzugt 48-53 Mol-%, Aminlipid;
5-15 Mol-%, wie 8-10 Mol-%, neutrales Lipid, wobei das neutrale Lipid bevorzugt DSPC ist; und
1,5-10 Mol-%, wie 2,5-4 Mol-%, PEG-Lipid,
wobei der Rest der Lipidkomponente Helferlipid, bevorzugt Cholesterin ist, und wobei das N/P-Verhältnis der LNP-Zusammensetzung 3-10, bevorzugt 3-8 $\pm$ 0,2 ist.

**17.** Verfahren nach einem der Ansprüche 1-16, wobei die mRNA eine modifizierte mRNA ist.

**18.** Verfahren nach einem der Ansprüche 1-17, wobei:

die mRNA einen offenen Leserahmen umfasst, der ein RNA-geführtes DNA-Bindemittel codiert,
wobei der offene Leserahmen einen Uridingehalt aufweist, der von seinem minimalen Uridingehalt bis 150 % des minimalen Uridingehalts reicht; oder
die mRNA einen offenen Leserahmen umfasst, der ein RNA-geführtes DNA-Bindemittel codiert, wobei der offene Leserahmen einen Uridindinukleotidgehalt aufweist, der von seinem minimalen Uridindinukleotidgehalt bis 150 % des minimalen Uridindinukleotidgehalts reicht.

**19.** Verfahren nach einem der Ansprüche 1-18, wobei die mRNA eine Sequenz mit zumindest 90 % Identität zu einer beliebigen von SEQ ID NO: 1, 4, 10, 14, 15, 17, 18, 20, 21, 23, 24, 26, 27, 29, 30, 50, 52, 54, 65 oder 66 umfasst, wobei die mRNA einen offenen Leserahmen umfasst, der ein RNA-geführtes DNA-Bindemittel codiert.

**20.** Verfahren nach einem der Ansprüche 5-19, wobei die gRNA eine modifizierte gRNA ist.

**21.** Verfahren nach Anspruch 20, wobei die gRNA eine Modifikation umfasst, die aus 2'-O-Methyl(2'-O-Me-)modifiziertem Nukleotid, einer Phosphorthioat(PS-)Bindung zwischen Nukleotiden; und einem 2'-Fluor(2'-F-)modifizierten Nukleotid gewählt ist.

**22.** Verfahren nach Anspruch 19 oder 20, wobei die gRNA ein 5'-Ende und ein 3'-Ende umfasst, wobei die gRNA zumindest eine Modifikation ausgewählt aus Folgendem umfasst:

(a) einer Modifikation an einem oder mehreren der ersten fünf Nukleotide an dem 5'-Ende;
(b) einer Modifikation an einem oder mehreren der letzten fünf Nukleotide an dem 3'-Ende;

(c) PS-Bindungen zwischen den ersten vier Nukleotiden an dem 5'-Ende;
(d) PS-Bindungen zwischen den letzten vier Nukleotiden an dem 3'-Ende;
(e) 2'-O-Me-modifizierten Nukleotiden an den ersten drei Nukleotiden an dem 5'-Ende; und
(f) 2'-O-Me-modifizierten Nukleotiden an den letzten drei Nukleotiden an dem 3'-Ende.

**23.** Verfahren nach einem der Ansprüche 1-22, ferner umfassend Erreichen von CRISPR-Cas-Gen-Editierung in der Stammzelle, HSPC oder HSPC-Population, wobei Gen-Editierung als prozentuale Editierung erreicht und gemessen wird und wobei die prozentuale Editierung zumindest 40 %, zumindest 60 %, zumindest 70 %, zumindest 80 %, zumindest 90 % oder zumindest 95 % der Gesamtzahl an Sequenzlesevorgängen ist.

**24.** Verfahren nach einem der Ansprüche 1-23, wobei das Aminlipid

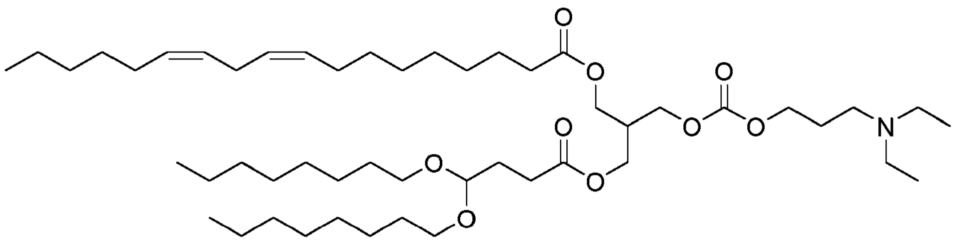

ist.

## Revendications

**1.** Procédé d'administration d'un ARNm à une cellule souche ou à une population de cellules souches, le procédé comprenant :

a. la préincubation d'un facteur sérique avec une composition LNP comprenant l'ARNm, un lipide aminé, un lipide auxiliaire, un lipide neutre et un lipide PEG, ledit lipide aminé étant le lipide A

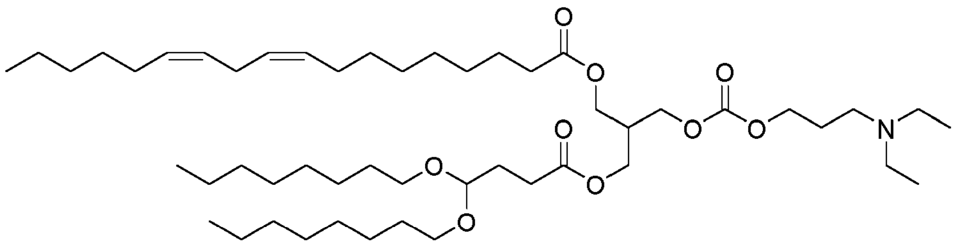

,

ou un analogue acétal en C4-C12 du lipide A ;
b. la mise en contact de la cellule souche ou de la population de cellules souches avec la composition LNP préincubée *in vitro* ; et
c. la culture de la cellule souche ou de la population de cellules souches *in vitro ;*

administrant ainsi l'ARNm à la cellule souche ou à la population de cellules souches.

**2.** Procédé de la revendication 1, dans lequel la cellule souche est une cellule souche et/ou progénitrice hématopoïétique (HSPC) et la population de cellules souches est une population de HSPC.

**3.** Procédé de la revendication 2, dans lequel la HSPC ou la population de HSPC est CD34+, de préférence CD34+CD90+.

**4.** Procédé de l'une quelconque des revendications 1 à 3, dans lequel l'ARNm code pour une nucléase Cas.

**5.** Procédé de la revendication 4, dans lequel la composition LNP comprend en outre un ARNg.

**6.** Procédé de la revendication 4 ou 5, dans lequel la nucléase Cas est une nucléase Cas de classe 2, telle que la nucléase Cas9, une Cas9 de *S. pyogenes,* ou une nucléase Cpfl.

**7.** Procédé de la revendication 5 ou 6, dans lequel l'ARNg est un ARN à double guide (ARNdg).

**8.** Procédé de la revendication 5 ou 6, dans lequel l'ARNg est un ARN à guide unique (ARNsg).

**9.** Procédé de l'une quelconque des revendications 1 à 8, dans lequel la survie cellulaire post-transfection est d'au moins 60 %, par exemple d'au moins 70 %, au moins 80 %, au moins 90 % ou au moins 95 %.

**10.** Procédé de l'une quelconque des revendications 1 à 9, dans lequel la composition LNP est préincubée avec un sérum, tel qu'un sérum de mammifère, de souris, de primate ou humain, ou avec un facteur sérique isolé.

**11.** Procédé de la revendication 10, dans lequel le facteur sérique isolé est une ApoE, telle que ApoE2, ApoE3, ApoE4 ou une protéine humaine recombinante.

**12.** Procédé de l'une quelconque des revendications 1 à 11, dans lequel l'étape de culture comprend la mise en contact des cellules avec un agent d'expansion de cellules souches.

**13.** Procédé de l'une quelconque des revendications 1 à 12, dans lequel la cellule souche, la HSPC ou la population de HSPC est une cellule ou un échantillon humain.

**14.** Procédé de l'une quelconque des revendications 1 à 13, dans lequel la composition LNP comprend en outre un acide nucléique matrice.

**15.** Procédé de l'une quelconque des revendications 1 à 14, dans lequel la composition LNP comprend : un composant ARN et un composant lipidique, dans lequel le composant lipidique comprend un lipide aminé, un lipide neutre, un lipide auxiliaire et un lipide furtif ; et dans lequel le rapport N/P est de 1 à 10, dans lequel le rapport N/P est un rapport de groupes amine chargés positivement du lipide aminé (N) et de groupes phosphate chargés négativement (P) du composant ARN.

**16.** Procédé de la revendication 15, dans lequel le composant lipidique comprend :

40 à 60 % en mole, par exemple 50 à 60 % en mole, de préférence 48 à 53 % en mole, de lipide aminé ;
5 à 15 % en mole, par exemple 8 à 10 % en mole, de lipide neutre, ledit lipide neutre étant de préférence la DSPC ; et
1,5 à 10 % en mole, par exemple 2,5 à 4 % en mole, de lipide PEG,
dans lequel le reste du composant lipidique est un lipide auxiliaire, de préférence le cholestérol, et dans lequel le rapport N/P de la composition LNP est de 3 à 10, de préférence de 3 à 8 $\pm$ 0,2.

**17.** Procédé de l'une quelconque des revendications 1 à 16, dans lequel l'ARNm est un ARNm modifié.

**18.** Procédé de l'une quelconque des revendications 1 à 17, dans lequel :

l'ARNm comprend un cadre de lecture ouvert codant pour un agent de liaison à l'ADN guidé ARN, dans lequel le cadre de lecture ouvert présente une teneur en uridine allant de sa teneur minimale en uridine à 150 % de la teneur minimale en uridine ; ou
l'ARNm comprend un cadre de lecture ouvert codant pour un agent de liaison à l'ADN guidé par ARN, dans lequel le cadre de lecture ouvert présente une teneur en dinucléotide d'uridine allant de sa teneur minimale en dinucléotide d'uridine à 150 % de la teneur minimale en dinucléotide d'uridine.

**19.** Procédé de l'une quelconque des revendications 1 à 18, dans lequel l'ARNm comprend une séquence présentant une identité d'au moins 90 % avec l'une quelconque des SEQ ID N° : 1, 4, 10, 14, 15, 17, 18, 20, 21, 23, 24, 26, 27, 29, 30, 50, 52, 54, 65 ou 66, ledit ARNm comprenant un cadre de lecture ouvert codant pour un agent de liaison à l'ADN guidé par ARN.

**20.** Procédé de l'une quelconque des revendications 5 à 19, dans lequel l'ARNg est un ARNg modifié.

**21.** Procédé de la revendication 20, dans lequel l'ARNg comprend une modification choisie parmi un nucléotide portant une modification 2'-O-méthyle (2'-O-Me), une liaison phosphorothioate (PS) entre nucléotides ; et un nucléotide portant une modification 2'-fluoro (2'-F).

**22.** Procédé de la revendication 19 ou 20, dans lequel l'ARNg comprend une extrémité 5' et une extrémité 3', dans lequel l'ARNg comprend au moins une modification choisie parmi :

(a) une modification au niveau d'un ou plusieurs des cinq premiers nucléotides à l'extrémité 5' ;
(b) une modification au niveau d'un ou plusieurs des cinq derniers nucléotides à l'extrémité 3' ;
(c) des liaisons PS entre les quatre premiers nucléotides à l'extrémité 5' ;
(d) des liaisons PS entre les quatre derniers nucléotides à l'extrémité 3' ;
(e) des nucléotides portant une modification 2'-O-Me au niveau des trois premiers nucléotides à l'extrémité 5' ; et
(f) des nucléotides portant une modification 2'-O-Me au niveau des trois derniers nucléotides à l'extrémité 3'.

**23.** Procédé de l'une quelconque des revendications 1 à 22, comprenant en outre la réalisation d'une édition génique CRISPR-Cas dans la cellule souche, la HSPC ou la population de HSPC, dans lequel l'édition génique est réalisée et mesurée en pourcentage d'édition, et dans lequel le pourcentage d'édition est d'au moins 40 %, d'au moins 60 %, d'au moins 70 %, d'au moins 80 %, d'au moins 90 % ou d'au moins 95 % du nombre total de lectures de séquences.

**24.** Procédé de l'une quelconque des revendications 1 à 23, dans lequel le lipide aminé est

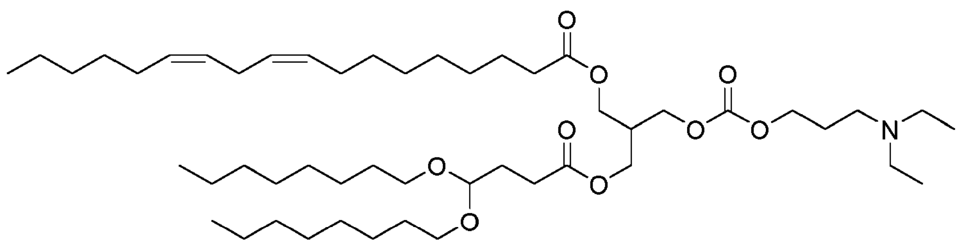

.

Fig. 1A

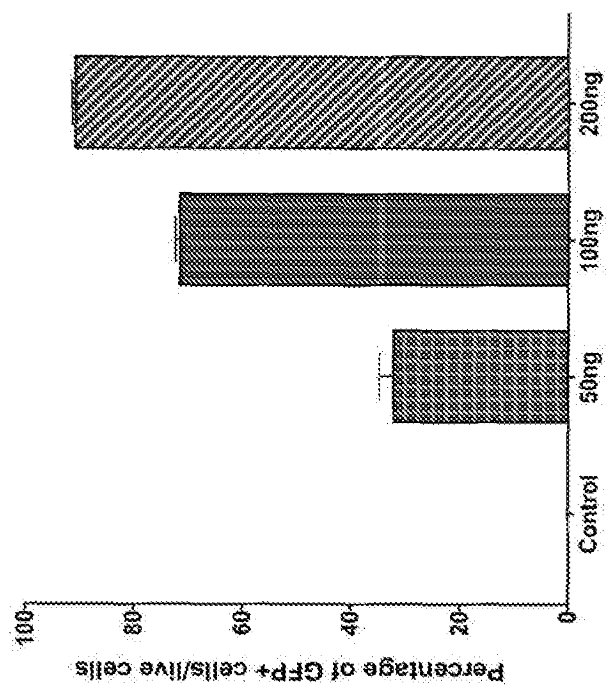
Percentage of GFP+ cells/live cells
100
80
60
40
20
0
Control
50ng
100ng
200ng

Fig. 1B

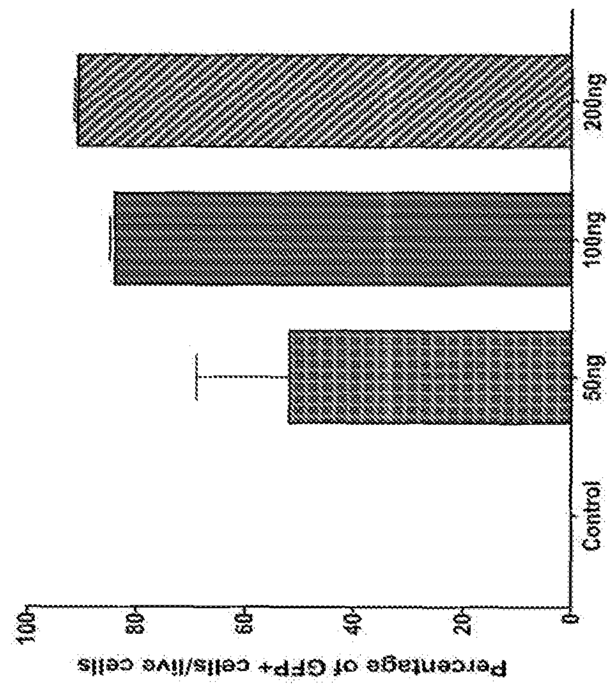
Percentage of GFP+ cells/live cells
100
80
60
40
20
0
Control
50ng
100ng
200ng

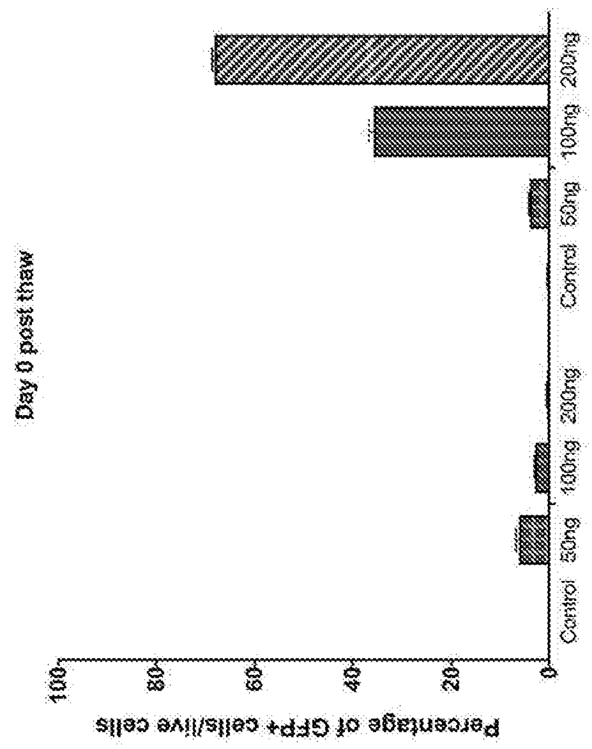
Day 0 post thaw
Percentage of GFP+ cells/live cells
100
80
60
40
20
0
Control
50ng
100ng
200ng
Control
50ng
100ng
200ng
no serum
6% mouse serum

Fig. 2A

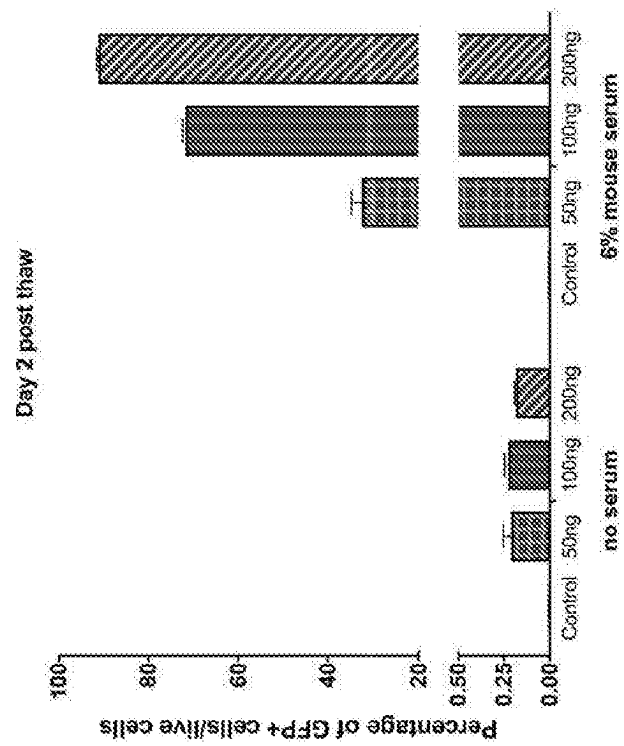
Day 2 post thaw
Percentage of GFP+ cells/live cells
100
80
60
40
20
0.50
0.25
0.00
Control
50ng
100ng
200ng
Control
50ng
100ng
200ng
no serum
6% mouse serum

Fig. 2B

Fig. 3B

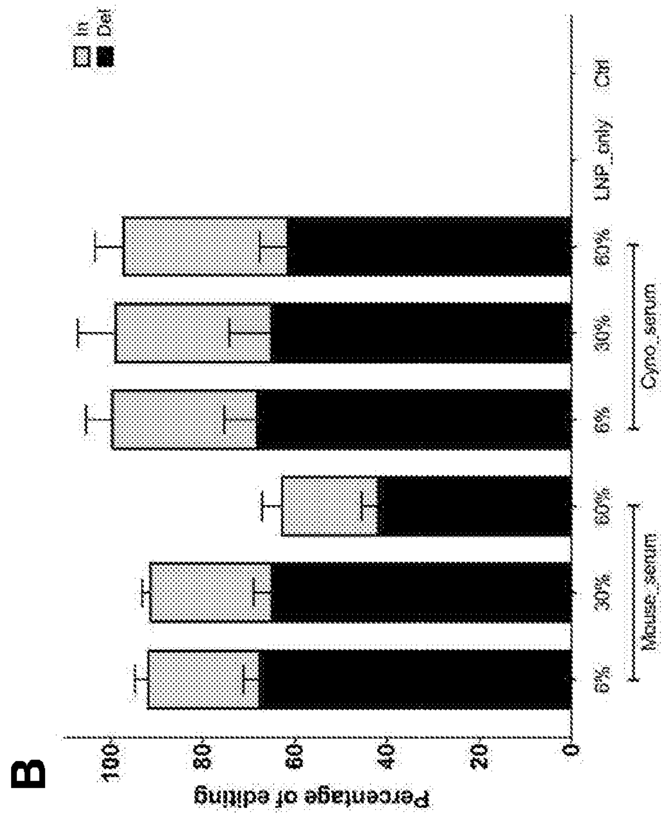
B
Percentage of editing
100
80
60
40
20
0
6%
30%
60%
6%
30%
60%
LNP_only
Ctrl
Mouse_serum
Cyno_serum
In
Del

Fig. 3A

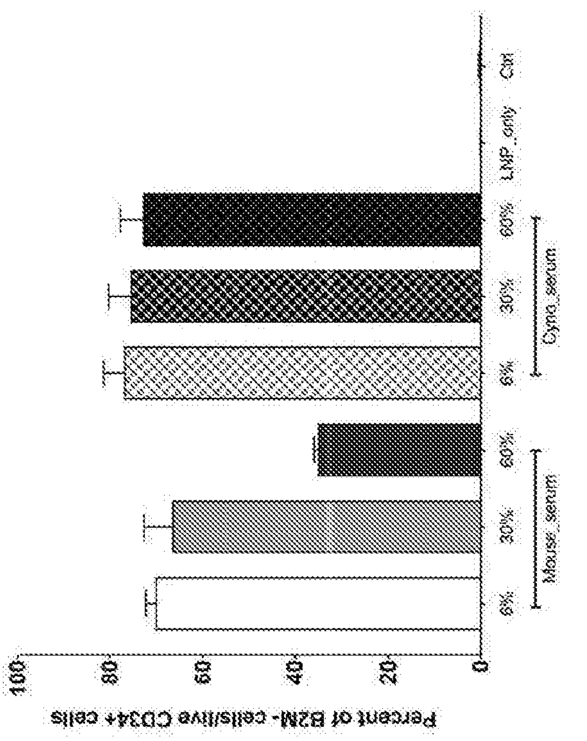
Percent of B2M- cells/live CD34+ cells
100
80
60
40
20
0
6%
30%
60%
6%
30%
60%
LNP_only
Ctrl
Mouse_serum
Cyno_serum

Fig. 4A

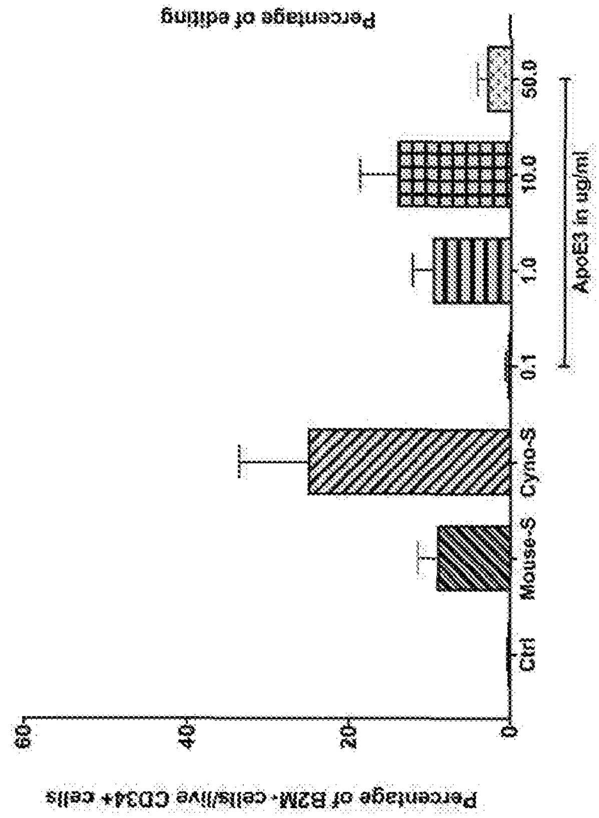
Percentage of B2M- cells/live CD34+ cells
0
20
40
60
Ctrl
Mouse-S
Cyno-S
0.1
1.0
10.0
50.0
ApoE3 in ug/ml

Fig. 4B

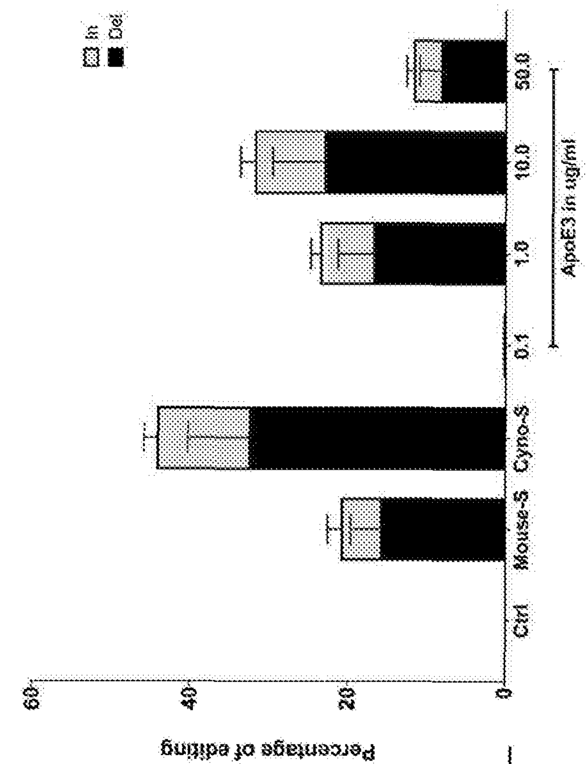
Percentage of editing
0
20
40
60
Ctrl
Mouse-S
Cyno-S
0.1
1.0
10.0
50.0
ApoE3 in ug/ml
In
Del

Fig. 5

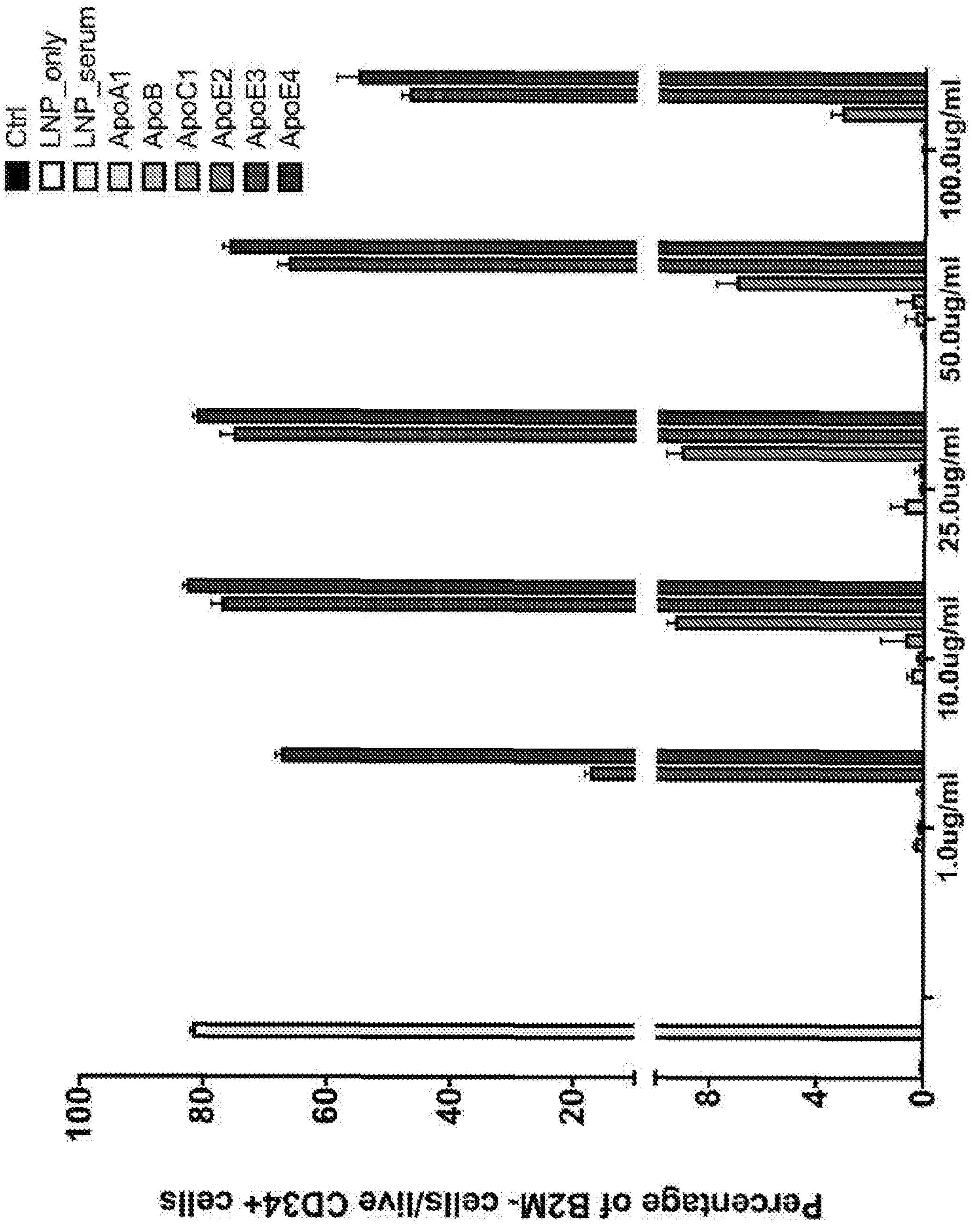
Ctrl
LNP_only
LNP_serum
ApoA1
ApoB
ApoC1
ApoE2
ApoE3
ApoE4
Percentage of B2M- cells/live CD34+ cells
100
80
60
40
20
8
4
0
1.0ug/ml
10.0ug/ml
25.0ug/ml
50.0ug/ml
100.0ug/ml

Fig. 6A

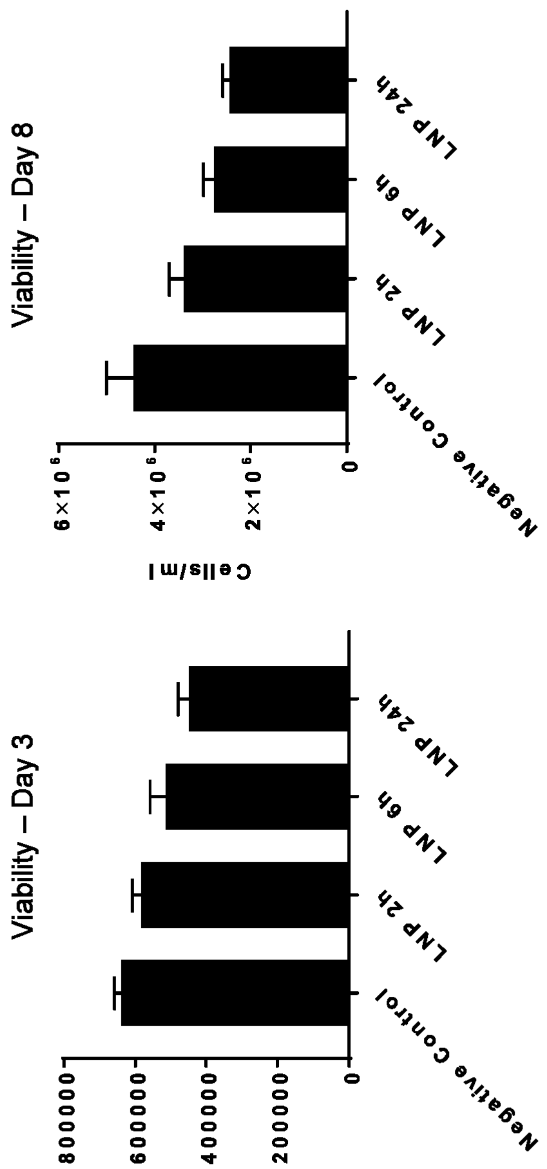
Viability – Day 3
Cells/ml
800000
600000
400000
200000
0
Negative Control
LNP 2h
LNP 6h
LNP 24h
Viability – Day 8
Cells/ml
6×10⁶
4×10⁶
2×10⁶
0
Negative Control
LNP 2h
LNP 6h
LNP 24h

Fig. 6B

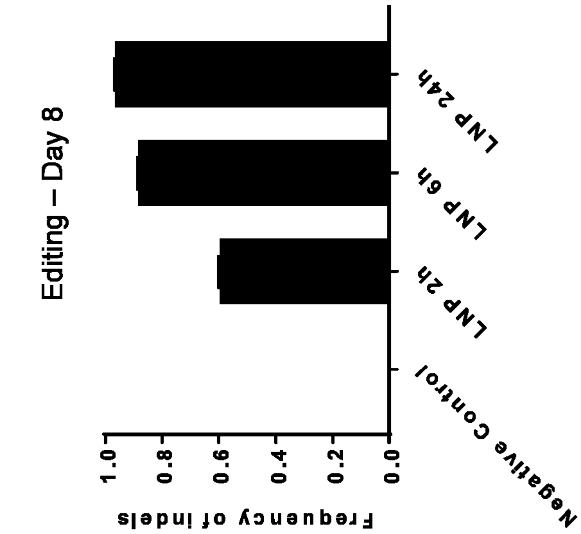
Editing – Day 8
Frequency of indels
1.0
0.8
0.6
0.4
0.2
0.0
Negative Control
LNP 2h
LNP 6h
LNP 24h

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010059401 A
- WO 2013110198 A
- WO 2017115268 A
- US 20160312198 A1
- US 20160312199 A1
- US 8889356 B
- US 20140186958 A1
- US 20150166980 A1
- US 9023649 B
- WO 2015089406 A
- US 20160304846 A
- US 62566232
- WO 2017077394 A
- WO 2015095340 A
- WO 2017173054 A
- WO 2014136086 A
- WO 2006007712 A
- WO 2016010840 A


### Non-patent literature cited in the description


- **DOULATOV et al.** *Cell Stem Cell*, 2012
- **ZETSCHE et al.** *Cell*, 2015, vol. 163, 1-13
- **MAKAROVA et al.** *Nat Rev Microbiol*, 2015, vol. 13 (11), 722-36
- **SHMAKOV et al.** *Molecular Cell*, 2015, vol. 60, 385-397
- **MAKAROVA et al.** *Nat. Rev. Microbiol.*, 2011, vol. 9, 467-477
- **MAKAROVA et al.** *Nat. Rev. Microbiol*, 2015, vol. 13, 722-36
- **ZETSCHE et al.** *Cell*, 22 October 2015, vol. 163 (3), 759-771
- **QI et al.** Repurposing CRISPR as an RNA-guided platform for sequence-specific control of gene expression. *Cell*, 2013, vol. 152, 1173-83
- **PEREZ-PINERA et al.** RNA-guided gene activation by CRISPR-Cas9-based transcription factors. *Nat. Methods*, 2013, vol. 10, 973-6
- **MALI et al.** CAS9 transcriptional activators for target specificity screening and paired nickases for cooperative genome engineering. *Nat. Biotechnol.*, 2013, vol. 31, 833-8
- **GILBERT et al.** CRISPR-mediated modular RNA-guided regulation of transcription in eukaryotes. *Cell*, 2013, vol. 154, 442-51
- **MEFFERD et al.** *RNA.*, 2015, vol. 21, 1683-9
- **SCHERER et al.** *Nucleic Acids Res.*, 2007, vol. 35, 2620-2628
- **KATIBAH et al.** *Proc Natl Acad Sci USA*, 2014, vol. 111 (33), 12025-30
- **ABBAS et al.** *Proc Natl Acad Sci USA*, 2017, vol. 114 (11), E2106-E2115
- **STEPINSKI et al.** Synthesis and properties of mRNAs containing the novel 'anti-reverse' cap analogs 7-methyl(3'-O-methyl)GpppG and 7-methyl(3'deoxy)GpppG. *RNA*, 2001, vol. 7, 1486-1495
- **GUO, P.** ; **MOSS, B.** *Proc. Natl. Acad. Sci. USA*, 1990, vol. 87, 4023-4027
- **MAO, X.** ; **SHUMAN, S.** *J. Biol. Chem.*, 1994, vol. 269, 24472-24479
- **BAUER et al.** *Science*, 2013, vol. 342 (6155), 253-257
- **SANKARAN VG et al.** *NEJM*, 2011, vol. 365, 807-814
- **SANKARAN VG et al.** A functional element necessary for fetal hemoglobin silencing. *NEJM*, 2011, vol. 365, 807-814
- **RAN et al.** *Nature*, 2015, vol. 520, 186-191
- **MAIER, M.A. et al.** Biodegradable Lipids Enabling Rapidly Eliminated Lipid Nanoparticles for Systemic Delivery of RNAi Therapeutics. *Mol. Ther.*, 2013, vol. 21 (8), 1570-78
- **ROMBERG et al.** *Pharmaceutical Research*, 2008, vol. 25 (1), 55-71
- **HOEKSTRA et al.** *Biochimica et Biophysica Acta*, 2004, vol. 1660, 41-52
- **J. MILTON HARRIS**. *Poly(ethylene glycol) chemistry: biotechnical and biomedical applications*, 1992